(19) Europäisches Patentamt
European Patent Office
Office européen des brevets

(11) EP 4 095 134 A1

(12) EUROPEAN PATENT APPLICATION

(43) Date of publication:
**30.11.2022 Bulletin 2022/48**

(21) Application number: **21175671.3**

(22) Date of filing: **25.05.2021**

(51) International Patent Classification (IPC):
***C07D 405/12*** (2006.01) ***C07D 405/14*** (2006.01)
***C07D 417/14*** (2006.01) ***C07D 401/12*** (2006.01)
***C07D 513/04*** (2006.01) ***A61K 31/4427*** (2006.01)
***A61K 31/506*** (2006.01) ***A61P 25/00*** (2006.01)

(52) Cooperative Patent Classification (CPC):
**C07D 405/12; A61P 25/00; C07D 401/12; C07D 405/14; C07D 417/14; C07D 513/04**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **UCL Business Ltd**
**London WC1E 6BT (GB)**

(72) Inventor: **BAYLE, Elliott**
**London W1T 4TJ (GB)**

(74) Representative: **J A Kemp LLP**
**80 Turnmill Street**
**London EC1M 5QU (GB)**

# (54) POLYCYCLIC COMPOUNDS FOR THE TREATMENT OF NEUROLOGICAL INDICATIONS

(57) The present invention relates to P2Y1 inhibiting compounds of formula (I) or (II) and pharmaceutical compositions comprising the same. The present invention further relates to the treatment of CNS disorders, in particular Alzheimer's disease, using the P2Y1 inhibiting compounds and pharmaceutical compositions of the invention.

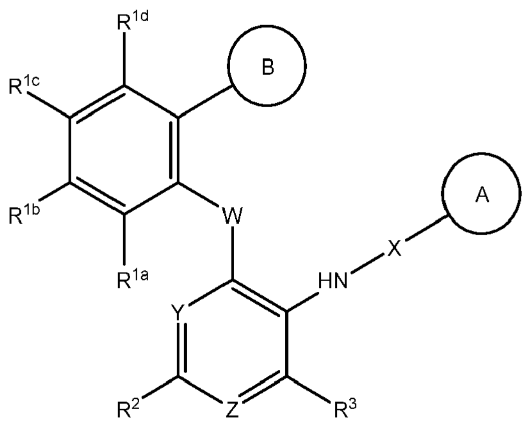

(I)

5

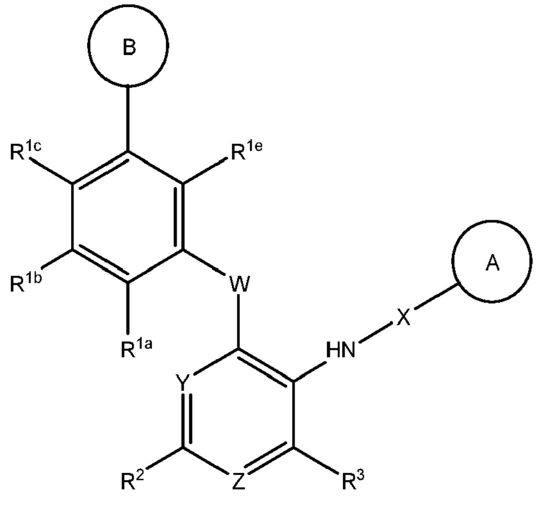

(II)

;

EP 4 095 134 A1

## Description

The present invention relates to P2Y1 inhibiting compounds and pharmaceutical compositions comprising the same. The present invention further relates to the treatment of CNS disorders, in particular Alzheimer's disease, using the P2Y1 inhibiting compounds and pharmaceutical compositions of the invention.

## Background to the invention

The P2Y1 receptor is a purinergic receptor that has been identified as a target in treating and/or preventing CNS disorders through the modulation of astrocyte hyperactivity. Examples of CNS disorders that may be susceptible to treatment and/or prevention by astroglial P2Y1 receptor inhibition include Alzheimer's disease, pain (both neuropathic and nociceptive), epilepsy, stroke (via neuroprotection), traumatic brain injury and amyotrophic lateral sclerosis. Non-limiting examples of literature evidence substantiating the principle of applying astroglial P2Y1 receptor inhibition for treating and/or preventing these CNS disorders includes:

- Alzheimer's disease - see (i) Delakate A, et al. Nat. Commun., 2014;5:5422; (ii) Reichenbach N, et al. J. Exp. Med. 2018;215(6):1649-1663; (iii) Reichenbach N, et al. EMBO Mol Med. 2019 11:e9665; and (iv) Shi A, et al. Cereb Cortex. 2020;30(3):1272-1290.
- Neuropathic pain - see (i) Barragán-Iglesias P, et al. Brain Res. 2016 1636:43-51; (ii) Mitchell R, et al. Mol Neurobiol. 2019 56(8):5917-5933; and (iii) Yang CT, et al. Sci Rep. 2019 9(1):16032.
- Nociceptive pain - see (i) Hockley JR, et al. J Neurosci. 2016 36(8):2364-76; (ii) Kwon SG, et al. Brain Res Bull. 2017 130:165-172; (iii) Mitchell R, et al. Mol Neurobiol. 2019 56(8):5917-5933; and (iv) Yang CT, et al. Sci Rep. 2019 9(1):16032.
- Epilepsy - see (i) Alvarez-Ferradas C, et al. Glia. 2015 63(9):1507-21; (ii) Alves M, et al. J Neurosci. 2019 39(27):5377-5392; (iii) Alves M, et al. Front Pharmacol. 2020 10:1558; (iv) Alves M, et al. Epilepsia. 2017 58(9):1603-1614; (v) Nikolic L, et al. Glia. 2018 66(12):2673-2683; and (vi) Wellmann M, et al. Front Cell Neurosci. 2018 12:446.
- Stroke (*via* neuroprotection) - see (i) Carmo MR, et al. Eur J Neurosci. 2014 39:614-622; (ii) Chin Y, et al. J Neuroinflammation. 2013 10:95; (iii) Forster D, Reiser G. Purinergic Signal. 2015 11:441-454; (iv) Fukumoto Y, et al. J Cereb Blood Flow Metab. 2019 39:2144-2156;(v) Kuboyama K, et al. J Cereb Blood Flow Metab. 2011 31:1930-1941; (vi) Giovanna, M., et al. PloS one 2014, vol. 9, 12 e115273; and (vii) Guo, H., et al. Molecular Medicine Reports, 2018 17, 1819-1824.
- Traumatic brain injury - see (i) Choo AM, et al. Brain. 2013 136:65-80; and (ii) Shinozaki Y, et al. Cell Rep. 2017 19:1151-1164.
- Amyotrophic lateral sclerosis - see (i) Cieślak M, et al. Purinergic Signal. 2019 15(1): 1-15; (ii) Izrael M, et al. Front Neurosci. 2020;14:824; (iii) Jiang MC, et al. Neuroscience. 2017 362:33-46; and (iv) Pehar M, et al. Curr Pharm Des. 2017 23:5010-5021.

Further CNS disorders associated with astrocytes include Huntington's disease, Parkinson's disease and frontotemporal dementia (see (i) Wilton DK & Stevens B. Neurobiol Dis. 2020 143; (ii) Miyazaki I & Asanuma M. Cells. 2020 9:2623; and (iii) Radford RA, et al. Front Cell Neurosci. 2015 9:414). These conditions therefore represent additional candidate CNS disorders for treatment and/or prevention through P2Y1 inhibition.

A number of P2Y1 inhibitors have previously been developed, as described in e.g. WO 2005/113511, WO 2005/113537, WO 2006/078621, WO 2008/023235, WO 2014/022253, WO 2014/022349, WO 2014/022343, Yang et al., J. Med. Chem. 2014 57(14) 6150-6164 and Peng et al., European Journal of Medicinal Chemistry 158 (2018) 302-310. However, each of these references concerns the use of P2Y1 inhibitors in treating thromboembolic disorders, and therefore seek only to provide compounds capable of entering, and circulating within, the blood stream. In contrast to achieving systemic delivery, providing active compounds that are capable of crossing the blood-brain barrier [BBB] is known to be extremely challenging (see Pardridge W M., Alzheimers Dement. 2009;5(5):427-43). For instance, as of 2009, it was determined that more than 98% of all small molecule drugs, and ~100% of all large molecule drugs, do not cross the BBB. This has the implication that drugs which might hypothetically be useful for treating CNS disorders will - in many cases - only be administrable to the CNS *via* highly invasive, and so often impractical, drug delivery methods such as transcranial drug delivery. Such an administration mode is disadvantageous, indeed to the extent of becoming practically infeasible, not only because of the need for neurosurgical intervention, but also due to problems with the extent to which the active agent is capable of diffusing from the injection site throughout the human brain.

It is desirable to identify new compounds that are capable of inhibiting the P2Y1 receptor in order to provide methods for the treatment of conditions susceptible to treatment by P2Y1 inhibition. It is particularly desirable to identify P2Y1 inhibitors that are capable of crossing the blood-brain barrier, i.e. to provide P2Y1 inhibitors that are CNS penetrants

and that are therefore susceptible to administration outside of the CNS (e.g. by oral administration), whilst still being able subsequently to access the CNS.

The present inventors have identified new P2Y1 inhibitors. They have further identified specific P2Y1 inhibitors that are capable of crossing the blood-brain barrier. The ability of these inhibitors to pass across the blood-brain barrier is surprising at least in view of the well-known wide inability of most small molecule active pharmaceutical ingredients (APIs) to demonstrate such properties, the absence of any suggestion in the existing literature that previously described P2Y1 inhibitors may have such properties, and, still further, the physicochemical properties of these compounds, being inconsistent with the accepted CNS drug-like chemical space and with *in silico* predictors of brain penetration such as the state-of-the-art CNS Multiparameter Optimization (CNS MPO) score (Wager, T.T et al. ACS Chem. Neurosci. 2010, 1, 435-449). Furthermore, the ability of the presently identified compounds to cross the blood-brain barrier, and thereafter exert clinically significant effects, following e.g. oral administration is not shared with some other P2Y1 inhibiting compounds. One particular focus of the present invention is Alzheimer's disease.

## Summary of the invention

The present invention provides compounds ('compounds of the invention'). Such compounds in particular include compounds of formula (I) or formula (II), as defined herein, as well as tautomers, N-oxides, pharmaceutically acceptable salts, and solvates thereof.

The present invention also provides a pharmaceutical composition comprising a compound of the invention, in association with one or more pharmaceutically acceptable carriers.

The present invention also provides a compound of the invention, or a pharmaceutical composition of the invention, for use as a medicament, and in particular for use in a method for treating and/or preventing a CNS disorder (typically a CNS disorder susceptible to treatment by P2Y1 inhibition).

The present invention also provides the use of a compound of the invention, or the pharmaceutical composition of the invention, for the manufacture of a medicament, in particular a medicament for use in a method for treating and/or preventing a CNS disorder.

The present invention also provides a method of treatment, in particular a method for treating and/or preventing a CNS disorder in a patient in need thereof, the method comprising administering a compound of the invention, or the pharmaceutical composition of the invention, to the patient.

## Definitions

Compounds of the present invention may have asymmetric centers. Compounds of the present invention containing an asymmetrically substituted atom may be isolated in optically active or racemic forms. It is well known in the art how to prepare optically active forms, such as by resolution of racemic forms or by synthesis from optically active starting materials. Geometric isomers of double bonds such as olefins and C=N double bonds can also be present in the compounds described herein, and all such stable isomers are contemplated. Cis and trans geometric isomers of the compounds are described and may be isolated as a mixture of isomers or as separated isomeric forms. All chiral, diastereomeric, racemic forms and all geometric isomeric forms of a structure are intended, unless the specific stereochemistry or isomeric form is specifically indicated. Hence, the compounds of the present invention include all possible chiral, diastereomeric and racemic forms thereof and all possible geometric isomers thereof. When no specific mention is made of the configuration (*cis, trans,* R, S, etc.) of a compound (or of an asymmetric carbon), then any one of the isomers or a mixture of more than one isomer is intended. Limitation to a particular asymmetric form of a compound is only intended where expressly indicated.

The processes for preparation can use racemates, enantiomers, or diastereomers as starting materials. When enantiomeric or diastereomeric products are prepared, they can be separated by conventional methods, for example, by chromatographic or fractional crystallization. The prepared compounds may be in the free or hydrate form.

Compounds of the present invention encompass both compounds in which: (a) all contained atoms are in their natural isotopic form ("natural isotopic form of the compound"); and (b) compounds in which one or more contained atoms are in a non-natural isotopic form ("unnatural variant isotopic form of the compound"), for instance compounds comprising isotopic replacement, enrichment, or depletion. An unnatural variant isotopic form of the compound may thus contain one or more artificial or uncommon isotopes such as deuterium ($^{2}H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{37}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms of the compound comprising radioisotopes may, for example, be used for drug and/or substrate tissue distribution studies. The radioactive isotopes tritium, i.e. $^{3}H$, and carbon-14, i.e. $^{14}C$, are particularly

useful for this purpose in view of their ease of incorporation and ready means of detection. Unnatural variant isotopic forms which incorporate deuterium i.e. $^2H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

The following are definitions of terms used in this specification. The initial definition provided for a group or term herein applies to that group or term throughout the present specification, individually or as part of another group, unless otherwise indicated.

Preferably, the molecular weight of compounds of the present invention is less than about 500, 550, 600, 650, 700, 750, or 800 grams per mole.

Preferably, the molecular weight is less than about 800 grams per mole. More preferably, the molecular weight is less than about 750 grams per mole. Even more preferably, the molecular weight is less than about 700 grams per mole.

The term "substituted" as used herein, means that any one or more hydrogens on the designated atom is replaced with a selection from the indicated group, provided that the designated atom's normal valency is not exceeded, and that the substitution results in a stable compound (for instance, avoiding unstable acetals or similar groups). When a substituent is keto (i.e., =O), then 2 hydrogens on the atom are replaced. When a ring system (e.g., carbocyclic or heterocyclic) is said to be substituted with a carbonyl group or a double bond, it is intended that the carbon atom of the carbonyl group or one carbon atom of the double bond be part of (i.e., within) the ring. Ring double bonds, as used herein, are double bonds that are formed between two adjacent ring atoms (e.g., C=C, C=N, or N=N).

When any variable (e.g., $R^4$, $R^{9A}$, etc.) occurs more than one time in any constituent or formula for a compound, its definition at each occurrence is independent of its definition at every other occurrence. Thus, for example, if a group is shown to be substituted with 0-3 $R^4$, then said group may optionally be substituted with up to three $R^4$ groups and $R^4$ at each occurrence is selected independently from the definition of $R^4$. Also, combinations of substituents and/or variables are permissible only if such combinations result in stable compounds.

When a bond to a substituent is shown to cross a bond connecting two atoms in a ring, then such substituent may be bonded to any atom on the ring. When a substituent is listed without indicating the atom via which such substituent is bonded to the rest of the compound of a given formula, then such substituent may be bonded via any atom in such substituent. Combinations of substituents and/or variables are permissible only if such combinations result in stable compounds.

As used herein, "alkyl" or "alkylene" is intended to include both branched and straight-chain saturated aliphatic hydrocarbon groups having the specified number of carbon atoms. For example, "$C_1$-$C_{10}$ alkyl" (or alkylene), is intended to include $C_1$, $C_2$, $C_3$, $C_4$, $C_5$, $C_6$, $C_7$, $C_8$, $C_9$, and $C_{10}$ alkyl groups. Additionally, for example, "$C_1$-$C_6$ alkyl" denotes alkyl having 1 to 6 carbon atoms. Examples of alkyl include, but are not limited to, methyl, ethyl, n-propyl, i-propyl, n-butyl, i-butyl, sec-butyl, t-butyl, n-pentyl, n-hexyl, 2-methylbutyl, 2-methylpentyl, 2-ethylbutyl, 3-methylpentyl, and 4-methylpentyl.

"Alkoxy" or "alkyloxy" represents an alkyl group as defined above with the indicated number of carbon atoms attached through an oxygen bridge. For example, "$C_1$-$C_6$ alkoxy" (or alkyloxy), is intended to include $C_1$, $C_2$, $C_3$, $C_4$, $C_5$, and $C_6$ alkoxy groups (and can also be written equivalently as -O-$C_{1-6}$ alkyl). Examples of alkoxy include, but are not limited to, methoxy, ethoxy, n-propoxy, i-propoxy, n-butoxy, s-butoxy, t-butoxy, n-pentoxy, and s-pentoxy.

As used herein, "carbocycle" refers to a monocyclic ring or polycyclic (e.g., bicyclic or tricyclic) ring system whose ring atoms consist of carbon atoms.

A polycyclic carbocycle may comprise fused rings (where a first ring and a second ring share two adjacent ring carbon atoms), bridged rings (where two non-adjacent ring carbon atoms of a first ring are shared with a second ring) and/or spirocyclic rings (where a first ring shares only a single ring carbon atom with a second ring). A tricyclic or higher order polycyclic carbocycle may comprise a mixture of fused, bridged and spirocyclic relationships between its constituent rings. A bicyclic carbocycle is one of fused, bridged and spirocyclic.

The carbocycle may be saturated (also referred to herein as cycloalkyl), partially unsaturated, or aromatic (also referred to herein as aryl).

The term "cycloalkyl" refers to a carbocycle in which all ring bonds between adjacent carbon atoms are single bonds (i.e. a saturated ring system). A typical cycloalkyl is $C_{3-10}$ cycloalkyl, which is intended to include $C_3$, $C_4$, $C_5$, $C_6$, $C_7$, $C_8$, $C_9$ and $C_{10}$ cycloalkyl groups. Example cycloalkyl groups include, but are not limited to, cyclopropyl, cyclobutyl, cyclopentyl, cyclohexyl, norbomyl, and the like.

Examples of carbocycles include $C_{3-10}$ carbocycles, such as monocyclic or bicyclic (including fused, bridged and spirocyclic where chemically possible in view of the number of carbon ring atoms) carbocycles, further including $C_{3-7}$ carbocycles, $C_{3-6}$ carbocycles, $C_{3-5}$ carbocycles and $C_{3-4}$ carbocycles.

Examples of carbocycles include, but are not limited to, cyclopropyl, cyclobutyl, cyclopentyl, cyclohexyl, cycloheptyl, adamantyl, cyclooctyl, [3.3.0]bicyclooctane, [4.3.0] bicyclononane, [4.4.0] bicyclodecane (decalin), [2.2.2] bicyclooctane, fluorenyl, phenyl, naphthyl, dihydronaphthyl, 1,2,3,4- tetrahydronaphthyl, indanyl, adamantyl, or tetrahydronaphthyl (tetralin). Preferred carbocycles, unless otherwise specified, are cyclopropyl, cyclobutyl, cyclopentyl, cyclohexyl, phenyl, naphthyl, and indanyl. When the term "carbocycle" is used, it is intended to include "aryl".

As used herein, the term "aryl" is intended to mean an aromatic moiety containing, if specified, the specified number of carbon atoms; for example phenyl or naphthyl.

As used herein, "heterocycle" or "heterocyclic" refers to a monocyclic ring or polycyclic (e.g., bicyclic or tricyclic) ring system whose ring atoms consist of carbon atoms and at least one heteroatom.

A polycyclic heterocycle may comprise fused rings (where a first ring and a second ring share two adjacent ring atoms), bridged rings (where two non-adjacent ring atoms of a first ring are shared with a second ring) and/or spirocyclic rings (where a first ring shares only a single ring atom with a second ring). A tricyclic or higher order polycyclic heterocycle may comprise a mixture of fused, bridged and spirocyclic relationships between its constituent rings. A bicyclic heterocycle is one of fused, bridged and spirocyclic. The heterocycle may be saturated, partially unsaturated, or aromatic (also referred to herein as heteroaryl).

The heterocycle may be saturated, partially unsaturated, or aromatic. When the term "heterocycle" is used, it is intended to include heteroaryl. The term "heterocycle" also includes cycloheteroalkyl.

The heterocycle contains at least one heteroatom ring atom. Typically, heteroatoms are selected from N, $NR^5$, O and $S(O)_p$, where p and $R^5$ are defined elsewhere herein. The at least one heteroatom is commonly 1-4 heteroatoms, for instance 1-3 heteroatoms, 1-2 heteroatoms or 1 heteroatom. It is preferred that when the total number of $S(O)_p$ and O ring atoms in the heterocycle exceeds 1, then these heteroatoms are not adjacent to one another. It is preferred that the total number of $S(O)_p$ and O ring atoms in the heterocycle is not more than 1.

Typically the heterocycle contains, in addition to heteroatom ring atoms, at least 1 carbon ring atom, preferably at least two ring atoms. In polycyclic heterocycles, typically each ring contains at least 1 carbon atom. In polycyclic heterocycles at least one ring of the polycyclic ring system contains at least one heteroatom ring atom (for avoidance of doubt, it is not necessary for each ring of a polycyclic heterocycles to contain at least one heteroatom).

Typical examples of heterocycles include 5- to 10-membered heterocycles, wherein "X-membered" means that the total number of ring atoms possessed by the heterocycle is X.

Examples of heterocycles include, but are not limited to, pyridyl, pyrimidinyl, pyrazinyl, pyridazinyl, triazinyl, furyl, quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, isothiazolyl, purinyl, carbazolyl, benzimidazolyl, 2,3-dihydrobenzofuranyl, 2,3-dihydrobenzothienyl, 2,3-dihydrobenzothienyl-S-oxide, 2,3-dihydrobenzothienyl-S-dioxide, benzoxazolin-2-on-yl, indolinyl, benzodioxolanyl, benzodioxane, 2-pyrrolidonyl, 2H,6H-1,5,2-dithiazinyl, 2H-pyrrolyl, 3H-indolyl, 4-piperidonyl, 4aH-carbazole, 4H-quinolizinyl, 6H-1,2,5-thiadiazinyl, acridinyl, azocinyl, benzofuranyl, benzothiofuranyl, benzothiophenyl, benzoxazolyl, benzoxazolinyl, benztriazolyl, benztetrazolyl, benzisoxazolyl, benzisothiazolyl, benzimidazalonyl, 4aH-carbazolyl, b-carbolinyl, chromanyl, chromenyl, cinnolinyl, decahydroquinolinyl, 2H,6H-1,5,2-dithiazinyl, dihydrofuro[2,3-b]tetrahydrofuran, furanyl, furazanyl, imidazolidinyl, imidazolinyl, imidazolopyridinyl, 1*H*-indazolyl, indolenyl, indolizinyl, isatinoyl, isobenzofuranyl, isochromanyl, isoindazolyl, isoindolinyl, isoindolyl, isoquinolinyl, isothiazolopyridinyl, isoxazolopyridinyl, morpholinyl, naphthyridinyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolopyridinyl, oxazolidinylperimidinyl, oxindolyl, phenanthridinyl, phenanthrolinyl, phenarsazinyl, phenazinyl, phenothiazinyl, phenoxathiinyl, phenoxazinyl, phthalazinyl, piperazinyl, piperidinyl, piperidonyl, pteridinyl, pyranyl, pyrazolidinyl, pyrazolinyl, pyrazolopyridinyl, pyridooxazole, pyridoimidazole, pyridothiazole, pyridinyl, pyrrolidinyl, pyrrolinyl, pyrrolyl, quinazolinyl, quinolinyl, 4*H*-quinolizinyl, quinoxalinyl, quinuclidinyl, carbolinyl, tetrahydrofuranyl, tetrahydroisoquinolinyl, tetrahydroquinolinyl, 6*H*-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolopyridinyl, thienothiazolyl, thienooxazolyl, thienoimidazolyl, thiophenyl, 1,2,3-triazolyl, 1,2,4-triazolyl, 1,2,5-triazolyl, 1,3,4-triazolyl, xanthenyl, tetrahydrothiophenyl, tetrahydropyranyl, and morpholinyl.

Heteroaryl groups include, without limitation, pyridyl, pyrimidinyl, pyrazinyl, pyridazinyl, triazinyl, furyl, quinolyl, isoquinolyl, thienyl, imidazolyl, thiazolyl, indolyl, pyrryl, oxazolyl, benzofuryl, benzothienyl, benzthiazolyl, isoxazolyl, pyrazolyl, triazolyl, tetrazolyl, indazolyl, 1,2,4-thiadiazolyl, isothiazolyl, benzothienyl, purinyl, carbazolyl, benzimidazolyl, 2,3-dihydrobenzofuranyl, 2,3-dihydrobenzothienyl, 2,3-dihydrobenzothienyl-S-oxide, 2,3-dihydrobenzothienyl-S-dioxide, benzoxazolin-2-on-yl, indolinyl, benzodioxolanyl, benzodioxane, and the like.

Non-limiting examples of exemplary heterocycles include: (a) pyridinyl, pyrimidinyl, furanyl, isoxazolyl, thiazolyl, thiadiazolyl, benzothiazolyl, thiazolopyridinyl, indolyl, benzodioxolyl, and benzimidazolyl (b) pyridinyl, pyrimidinyl, thiadiazolyl, thiazolopyridinyl, benzodioxolyl, and benzimidazolyl; and (c) thiazolopyridinyl.

The phrase "pharmaceutically acceptable" is employed herein to refer to those compounds, materials, compositions, and/or dosage forms which are, within the scope of sound medical judgment, suitable for use in contact with the tissues of human beings and animals, especially human beings, without excessive toxicity, irritation, allergic response, or other problem or complication, commensurate with a reasonable benefit/risk ratio.

The compounds of the present invention encompass pharmaceutically acceptable salts (in particular, pharmaceutically acceptable salts of compounds of formula (I) and of formula (II), as well as solvates, N-oxides and tautomers

thereof). As used herein, "pharmaceutically acceptable salts" refer to derivatives of the disclosed compounds wherein the parent compound (e.g., of formula (I) or of formula (II)) is modified by making pharmaceutically acceptable acid or base salts thereof. Examples of pharmaceutically acceptable salts include, but are not limited to, mineral or organic acid salts of basic groups such as amines; and alkali or organic salts of acidic groups such as carboxylic acids. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, and nitric; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, and isethionic, and the like.

The pharmaceutically acceptable salts of the present invention can be synthesized from the parent compound (e.g., of formula (I) or of formula (II)) which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, ethyl acetate, ethanol, isopropanol, or acetonitrile are preferred. Lists of suitable salts are found in Remington's Pharmaceutical Sciences, 17th ed., Mack Publishing Company, Easton, PA, 1985, p. 1418, the disclosure of which is hereby incorporated by reference.

Non-pharmaceutically acceptable salt forms of the compounds of the invention may be of use during the preparation of non-salt or pharmaceutically acceptable salt forms. Consequently, the invention also encompasses a non-pharmaceutically acceptable salt of a compound of formula (I) or formula (II).

The compounds of the present invention encompass solvates (in particular, solvates of compounds of formula (I) and of formula (II), as well as salts, N-oxides and tautomers thereof). Solvates include, and preferably are, hydrates. Methods of solvation are generally known in the art.

The compounds of the present invention encompass tautomers (in particular, tautomers of compounds of formula (I) and of formula (II), as well as salts, solvates and N-oxides thereof). Some compounds of the invention may exist in a plurality of tautomeric forms, in which hydrogen atoms are transposed to other parts of the molecules and the chemical bonds between the atoms of the molecules are consequently rearranged. It should be understood that all tautomeric forms, insofar as they may exist, are encompassed by the compounds of the present invention.

In cases wherein there are nitrogen atoms (e.g., amines) on compounds of the present invention (e.g. of formula (I) or formula (II)), these can be converted to N-oxides by treatment with an oxidizing agent (e.g., MCPBA and/or hydrogen peroxides) to afford other compounds of this invention. Thus, all shown nitrogen atoms are considered to cover both the shown nitrogen and its N-oxide ($N \rightarrow O$) derivative. The compounds of the present invention encompass N-oxides (in particular, N-oxides of compounds of formula (I) and of formula (II), as well as salts, solvates and tautomers thereof).

For avoidance of doubt, many compounds of the invention can exist simultaneously in the form of two or more of a tautomer, N-oxide, pharmaceutically acceptable salt, and solvate of a particular parent compound (e.g. a compound of formula (I) or formula (II)), and all such possible compounds are encompassed within the definition of "compound of the invention". Hence, a compound of the invention (to the extent chemically possible in view of the relevant formula (I) or formula (II) expressly includes any one of the following: (1) a compound of formula (I) or formula (II) (which may also be referred to herein as a "free base form" of the compound); (2) a tautomer of formula (I) or formula (II); (3) an N-oxide of formula (I) or formula (II); (4) a pharmaceutically acceptable salt of formula (I) or formula (II); (5) a solvate of formula (I) or formula (II); (6) an N-oxide of a tautomer of formula (I) or formula (II); (7) a pharmaceutically acceptable salt of a tautomer of formula (I) or formula (II); (8) a solvate of a tautomer of formula (I) or formula (II); (9) a pharmaceutically acceptable salt of an N-oxide of a tautomer of formula (I) or formula (II); (10) a solvate of an N-oxide of a tautomer of formula (I) or formula (II); (11) a solvate of a pharmaceutically acceptable salt of a tautomer of formula (I) or formula (II); (12) a solvate of a pharmaceutically acceptable salt of an N-oxide of a tautomer of formula (I) or formula (II); (13) a pharmaceutically acceptable salt of an N-oxide of formula (I) or formula (II); (14) a solvate of an N-oxide of formula (I) or formula (II); (15) a solvate of a pharmaceutically acceptable salt of an N-oxide of a tautomer of formula (I) or formula (II); and (16) a solvate of a pharmaceutically acceptable salt of formula (I) or formula (II).

Compounds of the present invention are, subsequent to their preparation, preferably isolated and purified to obtain a composition containing an amount by weight equal to or greater than 99% compound ("substantially pure"), which is then used or formulated as described herein. Such "substantially pure" compounds are also part of the present invention.

"Stable compound" and "stable structure" are meant to indicate a compound that is sufficiently robust to survive isolation to a useful degree of purity from a reaction mixture, and formulation into an efficacious therapeutic agent. It is preferred that compounds of the present invention do not contain aN-halo, $S(O)_2H$, or S(O)H.

Purely for the avoidance of doubt, it should be noted that wherever a compound is disclosed as being either a particular structure (e.g. a particular general chemical formula, such as of formula (I) or formula (II)) or a tautomer, N-oxide, pharmaceutically acceptable salt, or solvate thereof, then, unless expressly indicated to the contrary, any disclosed

further embodiment of that compound (e.g. "A compound ... according to ..."), and describing additional limitations to the recited structure, continues to embrace any such tautomer, N-oxide, pharmaceutically acceptable salt, or solvate thereof.

As used herein, "treating and/or preventing" or "treatment and/or prevention" of a disease-state in a mammal, particularly a human, include: (a) preventing the disease-state from occurring in a mammal, in particular, when such mammal is predisposed to the disease-state but has not yet been diagnosed as having it; (b) inhibiting the disease-state, i.e., slowing or arresting its development; and/or (c) relieving the disease-state, i.e., causing regression of the disease state or a reduction in associated symptoms.

"Therapeutically effective amount" is intended to include an amount of a compound that is effective to achieve a desirable effect in treating and/or preventing a disease-state. A desirable effect is typically clinically significant and/or measurable, for instance in the context of (a) preventing the disease-state from occurring in a mammal, in particular, when such mammal is predisposed to the disease-state but has not yet been diagnosed as having it; (b) inhibiting the disease-state, i.e., slowing or arresting its development; and/or (c) relieving the disease-state, i.e., causing regression of the disease state or a reduction in associated symptoms. The therapeutically effective amount may be one that is sufficient to achieve the desirable effect either when the compound is administered alone, or alternatively when it is administered in combination with one or more further APIs, which either are further compounds of the invention or are different from the compounds of the invention. Furthermore, a therapeutically effective amount is typically an amount that is sufficient to inhibit P2Y1 receptors, preferably P2Y1 receptors in the CNS, again when administered either alone or in combination with one or more further APIs (which may also target P2Y1 receptors, or alternatively may exert their pharmacological effects by a different mechanism). Thus, "therapeutically effective amount" is intended to include an amount of a combination of compounds that each are compounds of the invention that is effective to inhibit P2Y1 receptors, preferably P2Y1 receptors in the CNS. The combination of compounds is preferably a synergistic combination.

Synergy, as described, for example, by Chou and Talalay, Adv. Enzyme Regul. 1984, 22: 27-55, occurs when the effect (in this case, inhibition of P2Y1) of the compounds when administered in combination is greater than the additive effect of the compounds when administered alone as a single agent. In general, a synergistic effect is most clearly demonstrated at sub-optimal concentrations of the compounds. Synergy can be in terms of lower cytotoxicity, or some other beneficial effect of the combination compared with the individual components.

For avoidance of doubt, a "therapeutically effective amount" as recited herein can be achieved by any suitable dosage regimen, including but not limited to exemplary dosage regimens described elsewhere herein. Hence, for example, references herein to administering a therapeutically effective amount of a compound by a particular administration route including achieving the therapeutically effective amount via a single dose or by plural doses administered by the specified administration route. For instance, orally administering a therapeutically effective amount includes both orally administering a single dose and orally administering any plural number of doses, provided that a therapeutically effective amount is thereby achieved by oral administration.

The present invention further includes compositions comprising one or more compounds of the present invention and a pharmaceutically acceptable carrier.

A "pharmaceutically acceptable carrier" refers to media generally accepted in the art for the delivery of biologically active agents to animals, in particular, mammals. Pharmaceutically acceptable carriers are formulated according to a number of factors well within the purview of those of ordinary skill in the art. These include, without limitation: the type and nature of the active agent being formulated; the subject to which the agent-containing composition is to be administered; the intended route of administration of the composition; and, the therapeutic indication being targeted. Pharmaceutically acceptable carriers include both aqueous and non-aqueous liquid media, as well as a variety of solid and semi- solid dosage forms. Such carriers can include a number of different ingredients and additives in addition to the active agent, such additional ingredients being included in the formulation for a variety of reasons, e.g., stabilization of the active agent, binders, etc., well known to those of ordinary skill in the art. Descriptions of suitable pharmaceutically acceptable carriers, and factors involved in their selection, are found in a variety of readily available sources such as, for example, Remington’s Pharmaceutical Sciences, 17th ed., 1985, which is incorporated herein by reference in its entirety.

## Detailed description of the invention

The following aspects provide, in conjunction with the disclosure elsewhere herein, non-limiting exemplary embodiments of the present invention. For the avoidance of doubt, the present invention encompasses the subject-matter defined in any one of these aspects, any combination thereof such as those recited by dependency, and any combination thereof with features of the invention that are disclosed elsewhere herein.

*Aspects*

1. A compound that (a) is of formula (I) or formula (II):

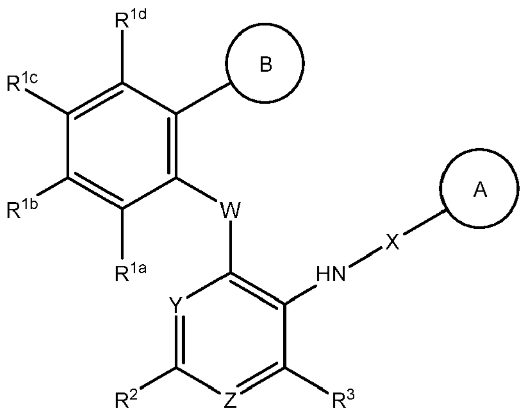

(I)

5

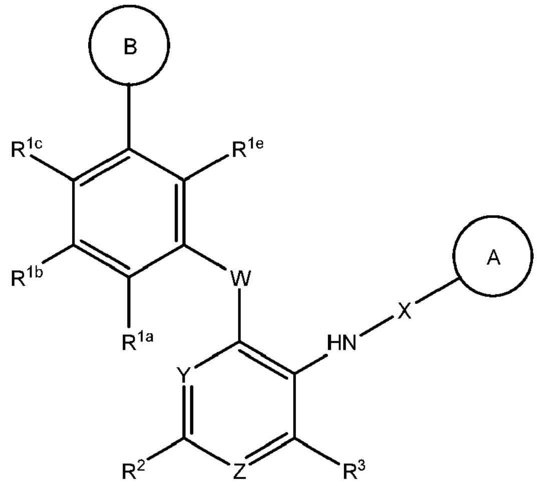

(II)

;

or (b) is a tautomer, N-oxide, pharmaceutically acceptable salt, or solvate thereof; wherein: ring A is $C_{3-10}$ carbocycle substituted with 0-4 $R^4$, or is 5- to 10-membered heterocycle comprising carbon ring atoms and 1-4 heteroatom ring atoms selected from N, $NR^5$, O and $S(O)_p$, wherein said heterocycle is substituted with 0-4 $R^4$, with the proviso that ring A is not:

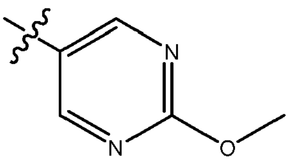

or

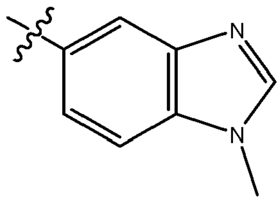

,

and with the proviso that when ring A is a 2-pyridinyl ring of formula (III), there is no $R^4$ substituent at the pyridinyl 3-position marked by * in formula (III)

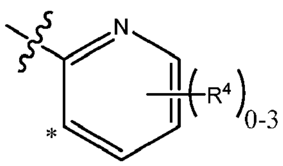

(III)

;

ring B is selected from:

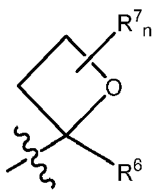

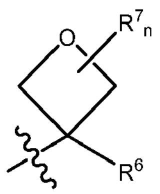

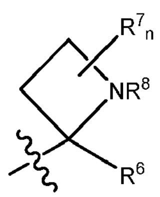

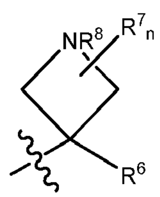

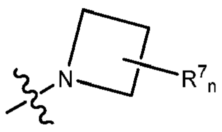 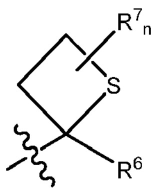 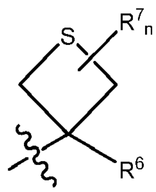 ;

W is O, NH, or S;
X is -CO-NH- or bond;
Y is N or CH;
Z is N or CH;
$R^{1a}$ and $R^{1b}$ are, independently at each occurrence, H, F, Cl, Br, -OH, or $-C_{1-6}$ alkyl substituted with 0-3 substituents selected from F or Cl;
$R^{1c}$, $R^{1d}$ and $R^{1e}$ are, independently at each occurrence, H, F, Cl, Br, or $-C_{1-6}$ alkyl substituted with 0-3 substituents selected from F or Cl;
$R^2$ is H, F, Cl, Br, or $-C_{1-6}$ alkyl substituted with 0-3 substituents selected from F or Cl;
$R^3$ is H or OH;
$R^4$ is, independently at each occurrence, F, Cl, Br, $-C_{rA}F_{(2rA+1)}$, $-C_{rA}F_{2rA}H$, $-(CR^{9A}{}_2)_{tA}-NR^{10}R^{11}$, -CN, $-OC_{rA}F_{(2rA+1)}$, $-OC_{rA}F_{2rA}H$, $-SF_5$, $-C_{1-6}$ alkyl substituted with 0-3 substituents selected from $R^{12}$, $-C_{3-6}$ cycloalkyl substituted with 0-3 substituents selected from $R^{12}$, $-O-C_{1-6}$ alkyl substituted with 0-3 substituents selected from $R^{12}$, $-O-C_{3-6}$ cycloalkyl substituted with 0-3 substituents selected from $R^{12}$, or 3- to 6- membered heterocyclic ring comprising: carbon ring atoms and 1-3 heteroatom ring atoms selected from N, $NR^5$, O and $S(O)_p$, wherein said heterocyclic ring is substituted with 0-3 substituents selected from F, Cl, $-C_{rAA}F_{(2rAA+1)}$, $-C_{rAA}F_{2rAA}H$, or $C_{1-6}$ alkyl substituted with 0-3 substituents selected from $R^{12}$;
alternatively, two $R^4$ groups on the same carbon ring atom combine to form a $C_{3-6}$ carbocycle substituted with 0-2 substituents selected from $R^{13}$;
$R^5$ is, independently at each occurrence, H, $-C_{rB}F_{(2rB+1)}$, $-C_{rB}F_{2rB}H$ or $-C_{1-6}$ alkyl substituted with 0-3 substituents selected from F or Cl;
$R^6$ is H, $-C_{rC}F_{(2rC+1)}$, -CrcF2rcH, or $C_{1-6}$ alkyl;
$R^7$ is $-C_{rD}F_{(2rD+1)}$, $-C_{rD}F_{2rD}H$, or $C_{1-6}$ alkyl;

Alternatively, two $R^7$ groups on the same carbon ring atom form =O;
$R^8$ is H, $-C_{rE}F_{(2rE+1)}$, $-C_{rE}F_{2rE}H$, $C_{1-6}$ alkyl substituted with 0-3 substituents selected from F and Cl, or $-(CR^{9B}{}_2)_{tB}-C_{3-10}$ carbocycle substituted with 0-3 substituents selected from F, Cl or $C_{1-6}$ alkyl;
$R^{9A}$ is, independently at each occurrence, H, $-C_{rF}F_{(2rF+1)}$, $-C_{rF}F_{2rF}H$, or $C_{1-6}$ alkyl;
alternatively, two $R^{9A}$ groups on the same carbon atom combine to form a 3- to 7-membered carbocyclic ring;
$R^{9B}$ is, independently at each occurrence, H, $-C_{rG}F_{(2rG+1)}$, $-C_{rG}F_{2rG}H$, or $C_{1-6}$ alkyl;
alternatively, two $R^{9B}$ groups on the same carbon atom combine to form a 3- to 7-membered carbocyclic ring;
$R^{10}$ is $-C_{1-6}$ alkyl substituted with 0-3 substituents selected from F or Cl;
$R^{11}$ is H or $-C_{1-6}$ alkyl substituted with 0-3 substituents selected from F or Cl;
alternatively, $R^{10}$ and $R^{11}$ on the same nitrogen atom combine to form a 3- to 6-membered heterocyclic ring comprising: the said nitrogen atom as a heteroatom ring atom, one or more carbon ring atoms and 0, 1 or 2 further heteroatom ring atoms selected from N, $NR^{14}$, O and $S(O)_p$, wherein said heterocyclic ring is substituted with 0-3 substituents selected from F or Cl;
$R^{12}$ is, independently at each occurrence, F, Cl, $-CF_3$, $-CF_2H$, $-C_2F_5$, $-C_2F_4H$, $-OCH_3$,$-OCH_2CH_3$, $-N(CH_3)_2$, $-N(CH_3)(CH_2CH_3)$, $-N(CH_2CH_3)_2$;
$R^{13}$ is, independently at each occurrence, F or Cl;
$R^{14}$ is H, $-C_{rH}F_{(2rH+1)}$, $-C_{rH}F_{2rH}H$ or $-C_{1-6}$ alkyl substituted with 0-3 substituents selected from F or Cl;
n is 0, 1 or 2;
p is, independently at each occurrence, 0, 1 or 2;
rA is, independently on each substituent in which it occurs, 1-6;
rAA is, independently on each substituent in which it occurs, 1-6;
rB is, independently on each substituent in which it occurs, 1-6;
rC is, independently on each substituent in which it occurs, 1-6;
rD is, independently on each substituent in which it occurs, 1-6;
rE is, independently on each substituent in which it occurs, 1-6;
rF is, independently on each substituent in which it occurs, 1-6;

rG is, independently on each substituent in which it occurs, 1-6;
rH is, independently on each substituent in which it occurs, 1-6;
tA is, independently at each occurrence, 1, 2 or 3; and
tB is, independently at each occurrence, 1, 2 or 3.

2. The compound according to aspect 1, wherein:
ring A is a phenyl ring substituted with 0-4 $R^4$ or a napthyl ring substituted with 0-4 $R^4$.

3. The compound according to aspect 2, wherein:
ring A is a phenyl ring substituted with 0-4 $R^4$.

4. The compound according to aspect 2 or 3, wherein:
ring A is substituted with 0-3 $R^4$.

5. The compound according to aspect 4, wherein ring A is substituted with 1-3 $R^4$.

6. The compound according to aspect 4, wherein ring A is unsubstituted.

7. The compound according to aspect 4, wherein ring A is substituted with 1 $R^4$.

8. The compound according to aspect 4, wherein ring A is substituted with 2 $R^4$.

9. The compound according to aspect 4, wherein ring A is substituted with 3 $R^4$.

10. The compound according to any one of aspects 1 to 9, wherein:
$R^4$ is, independently at each occurrence, F, Cl, $-C_{rA}F_{(2rA+1)}$, $-C_{rA}F_{2rA}H$, $-CH_2-NR^{10}R^{11}$, -CN, $-OC_{rA}F_{(2rA+1)}$, $-OC_{rA}F_{2rA}H$, $-SF_5$, $-C_{1-6}$ alkyl substituted with 0-2 substituents selected from $R^{12}$, $-C_{3-6}$ cycloalkyl substituted with 0-2 substituents selected from $R^{12}$, $-O-C_{1-6}$ alkyl substituted with 0-2 substituents selected from $R^{12}$, $-O-C_{3-6}$ cycloalkyl substituted with 0-2 substituents selected from $R^{12}$, or 3- to 6- membered heterocyclic ring comprising: carbon ring atoms and 1 heteroatom ring atom selected from $NR^5$ and O, wherein said heterocyclic ring is substituted with 0-2 substituents selected from F, Cl, $-C_{rAA}F_{(2rAA+1)}$, $-C_{rAA}F_{2rAA}H$, or $C_{1-6}$ alkyl substituted with 0-2 substituents selected from $R^{12}$.

11. The compound according to aspect 10, wherein:

$R^4$ is, independently at each occurrence, F, Cl, $-C_{rA}F_{(2rA+1)}$, $-C_{rA}F_{2rA}H$, $-CH_2-NR^{10}R^{11}$, -CN, $-OC_{rA}F_{(2rA+1)}$, $-OC_{rA}F_{2rA}H$, $-SF_5$, $-C_{1-3}$ alkyl substituted with 0-2 substituents selected from $R^{12}$, $-O-C_{1-3}$ alkyl substituted with 0-2 substituents selected from $R^{12}$, $O-C_{3-5}$ cycloalkyl substituted with 0-2 substituents selected from $R^{12}$, or 3- to 5- membered heterocyclic ring comprising: carbon atoms and 1 heteroatom selected from O, wherein said heterocyclic ring is substituted with 0-2 substituents selected from F, Cl, $-C_{rAA}F_{(2rAA+1)}$, $-C_{rAA}F_{2rAA}H$, or $C_{1-3}$ alkyl substituted with 0-2 substituents selected from $R^{12}$;
$R^{10}$ and $R^{11}$ on the same nitrogen atom combine to form a 3- to 5- membered heterocyclic ring comprising: carbon ring atoms and 1-2 heteroatom ring atoms selected from N, $NR^{14}$ and O and $S(O)_p$, wherein said heterocyclic ring is substituted with 0-3 substituents selected from F or Cl;$R^{12}$ is F, Cl, $-CF_3$, $-CF_2H$, $-C_2F_5$, or $-C_2F_4H$,;
$R^{14}$ is H, $-CH_3$ or $-CH_2CF_3$;
rA is, independently on each substituent in which it occurs, 1-3; and
rAA is, independently on each substituent in which it occurs, 1-3.

12. The compound according to aspect 11, wherein:
$R^4$ is, independently at each occurrence, F, Cl, $-CF_3$, $-CF_2H$, $-C_2F_5$, $-C_2F_4H$,

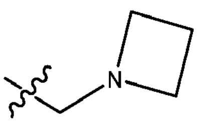 , 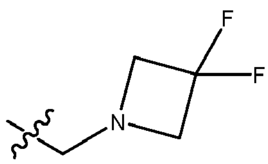 , 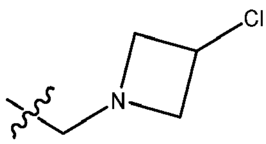 , 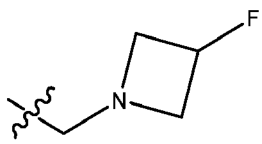 ,

-CN, - $OCF_3$, $-OC_2F_5$, $-OCF_2H$, $-OC_2F_4H$, $-SF_5$, $-C_{1-3}$ alkyl substituted with 0-2 substituents selected from $R^{12}$, $-OCH_3$, $-OC_2H_5$,

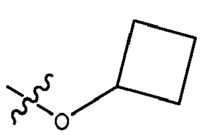 , 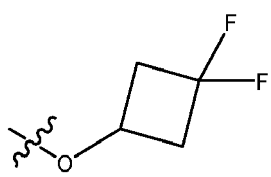 ,

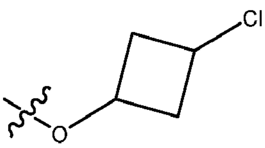, 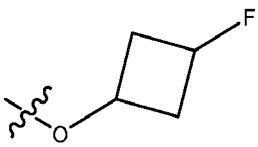, 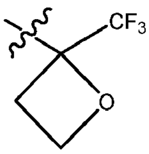, 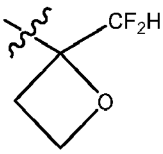, 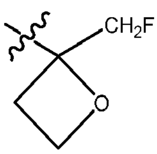,

or

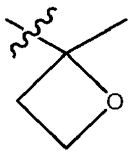;

and $R^{12}$ is F, Cl or $-CF_3$.

13. The compound according to aspect 12, wherein:

$R^4$ is, independently at each occurrence, F, Cl, $-CF_3$, $-CF_2H$, $-C_2F_5$, $-C_2F_4H$,

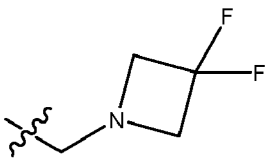, 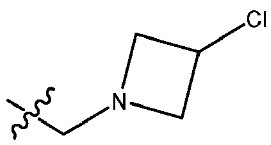, 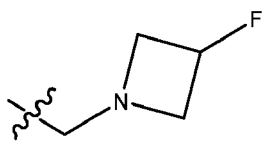,

$-CN$, $-OCF_3$, $-OC_2F_5$,$-OCF_2H$, $-OC_2F_4H$, $-SF_5$, $-C_{1-3}$ alkyl substituted with 0-2 substituents selected from $R^{12}$,$-OCH_3$, $-OC_2H_5$,

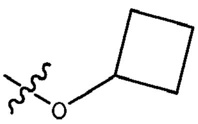, 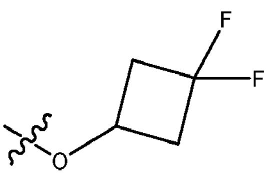, 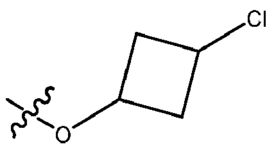,

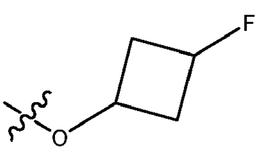, 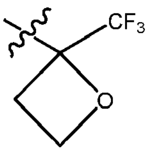, 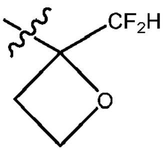, 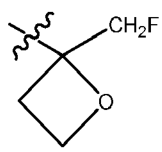, or 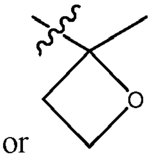;

and $R^{12}$ is F, Cl or $-CF_3$.

14. The compound according to aspect 13, wherein:

$R^4$ is, independently at each occurrence, F, Cl, $-CF_2H$, $-C_2F_5$, $-C_2F_4H$, $-OCF_3$,$-OC_2F_5$, $-OCF_2H$, $-OC_2F_4H$, $-SF_5$, $-C_{1-3}$ alkyl substituted with 0-2 substituents selected from $R^{12}$,

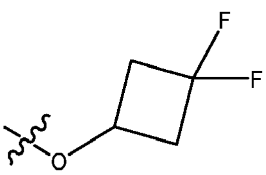, 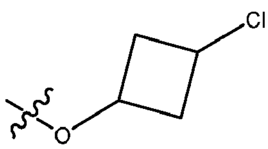, 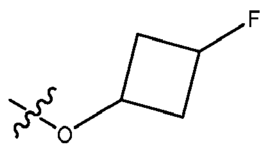, 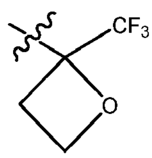,

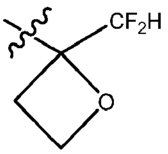, or 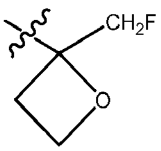 ;

and $R^{12}$ is F, Cl or $-CF_3$.

15. The compound according to aspect 14, wherein:

$R^4$ is, independently at each occurrence, F, Cl, $-OCF_3$, $-SF_5$, $-CH(CH_3)_2$,$-C(CF_3)(CH_3)_2$,

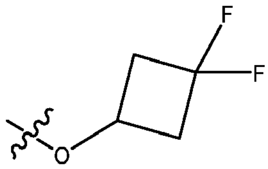, or 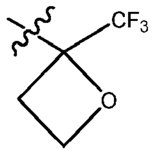 .

16. The compound according to any one of aspects 2 to 15, wherein ring A is

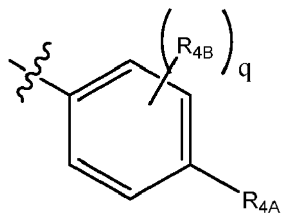;

wherein:

$R_{4A}$ and $R_{4B}$, at each occurrence, are independently an $R_4$ group as defined in any one of aspects 1 and 10 to 15; and
q is 0-3.

17. The compound according to aspect 16, wherein q is 0-2.
18. The compound according to aspect 16, wherein q is 2.
19. The compound according to aspect 16, wherein q is 1.
20. The compound according to aspect 16, wherein q is 0.
21. The compound according to any one of aspects 16 to 19, wherein $R_{4B}$ is independently at each occurrence $R_4$ as defined in any one of aspects 10 to 15.
22. The compound according to aspect 21, wherein $R_{4B}$ is independently at each occurrence F or Cl.
23. The compound according to any one of aspects 16 to 22, wherein $R_{4A}$ is as defined in any one of aspects 10 to 15.
24. The compound according to aspect 1, wherein:
ring A is selected from:

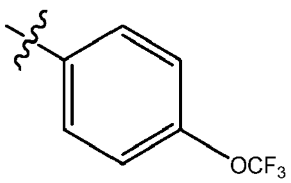 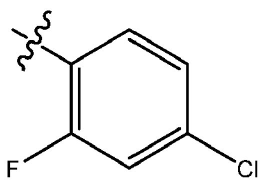 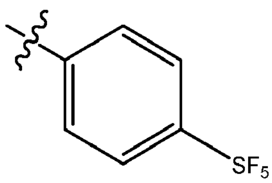 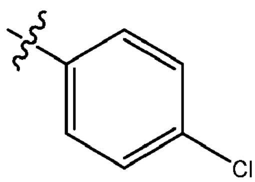

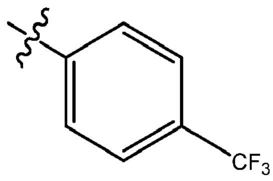 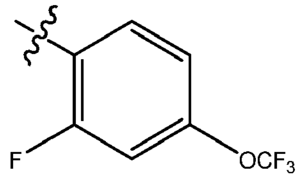 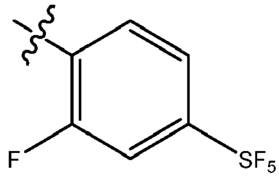

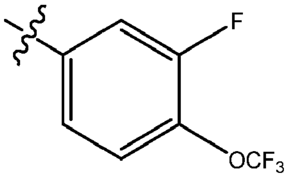 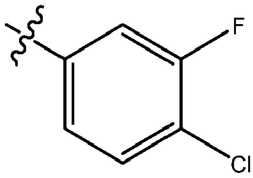 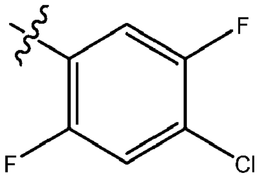

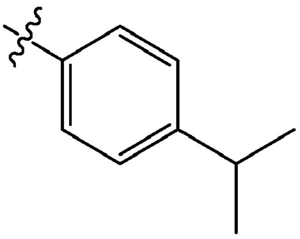 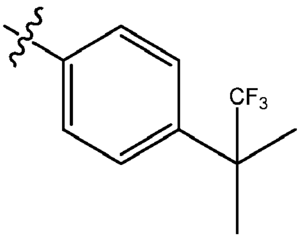 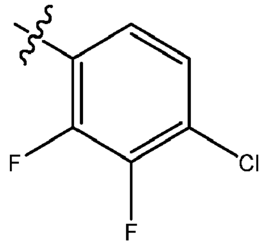

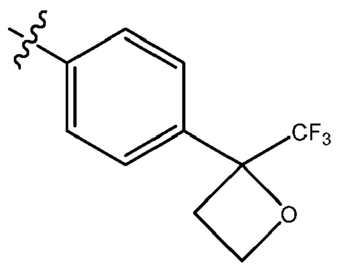 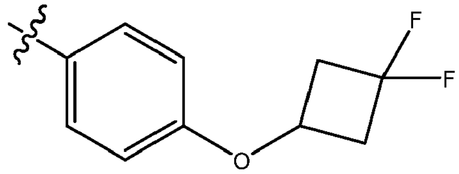

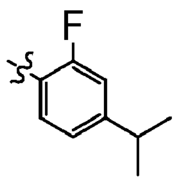 .

25. The compound according to aspect 1, wherein:
ring A is selected from:

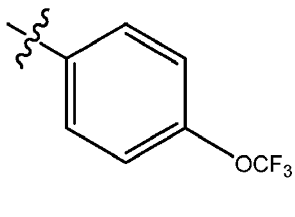 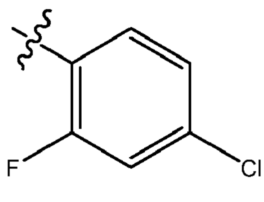 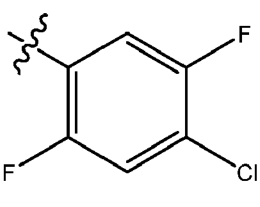

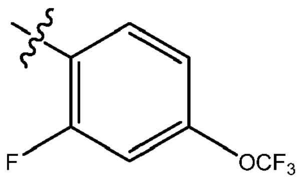 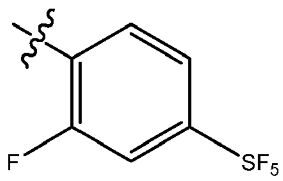 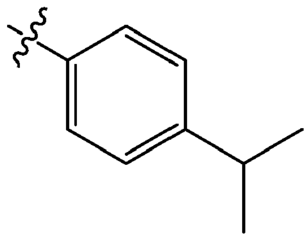

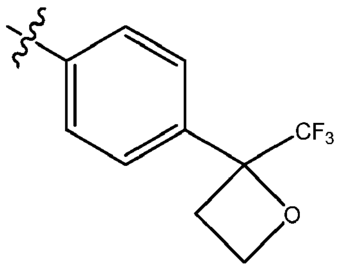 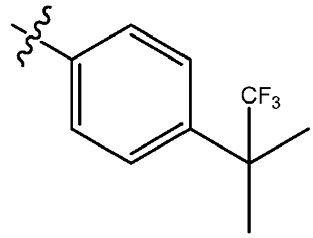

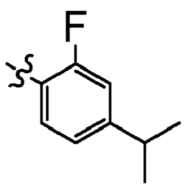.

26. The compound according to aspect 1, wherein:
ring A is $C_{3-10}$ cycloalkyl substituted with 0-4 $R^4$.
27. The compound according to aspect 26, wherein:
ring A is $C_{3-6}$ cycloalkyl substituted with 0-4 $R^4$.
28. The compound according to aspect 27, wherein:
ring A is $C_{3-4}$ cycloalkyl substituted with 0-4 $R^4$.
29. The compound according to any one of aspects 26 to 28, wherein the ring A is substituted with 0-3 $R^4$.
30. The compound according to aspect 29, wherein the ring A is substituted with 0-2 $R^4$.
31. The compound according to any one of aspects 26 to 30, wherein:
ring A is substituted at least with two $R^4$ groups on the same carbon ring atom; and
said two $R^4$ groups on the same carbon ring atom combine to form a $C_{3-6}$ carbocycle substituted with 0-2 substituents selected from $R^{13}$.
32. The compound according to aspect 31, wherein:
two $R^4$ groups on the same carbon ring atom combine to form a $C_{3-4}$ carbocycle substituted with 0-2 substituents selected from $R^{13}$.
33. The compound according to aspect 31 or 32, wherein $R^{13}$ is F.
34. The compound according to aspect 1, wherein ring A is a 5- to 10-membered heterocycle comprising carbon ring atoms and 1-4 heteroatom ring atoms selected from N, $NR^5$, O and $S(O)_p$, wherein said heterocycle is substituted with 0-4 $R^4$.
35. The compound according to aspect 34, wherein:
ring A is substituted with 0-3 $R^4$.
36. The compound according to aspect 35, wherein ring A is unsubstituted.
37. The compound according to aspect 35, wherein ring A is substituted with 1 $R^4$.
38. The compound according to aspect 35, wherein ring A is substituted with 2 $R^4$.
39. The compound according to any one of aspects 34 to 38, wherein the heteroatom ring atoms of the heterocycle that is ring A are selected from N, $NR^5$, O and S.
40. The compound according to any one of aspects 34 to 39, wherein the heterocycle that is ring A contains 1-3 heteroatom rings atoms.
41. The compound according to any one of aspects 34 to 40, wherein the heterocycle that is ring A is selected from pyridinyl, pyrimidinyl, furanyl, isoxazolyl, thiazolyl, thiadiazolyl, benzothiazolyl, thiazolopyridinyl, indolyl, benzodioxolyl, benzimidazolyl, and pyridoimidazolyl.
42. The compound according to aspect 41, wherein the heterocycle that is ring A is selected from pyridinyl, pyrimidinyl,

thiadiazolyl, thiazolopyridinyl, benzodioxolyl, and benzimidazolyl.

43. The compound according to aspect 42, wherein the heterocycle that is ring A is thiazolopyridinyl.

44. The compound according to any one of aspects 34 to 43, wherein:

$R^4$ is, independently at each occurrence, F, Cl, $-C_{rA}F_{(2rA+1)}$, $-C_{rA}F_{2rA}H$, $C_{1-6}$ alkyl substituted with 0-3 substituents selected from $R^{12}$, $-C_{3-6}$ cycloalkyl substituted with 0-3 substituents selected from $R^{12}$, $-O-C_{1-6}$ alkyl substituted with 0-3 substituents selected from $R^{12}$, $-O-C_{3-6}$ cycloalkyl substituted with 0-3 substituents selected from $R^{12}$, or 3- to 6- membered heterocyclic ring comprising: carbon ring atoms and 1-3 heteroatom ring atoms selected from N, $NR^5$, O and $S(O)_p$, wherein said heterocyclic ring is substituted with 0-3 substituents selected from F, Cl, $-C_{rAA}F_{(2rAA+1)}$, $-C_{rAA}F_{2rAA}H$, or $C_{1-6}$ alkyl substituted with 0-3 substituents selected from $R^{12}$.

45. The compound according to aspect 44, wherein:

$R^4$ is, independently at each occurrence, F, Cl, $-C_2F_5$, $-CF_2H$, $-C_2F_4H$, $-C_{1-3}$ alkyl substituted with 0-2 substituents selected from $R^{12}$, $-OCH_3$, $-OC_2H_5$,

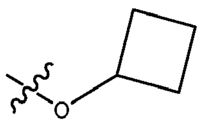,
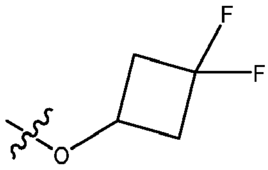,
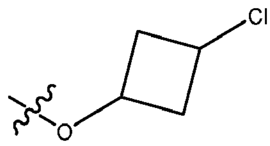,
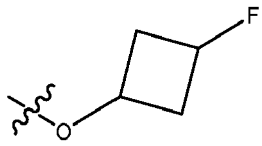

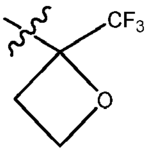,
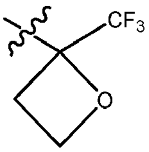,
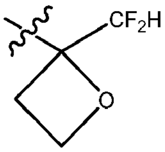, or
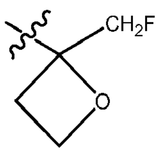;

and $R^{12}$ is F, Cl or $-CF_3$.

46. The compound according to aspect 45, wherein:

ring A is:

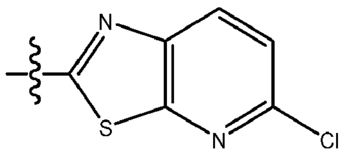.

47. The compound according to any one of aspects 1 to 46, wherein:

ring B is selected from:

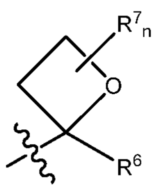
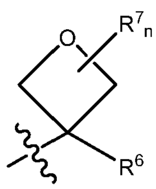
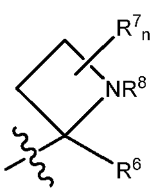
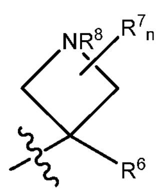

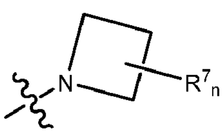

48. The compound according to aspect 47, wherein:

ring B is selected from:

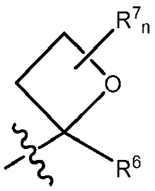 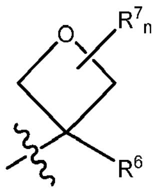 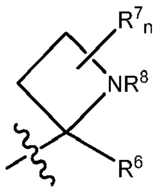 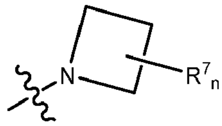.

49. The compound according to aspect 48, wherein ring B is

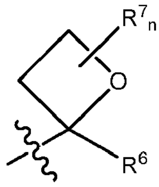.

50. The compound according to aspect 48, wherein ring B is

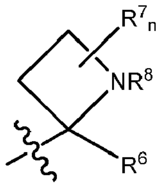.

51. The compound according to any one of aspects 1 to 50, wherein:
$R^6$ is H, $-C_{rC}F_{(2rC+1)}$, $-C_{rC}F_{2rC}H$, or $C_{1-2}$ alkyl, wherein rC is 1 or 2.

52. The compound according to aspect 51, wherein
$R^6$ is H, $-CF_2H$, or $-CH_3$.

53. The compound according to aspect 52, wherein:
$R^6$ is H.

54. The compound according to aspect 52, wherein:
$R^6$ is $-CF_2H$.

55. The compound according to aspect 52, wherein:
$R^6$ is $-CF_3$.

56. The compound according to any one of aspects 1 to 55, wherein $R^7$ is: $-C_{rD}F_{(2rD+1)}$, $-C_{rD}F_{2rD}H$, or $C_{1-2}$ alkyl, wherein rD is 1 or 2; or two $R^7$ groups on the same carbon ring atom form =O.

57. The compound according to aspect 56, wherein $R^7$ is: $-CF_2H$, or $-CH_3$, or two $R^7$ groups on the same carbon ring atom form =O.

58. The compound according to any one of aspects 1 to 57, wherein n is 0.

59. The compound according to any one of aspects 1 to 57, wherein n is 1.

60. The compound according to any one of aspects 1 to 57, wherein n is 2.

61. The compound according to any one of aspects 1 to 60, wherein $R^8$ is $C_{1-6}$ alkyl substituted with 0-3 substituents selected from F and Cl, or
$-(CR^{9B}_2)_{tB}-C_{3-10}$ carbocycle substituted with 0-3 substituents selected from F, Cl or $C_{1-6}$ alkyl.

62. The compound according to aspect 61, wherein $R^8$ is $C_{1-6}$ alkyl substituted with 0-3 F, $-CH_2$-phenyl substituted with 0-3 substituents selected from F, Cl or $C_{1-6}$ alkyl, or $-CH_2-C_{3-6}$ cycloalkyl substituted with 0-3 substituents selected from F, Cl or $C_{1-6}$ alkyl.

63. The compound according to aspect 62, wherein $R^8$ is $C_{1-6}$ alkyl substituted with 0-2 F, benzyl or -methylcyclopropyl, especially $C_{1-6}$ alkyl or benzyl.

64. The compound according to any one of aspects 1 to 49 and 51 to 63, wherein:
ring B is:

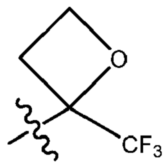

65. The compound according to any one of aspects 1 to 48 and 50 to 63, wherein:
ring B is:

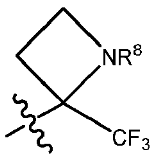 or 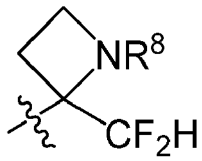.

66. The compound according to aspect 65, wherein:
ring B is selected from:

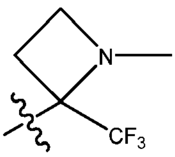 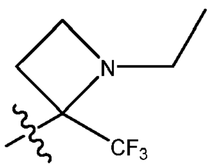 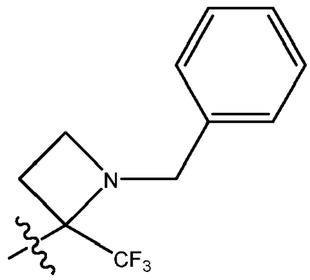 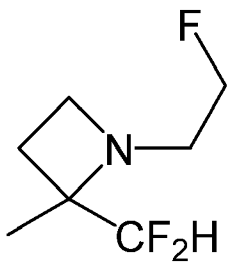.

67. The compound according to aspect 66, wherein:
ring B is:

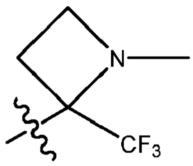.

68. The compound according to any one of aspects 1 to 67, wherein:
W is O.
69. The compound according to any one of aspects 1 to 68, wherein:
X is -CO-NH-.
70. The compound according to any one of aspects 1 to 69, wherein:
Y is N.
71. The compound according to any one of aspects 1 to 70, wherein:
Z is CH.
72. The compound according to any one of aspects 1 to 71, wherein rA is, independently on each substituent in which it occurs, 1-3.
73. The compound according to aspect 72, wherein rA is, independently on each substituent in which it occurs, 1 or 2.
74. The compound according to any one of aspects 1 to 73, wherein rAA is, independently on each substituent in which it occurs, 1-3.
75. The compound according to aspect 74, wherein rAA is, independently on each substituent in which it occurs, 1 or 2.
76. The compound according to any one of aspects 1 to 75, wherein rB is, independently on each substituent in which it occurs, 1-3.
77. The compound according to aspect 76, wherein rB is, independently on each substituent in which it occurs, 1 or 2.

78. The compound according to any one of aspects 1 to 77, wherein rC is, independently on each substituent in which it occurs, 1-3.
79. The compound according to aspect 78, wherein rC is, independently on each substituent in which it occurs, 1 or 2.
80. The compound according to any one of aspects 1 to 79, wherein rD is, independently on each substituent in which it occurs, 1-3.
81. The compound according to aspect 80, wherein rD is, independently on each substituent in which it occurs, 1 or 2.
82. The compound according to any one of aspects 1 to 81, wherein rE is, independently on each substituent in which it occurs, 1-3.
83. The compound according to aspect 82, wherein rE is, independently on each substituent in which it occurs, 1 or 2.
84. The compound according to any one of aspects 1 to 83, wherein rF is, independently on each substituent in which it occurs, 1-3.
85. The compound according to aspect 84, wherein rF is, independently on each substituent in which it occurs, 1 or 2.
86. The compound according to any one of aspects 1 to 85, wherein rG is, independently on each substituent in which it occurs, 1-3.
87. The compound according to aspect 86, wherein rG is, independently on each substituent in which it occurs, 1 or 2.
88. The compound according to any one of aspects 1 to 87, wherein rH is, independently on each substituent in which it occurs, 1-3.
89. The compound according to aspect 88, wherein rH is, independently on each substituent in which it occurs, 1 or 2.
90. The compound according to any one of aspects 1 to 89, wherein tA is 0.
91. The compound according to any one of aspects 1 to 89, wherein tA is 1.
92. The compound according to any one of aspects 1 to 91, wherein tB is 0.
93. The compound according to any one of aspects 1 to 91, wherein tB is 1.
94. The compound according to any one of aspects 1 to 93, wherein neither $R^{1a}$ nor $R^{1b}$ is -OH.
95. The compound according to any one of aspects 1 to 94, wherein the formula (I) or formula (II) is formula (I).
96. The compound according to aspect 95, wherein:
$R^{1a}$, $R^{1b}$, $R^{1c}$ and $R^{1d}$ are independently selected from H, F, Cl, $-CH_3$, and $-C_2H_5$.
97. The compound according to aspect 96, wherein:
$R^{1a}$, $R^{1b}$, $R^{1c}$ and $R^{1d}$ are independently selected from H, F and $-CH_3$.
98. The compound according to aspect 97, wherein:
$R^{1a}$, $R^{1b}$, $R^{1c}$ and $R^{1d}$ are independently selected from H or F.
99. The compound according to any one of aspects 95 to 98, wherein:
$R^{1d}$ is H.
100. The compound according to any one of aspects 95 to 99, wherein:
$R^{1a}$ is H.
101. The compound according to any one of aspects 95 to 100, wherein:
$R^{1c}$ is H.
102. The compound according to aspect 101, wherein:
$R^{1a}$, $R^{1b}$, $R^{1c}$ and $R^{1d}$ are H.
103. The compound according to any one of aspects 1 to 94, wherein the formula (I) or formula (II) is formula (II).
104. The compound according to aspect 103, wherein:
$R^{1a}$, $R^{1b}$, $R^{1c}$ and $R^{1e}$ are independently selected from H, F, Cl, $-CH_3$, and $-C_2H_5$.
105. The compound according to aspect 104, wherein:
$R^{1a}$, $R^{1b}$, $R^{1c}$ and $R^{1e}$ are independently selected from H, F and $-CH_3$.
106. The compound according to aspect 105, wherein:
$R^{1a}$, $R^{1b}$, $R^{1c}$ and $R^{1e}$ are independently selected from H or $-CH_3$.
107. The compound according to aspect 106, wherein:
$R^{1a}$, $R^{1b}$ and $R^{1c}$ are H.
108. The compound according to any one of aspects 103 to 107, wherein:
$R^{1e}$ is $-CH_3$.
109. The compound according to any one of aspects 1 to 108, wherein:
$R^2$ is H, F, Cl, or $-C_{1-6}$ alkyl.
110. The compound according to aspect 109, wherein:
$R^2$ is H, Cl, $-CH_3$, or $-C_2H_5$.
111. The compound according to aspect 110, wherein:
$R^2$ is H, Cl, or $-CH_3$.
112. The compound according to aspect 111, wherein:
$R^2$ is $-CH_3$.
113. The compound according to aspect 111, wherein:

$R^2$ is H.

114. The compound according to any one of aspects 1 to 113, wherein:

$R^3$ is H.

115. The compound according to aspect 1, wherein the formula (I) or formula (II) is formula (I):

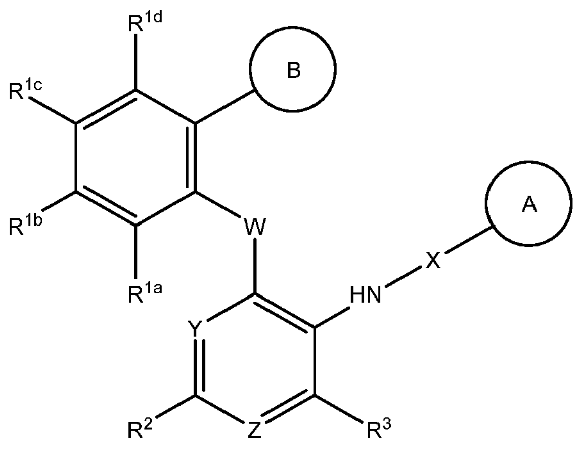

(I)

wherein:

ring A is

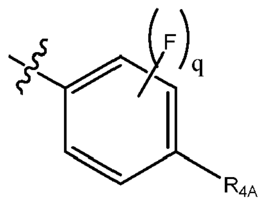;

ring B is selected from:

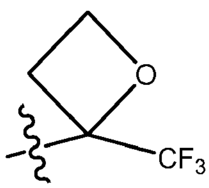

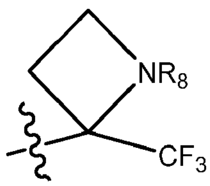

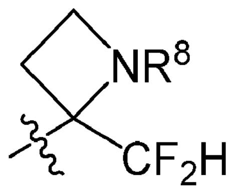;

W is O;

X is -CO-NH-;

Y is N;

Z is CH;

$R^{1a}$ and $R^{1d}$ are H;

$R^{1b}$ and $R^{1c}$ are independently selected from H or F;

$R^2$ is H, Cl or $-CH_3$;

$R^3$ is H;

$R^{4A}$ is F, Cl, $-OCF_3$, $-SF_5$, $-C_{1-3}$ alkyl substituted with 0-2 $CF_3$ substituents,

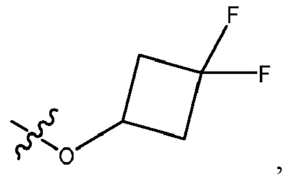

, or

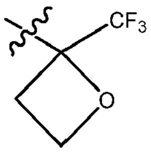

;

q is 0, 1 or 2; and
$R^8$ is $-CH_3$, $-C_2H_5$, $-CH_2CH_2F$, $-CH_2C(CH_3)_3$, benzyl or -methylcyclopropyl.

116. The compound according to aspect 1, wherein the formula (I) or formula (II) is formula (I):

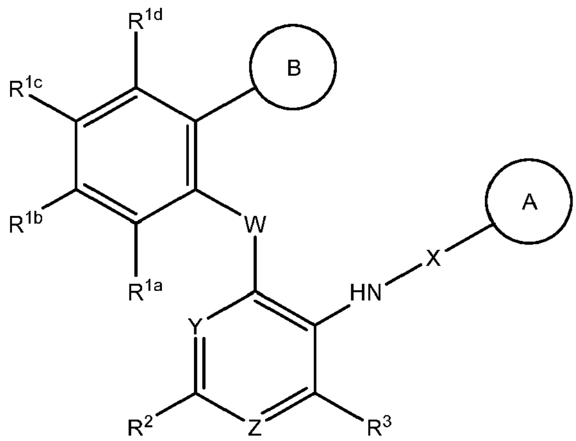

(I)

wherein:

ring A is:

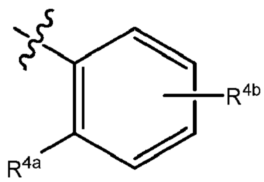

ring B is selected from:

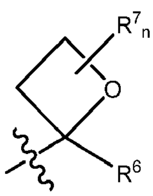

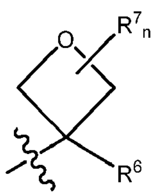

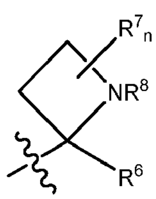

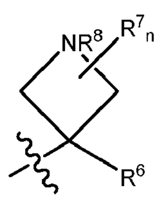

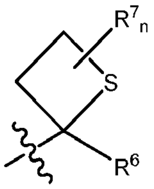

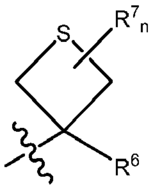

;

W is O;
X is -CO-NH-;
Y is N;
Z is CH;
$R^{1a}$ and $R^{1d}$ are H;
$R^{1b}$ and $R^{1c}$ are, independently at each occurrence, H, F, Cl, Br, or $-CH_3$;
$R^2$ is H, Cl, $-CH_3$;
$R^3$ is H;
$R^{4a}$ is H, F, Cl, Br, or -CN;
$R^{4b}$ is F, Cl, Br, $-SF_5$, $-C_{1-3}$ alkyl substituted with 0-3 substituents selected from F or Cl, $-O-C_{1-6}$ alkyl substituted with 0-3 substituents selected from F or Cl;
$R^6$ is H, $-C_rF_{(2r+1)}$, $-C_rF_{2r}H$, or $C_{1-3}$ alkyl; and
$R^7$ is $-C_rF_{(2r+1)}$, $-C_rF_{2r}H$, or $C_{1-3}$ alkyl;
$R^8$ is H, $-C_rF_{(2r+1)}$, $-C_rF_{2r}H$, $-C_rH_{2r}F$, $C_{1-6}$ alkyl, $-CH_2$-phenyl, or $-CH_2-C_{3-6}$ cycloalkyl;
n is 0, 1 or 2;
r is, independently on each substituent in which it occurs, 1-4.

117. The compound according to aspect 116 wherein:
ring B is selected from:

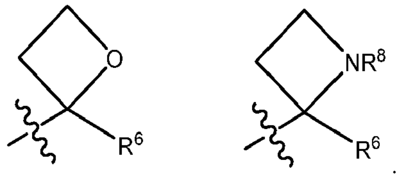

118. The compound according to any one of aspects 1 to 117, wherein the formula (I) is not:

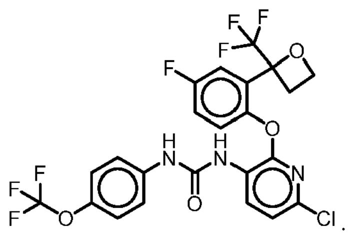

119. The compound according to aspect 118, wherein, when Y is N and Z is CH, then: $R^2$ is not F, Cl or Br.
120. The compound according to aspect 118 or 119, wherein, when Y is N and Z is CH, then:
$R^{1c}$ is not F, Cl or Br.
121. The compound according to any one of aspects 118 to 120, wherein, when Y is N and Z is CH, then;
$R^{1a}$, $R^{1a}$, $R^{1b}$, $R^{1c}$, $R^{1d}$, $R^{1e}$ are each not F, Cl or Br.
122. The compound according to any one of aspects 118 to 121, wherein, when Y is N and Z is CH, then:
either none or only one of $R^{1a}$, $R^{1a}$, $R^{1b}$, $R^{1c}$, $R^{1d}$, $R^{1e}$, $R^2$ and $R^3$ is F, Cl or Br.
123. The compound according to aspect 118, wherein:

- $R^2$ is not F, Cl or Br; or
- $R^{1c}$ is not F, Cl or Br; or
- $R^{1a}$, $R^{1a}$, $R^{1b}$, $R^{1c}$, $R^{1d}$, $R^{1e}$ are each not F, Cl or Br; or
- either none or only one of $R^{1a}$, $R^{1a}$, $R^{1b}$, $R^{1c}$, $R^{1d}$, $R^{1e}$, $R^2$ and $R^3$ is F, Cl or Br.

124. The compound according to aspect 1, wherein the formula (I) or formula (II) is selected from:

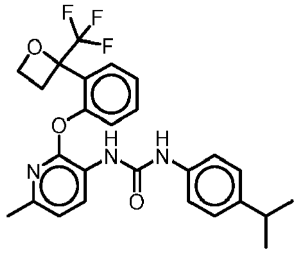,
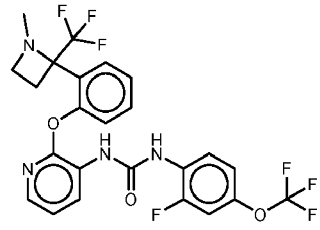,
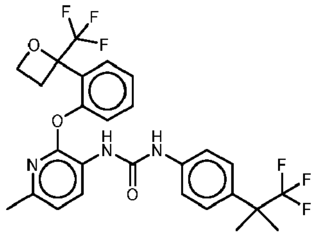

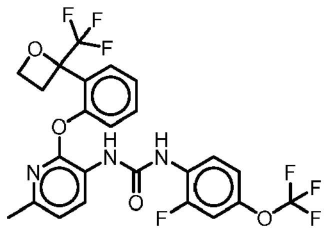,
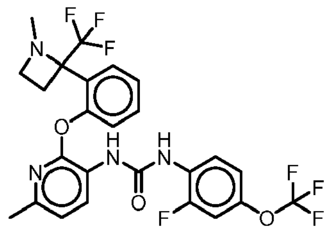,
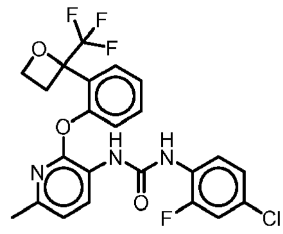,

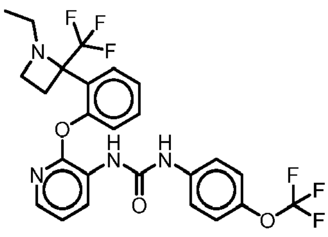,
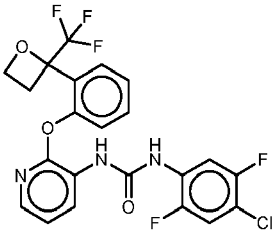,
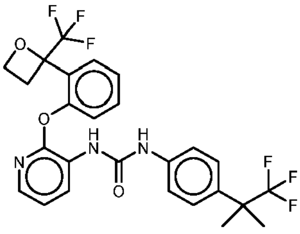,

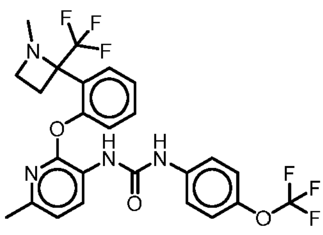,
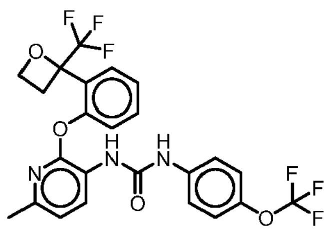,
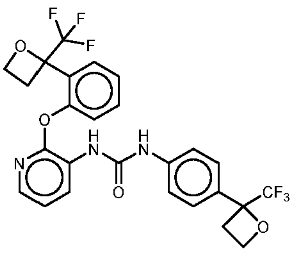,

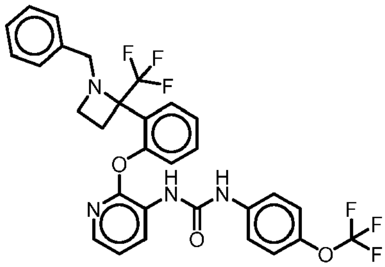,
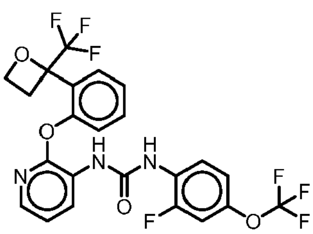,
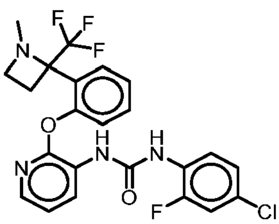,

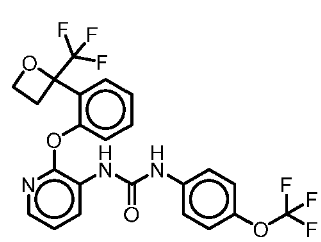,
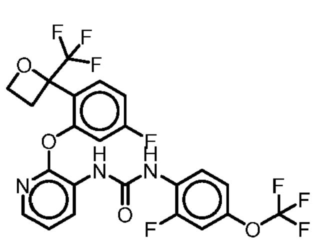,
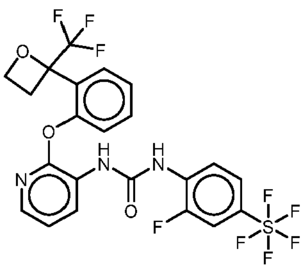

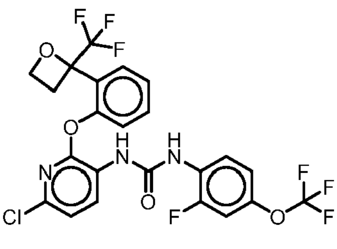, 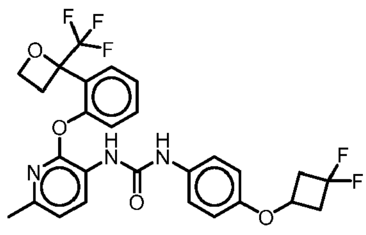, 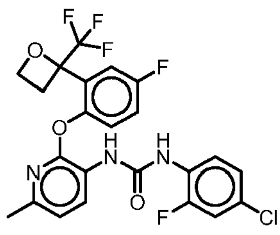,

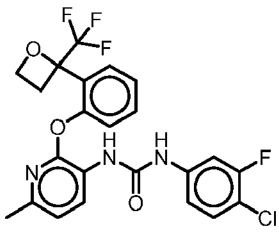, 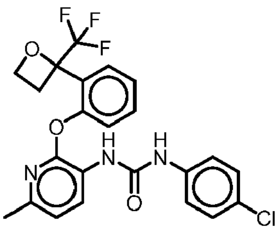, 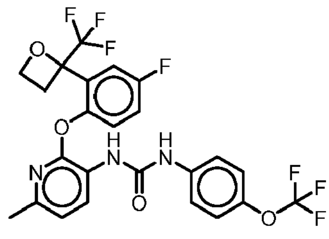,

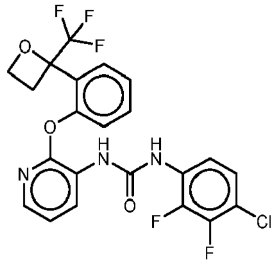, 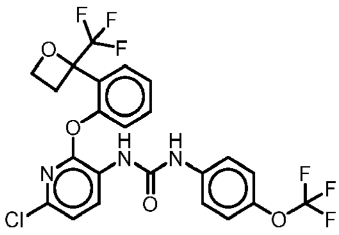, 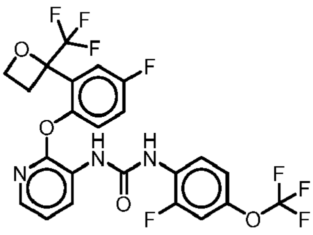,

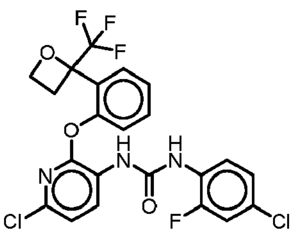, 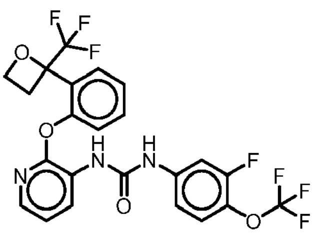, 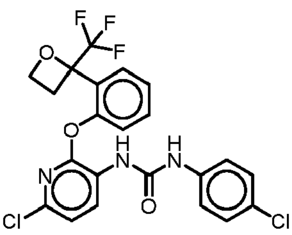,

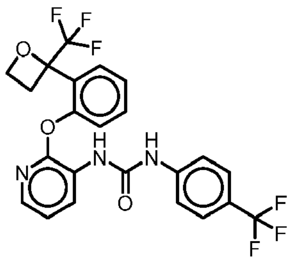, 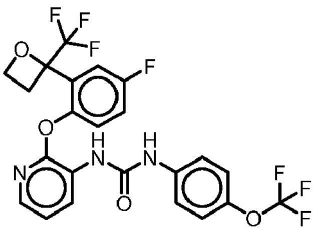, 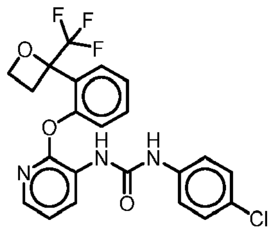,

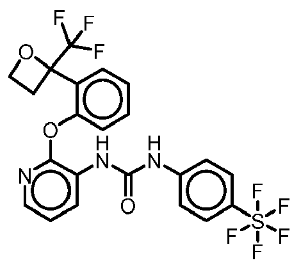, 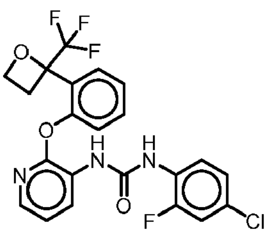, 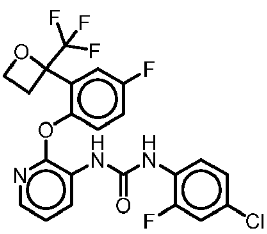,

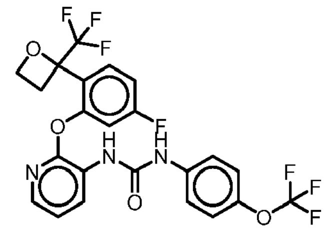, 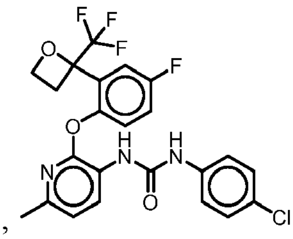, 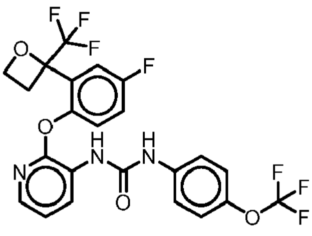,

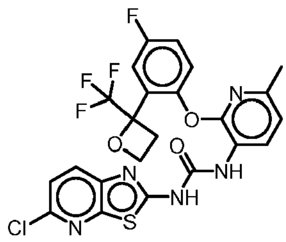, 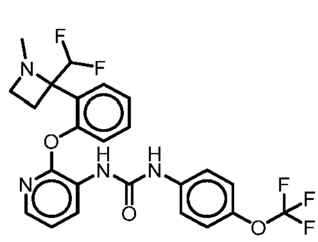, 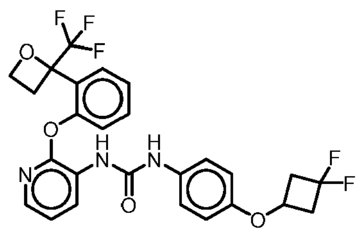,

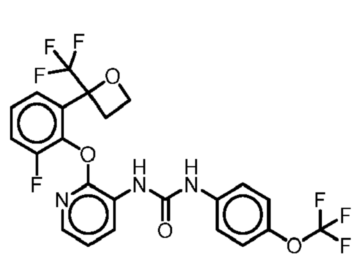, 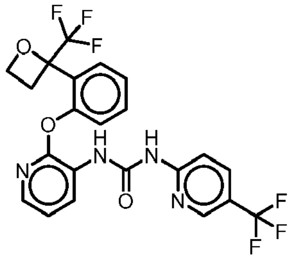, 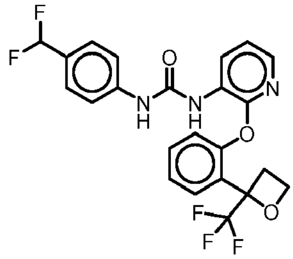,

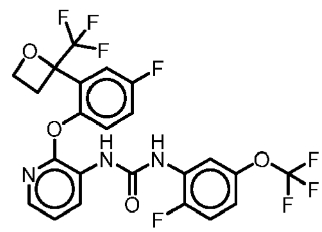, 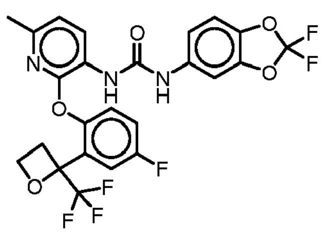, 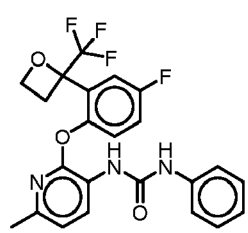,

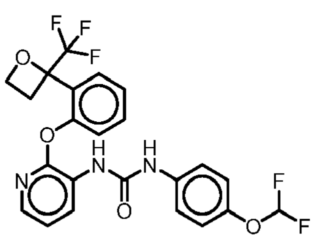, 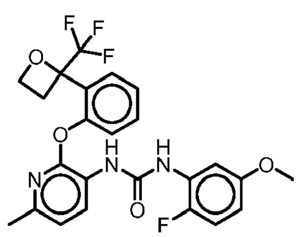, 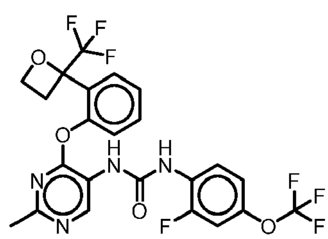,

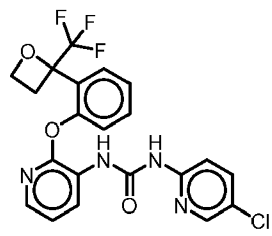, 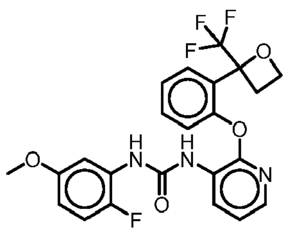, 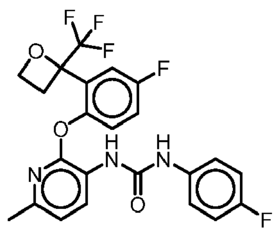,

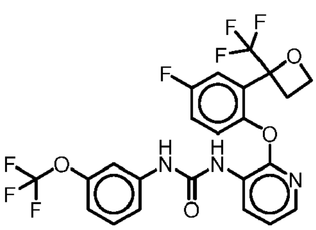, 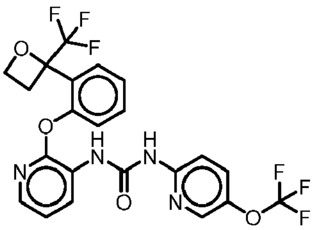, 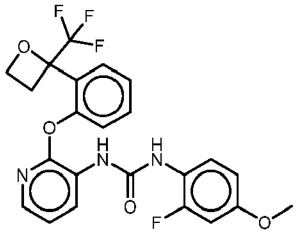,

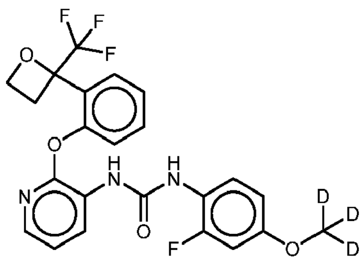, 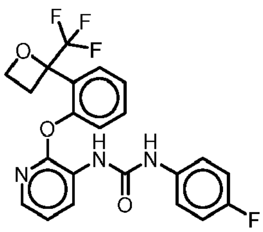, 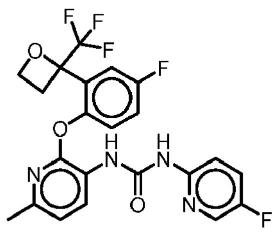,

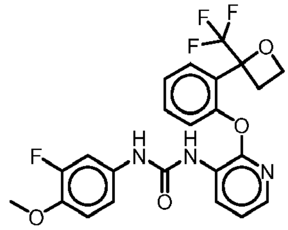, 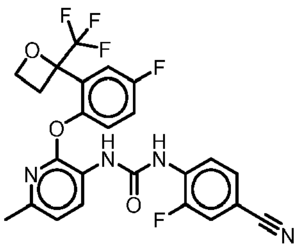, 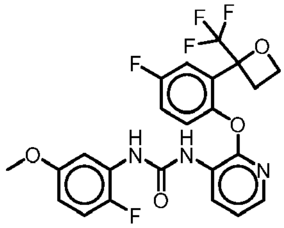,

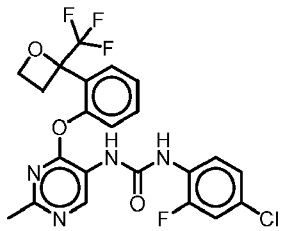, 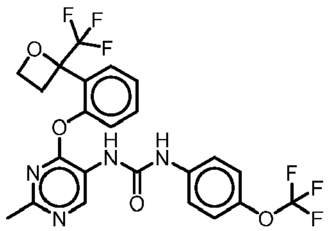, 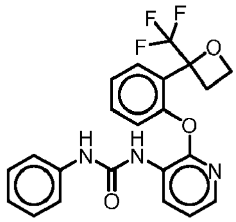,

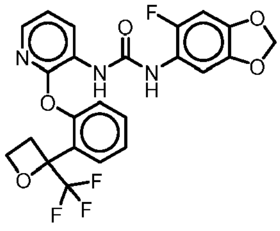, 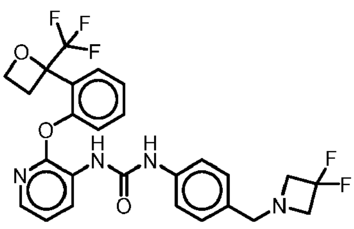, 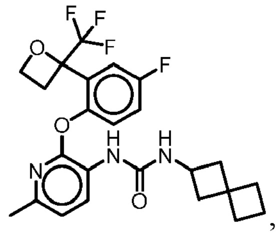,

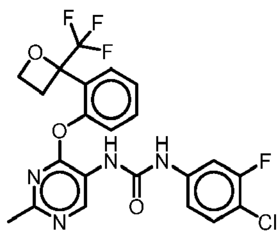, 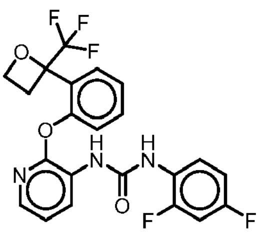, 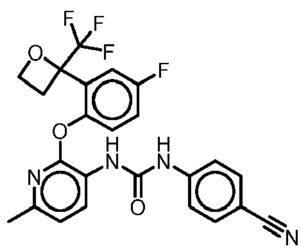,

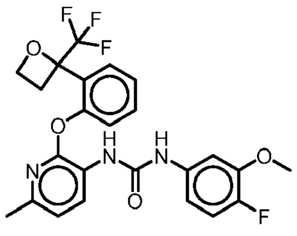, 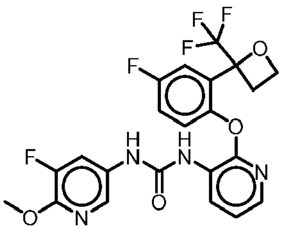, 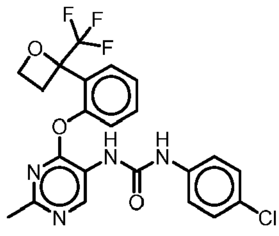,

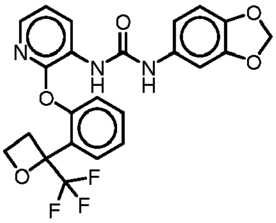, 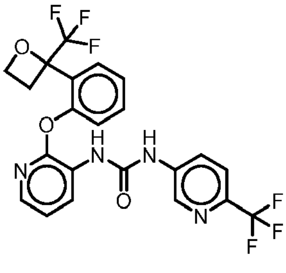, 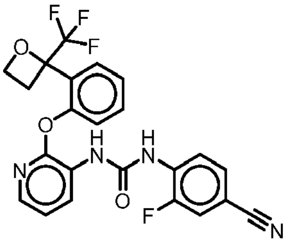,

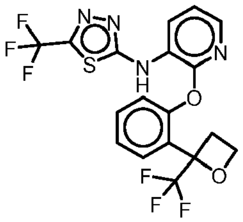, 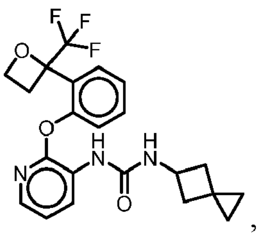, 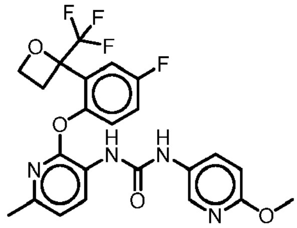, 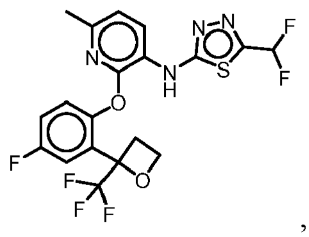,

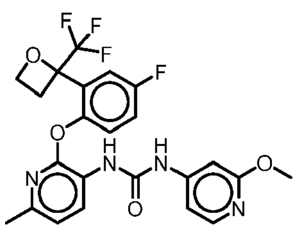, 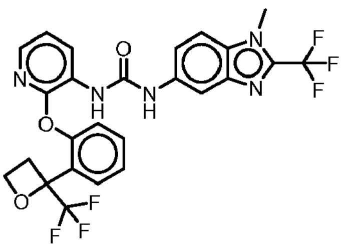, 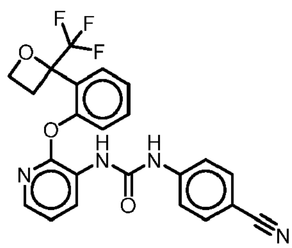,

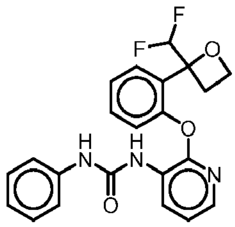, 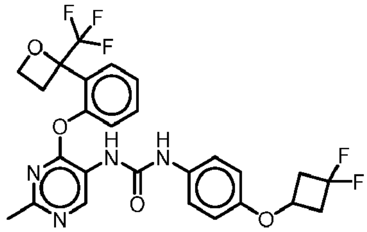, 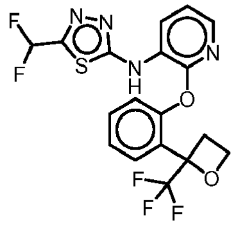,

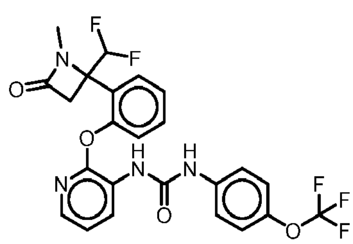, 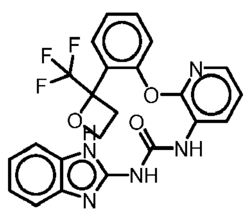, 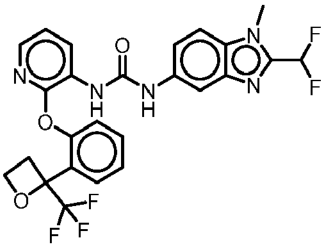,

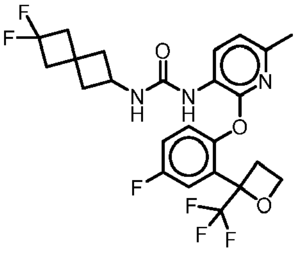, 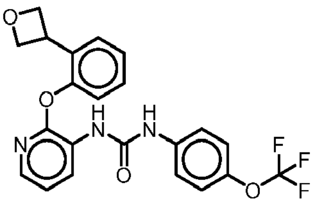, 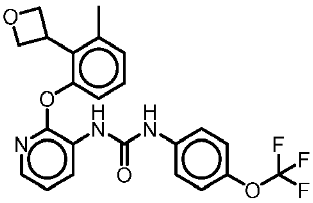,

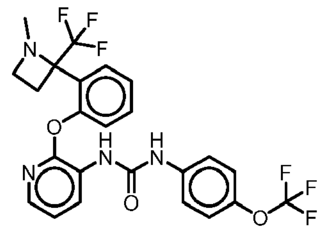, 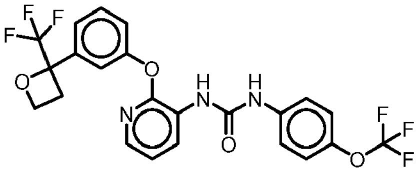,

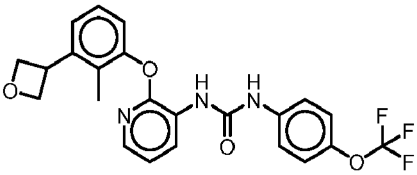, 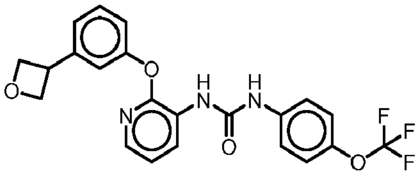,

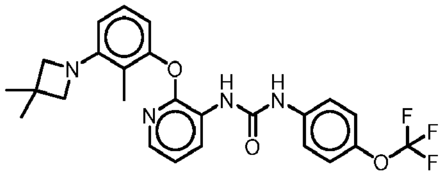, 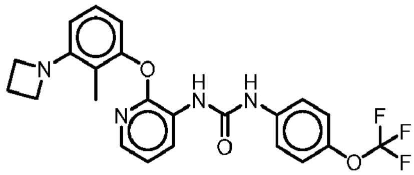,

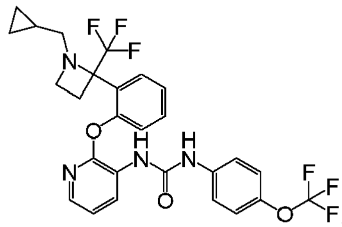, 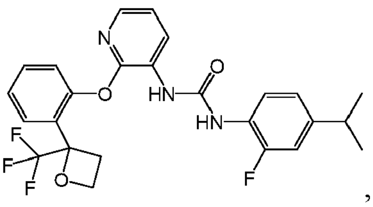, 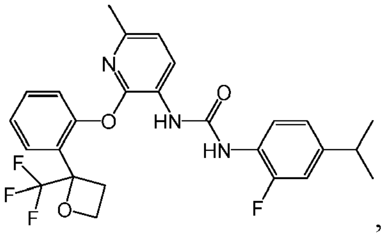,

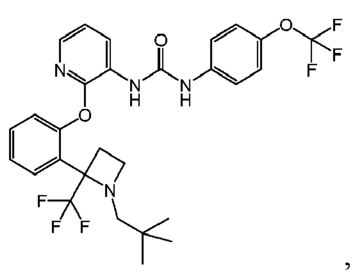, 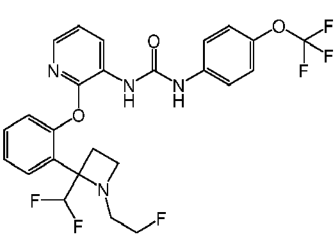, 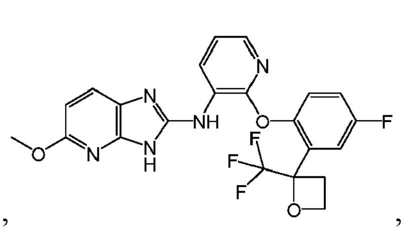,

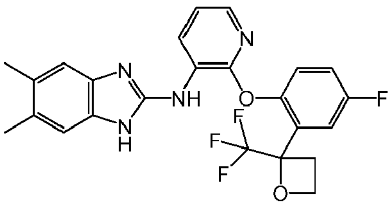,
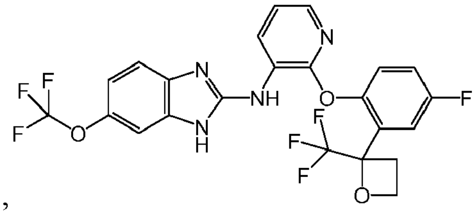.

125. The compound according to aspect 1, wherein the formula (I) or formula (II) is selected from:

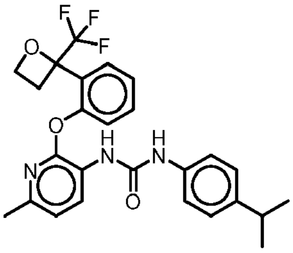,
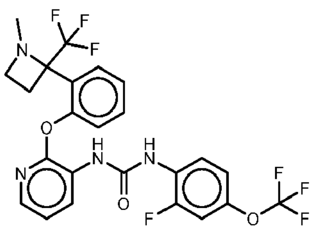,
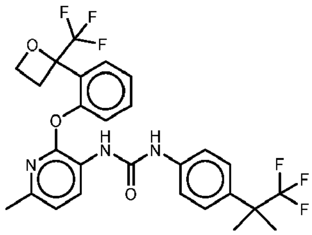

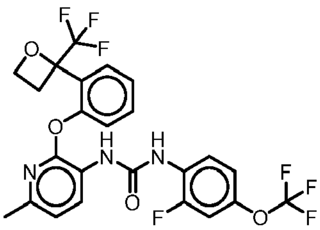,
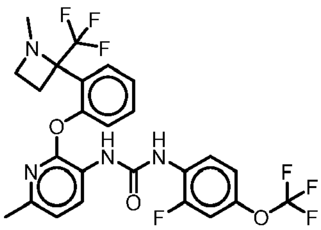,
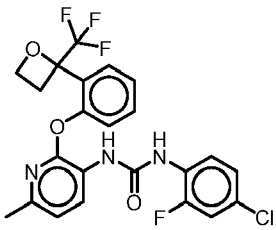,

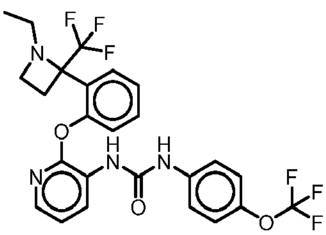,
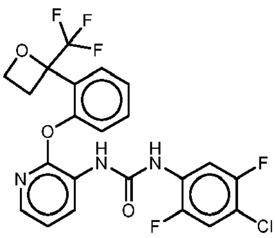,
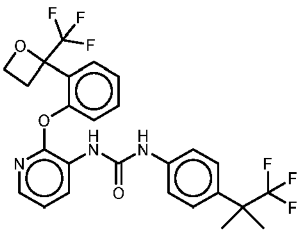,

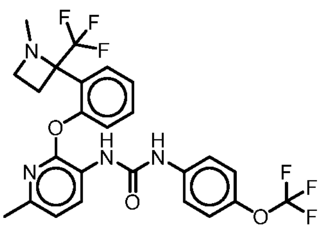,
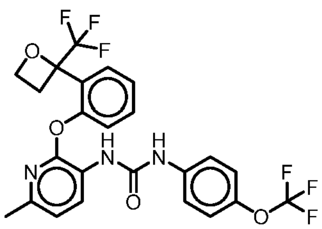,
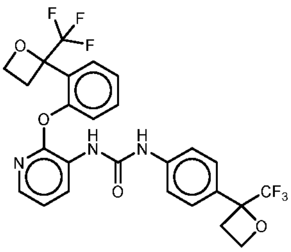,

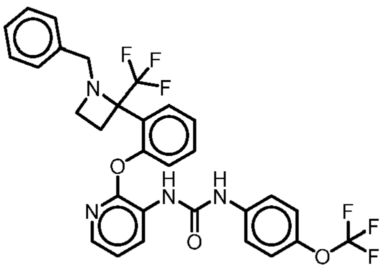,
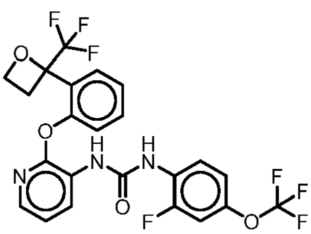,
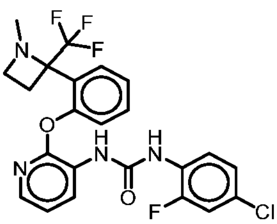,

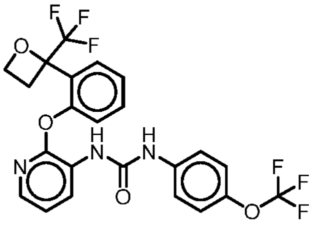,

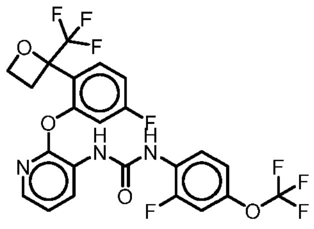,

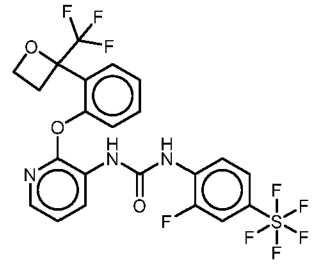

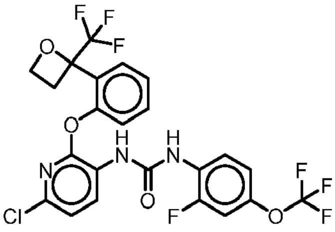,

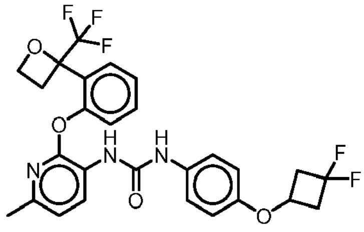,

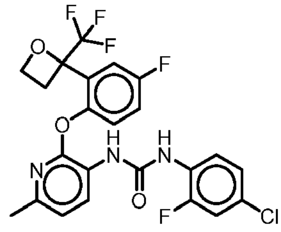,

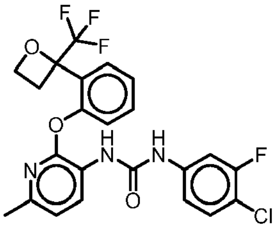,

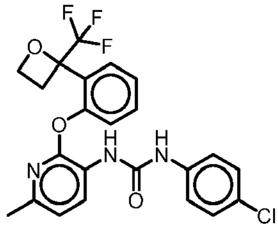,

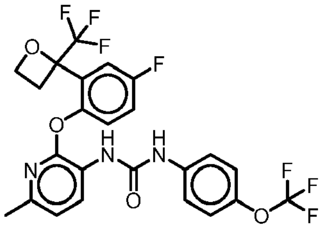,

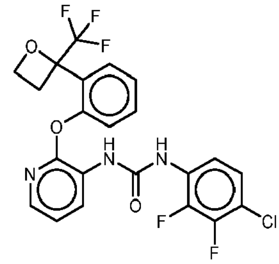,

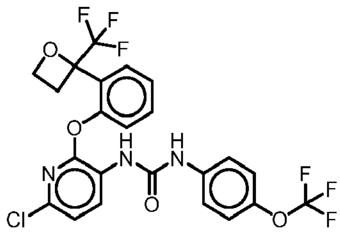,

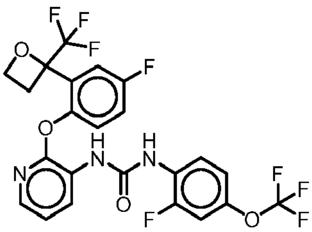,

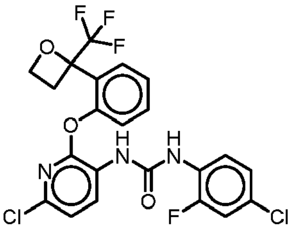,

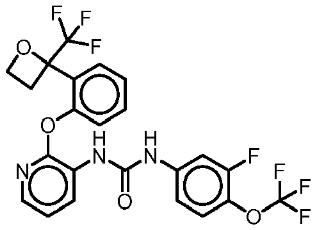,

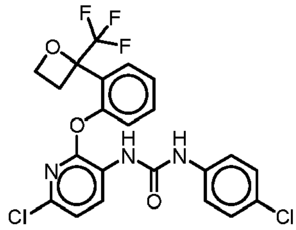,

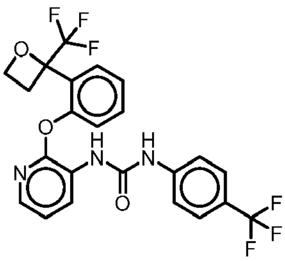,

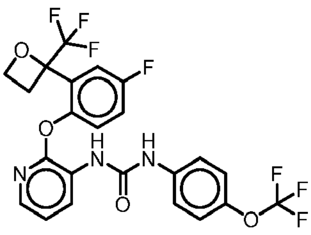,

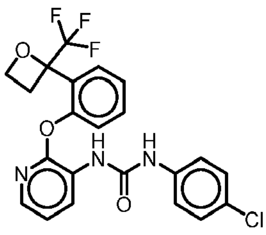,

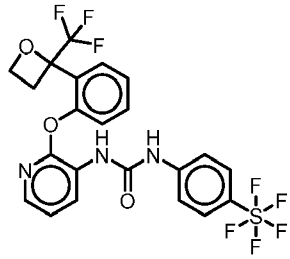,

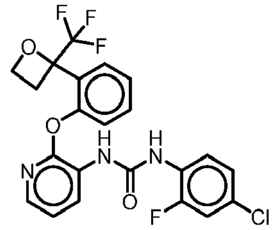,

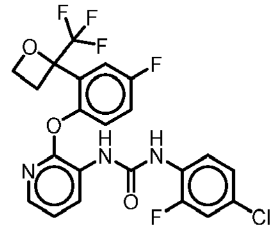,

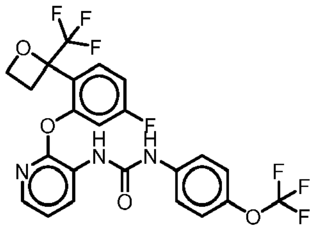,

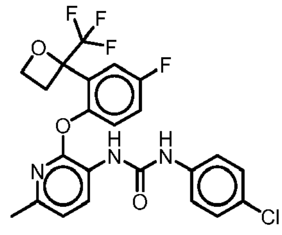,

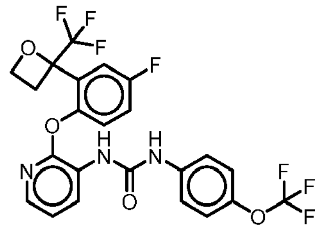,

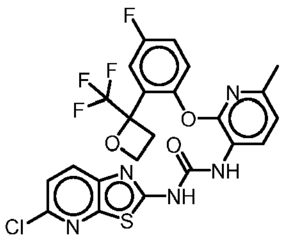,

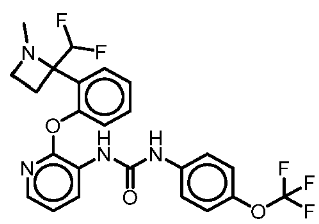,

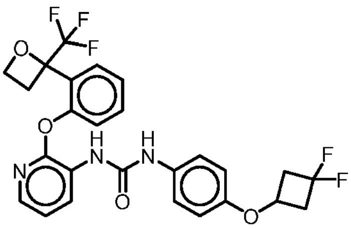,

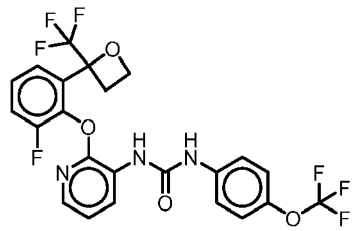,

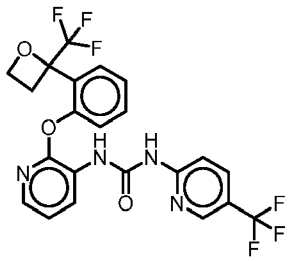,

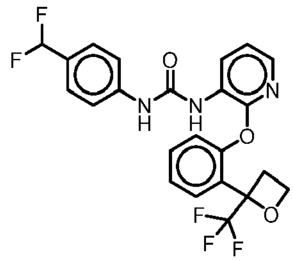,

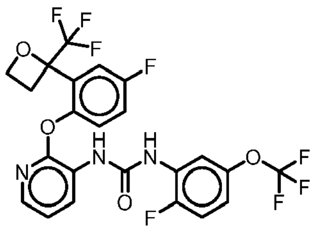,

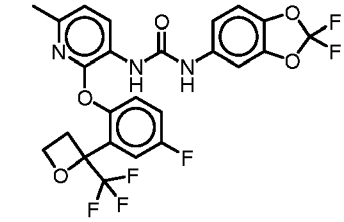,

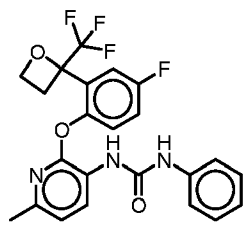,

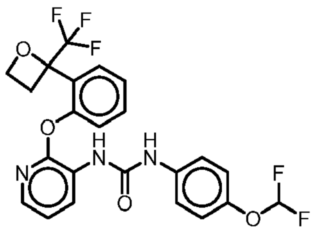,

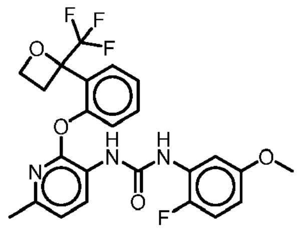,

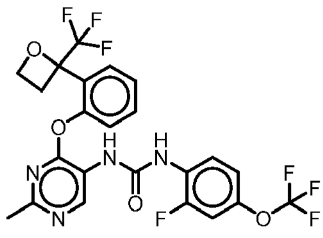,

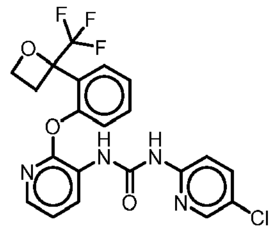,
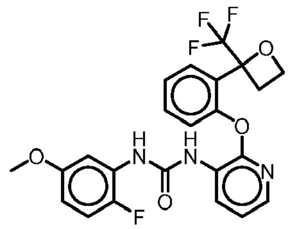,
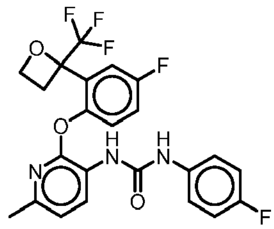,

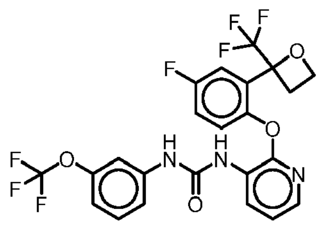,
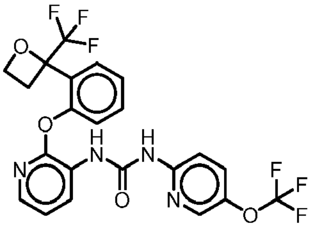,
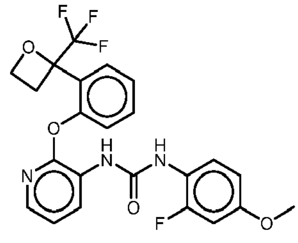,

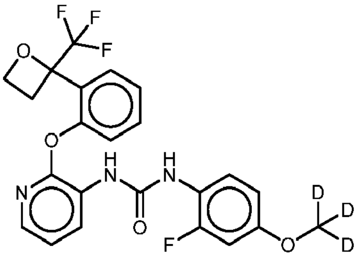,
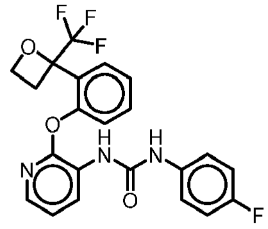,
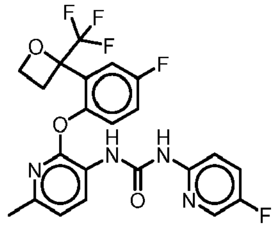,

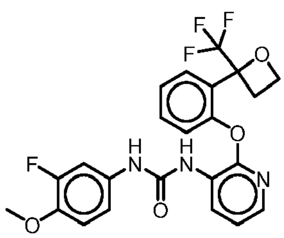,
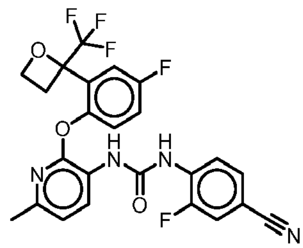,
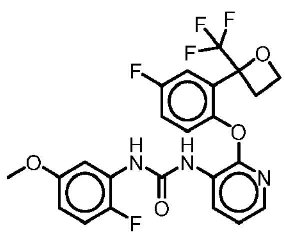,

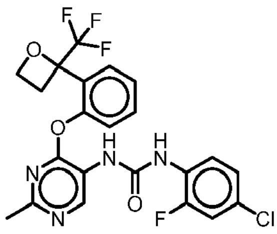,
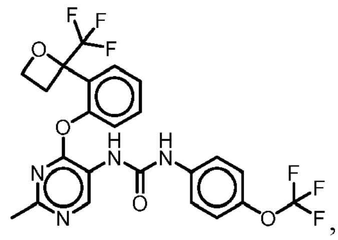,
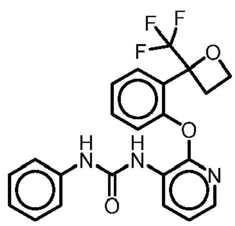,

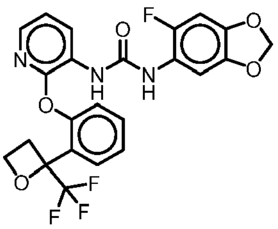,
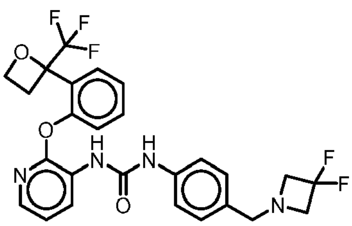,
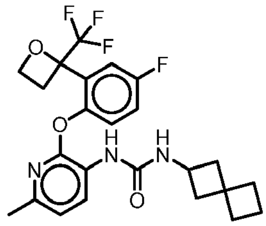,

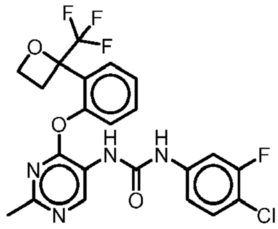,
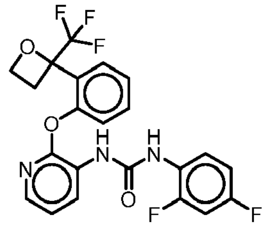,
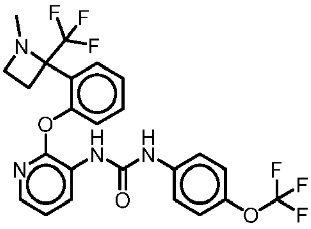,

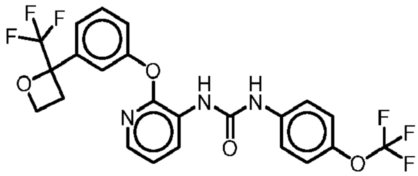,
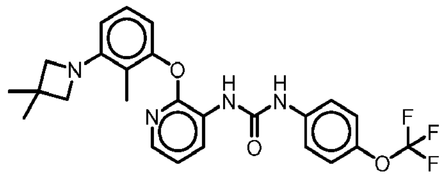,

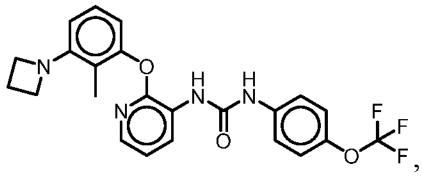,
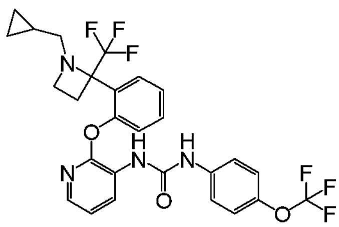,
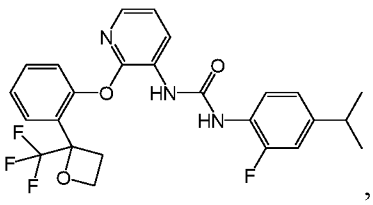,

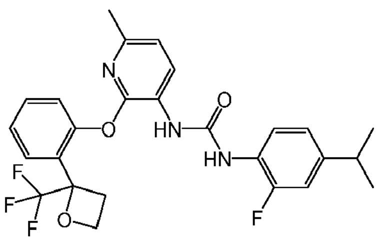,
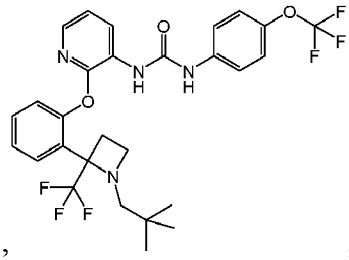,
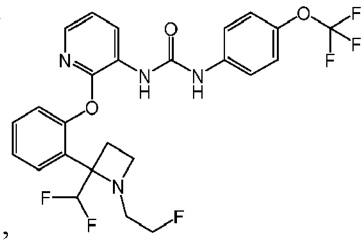,

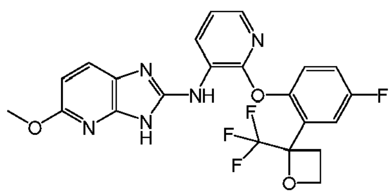.

126. The compound according to aspect 1, wherein the formula (I) or formula (II) is selected from:

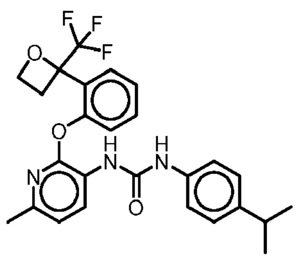,
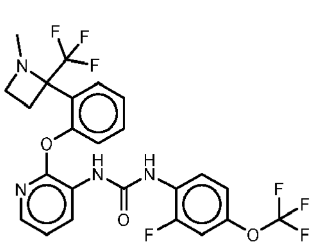,
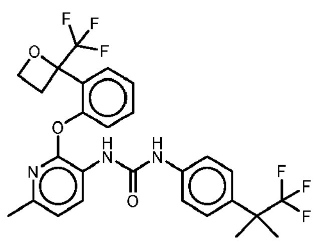

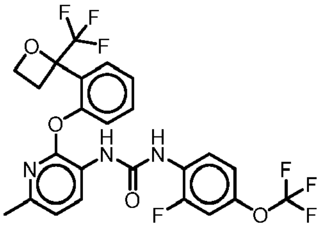,
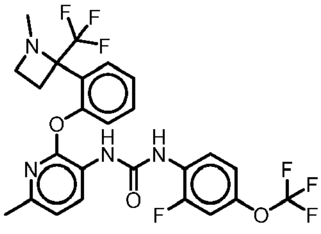,
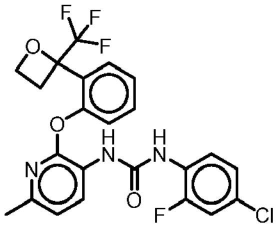,

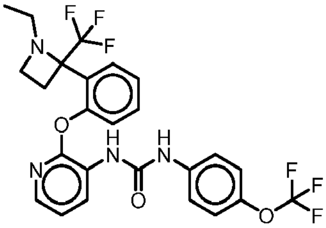,
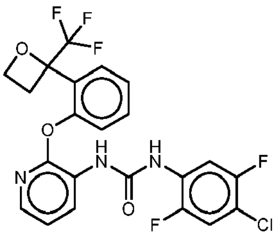,
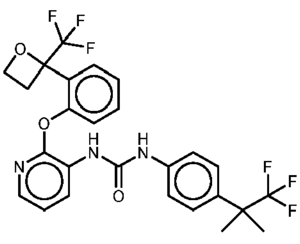,

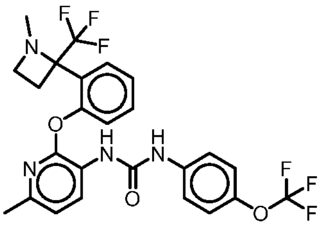,
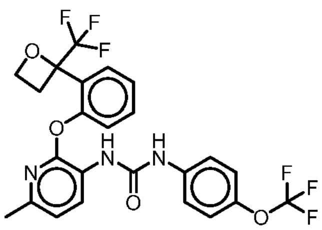,
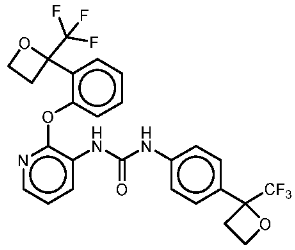,

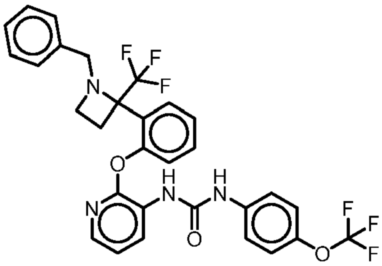,
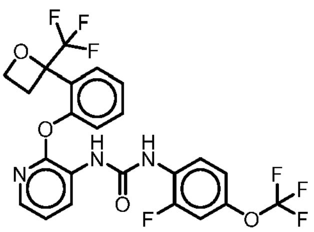,
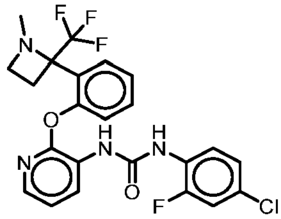,

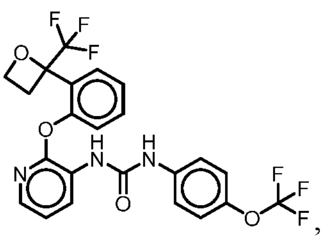,
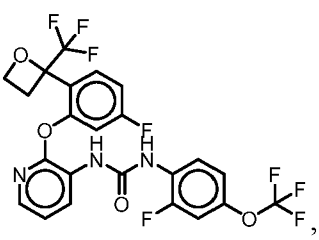,
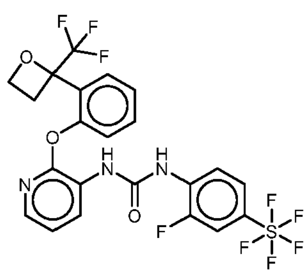

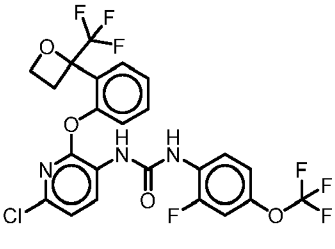,
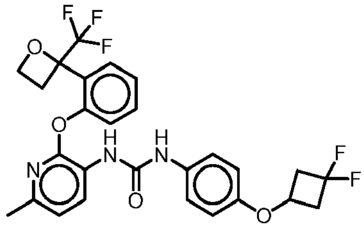,
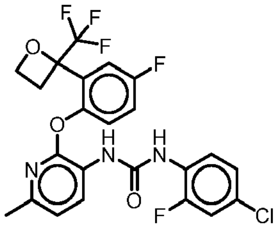,

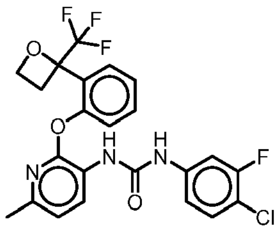,
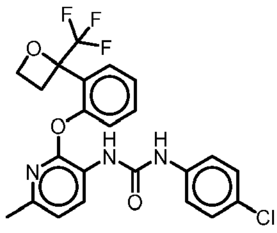,
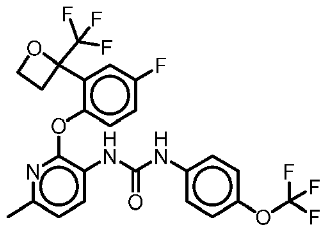,

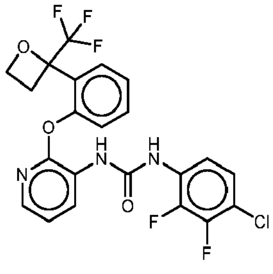,
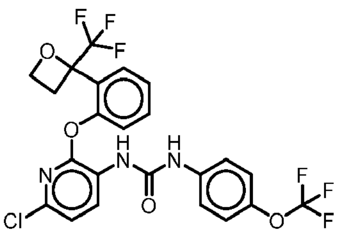,
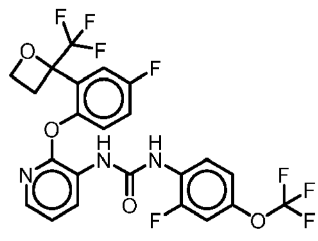,

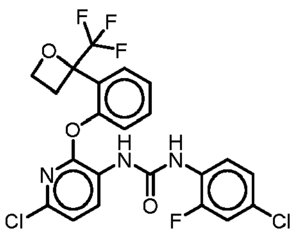,
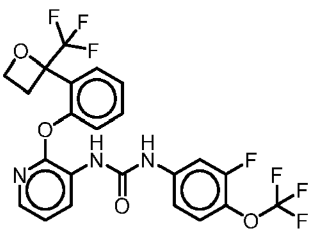,
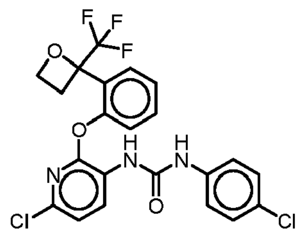,

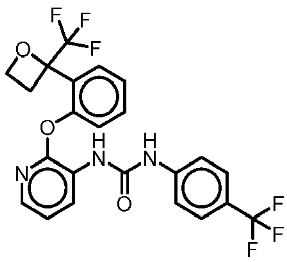,
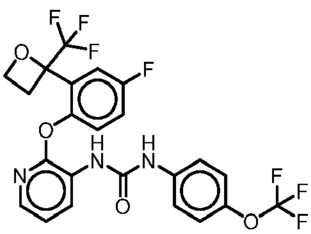,
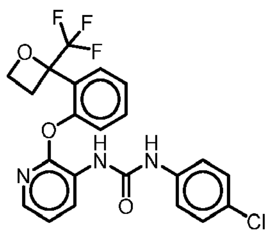,

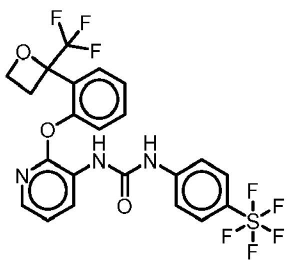,
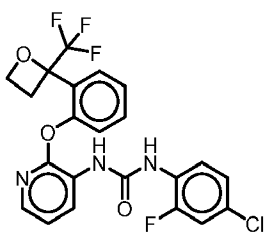,
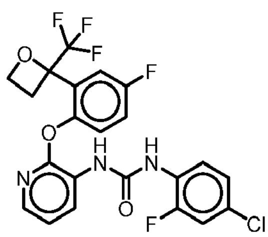,

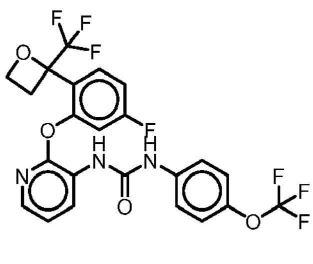,
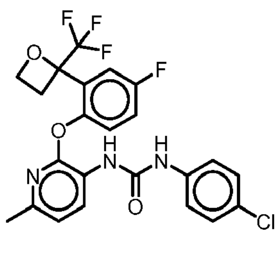,
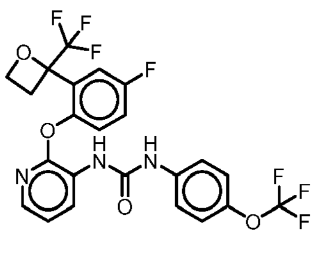,

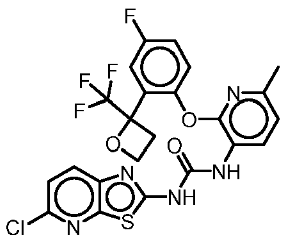 ,

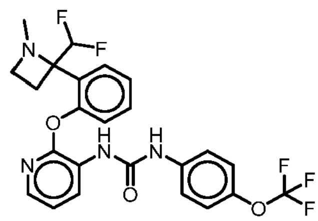 ,

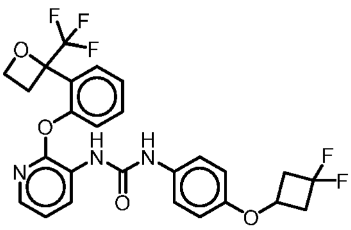 ,

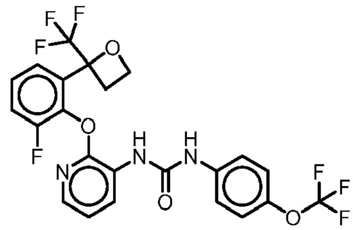 ,

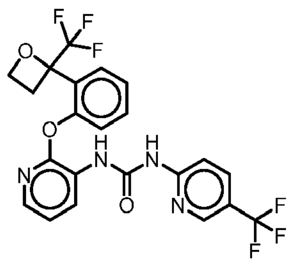 ,

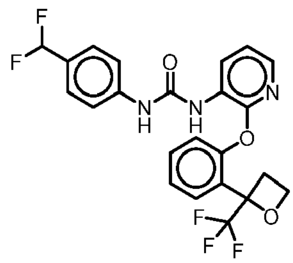 ,

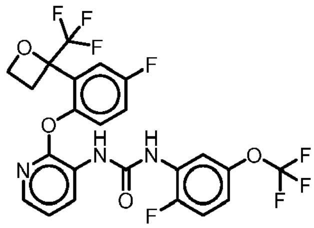 ,

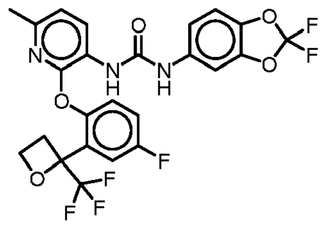 ,

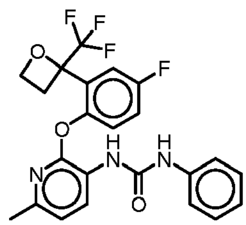 ,

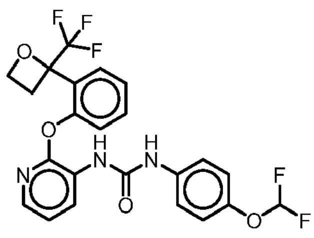 ,

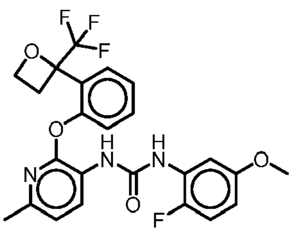 ,

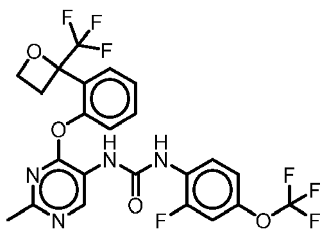 ,

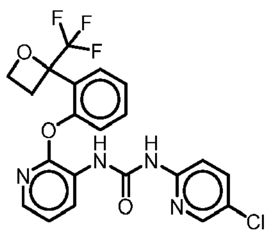 ,

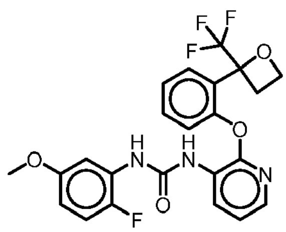 ,

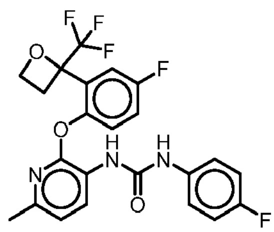 ,

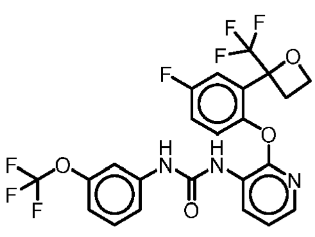 ,

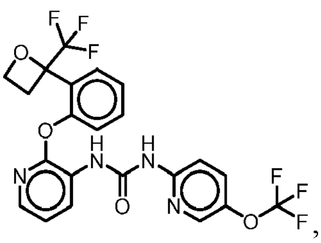 ,

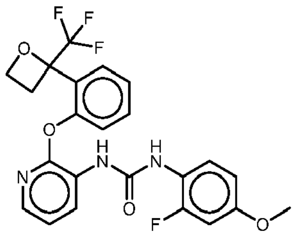 ,

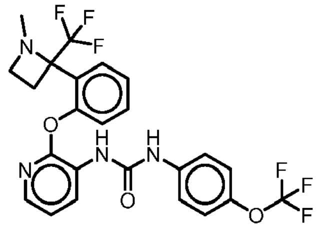, 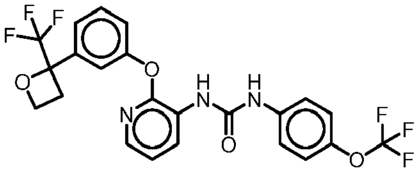, 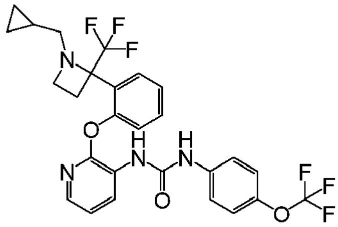,

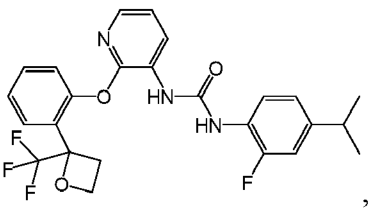, 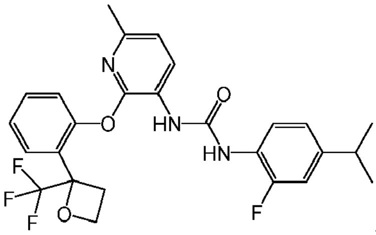, 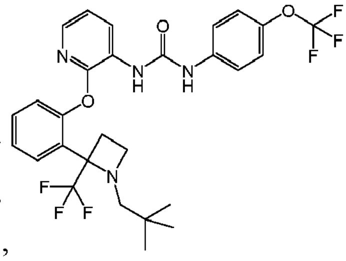,

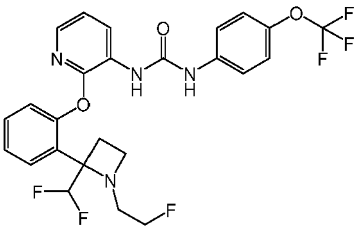.

127. The compound according to aspect 1, wherein the formula (I) or formula (II) is selected from:

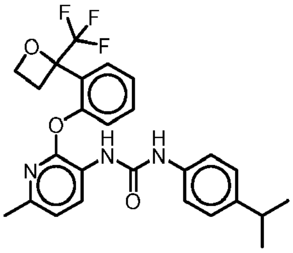, 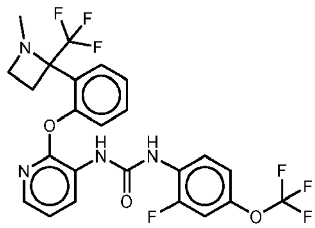, 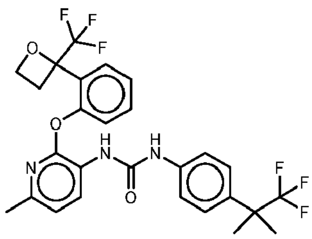

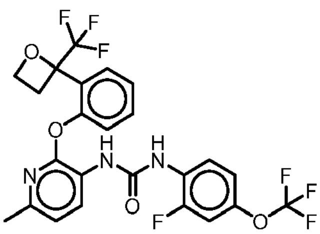, 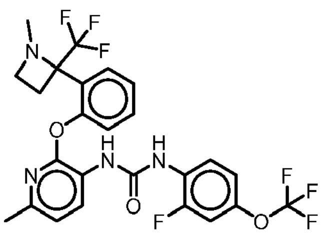, 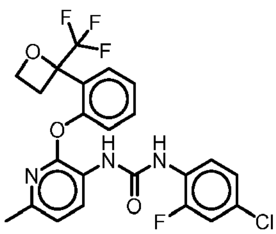,

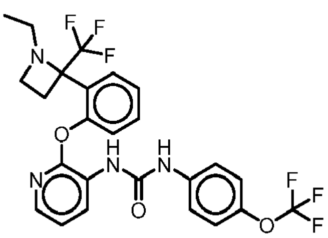, 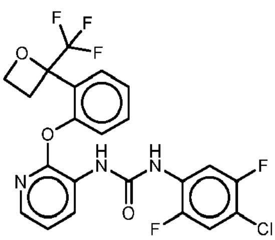, 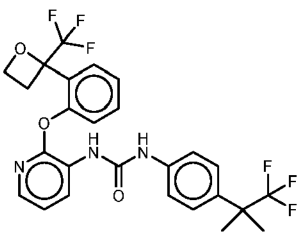,

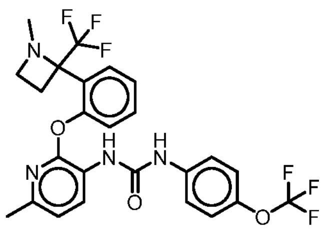,
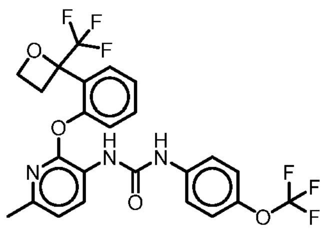,
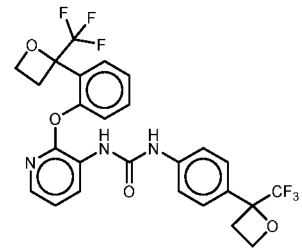,

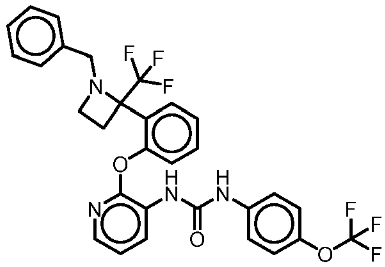,
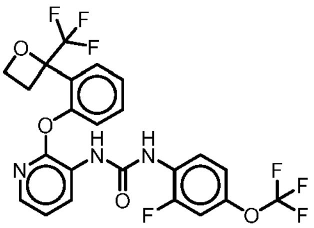,
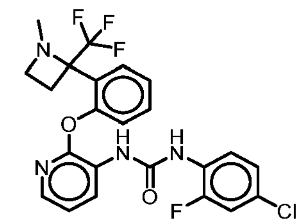,

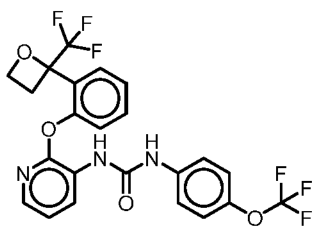,
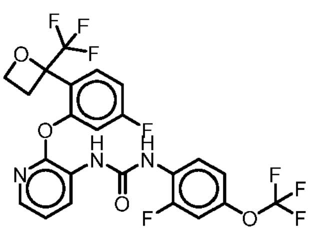,
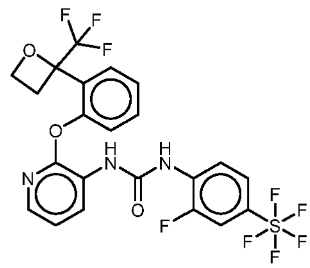

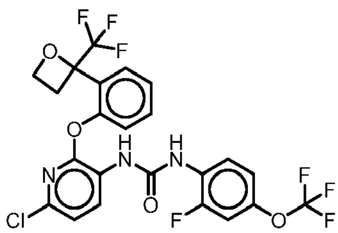,
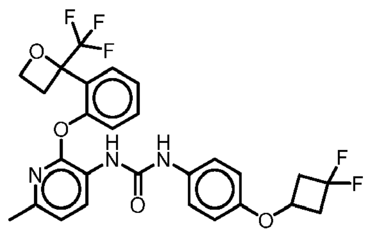,
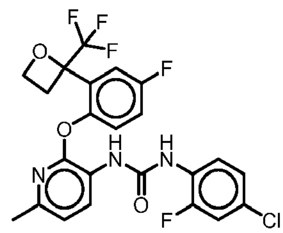,

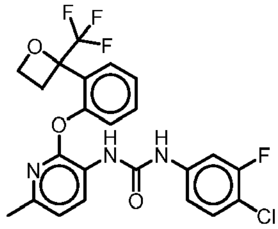,
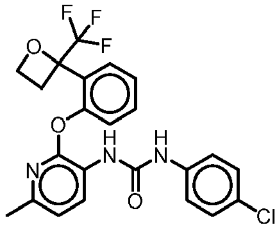,
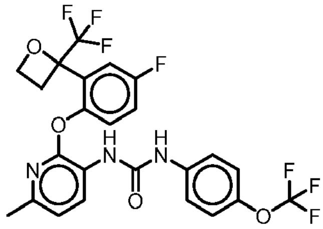,

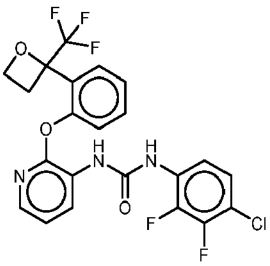,
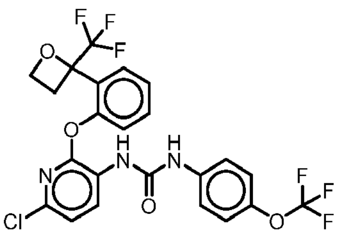,
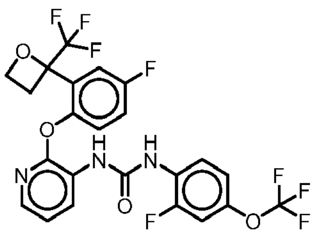,

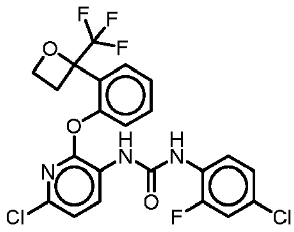,
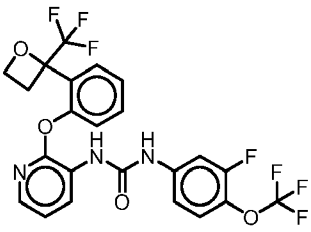,
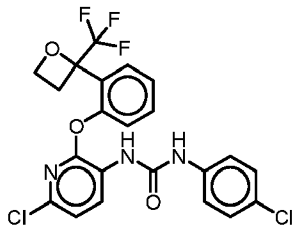,

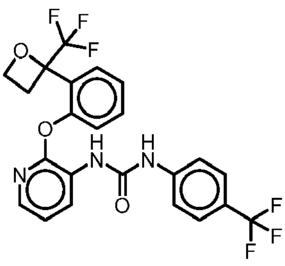,
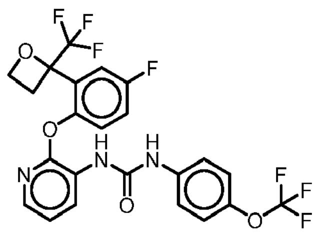,
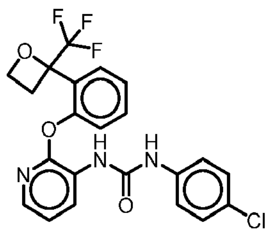,

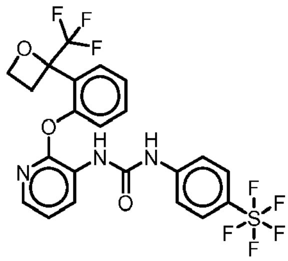,
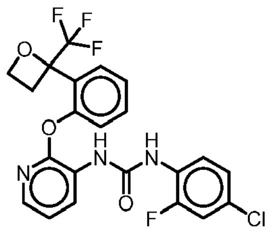,
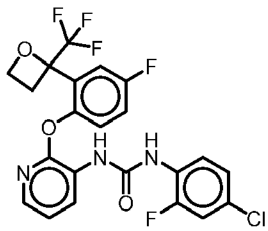,

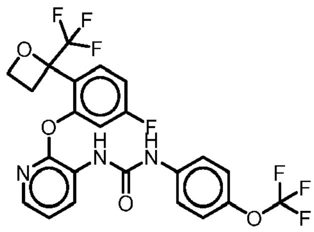,
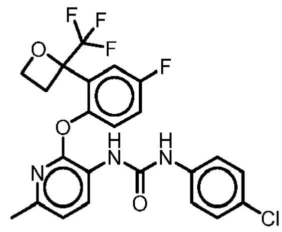,
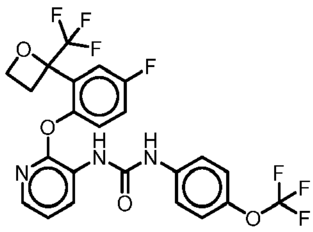,

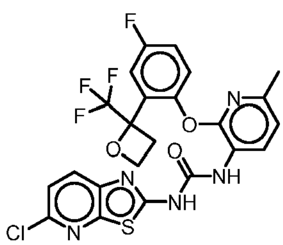,
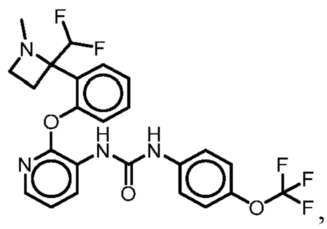,
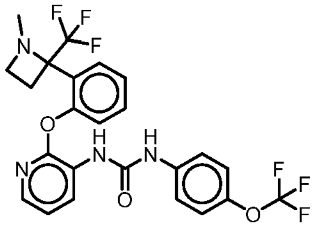,

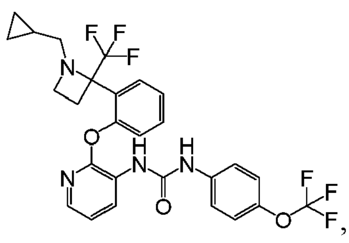,
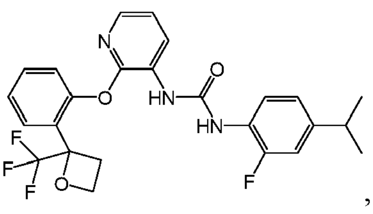,
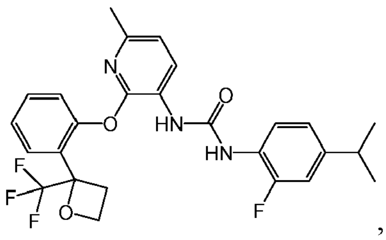,

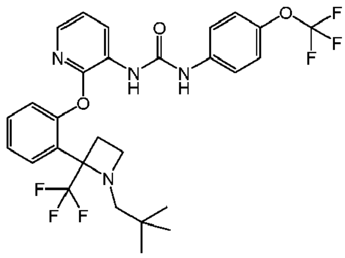, 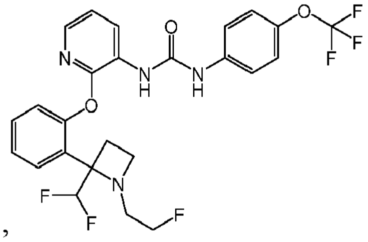.

128. The compound according to aspect 1, wherein the formula (I) or formula (II) is selected from:

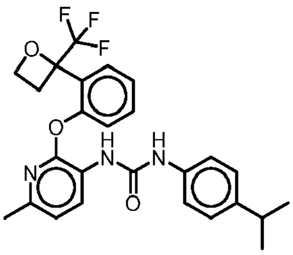, 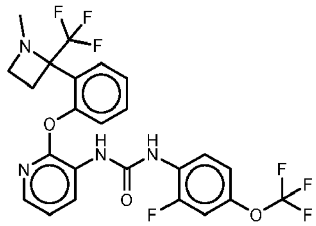, 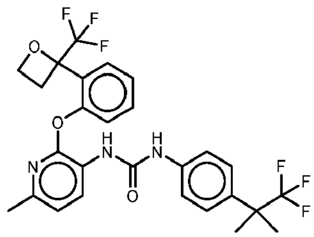

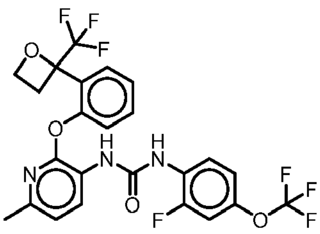, 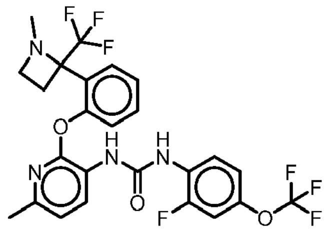, 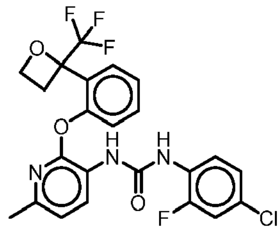,

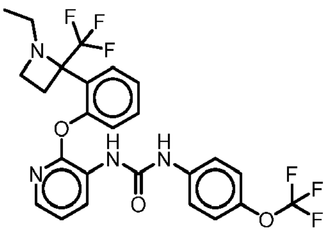, 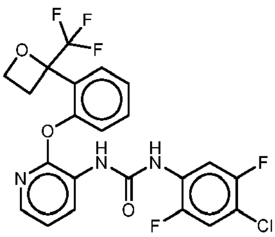, 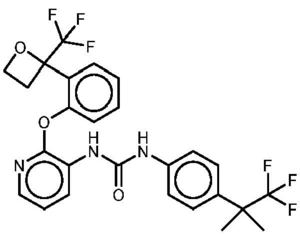,

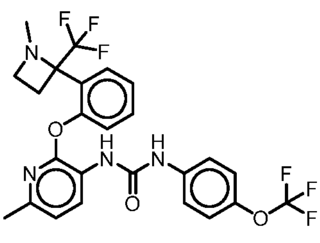, 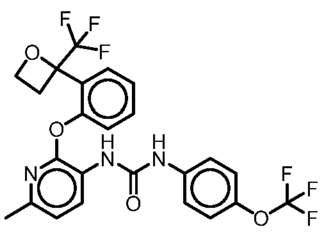, 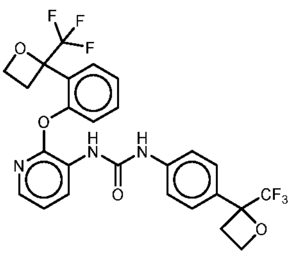,

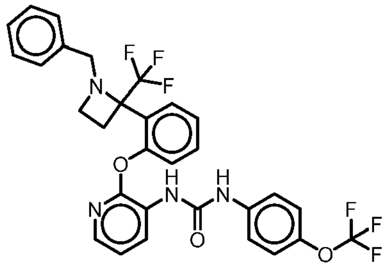,
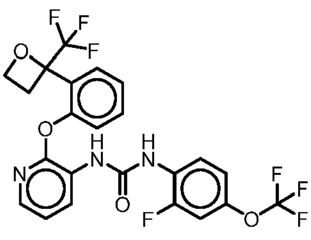,
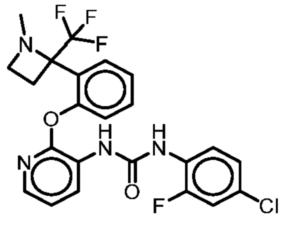,

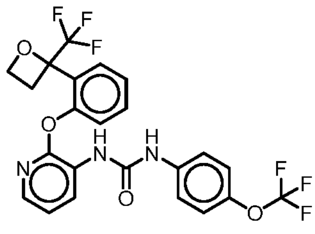,
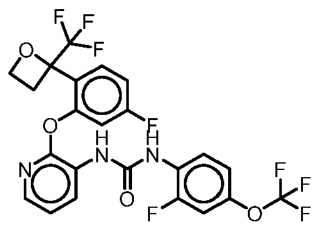,
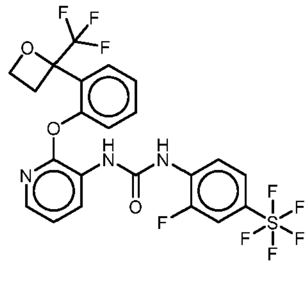

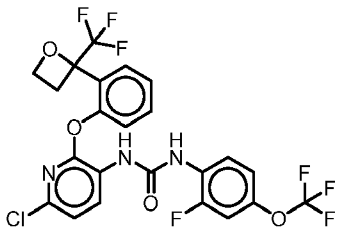,
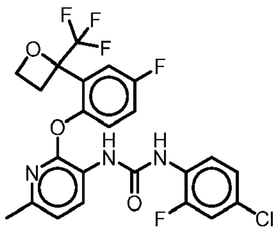,
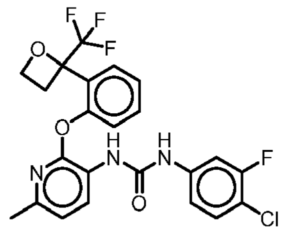,

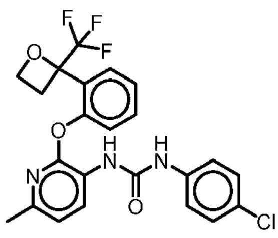,
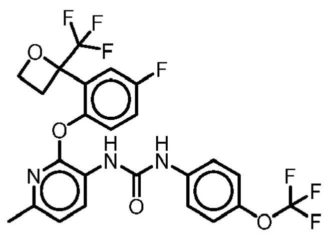,
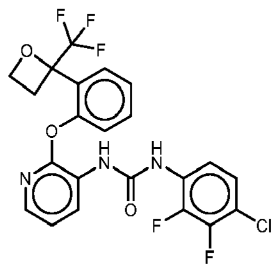,

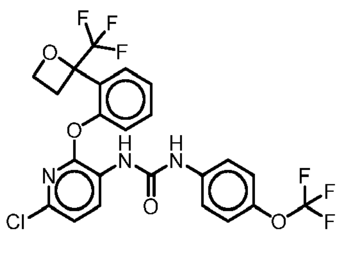,
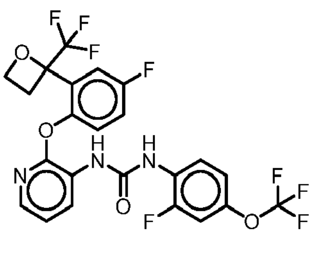,
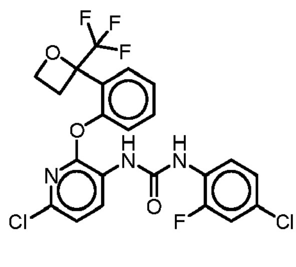,

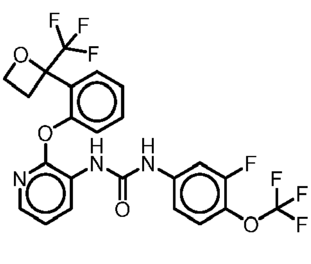,
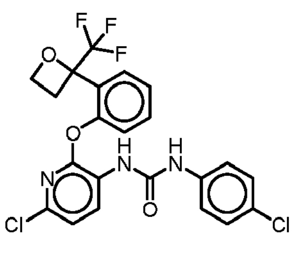,
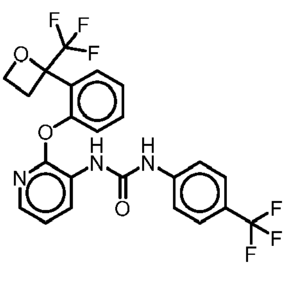,

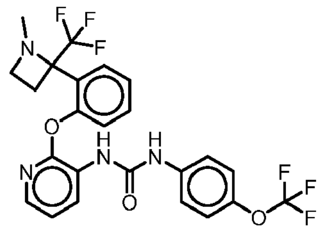, 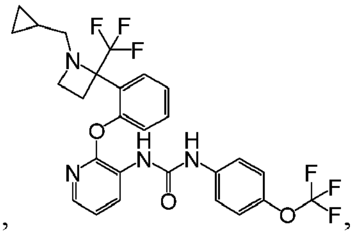, 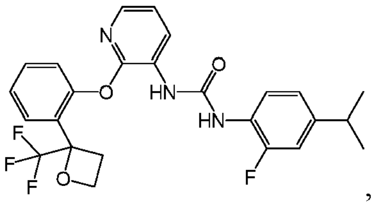,

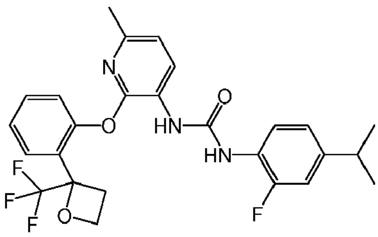, 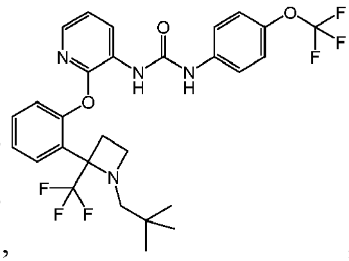, 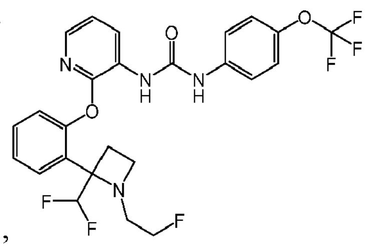.

129. The compound according to aspect 1, wherein the formula (I) or formula (II) is selected from:

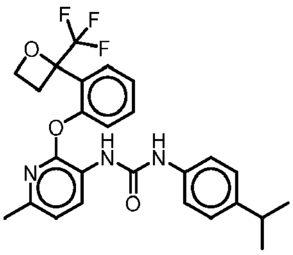, 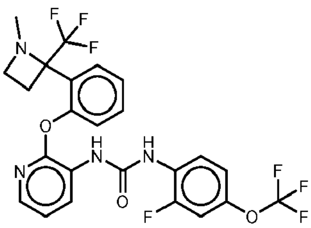, 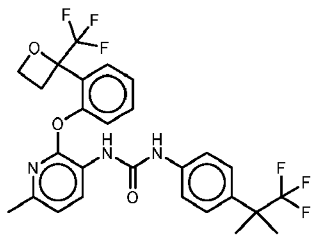

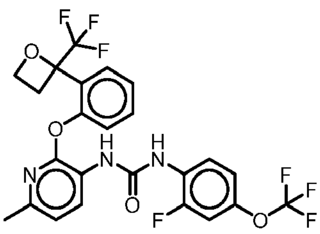, 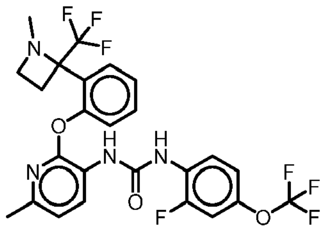, 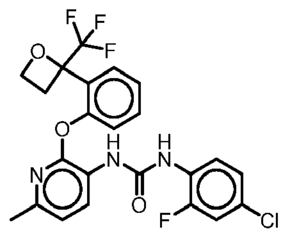,

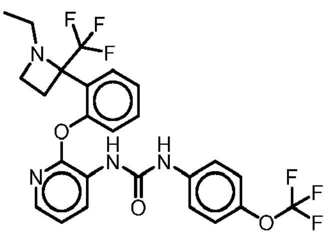, 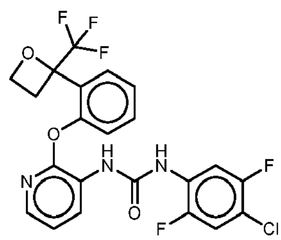, 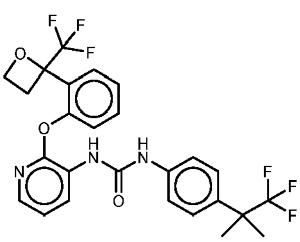,

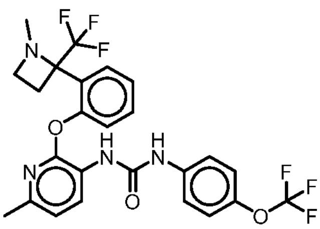, 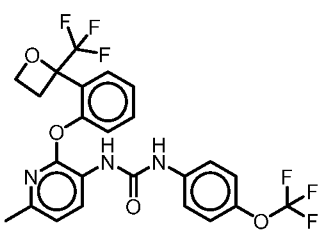, 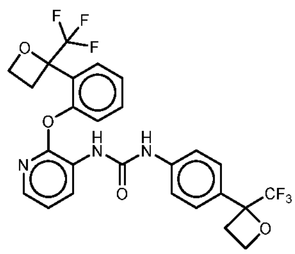,

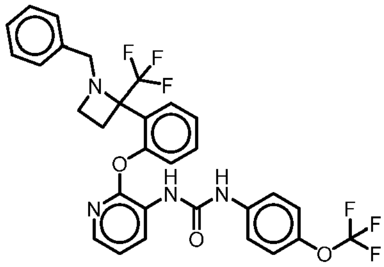, 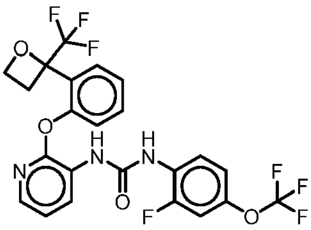, 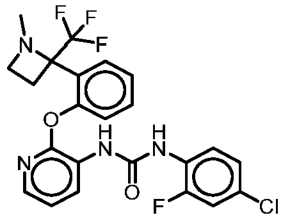,

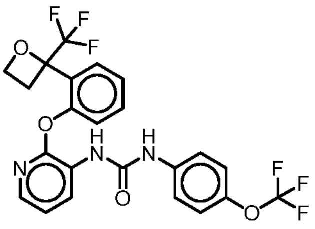, 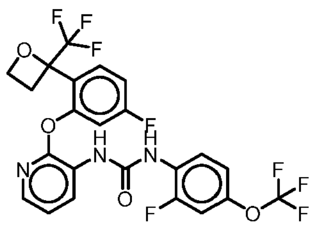, 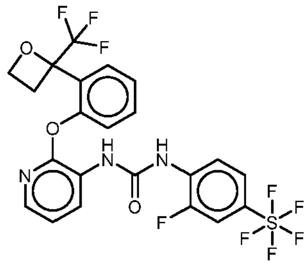

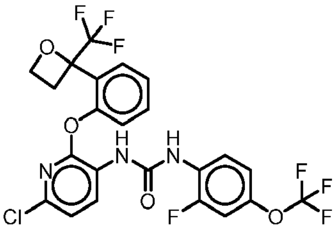, 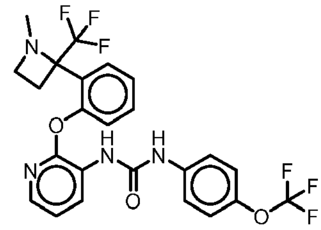, 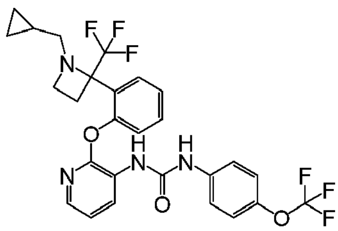,

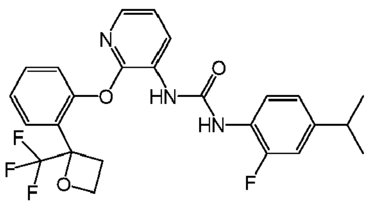, 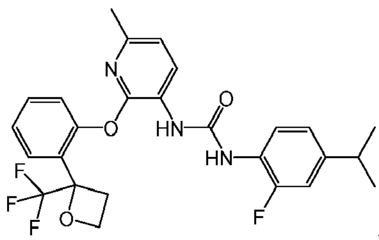, 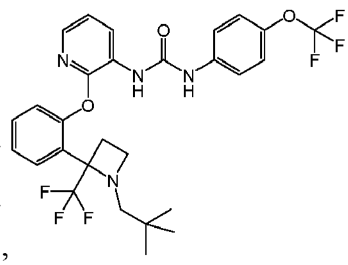.

130. The compound according to aspect 1, wherein the formula (I) or formula (II) is selected from:

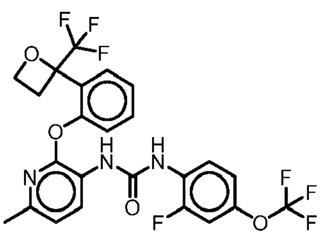, 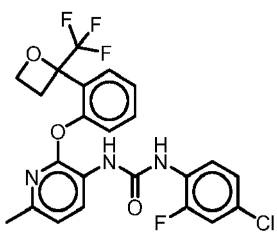, 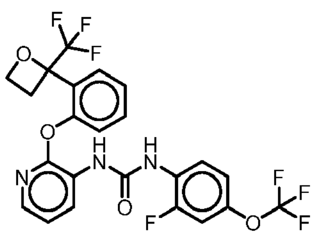,

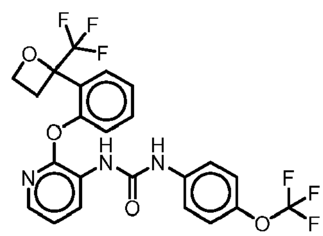, 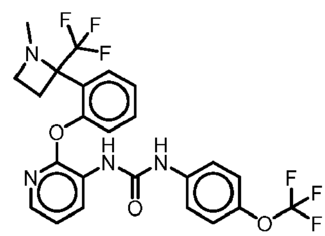.

131. The compound according to aspect 1, wherein the formula (I) or formula (II) is selected from:

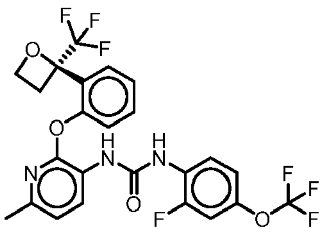,
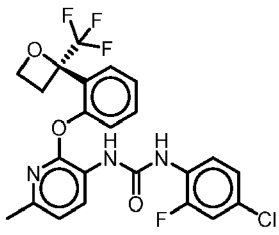,
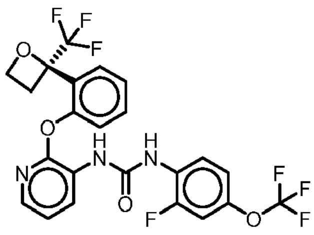

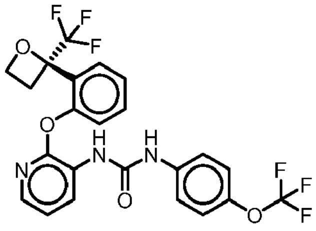,
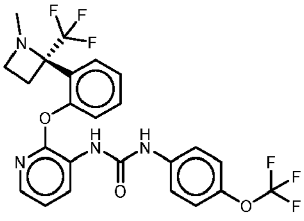,
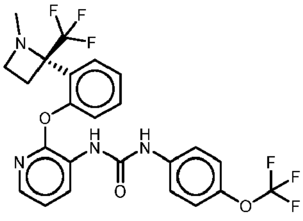.

132. The compound according to aspect 1, wherein the formula (I) or formula (II) is:

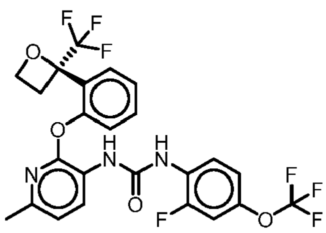.

133. The compound according to aspect 1, wherein the formula (I) or formula (II) is:

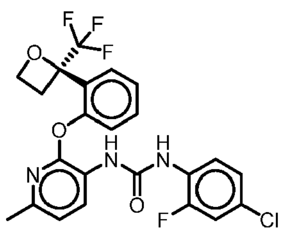.

134. The compound according to aspect 1, wherein the formula (I) or formula (II) is:

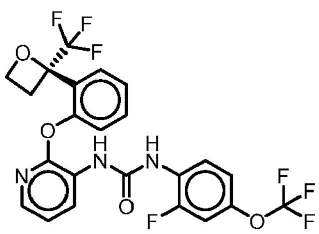.

135. The compound according to aspect 1, wherein the formula (I) or formula (II) is:

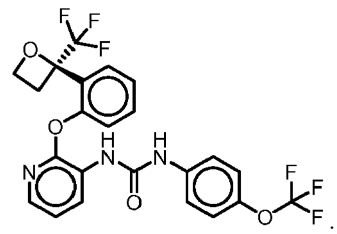

136. The compound according to aspect 1, wherein the formula (I) or formula (II) is:

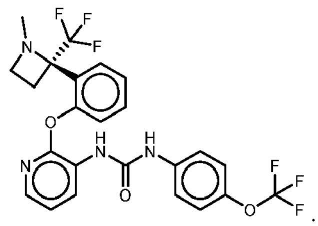

137. The compound according to aspect 1, wherein the formula (I) or formula (II) is:

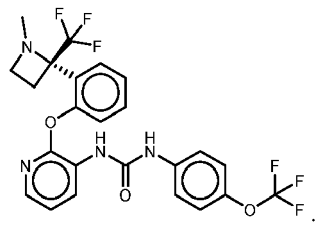

138. A compound that: (a) is of formula (I) or formula (II):

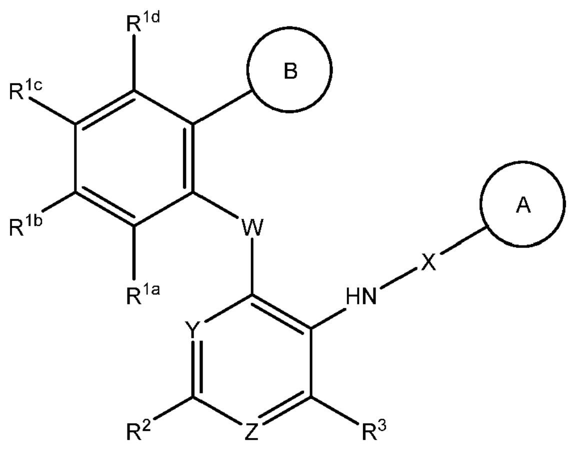

(I)

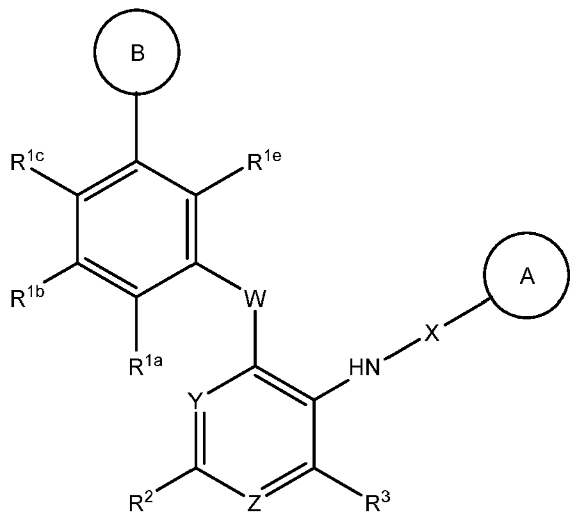

(II) ;

or (b) is a tautomer, N-oxide, pharmaceutically acceptable salt, or solvate thereof, wherein:

ring A is $C_{3-10}$ carbocycle substituted with 0-4 $R^4$, or is 5- to 10-membered heterocycle comprising carbon ring atoms and 1-4 heteroatom ring atoms selected from N, $NR^5$, O and $S(O)_p$, wherein said heterocycle is substituted with 0-4 $R^4$;

ring B is:

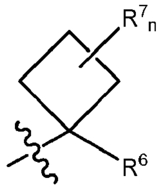

;

W is O, NH, or S;
X is -CO-NH- or bond;
Y is N or CH;
Z is N or CH;
$R^{1a}$ and $R^{1b}$ are, independently at each occurrence, H, F, Cl, Br, -OH, or -$C_{1-6}$ alkyl substituted with 0-3 substituents selected from F or Cl;
$R^{1c}$, $R^{1d}$ and $R^{1e}$ are, independently at each occurrence, H, F, Cl, Br, or -$C_{1-6}$ alkyl substituted with 0-3 substituents selected from F or Cl;
$R^2$ is H, F, Cl, Br, or -$C_{1-6}$ alkyl substituted with 0-3 substituents selected from F or Cl;
$R^3$ is H or OH;
$R^4$ is, independently at each occurrence, F, Cl, Br, $-C_{rA}F_{(2rA+1)}$, $-C_{rA}F_{2rA}H$, - $(CR^{9A}{}_2)_{tA}$-$NR^{10}R^{11}$, -CN, $-OC_{rA}F_{(2rA+1)}$, $-OC_{rA}F_{2rA}H$, $-SF_5$, -$C_{1-6}$ alkyl substituted with 0-3 substituents selected from $R^{12}$, -$C_{3-6}$ cycloalkyl substituted with 0-3 substituents selected from $R^{12}$, -O-$C_{1-6}$ alkyl substituted with 0-3 substituents selected from $R^{12}$, O-$C_{3-6}$ cycloalkyl substituted with 0-3 substituents selected from $R^{12}$, or 3- to 6- membered heterocyclic ring comprising: carbon ring atoms and 1-3 heteroatom ring atoms selected from N, $NR^5$, O and $S(O)_p$, wherein said heterocyclic ring is substituted with 0-3 substituents selected from F, Cl, $-C_{rAA}F_{(2rAA+1)}$, $-C_{rAA}F_{2rAA}H$, or $C_{1-6}$ alkyl substituted with 0-3 substituents selected from $R^{12}$;
alternatively, two $R^4$ groups on the same carbon ring atom combine to form a $C_{3-6}$ carbocycle substituted with 0-2 substituents selected from $R^{13}$;
$R^5$ is, independently at each occurrence, H, $-C_{rB}F_{(2rB+1)}$, $-C_{rB}F_{2rB}H$ or -$C_{1-6}$ alkyl substituted with 0-3 substituents selected from F or Cl;
$R^6$ is H, $-C_{rC}F_{(2rC+1)}$, -CrcF2rcH, or $C_{1-6}$ alkyl;
$R^7$ is $-C_{rD}F_{(2rD+1)}$, -CrDF2rDH, or $C_{1-6}$ alkyl;
alternatively, two $R^7$ groups on the same carbon ring atom form =O;
$R^{9A}$ is, independently at each occurrence, H, $-C_{rF}F_{(2r+1)}$, $-C_{rF}F_{2rF}H$, or $C_{1-6}$ alkyl;
alternatively, two $R^9$ groups on the same carbon atom combine to form a 3- to 7-membered carbocyclic ring;
$R^{10}$ is -$C_{1-6}$ alkyl substituted with 0-3 substituents selected from F or Cl;
$R^{11}$ is H or -$C_{1-6}$ alkyl substituted with 0-3 substituents selected from F or Cl;
alternatively, $R^{10}$ and $R^{11}$ on the same nitrogen atom combine to form a 3- to 6-membered heterocyclic ring comprising: the said nitrogen atom as a heteroatom ring atom, one or more carbon ring atoms and 0, 1 or 2 further heteroatom ring atoms selected from N, $NR^{14}$, O and $S(O)_p$, wherein said heterocyclic ring is substituted with 0-3 substituents selected from F or Cl;
$R^{12}$ is, independently at each occurrence, F, Cl, $-CF_2H$, $-C_2F_5$, $-C_2F_4H$, $-OCH_3$, $-OCH_2CH_3$, $-N(CH_3)_2$, $-N(CH_3)(CH_2CH_3)$, $-N(CH_2CH_3)_2$;
$R^{13}$ is, independently at each occurrence, F or Cl;
$R^{14}$ is H, $-C_{rH}F_{(2rH+1)}$, $-C_{rH}F_{2rH}H$ or -$C_{1-6}$ alkyl substituted with 0-3 substituents selected from F or Cl;
n is 0, 1 or 2;
p is, independently at each occurrence, 0, 1 or 2;
rA is, independently on each substituent in which it occurs, 1-6;
rAA is, independently on each substituent in which it occurs, 1-6;
rB is, independently on each substituent in which it occurs, 1-6;
rC is, independently on each substituent in which it occurs, 1-6;
rD is, independently on each substituent in which it occurs, 1-6;
rF is, independently on each substituent in which it occurs, 1-6;
rH is, independently on each substituent in which it occurs, 1-6; and
tA is, independently at each occurrence, 1, 2 or 3.

139. The compound according to aspect 138, wherein ring A, W, X, Y, Z, $R^{1a}$, $R^{1b}$, $R^{1c}$, $R^{1d}$ $R^{1e}$, $R^2$, $R^3$, $R^4$, $R^6$,

$R^7$, $R^{10}$, $R^{11}$, $R^{12}$, $R^{14}$, n, rA, rAA, rB, rC, rD, rF, rH and tA are as defined in any one of aspects 2 to 123.

140. The compound according to aspect 138 or 139, wherein ring A is not:

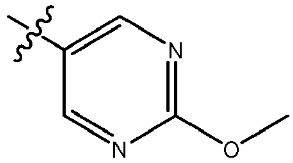

or

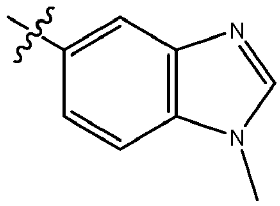

.

141. The compound according to any one of aspects 138 to 140, wherein when ring A is a 2-pyridinyl ring of formula (III), there is no $R^4$ substituent at the pyridinyl 3-position marked by * in formula (III)

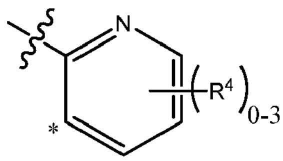

(III)

.

142. The compound according to any of aspects 138 or 141, wherein $R^6$ is $C_{1-6}$ alkyl.

143. The compound according to aspect 142, wherein $R^6$ is $CH_3$.

144. The compound according to aspect 143, wherein the formula (I) or formula (II) is:

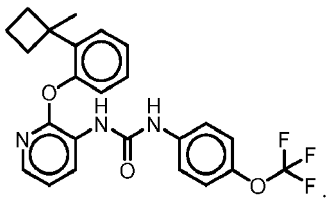

.

145. The compound according to any one of aspects 1 to 144, wherein the compound is a CNS penetrant.

146. The compound according to aspect 145, wherein the compound that is a CNS penetrant achieves a brain to plasma ratio that is 0.3 or greater when measured in accordance with the method defined in Reference Example 1.

147. The compound according to aspect 146, wherein the compound that is a CNS penetrant achieves a brain to plasma ratio that is 0.5 or greater when measured in accordance with the method defined in Reference Example 1.

148. The compound according to aspect 147, wherein the compound that is a CNS penetrant achieves a brain to plasma ratio that is 1.0 or greater when measured in accordance with the method defined in Reference Example 1.

149. The compound according to any one of aspects 1 to 148, wherein all of the atoms of the compound are in their natural isotopic form.

150. The compound according to any one of aspects 1 to 149, which is a tautomer of formula (I) or formula (II).

151. The compound according to any one of aspects 1 to 150, which is an N-oxide of: (a) formula (I) or formula (II); or (b) the tautomer according to aspect 150.

152. The compound according to any one of aspects 1 to 151, which is a pharmaceutically acceptable salt of: (a) formula (I) or formula (II); (b) the tautomer according to aspect 150; or (c) the N-oxide according to aspect 151.

153. The compound according to any one of aspects 1 to 152, which is a solvate of: (a) formula (I) or formula (II); (b) the tautomer according to aspect 150; (c) the N-oxide according to aspect 151; or (d) the pharmaceutically acceptable salt according to aspect 152.

154. The compound according to any one of aspects 1 to 149, which is a compound of formula (I) or formula (II).

155.A pharmaceutical composition comprising a compound according to any one of aspects 1 to 154 in association with one or more pharmaceutically acceptable carriers.

156. The pharmaceutical composition according to aspect 155, which is a solid dosage form suitable for oral administration.

157. The pharmaceutical composition according to aspect 155, which is a liquid dosage form suitable for oral administration.

158. A compound according to any of aspects 1 to 154, or a pharmaceutical composition according to any one of

aspects 155 to 157, for use as a medicament.

159. The use of a compound according to any one of aspects 1 to 154, or the pharmaceutical composition according to any one of aspects 155 to 157, for the manufacture of a medicament.

160.A method of treatment for a patient in need thereof, the method comprising administering a compound according to any one of aspects 1 to 154, or the pharmaceutical composition according to any one of aspects 155 to 157, to the patient.

161. A compound according to any of aspects 1 to 154, or a pharmaceutical composition according to any one of aspects 155 to 157, for use in a method for treating and/or preventing a CNS disorder in a patient.

162. The use of a compound according to any one of aspects 1 to 154, or the pharmaceutical composition according to any one of aspects 155 to 157, for the manufacture of a medicament for use in a method for treating and/or preventing a CNS disorder in a patient.

163.A method for treating and/or preventing a CNS disorder in a patient in need thereof, the method comprising administering a compound according to any one of aspects 1 to 154, or the pharmaceutical composition according to any one of aspects 155 to 157, to the patient.

164. The compound or pharmaceutical composition for use according to aspect 161, use according to aspect 162, or method according to aspect 163, wherein the CNS disorder is a CNS disorder susceptible to treatment by P2Y1 inhibition.

165. The compound or pharmaceutical composition for use, use, or method according to aspect 164, wherein the CNS disorder is Alzheimer's disease.

166. The compound or pharmaceutical composition for use, use, or method according to aspect 165, wherein the Alzheimer's disease is early-onset Alzheimer's disease.

167. The compound or pharmaceutical composition for use, use, or method according to aspect 165, wherein the Alzheimer's disease is late-onset Alzheimer's disease.

168. The compound or pharmaceutical composition for use, use, or method according to aspect 164, wherein the CNS disorder is pain.

169. The compound or pharmaceutical composition for use, use, or method according to aspect 168, wherein the pain comprises neuropathic pain.

170. The compound or pharmaceutical composition for use, use, or method according to aspect 168, wherein the pain comprises nociceptive pain.

171. The compound or pharmaceutical composition for use, use, or method according to aspect 164, wherein the CNS disorder is epilepsy.

172. The compound or pharmaceutical composition for use, use, or method according to aspect 164, wherein the CNS disorder is stroke.

173. The compound or pharmaceutical composition for use, use, or method according to aspect 172, wherein the treating and/or preventing said stroke comprises treating and/or preventing said stroke by inhibiting P2Y1 receptors expressed on astrocytes.

174. The compound or pharmaceutical composition for use, use, or method according to aspect 164, wherein the CNS disorder is traumatic brain injury.

175. The compound or pharmaceutical composition for use, use, or method according to aspect 164, wherein the CNS disorder is amyotrophic lateral sclerosis.

176. The compound or pharmaceutical composition for use, use, or method according to aspect 164, wherein the CNS disorder is Huntington's disease.

177. The compound or pharmaceutical composition for use, use, or method according to aspect 164, wherein the CNS disorder is Parkinson's disease.

178. The compound or pharmaceutical composition for use, use, or method according to aspect 164, wherein the CNS disorder is frontotemporal dementia.

179. The compound or pharmaceutical composition for use, use, or method according to any one of aspects 158 to 178, comprising orally administering the compound or the pharmaceutical composition.

180. The compound or pharmaceutical composition for use, use, or method according to aspect 179, wherein the method comprises orally administering a therapeutically effective amount of the compound or the pharmaceutical composition.

181. The compound or pharmaceutical composition for use, use, or method according to any one of aspects 160 to 180, wherein the patient is a human.

182. The compound or pharmaceutical composition for use, use, or method according to any one of aspects 160 to 181, wherein the method for treating and/or preventing a CNS disorder is a method for treating a CNS disorder.

183. The compound or pharmaceutical composition for use, use, or method according to any one of aspects 160 to 181, wherein the method for treating and/or preventing a CNS disorder is a method for preventing a CNS disorder.

*Compounds of the invention*

The compounds of the present invention are as defined in the detailed aspects 1 to 154.

With respect to ring A, it is noted, for avoidance of doubt, that the said 1-4 heteroatom ring atoms of a 5- to 10-membered heterocycle are members of the ring or rings of the heterocycle (and not, for instance, mere substituents of a carbon ring atom). The same applies to any other heterocycle described herein. Further, to the extent that a ring A that is a 5- to 10-membered heterocycle has one or more non-hydrogenic groups attached to ring atoms, these are defined by the group(s) $R^4$. Accordingly, by way of example, a structure such as

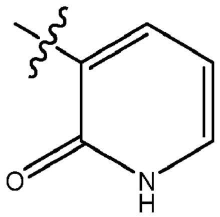

is not encompassed in the definition of ring A as the "O" atom is not a member of the ring of the heterocycle (it is not a heteroatom *ring* atom) and =O is not a group $R^4$.

Further preferred sub-aspects of the compounds of the present invention are provided below. Additionally, it is emphasized that all novel intermediates produced the synthesis of compounds of the present invention are also part of the invention, including but not limited to all of the compounds disclosed in the present Examples section.

*CNS penetration*

The compounds of the invention are typically capable of penetrating the CNS, e.g. following administration to a subject to a location other than the CNS. Hence, when used according to the invention, the compounds can typically exert a desired therapeutic effect without the need for administration directly to the CNS. Such a compound is also referred to herein as a "CNS penetrant". Most generally, a CNS penetrant is a compound that penetrates the CNS after administration to a location other than the CNS (e.g., in a therapeutically significant concentration, e.g. to thereby exert a therapeutically relevant effect).

In one preferred aspect, a compound is a CNS penetrant when the ratio of the concentration of the compound in the brain to the concentration of the compound in the plasma of a test subject (for instance, a mouse, such as a C57B16/J mouse) is at least 0.3 (preferably at least 0.5, further preferably at least 0.7, most preferably at least 1.0) at a time of from 2-4 hours following administration (preferably at a time of 2 hours following administration).

It is particularly preferred that the concentration of the compound in the brain and the concentration of the compound in the plasma be obtained by the following procedure:

- formulate compound in a vehicle;
- administer the compound to a mouse;
- cull the mouse at 2 hours following dosing, collect brain and plasma;
- prepare plasma samples for bioanalysis;
- prepare brain tissue samples for bioanalysis by homogenisation;
- determine the concentration of the compound in each of the brain tissue and plasma samples.

It is still more preferred that the concentration of the compound in the brain and the concentration of the compound in the plasma be obtained by the following procedure:

- construct a LC-MS/MS standard curve for the compound, having a range of 0.5ng/mL to 2000ng/mL
- formulate compound in a vehicle comprising DMSO, Kolliphor EL and HPCD (20% w/v) to a final dose of 0.4mg/kg;
- administer the compound via an intravenous route to a C57B16/J mouse, optionally with up to four other compounds for analysis;
- cull the mouse at 2 hours following dosing, collect brain and plasma;
- prepare plasma samples for bioanalysis in a 1:10 dilution in plasma;
- prepare brain tissue samples for bioanalysis by homogenizing the brain in 0.1M phosphate buffer solution at a ratio of 1:4;
- add internal standard to the samples and then centrifuge for 30 minutes at 4000rpm;
- transfer supernatant, dilute with an equal volume of water and mixed thoroughly;

- analyse the supernatant samples by LC-MS/MS analysis;
- using the standard curve, convert the test peak area of the compound in each of the samples, as obtained by the LC-MS/MS analysis, into a concentration;
- determine whether the compound is a CNS penetrant by assessing if the brain to plasma ratio is 0.3 or greater (preferably at least 0.5, further preferably at least 0.7, most preferably at least 1.0).

Further preferably, the compounds of the present invention are CNS penetrants according to one (or more) of the methods disclosed in Reference Example 1 and Example 2.

*Dosage and formulation*

The compounds of the invention, when used in a method of treatment, can be administered alone, but generally will be administered with a pharmaceutical carrier selected on the basis of the chosen route of administration and standard pharmaceutical practice. Thus, preferably, the compounds of the present invention can be included in a pharmaceutical composition as defined in aspect 155, 156 or 157. Preferably the pharmaceutical composition is suitable for oral administration. One exemplary such pharmaceutical composition is a solid dosage form suitable for oral administration (e.g., a tablet, capsule (each of which includes a sustained release or timed release formulation), pill, powder, or granule). Another exemplary such pharmaceutical composition is a liquid dosage form suitable for oral administration (e.g., an elixir, tincture, suspension, syrup, solution or emulsion).

Preferably, the compounds or pharmaceutical composition of the invention are for use in a method of treatment comprising administering a therapeutically effective amount of the compound or pharmaceutical composition, and particularly preferably a method of treatment comprising orally administering a therapeutically effective amount of the compound or pharmaceutical composition. Thus, the compounds of the invention are preferably administered in solid dosage forms for oral administration (such as tablets, capsules (each of which includes sustained release or timed release formulations), pills, powders, or granules) or as liquid dosage forms for oral administration (such as, elixirs, tinctures, suspensions, syrups, solutions and emulsions).

However, the compounds of the invention may also be administered in intravenous (bolus or infusion), intraperitoneal, subcutaneous, or intramuscular form, all using dosage forms well known to those of ordinary skill in the pharmaceutical arts.

The dosage regimen for the compounds of the invention will, of course, vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. A physician or veterinarian can determine and prescribe the effective amount of the drug required to prevent, counter, or arrest the progress of the thromboembolic disorder.

By way of general guidance, the daily oral dosage of each active ingredient, when used for the indicated effects, will range between about 0.001 to 1000 mg/kg of body weight, preferably between about 0.01 to 100 mg/kg of body weight per day, and most preferably between about 1.0 to 20 mg/kg/day.

Intravenously, the most preferred doses will range from about 1 to about 10 mg/kg/minute during a constant rate infusion. Compounds of the invention may be administered in a single daily dose, or the total daily dosage may be administered in divided doses of two, three, or four times daily.

Compounds of the invention can be administered in intranasal form via topical use of suitable intranasal vehicles, or via transdermal routes, using transdermal skin patches. When administered in the form of a transdermal delivery system, the dosage administration will, of course, be continuous rather than intermittent throughout the dosage regimen.

The compounds are typically administered in admixture with suitable pharmaceutical diluents, excipients, or carriers (collectively referred to herein as pharmaceutical carriers) suitably selected with respect to the intended form of administration, that is, oral tablets, capsules, elixirs, syrups and the like, and consistent with conventional pharmaceutical practices.

For instance, for oral administration in the form of a tablet or capsule, the active drug component can be combined with an oral, non-toxic, pharmaceutically acceptable, inert carrier such as lactose, starch, sucrose, glucose, methyl cellulose, magnesium stearate, dicalcium phosphate, calcium sulfate, mannitol, sorbitol and the like; for oral administration in liquid form, the oral drug components can be combined with any oral, non-toxic, pharmaceutically acceptable inert carrier such as ethanol, glycerol, water, and the like. Moreover, when desired or necessary, suitable binders, lubricants, disintegrating agents, and coloring agents can also be incorporated into the mixture. Suitable binders include starch, gelatin, natural sugars such as glucose or beta-lactose, com sweeteners, natural and synthetic gums such as acacia, tragacanth, or sodium alginate, carboxymethylcellulose, polyethylene glycol, waxes, and the like. Lubricants used in these dosage forms include sodium oleate, sodium stearate, magnesium stearate, sodium benzoate, sodium acetate, sodium chloride, and the like. Disintegrators include, without limitation, starch, methyl cellulose, agar,

bentonite, xanthan gum, and the like.

The compounds of the present invention can also be administered in the form of liposome delivery systems, such as small unilamellar vesicles, large unilamellar vesicles, and multilamellar vesicles. Liposomes can be formed from a variety of phospholipids, such as cholesterol, stearylamine, or phosphatidylcholines.

Compounds of the present invention may also be coupled with soluble polymers as targetable drug carriers. Such polymers can include polyvinylpyrrolidone, pyran copolymer, polyhydroxypropylmethacrylamide-phenol, polyhydroxyethylaspartamidephenol, or polyethyleneoxide-polylysine substituted with palmitoyl residues. Furthermore, the compounds of the present invention may be coupled to a class of biodegradable polymers useful in achieving controlled release of a drug, for example, polylacetic acid, polyglycolic acid, copolymers of polylacetic and polyglycolic acid, polyepsilon caprolactone, polyhydroxy butyric acid, polyorthoesters, polyacetals, polydihydropyrans, polycyanoacylates, and crosslinked or amphipathic block copolymers of hydrogels.

Dosage forms (pharmaceutical compositions) suitable for administration may contain from about 1 milligram to about 100 milligrams of active ingredient per dosage unit. In these pharmaceutical compositions the active ingredient will ordinarily be present in an amount of about 0.5-95% by weight based on the total weight of the composition.

Gelatin capsules may contain the active ingredient and powdered carriers, such as lactose, starch, cellulose derivatives, magnesium stearate, stearic acid, and the like. Similar diluents can be used to make compressed tablets. Both tablets and capsules can be manufactured as sustained release products to provide for continuous release of medication over a period of hours. Compressed tablets can be sugar coated or film coated to mask any unpleasant taste and protect the tablet from the atmosphere, or enteric coated for selective disintegration in the gastrointestinal tract.

Liquid dosage forms for oral administration can contain coloring and flavoring to increase patient acceptance.

In general, water, a suitable oil, saline, aqueous dextrose (glucose), and related sugar solutions and glycols such as propylene glycol or polyethylene glycols are suitable carriers for parenteral solutions. Solutions for parenteral administration preferably contain a water soluble salt of the active ingredient, suitable stabilizing agents, and if necessary, buffer substances. Antioxidizing agents such as sodium bisulfite, sodium sulfite, or ascorbic acid, either alone or combined, are suitable stabilizing agents. Also used are citric acid and its salts and sodium EDTA. In addition, parenteral solutions can contain preservatives, such as benzalkonium chloride, methyl-or propyl-paraben, and chlorobutanol.

Suitable pharmaceutical carriers are described in Remington's Pharmaceutical Sciences, Mack Publishing Company, a standard reference text in this field.

*Compound/composition for use as medicament*

In an embodiment, the compound or pharmaceutical composition of the present invention is for use as a medicament, as defined in aspect 158.

*Compound/composition for in method for treating and/or preventing a CNS disorder*

In another embodiment, the compound or pharmaceutical composition is for use in a method for treating and/or preventing a CNS disorder, as defined in aspects 161 and 164-182.

*CNS disorders*

Typically, the CNS disorder to be treated and/or prevented in the present invention is a CNS disorder susceptible to treatment by P2Y1 inhibition.

Further preferably, the CNS disorder to be treated and/or prevented is selected from Alzheimer's disease, pain, epilepsy, stroke, traumatic brain injury, amyotrophic lateral sclerosis, Huntington's disease, Parkinson's disease and frontotemporal dementia.

Further preferably, the CNS disorder to be treated and/or prevented is selected from Alzheimer's disease, pain, epilepsy, stroke, traumatic brain injury and amyotrophic lateral sclerosis.

Further preferably still, the CNS disorder to be treated and/or prevented is selected from Alzheimer's disease, pain, epilepsy and stroke.

In one (particularly) preferred aspect of the present invention, the CNS disorder to be treated and/or prevented is Alzheimer's disease. The Alzheimer's disease may be either early-onset or late-onset Alzheimer's disease.

Alzheimer's disease is a dementia-causing neurodegenerative disease. It is an irreversible, progressive brain disorder that causes a slow decline in memory, thinking and reasoning skills before ultimately leading to death. The disease pathology is associated with the formation of abnormal protein agglomerations in the brain tissue known as "plaques" and "tangles" which disrupt neuron functioning, lead to the loss of neuronal connections, and ultimately lead to neuron death.

A number of treatments are available to improve the symptoms of Alzheimer's disease, namely treatment with

acetylcholinesterase (AChE) inhibitors (such as donepezil, galantamine and rivastigmine) or with memantine. However, new treatments for Alzheimer's disease would be highly desirable.

Pathological astrocytic network activity (intracellular calcium waves and single-cell astrocyte hyperactivity) is observed in Alzheimer's disease. Recent reports have connected such pathological activity with purinergic signaling (Delakate A, et al. Nat. Commun., 2014;5:5422). Such studies have identified that the P2Y1 receptor (a purinergic receptor) is particularly strongly expressed in the reactive astrocytes surrounding plaques, and have demonstrated that P2Y1 inhibition is capable of normalizing astrocyte activity. The studies further suggest that such that P2Y1 inhibition may represent a potential therapeutic strategy for ameliorating inflammation and cognitive decline associated with Alzheimer's disease.

A proof-of-concept study has been carried out to demonstrate the application of P2Y1 inhibition in murine models of Alzheimer's disease (Reichenbach N, et al. J. Exp. Med. 2018;215(6): 1649-1663). This study demonstrated that chronic *intracerebroventricular* infusion of P2Y1 inhibitors normalizes astroglial and neuronal network dysfunction and is capable of protecting against decline of spatial learning and memory.

Intracerebroventricular infusion is an invasive mode of drug delivery, used when a pharmaceutical agent is either empirically known or otherwise believed - due to its physicochemical properties - to be incapable of crossing the blood-brain barrier. Such an administration mode is disadvantageous, not only because of the need for neurosurgical intervention, but also due to problems with the extent to which the active agent is capable of diffusing from the injection site throughout the human brain (Pardridge W M., Alzheimers Dement. 2009;5(5):427-43).

It is desirable to provide new P2Y1 inhibitors for use in a method of treating Alzheimer's disease. It is particularly desirable to provide P2Y1 inhibitors for use in a method of treating Alzheimer's disease that are capable of crossing blood-brain barrier, i.e. to provide a P2Y1 inhibitor for use in a method of treating Alzheimer's disease that is a CNS penetrant. This will enable the P2Y1 inhibitor to be administered to the patient in a simple manner (e.g., by oral administration), whilst still being able subsequently to access the CNS.

In another preferred aspect of the invention, the CNS disorder to be treated and/or prevented is pain. The pain that is treated and/or prevented may comprise or consist of neuropathic pain. The pain that is treated and/or prevented may comprise or consist of nociceptive pain.

In another preferred aspect of the invention, the CNS disorder to be treated and/or prevented is epilepsy.

In another preferred aspect of the invention, the CNS disorder to be treated and/or prevented is stroke. The treatment and/or prevention of stroke according to the present invention is typically achieved by neuroprotection within the CNS (e.g., by inhibition of P2Y1 receptors expressed on astrocytes), rather than through the treatment and/or prevention of thromboembolism-induced stroke mediated via blood clotting.

In another preferred aspect of the invention, the CNS disorder to be treated and/or prevented is traumatic brain injury.

In another preferred aspect of the invention, the CNS disorder to be treated and/or prevented is amyotrophic lateral sclerosis.

*Use for manufacture of a medicament*

The present invention provides the use of a compound or pharmaceutical composition according to the invention for the manufacture of a medicament, preferably for use in a method for treating and/or preventing a CNS disorder, as defined in aspects 159, 162 and 164 to 182.

It will be understood that all preferred disclosure provided above in connection with the compound or pharmaceutical composition according the invention for use according to the invention is equally contemplated as preferred in the context of the use of a compound or pharmaceutical composition according to the invention, for the manufacture of a medicament according to the present invention.

*Method of treatment*

The present invention provides a method of treatment, preferably a method for treating and/or preventing a CNS disorder according the invention, as defined in aspects 160 and 163 to 182.

It will be understood that all preferred disclosure provided above in connection with the compound or pharmaceutical composition according the invention for use according to the invention is equally contemplated as preferred in the context of the method of treatment according to the present invention.

## Examples

*Reference Example 1*

A protocol for determining CNS penetration (i.e. to determine whether a test compound is a "CNS penetrant") is as follows:

- construct a LC-MS/MS standard curve for the compound, having a range of 0.5ng/mL to 2000ng/mL
- formulate compound in a vehicle comprising DMSO, Kolliphor EL and HPCD (20% w/v) to a final dose of 0.4mg/kg;
- administer the compound via an intravenous route to a C57B16/J mouse, optionally with up to four other compounds for analysis;
- cull the mouse at 2 hours following dosing, collect brain and plasma;
- prepare plasma samples for bioanalysis in a 1:10 dilution in plasma;
- prepare brain tissue samples for bioanalysis by homogenizing the brain in 0.1M phosphate buffer solution at a ratio of 1:4;
- add internal standard to the samples and then centrifuge for 30 minutes at 4000rpm;
- transfer supernatant, dilute with an equal volume of water and mixed thoroughly;
- analyse the supernatant samples by LC-MS/MS analysis;
- using the standard curve, convert the test peak area of the compound in each of the samples, as obtained by the LC-MS/MS analysis, into a concentration;
- determine whether the compound is a CNS penetrant by assessing if the brain to plasma ratio is 0.3 or greater (preferably at least 0.5, further preferably at least 0.7, most preferably at least 1.0).

*Example 1*

P2Y1 inhibition data for selected examples of the invention.

*Measurement of IC50 values*

IC50 values were determined using a cell based assay in stably transfected cells overexpressing the P2Y1 receptor. The assay system is a high throughput resonance transfer (HTRF) assay measuring accumulation of the intracellular messenger inositol monophosphate (IP1), generated intracellularly following activation of P2Y1. Activity of test compounds was generated by measuring antagonism of the increase in IP1 generated by addition of a selective P2Y1 agonist, MRS2365.

P2Y1 overexpressing cells were maintained in T175 flasks. The day before the assay, these cells were split and 20μl cell suspension transferred to each well of white 384-well assay plates at a cell density of $0.5x10^6$ cells/mL. Plates were pulse centrifuged, then incubated overnight at 37°C / 5% $CO_2$ prior to assay.

On day of assay, medium was removed from cells and replaced with stimulation buffer. Compounds were prepared in 100% DMSO for addition to cells using the Tecan D300e digital dispenser; compounds were prepared in cassette heads and added directly to wells in the required concentration range, to give a 10-point concentration response curve from 10μM with semi-log dilutions and a final DMSO concentration of 0.1%. Positive control antagonist curves with standard compounds were included on each plate. Plates were incubated with antagonists for 1 hour prior to addition of 5nM of the agonist MRS2365, dispensed using a Bravo liquid handler. Following agonist addition, plates were incubated for 60 minutes prior to assay. Both incubations were carried out at 37°C / 5% $CO_2$.

For the assay, IP1 accumulation was quantified using the IPOne kit according to manufacturer's instructions. In short, a standard curve was prepared for IP 1 quantification, and cells lysed using kit lysis buffer. Assay reagents were added to the lysates, and incubated for 1 hour at room temperature without spinning or shaking. Plates were analysed on a BMG Pherastar plate reader, and IP1 signal generated in cells interpolated from the standard curve.

For all reported results, N is greater than or equal to 4. An individual N refers to one assay run with a single weighing of compound. A minimum of two assay runs were performed for each of two independent weighings of compound.

*Results (racemic)*

| Structure | ID | IC50(nM) |
|---|---|---|
| 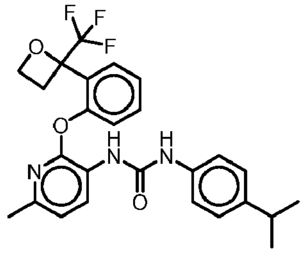 | S1 | 20 |
| 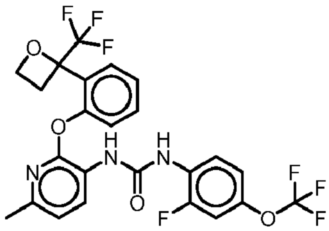 | S2 | 33 |
| 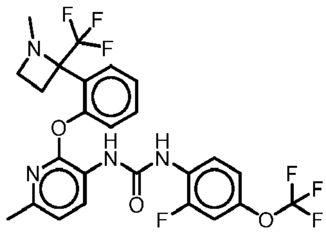 | S3 | 40 |
| 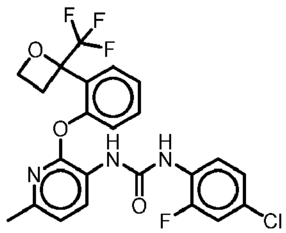 | S4 | 43 |
| 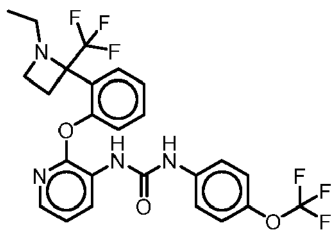 | S5 | 43 |
| 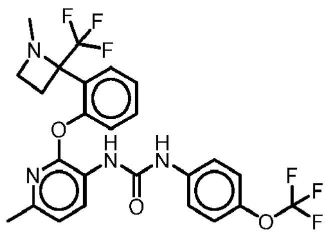 | S6 | 50 |

(continued)

| Structure | ID | IC50(nM) |
|---|---|---|
| 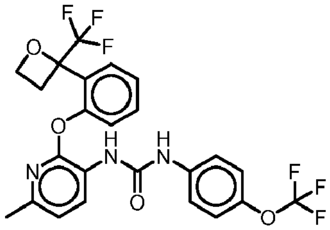 | S7 | 55 |
| 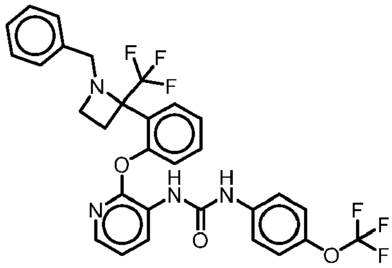 | S8 | 64 |
| 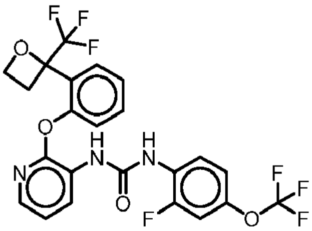 | S9 | 66 |
| 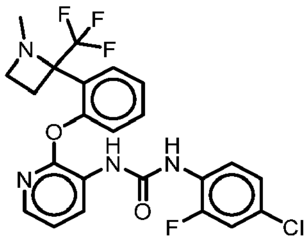 | S10 | 78 |
| 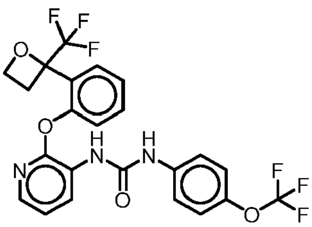 | S11 | 84 |
| 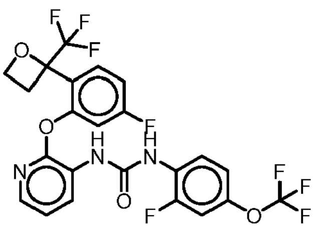 | S12 | 87 |

(continued)

| Structure | ID | IC50(nM) |
|---|---|---|
| 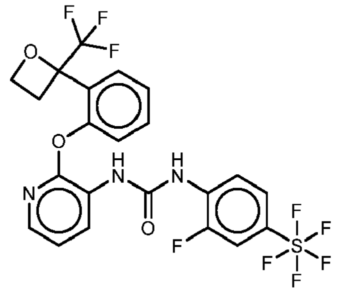 | S13 | 93 |
| 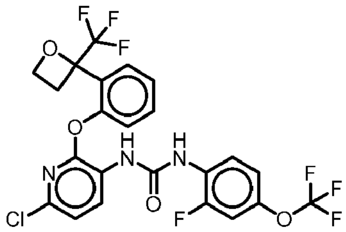 | S14 | 94 |
| 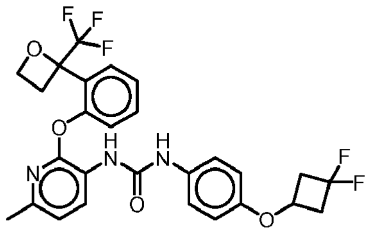 | S15 | 105 |
| 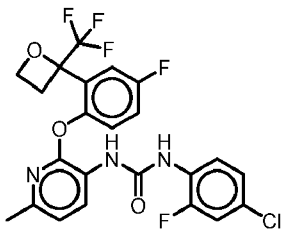 | S16 | 108 |
| 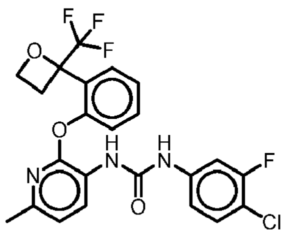 | S17 | 114 |
| 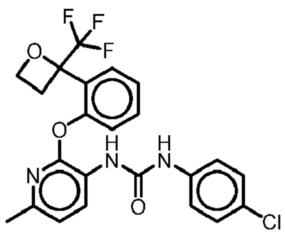 | S18 | 118 |

(continued)

| Structure | ID | IC50(nM) |
|---|---|---|
| 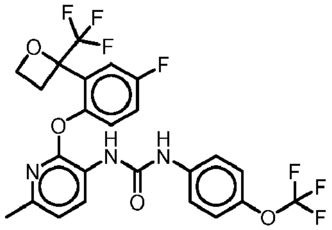 | S19 | 120 |
| 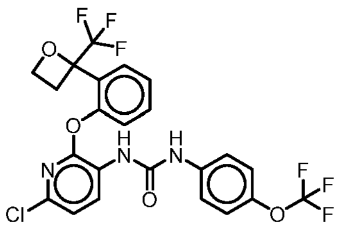 | S20 | 125 |
| 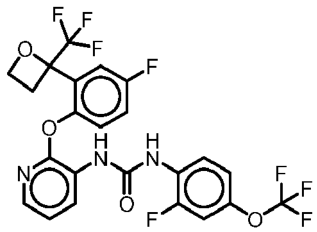 | S21 | 140 |
| 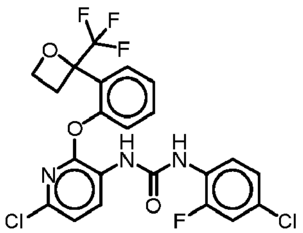 | S22 | 141 |
| 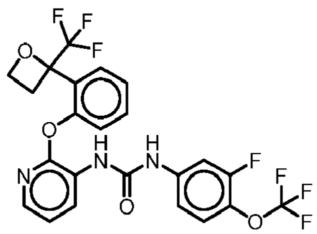 | S23 | 145 |
| 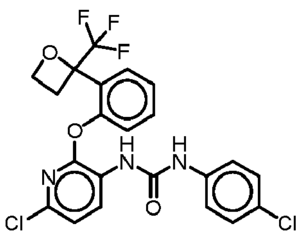 | S24 | 145 |

(continued)

| Structure | ID | IC50(nM) |
|---|---|---|
| 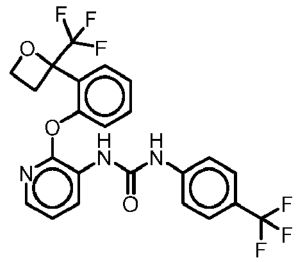 | S25 | 146 |
| 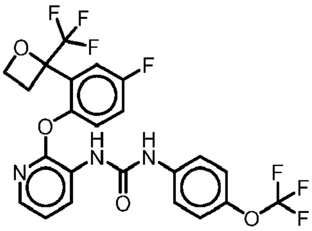 | S26 | 152 |
| 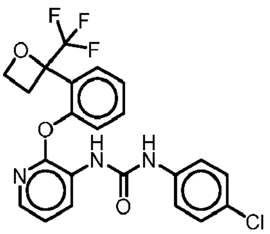 | S27 | 153 |
| 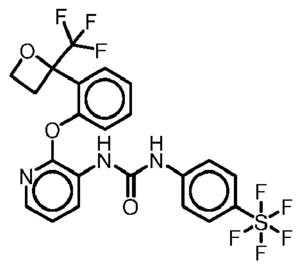 | S28 | 157 |
| 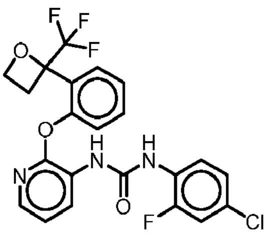 | S29 | 165 |
| 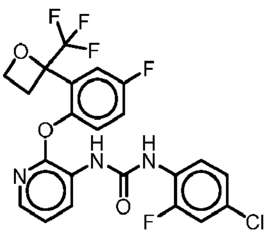 | S30 | 166 |

(continued)

| Structure | ID | IC50(nM) |
|---|---|---|
| 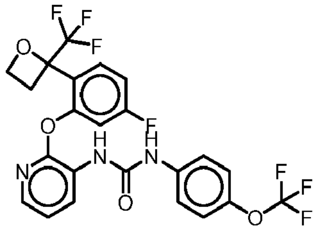 | S31 | 168 |
| 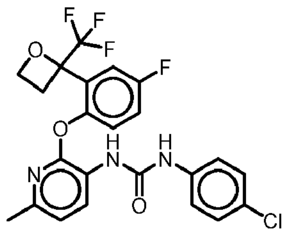 | S32 | 169 |
| 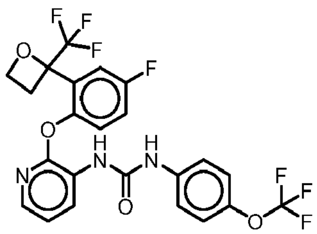 | S33 | 179 |
| 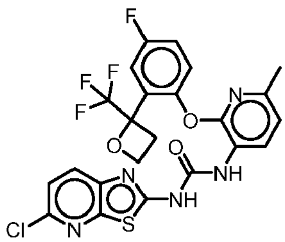 | S34 | 196 |
| 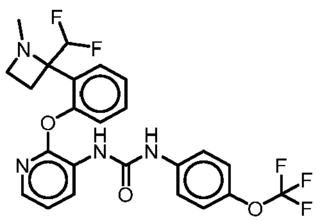 | S35 | 197 |
| 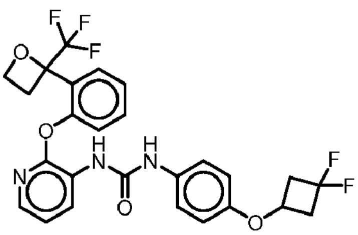 | S36 | 207 |

(continued)

| Structure | ID | IC50(nM) |
|---|---|---|
| 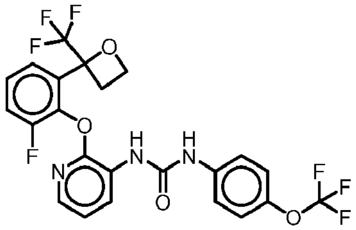 | S37 | 220 |
| 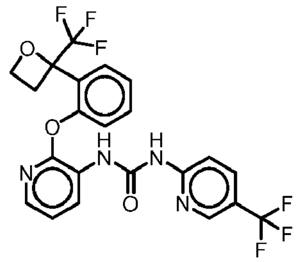 | S38 | 248 |
| 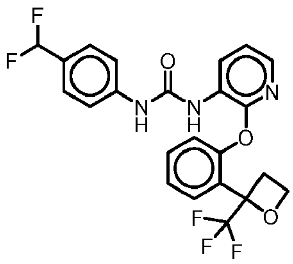 | S39 | 259 |
| 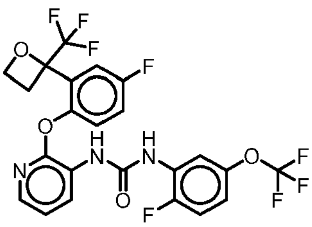 | S40 | 278 |
| 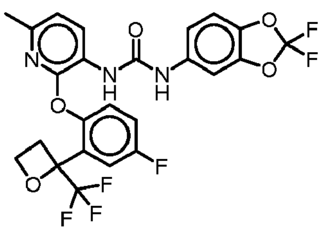 | S41 | 287 |
| 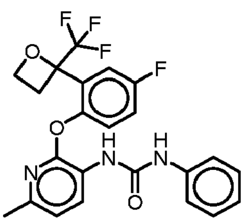 | S42 | 324 |

(continued)

| Structure | ID | IC50(nM) |
|---|---|---|
| 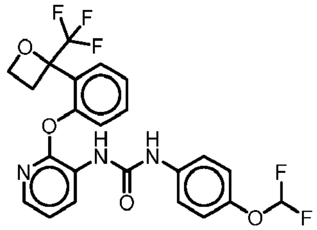 | S43 | 334 |
| 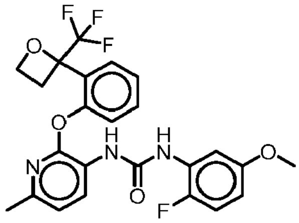 | S44 | 348 |
| 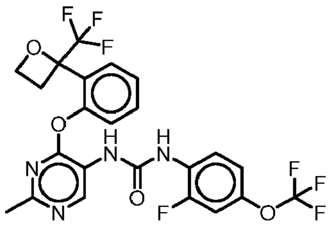 | S45 | 382 |
| 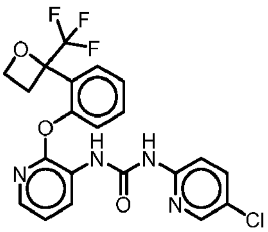 | S46 | 384 |
| 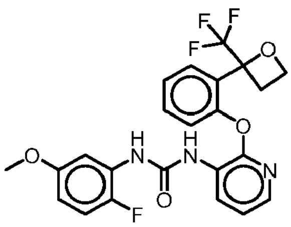 | S47 | 392 |
| 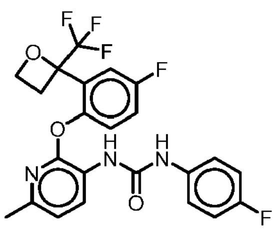 | S48 | 408 |

(continued)

| Structure | ID | IC50(nM) |
|---|---|---|
| 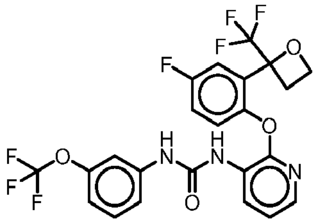 | S49 | 419 |
| 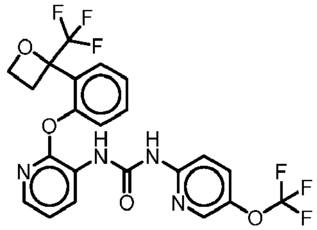 | S50 | 465 |
| 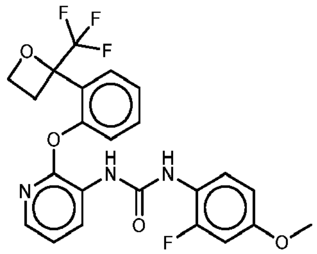 | S51 | 473 |
| 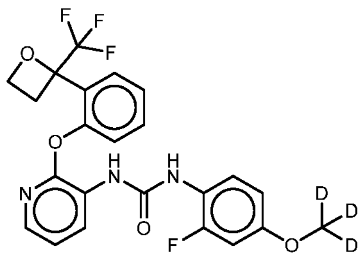 | S52 | 500 |
| 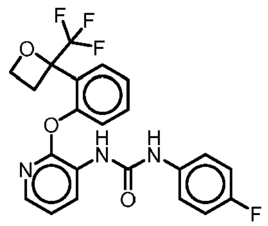 | S53 | 522 |
| 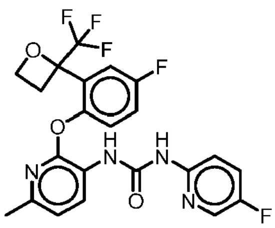 | S54 | 549 |

(continued)

| Structure | ID | IC50(nM) |
|---|---|---|
| 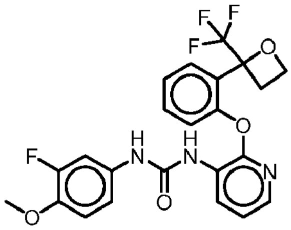 | S55 | 560 |
| 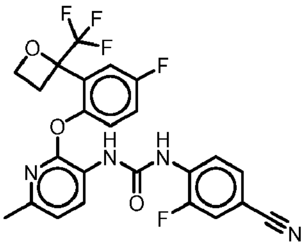 | S56 | 587 |
| 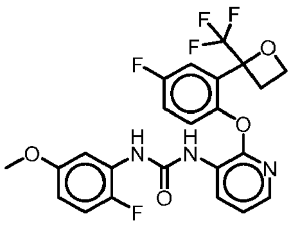 | S57 | 589 |
| 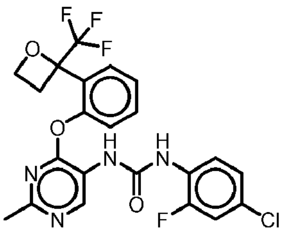 | S58 | 639 |
| 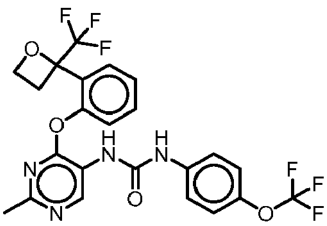 | S59 | 644 |
| 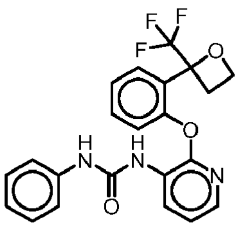 | S60 | 735 |

(continued)

| Structure | ID | IC50(nM) |
|---|---|---|
| 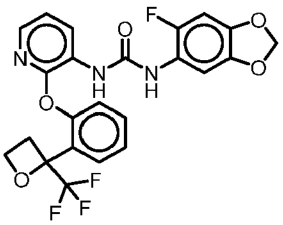 | S61 | 768 |
| 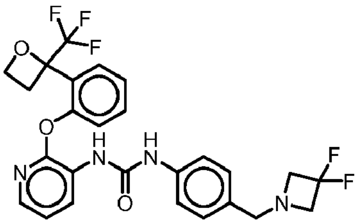 | S62 | 807 |
| 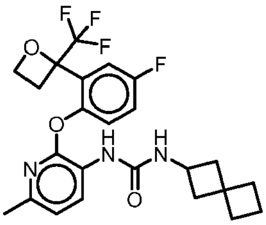 | S63 | 844 |
| 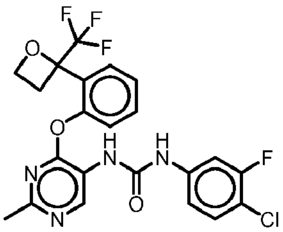 | S64 | 926 |
| 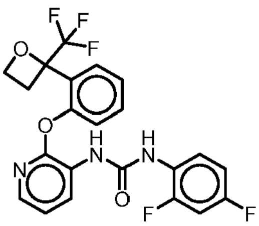 | S65 | 983 |
| 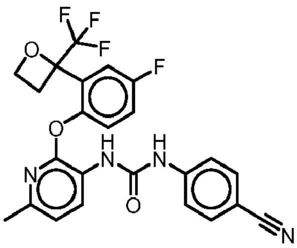 | S66 | 1020 |

(continued)

| Structure | ID | IC50(nM) |
|---|---|---|
| 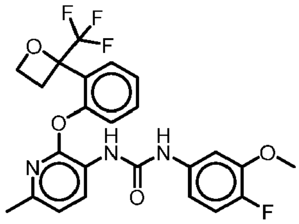 | S67 | 1058 |
| 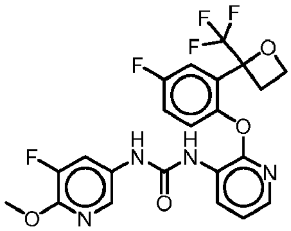 | S68 | 1334 |
| 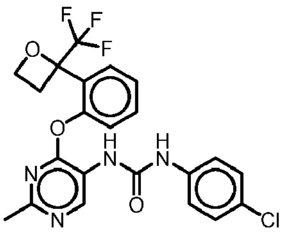 | S69 | 1413 |
| 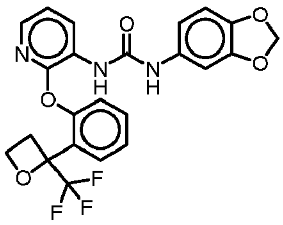 | S70 | 1453 |
| 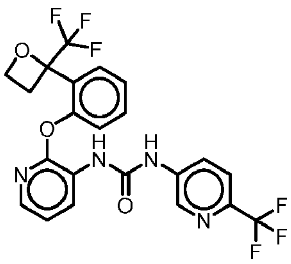 | S71 | 1484 |
| 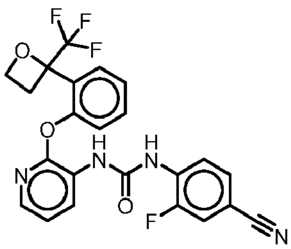 | S72 | 1491 |

(continued)

| **Structure** | **ID** | **IC50(nM)** |
|---|---|---|
| 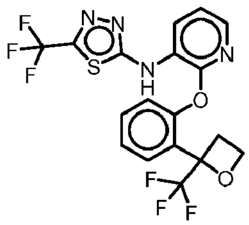 | S73 | 1692 |
| 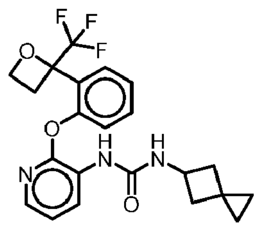 | S74 | 2135 |
| 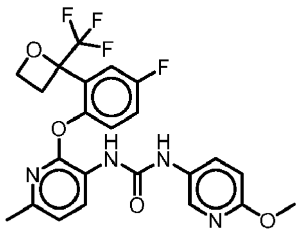 | S75 | 2650 |
| 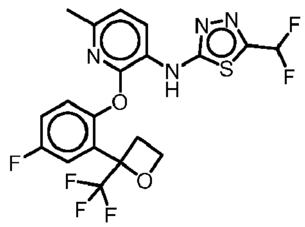 | S76 | 3054 |
| 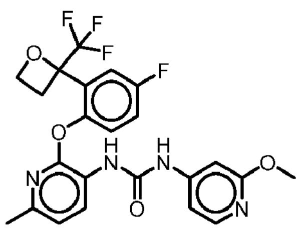 | S77 | 3142 |
| 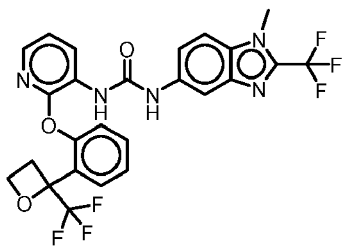 | S78 | 3159 |

(continued)

| Structure | ID | IC50(nM) |
|---|---|---|
| 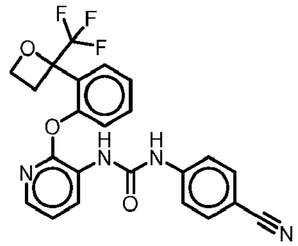 | S79 | 3168 |
| 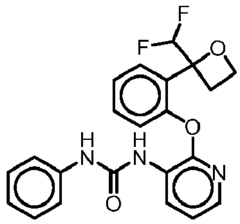 | S80 | 3277 |
| 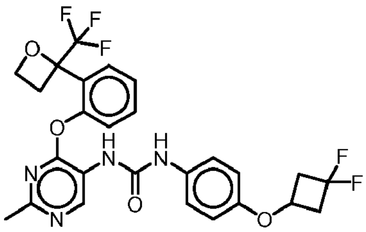 | S81 | 3527 |
| 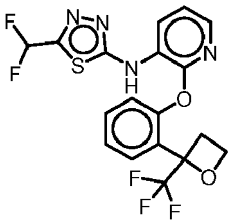 | S82 | 4532 |
| 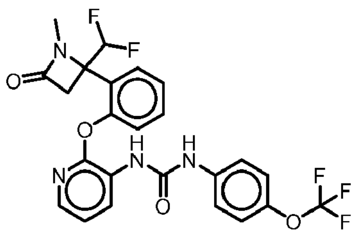 | S83 | 4706 |
| 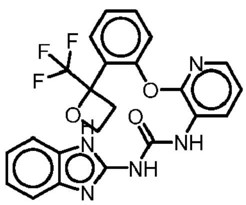 | S84 | 4938 |

(continued)

| Structure | ID | IC50(nM) |
|---|---|---|
| 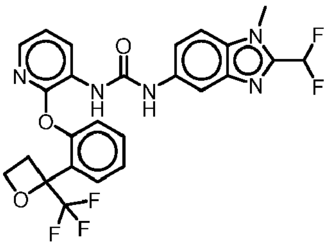 | S85 | 5576 |
| 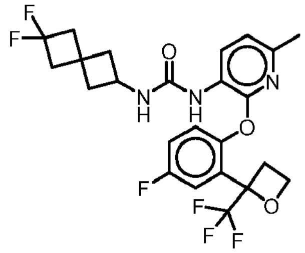 | S86 | 6047 |
| 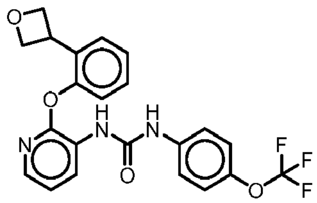 | S87 | 4589 |
| 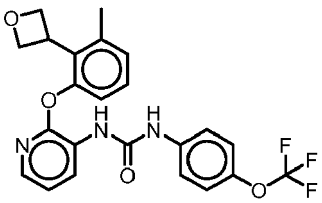 | S88 | 1163 |
| 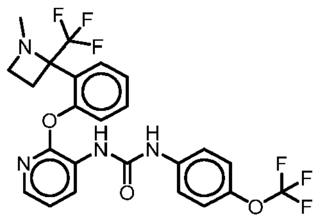 | S89 | 71 |
| 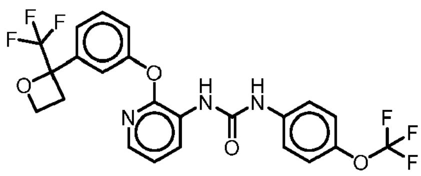 | S90 | 322 |
| 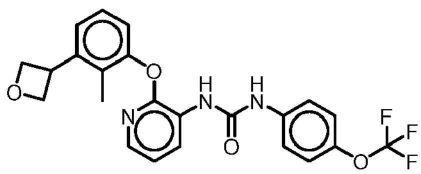 | S91 | 1369 |

(continued)

| Structure | ID | IC50(nM) |
|---|---|---|
| 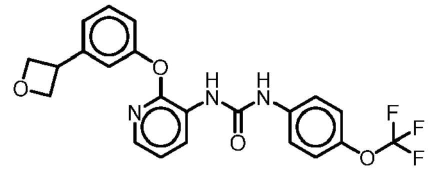 | S92 | 2792 |
| 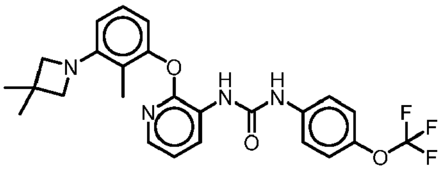 | S93 | 683 |
| 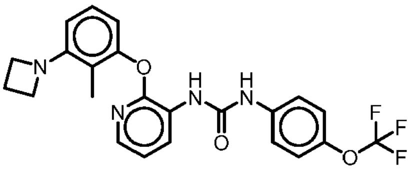 | S94 | 889 |
| 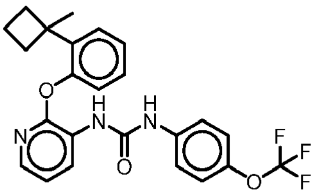 | S95 | 36 |
| 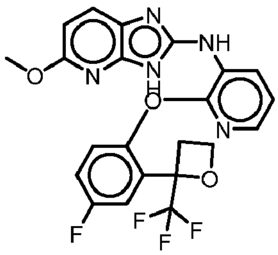 | S96 | ** |
| 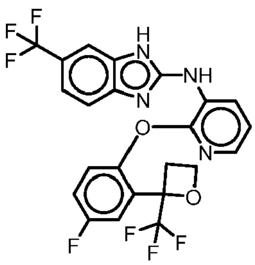 | S97 | 1010 |
| 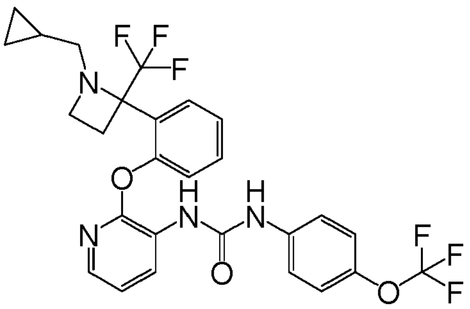 | S116 | 45 |

(continued)

| Structure | ID | IC50(nM) |
|---|---|---|
| 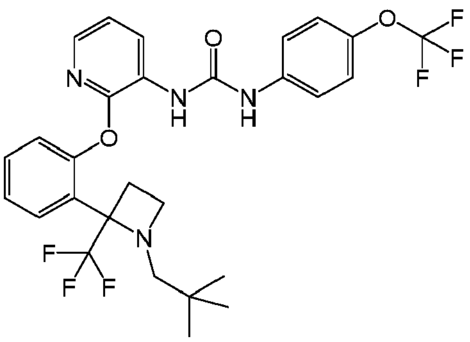 | S117 | 39 |
| 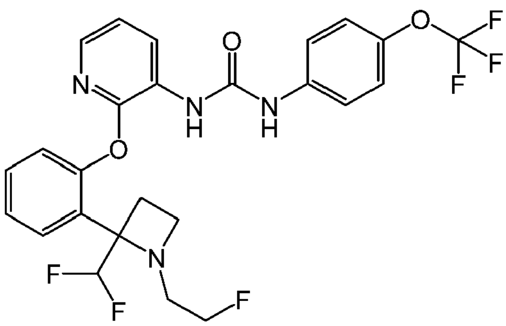 | S118 | 121 |
| 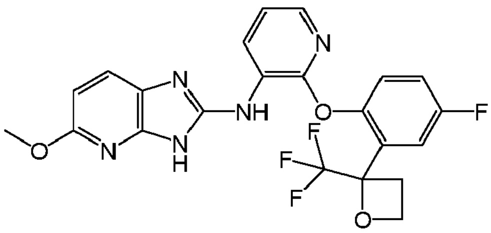 | S119 | 721 |
| 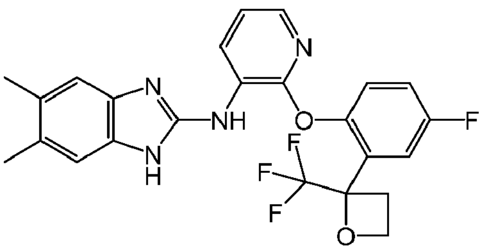 | S120 | 1240 |
| 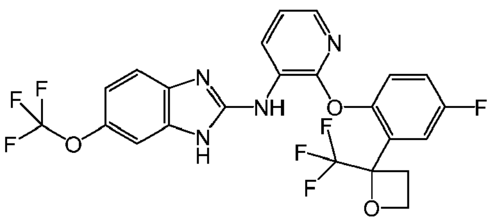 | S121 | 1440 |
| *All values rounded to nearest whole number<br>**Full dose-response curve not generated due to relatively high concentration of compound needed to achieve inhibition under tested conditions - inhibition of 56% measured at maximum tested concentration of 10000 nM | | |

*Results (single enantiomer)*

| Structure | ID | IC50 (nM)* |
|---|---|---|
| 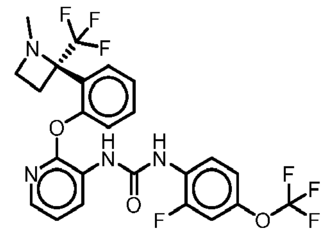 | S98 | 26 |
| 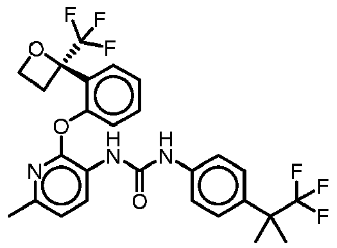 | S99 | 29 |
| 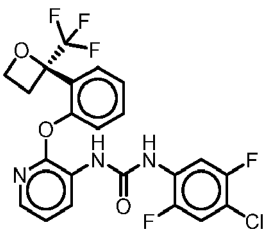 | S100 | 44 |
| 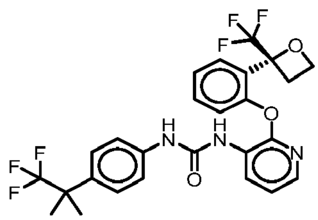 | S101 | 48 |
| 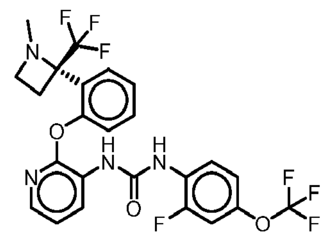 | S102 | 52 |
| 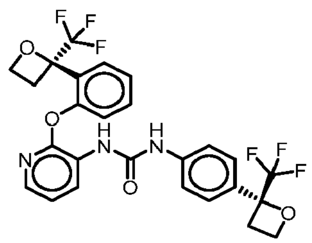 | S103 | 59 |

(continued)

| Structure | ID | IC50 (nM)* |
|---|---|---|
| 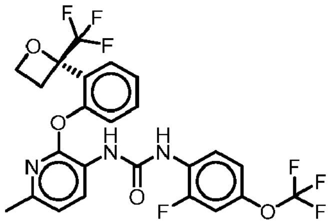 | S104 | 68 |
| 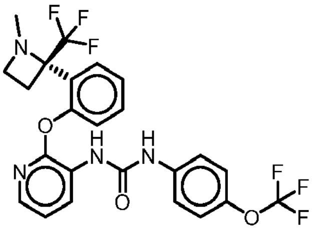 | S105 | 77 |
| 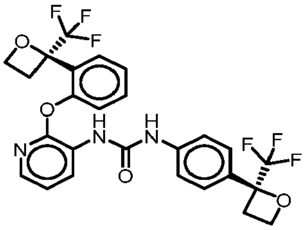 | S106 | 103 |
| 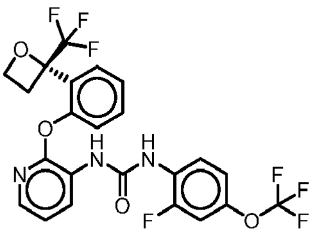 | S107 | 113 |
| 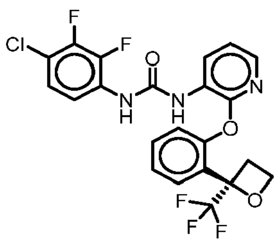 | S108 | 125 |
| 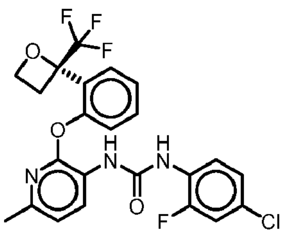 | S109 | 126 |

(continued)

| Structure | ID | IC50 (nM)* |
|---|---|---|
| 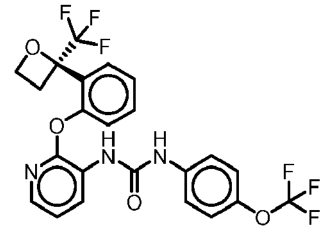 | S110 | 68 |
| 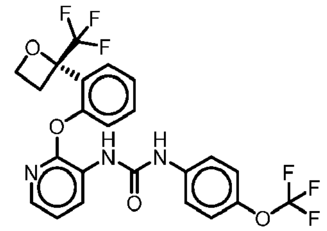 | S111 | 119 |
| 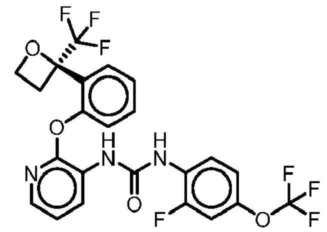 | S112 | 38 |
| 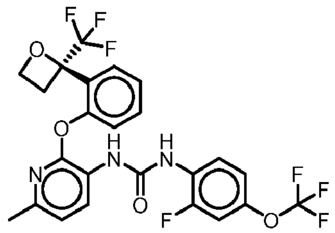 | S113 | 34 |
| 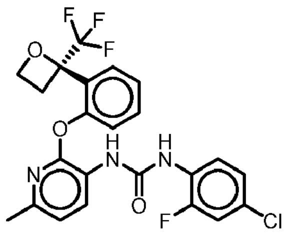 | S114 | 41 |
| 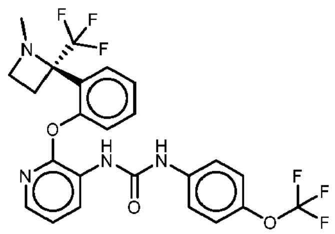 | S115** | 39 |

(continued)

| Structure | ID | IC50 (nM)* |
|---|---|---|
| 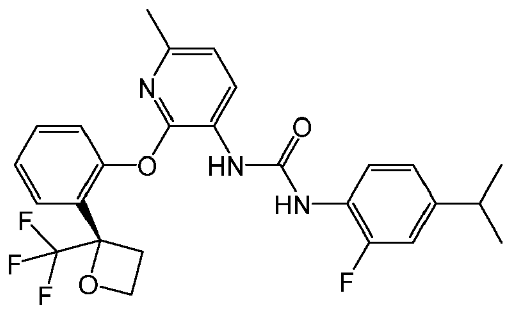 | S122 | 18 |
| 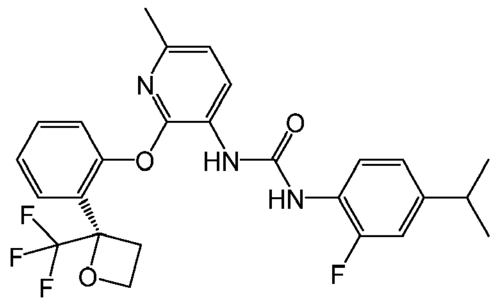 | S123 | 33 |
| 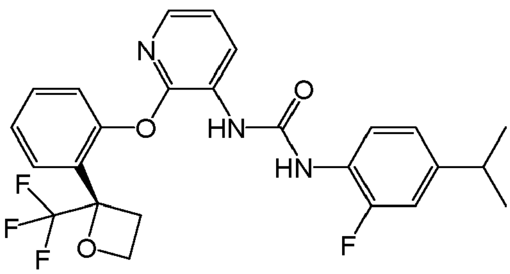 | S124 | 20 |
| *All values rounded to nearest whole number<br>**Absolute configuration not experimentally established, but provisionally assigned as R in line with known oxetanes | | |

*Example 2*

This example compares the *in silico* predicted CNS penetration properties of selected compounds with their observed CNS penetration properties. Also provided are P2Y1 inhibition data for each compound.

*In silico prediction of CNS penetration*

There is a body of modern research aiming to rationalise and predict whether a compound would be CNS penetrant (CNS+). There are a number of predictive models that essentially focus on a core group of molecular and physicochemical properties of the molecules, e.g. size (as measures by mol wt), lipophilicity (clogP, clogD), polarity (TPSA), H-bonding (HBA, HBD), aromatic rings (NAr), rotatable bonds (NRB) and ionisation state (pKa). These models have been constructed through the analysis of large data sets of experimentally measured brain exposures of drug-like molecules in rodent models. These *in silico* models have become adopted as good practice in the design of molecules that require CNS+ as an important feature in their use.

A state-of-the-art model is the CNS multiparameter optimisation (CNS MPO) score (Wager et al., ACS Chem. Neurosci. 2010, 1, 435-449), which combines features of these properties underpinned by a probabilistic analysis (i.e. soft failings are allowed, rather than binary yes/no cut-off). The novel CNS MPO algorithm showed that 74% of marketed CNS drugs display a high CNS MPO score (MPO desirability score ≥ 4, using a scale of 0-6). This analysis suggests that this algorithm could potentially be used to identify compounds with a higher probability of successfully testing hypotheses in the clinic.

However, application of the CNS MPO to the compound 1-(4-(trifluoromethoxy)phenyl)-3-(2-(2-(2-(trifluoromethyl)oxetan-2-yl)phenoxy)pyridin-3-yl)urea (*i.e.* S11):

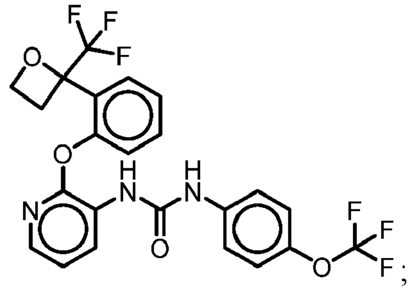

shows a low score, which predicts that it is unlikely to have CNS+:

| CNS MPO Calculator | | |
|---|---|---|
| **Property*** | **Value** | **T0** |
| ClogP | 4.5 | 0.250 |
| ClogD | 4.5 | 0.000 |
| TPSA | 82 | 1.000 |
| MW | 513 | 0.00 |
| HBD | 2 | 0.500 |
| pKa | 0** | 1.000 |
| **CNS MPO** | | **2.8** |
| **calculated using StarDrop™, Optibrium Ltd. Version: 6.6.3.23027*<br>***S11 is a neutral molecule and does not contain a group that would expected to be ionized at physiologically relevant pH. Hence, the pKa value has been set as zero.* | | |

*Method of assessing CNS penetration:*

*Via intravenous administration in cassette dosing format (entries 1 and 2 below)*

To assess penetration of compounds into the mouse central nervous system, intravenous pharmacokinetic studies were undertaken in C57B16/J mice. In each study, up to five compounds were formulated in a vehicle comprising DMSO, Kolliphor EL and HPCD (20% w/v) to a final dose for each compound of 0.4mg/kg. Compounds were then combined into a single dosing solution and administered together via the intravenous route into 24 mice. Total compound dose administered to each mouse was 2mg/kg. Mice were culled at 0.08, 0.25, 0.5, 1, 2, 4, 8 and 24 hours post dosing, and brain and plasma collected for bioanalysis.

For bioanalysis, compounds were prepared in DMSO and aliquoted together to ensure a final concentration of 1mg/mL per compound in DMSO. Analytical standards were prepared from the initial 1mg/mL stock solution of compound, with a 12-point standard curve range from 0.5ng/mL to 2000ng/mL, with independent quality control tests from 1ng/mL to 1000ng/mL run to validate the standards. All standards and QC samples were matrix matched to eliminate possible suppression. Plasma samples were prepared with a 1:10 dilution in plasma. Brain tissue samples were homogenized in 0.1M phosphate buffer solution at a ratio of 1:4, and used without further dilution. Dose solutions were diluted in a control matrix to a relative concentration that fell on the standard curve. Cold acetonitrile containing internal standard was added to all samples, standards, dose samples and QC samples; samples were then centrifuged for 30 minutes at 4000rpm. After centrifugation, supernatant was transferred to a new 96-well plate, diluted with an equal volume of water and mixed thoroughly before LC-MS analysis.

Supernatents were analysed by LC-MS/MS. From the analysis data a response ratio was obtained using the formula Response Ratio = Test Peak Area/Internal Standard Peak Area. From the standard curve, the test peak area was converted to concentration of test compound.

To establish CNS penetration, the ratio of the concentration of each compound in the plasma samples to the brain samples was assessed.

*Via single compound oral or intravenous administration (entries 3 to 9 below)*

To assess pharmacokinetic properties and CNS penetration of compounds, compounds were administered to mice via the oral and intravenous routes. For oral dosing, compound solutions were prepared in a suitable vehicle (such as 10% PEG400/5% Kolliphor EL/85% $H_2O$) at a concentration of 0.5mg/mL. Compounds were dosed via oral gavage at a dosing volume of 10mL/kg to C57B16/J mice, for a final dose of 5mg/kg. Animals were culled at 8 timepoints to 24 hours, and plasma and brain samples collected for bioanalysis.

For intravenous dosing, compound solutions were prepared in a suitable vehicle (such as 10% DMSO/90%HPCD (20%)) at a concentration of 0.2mg/mL. Compound was administered via the intravenous route in a dosing volume of 5mL/kg, for a final dose of 1mg/kg. Animals were culled at various timepoints out to 24 hours, and plasma and brain collected for bioanalysis.

For bioanalysis, compound was prepared in DMSO at a final concentration of 1mg/mL. Analytical standards were prepared from the initial 1mg/mL stock solution of compound, with a 12-point standard curve range from 0.5ng/mL to 2000ng/mL, with independent quality control tests from 1ng/mL to 1000ng/mL run to validate the standards. All standards and QC samples were matrix matched to eliminate possible suppression. Plasma samples were prepared with a 1:10 dilution in plasma. Brain tissue samples were homogenized in 0.1M phosphate buffer solution at a ratio of 1:4, and used without further dilution. Dose solutions were diluted in a control matrix to a relative concentration that fell on the standard curve. Cold acetonitrile containing internal standard was added to all samples, standards, dose samples and QC samples; samples were then centrifuged for 30 minutes at 4000rpm. After centrifugation, supernatant was transferred to a new 96-well plate, diluted with an equal volume of water and mixed thoroughly before LC-MS analysis.

Supernatents were analysed by LC-MS/MS. From the analysis data a response ratio was obtained using the formula Response Ratio = Test Peak Area/Internal Standard Peak Area. From the standard curve, the test peak area was converted to concentration of test compound.

To establish CNS penetration, the ratio of the concentration of each compound in the plasma samples to the brain samples was assessed.

Measurement of IC50 values:

As for example 1 above.

*Results:*

| Entry | Compound | IC50[a] (nM) | MPO[b] (0-6) | CNS penetration[c] |
|---|---|---|---|---|
| 1[f] | 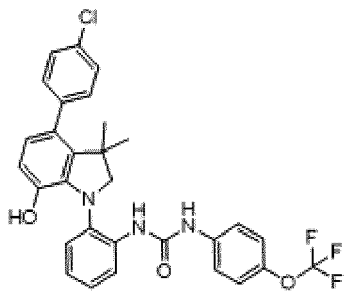 | 33 | 2.2 | BLLQ[d] iv |
| 2[g] | 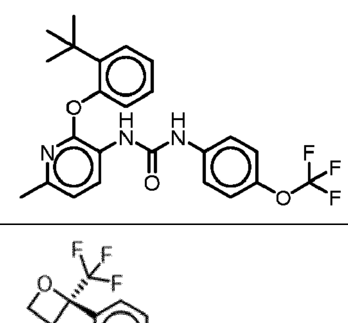 | 36 | 2.8 | BLLQ[d] iv |
| 3 | 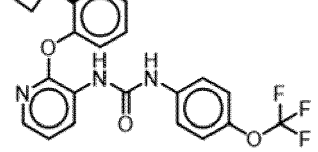 | 68 | 2.8 | 2.6 po<br>3.3 $AUC_{0-24h}$ iv<br>2.8 @ t =2 h iv |

(continued)

| Entry | Compound | IC50[a] (nM) | MPO[b] (0-6) | CNS penetration[c] |
|---|---|---|---|---|
| **4** | 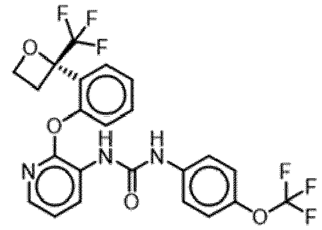 | 119 | 2.8 | 2.4 po<br>3.1 $AUC_{0-24h}$ iv<br>3.2 @ t =2 h iv |
| **5** | 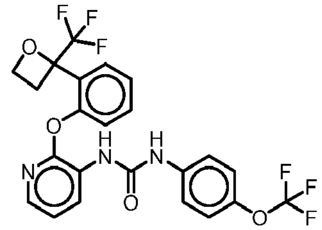 | 84 | 2.8 | 1.9 po<br>2.1 $AUC_{0-24h}$ iv<br>1.7 @ t =2 h iv |
| **6** | 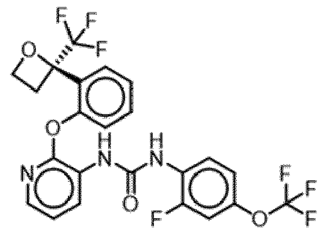 | 38 | 2.7 | 4.3 po |
| **7** | 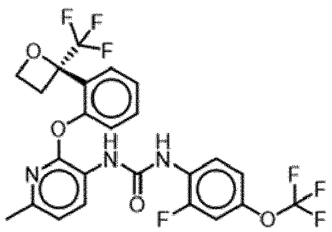 | 34 | 2.6 | 1.7 po |
| **8** | 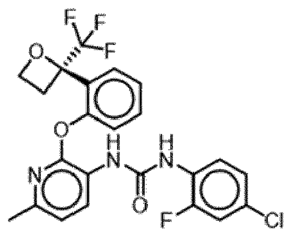 | 41 | 2.5 | 1.3 po |

(continued)

| Entry | Compound | IC50[a] (nM) | MPO[b] (0-6) | CNS penetration[c] |
|---|---|---|---|---|
| 9[e] | 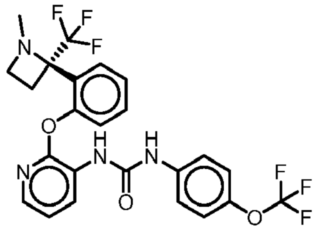 | 39 | 2.9 | 2.4 po (value at t = 2h) |
| [a] All values rounded to nearest whole number<br>[b] All values presented to two significant figures<br>[c] iv (0.4 mg/kg): total brain to plasma ratio (mouse) at t = 2 hr: p.o (5 mg/kg): total brain to plasma ratio (mouse) AUC 0-24 h; all values presented to two significant figures<br>[d] BLLQ = below lower limit of quantification<br>[e] Single enantiomer of unknown absolute configuration; but provisionally assigned as R in line with known oxetanes.<br>[f] Detailed in WO 2014/022343 and Yang et al., J. Med. Chem. 2014 57(14) 6150-6164; identified herein as compound RS 1<br>[g] Detailed in Peng et al., European Journal of Medicinal Chemistry 158 (2018) 302-310; identified herein as compound RS2 | | | | |

For the avoidance of doubt, it is noted that whether a compound is administered via an intravenous route or an oral route cannot be expected to have any significant effect on the CNS penetration properties as determined herein (i.e. as a ratio of the concentration of the compound in the brain relative to the concentration of the compound in the plasma). Thus, substantially equivalent CNS penetration results would be achieved for each compound regardless of the choice between the two administration modes used herein. This is evidenced by the above entries 3-5, which demonstrate consistent results when measured for their CNS penetration following administration via both an intravenous route or an oral route. These data further demonstrates that consistent (and therefore comparable) results are achieved when CNS penetration is measured at a fixed time point (i.e. 2 hours) or as $AUC_{0-24}$.

**Compound synthesis**

Detailed procedures for the synthesis of selected exemplary compounds of the invention are provided below. Other compounds of the invention and any reference compounds can routinely be synthesised by those skilled in the art, applying common general knowledge in the field of organic synthesis and with reference to the comparable synthetic steps indicated for the selected compounds discussed below.

**General procedure for preparation of phenols**

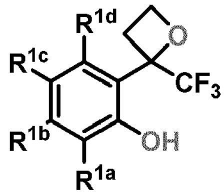

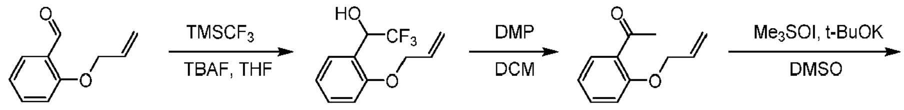


1 2 3

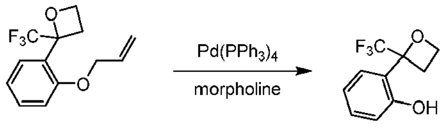


4 5

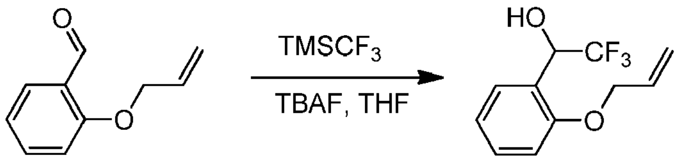


1 2

To a solution of compound **1** (60 g, 370 mmol, 1.0 eq.) in THF (1200 mL) was added $TMSCF_3$ (131.51 g, 925 mmol, 2.5 eq.) and TBAF (1.0 M, 925 mL, 2.5 eq). The mixture was stirred at 25 °C for 12 hr. LC-MS showed compound **1** was fully consumed and the desired mass was detected. The reaction mixture was quenched with water (1000 mL) and then diluted with ethyl acetate (300 mL). The aqueous layer was further extracted with ethyl acetate (300 mL x3), the organics combined, dried over $Na_2SO_4$, filtered and the filtrate concentrated under reduced pressure to give a residue. The residue was purified by column chromatography ($SiO_2$, petroleum ether/ethyl acetate = 20/ 1 to 2/ 1) to give compound **2** (160 g, 94%) as a yellow solid. TLC: petroleum ether: ethyl acetate = 10:1, $R_f$ = 0.49; LC-MS: m/z 231.0 $[M-H]^-$; $^1H$ NMR (400MHz, $CDCl_3$) $\delta$ = 7.48 (d, *J* = 7.5 Hz, 1H), 7.44 - 7.32 (m, 1H), 7.06 (t, *J* = 7.5 Hz, 1H), 6.97 (d, *J* = 8.3 Hz, 1H), 6.19 - 5.97 (m, 1H), 5.52 - 5.26 (m, 3H), 4.63 (d, *J* = 5.1 Hz, 2H), 4.11 - 4.01 (m, 1H).

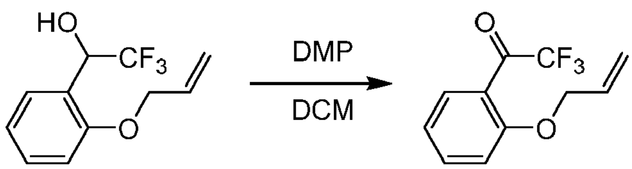


2 3

To a solution of compound **2** (160 g, 689 mmol, 1.0 eq.) in DCM (600 mL) then DMP (350.7 g, 827 mmol, 256 mL, 1.2 eq.) was added at 0°C. The mixture was stirred at 20 °C for 12 hr. LC-MS showed compound **2** was fully consumed and the desired mass was detected. The reaction mixture was concentrated under reduced pressure to give a crude residue. The residue was purified by column chromatography ($SiO_2$, petroleum ether/ ethyl acetate = 30/ 1 to 3/ 1) to give compound **3** (140 g, 93%) as a yellow oil. TLC: petroleum ether: ethyl acetate = 5:1, $R_f$ = 0.65; LC-MS: m/z 231.0 $[M+H]^+$; $^1H$ NMR (400MHz, $CDCl_3$) $\delta$ = 7.70 (d, *J* = 7.70 Hz, 1H), 7.56 - 7.63 (m, 1H), 7.00 - 7.13 (m, 2H), 6.00 - 6.15 (m, 1H), 5.49 (dd, *J* = 17.24, 1.22 Hz, 1H), 5.36 (dd, *J* = 10.57, 0.92 Hz, 1H), 4.69 (d, *J* = 5.13 Hz, 2H).

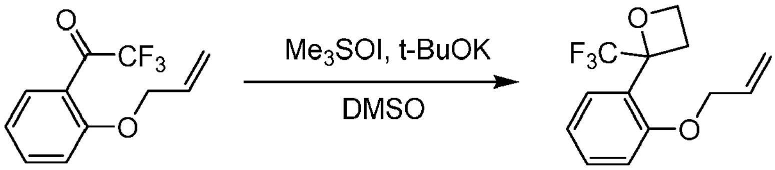


3 4

Two parallel reactions:

To a solution of trimethylsulfoxonium iodide (172.1 g, 781.99 mmol, 4.0 eq) in DMSO (750 mL) was added t-BuOK (87.75 g, 781.99 mmol, 4.0 eq.) at 20 °C. The mixture was stirred at 20°C for 10 min, and then a solution of compound **3** (45 g, 195.50 mmol, 1.0 eq.) in DMSO (450 mL) was added. The resulting mixture was stirred at 30 °C for 12 hr. TLC indicated compound **4** was consumed completely and one new spot had formed. The reaction mixture was quenched with $H_2O$ (1200 mL) at 0 °C, and then extracted with ethyl acetate (600 mL x4). The combined organic layers were dried over $Na_2SO_4$, filtered and the filtrate concentrated under reduced pressure to give a residue. The combined residues of both reactions were purified by column chromatography ($SiO_2$, petroleum ether/ ethyl acetate = 50/1 to 5/ 1) to give compound **4** (75.0 g, 75%) as a yellow oil. TLC: petroleum ether: ethyl acetate = 10:1. $R_f$ = 0.47; LC-MS: m/z 257.1 $[M-H]^-$; $^1H$ NMR (400MHz, $CDCl_3$) $\delta$ = 7.38 (dd, *J* = 7.63, 1.38 Hz, 1H), 7.16 - 7.27 (m, 1H), 6.86 - 6.96 (m, 1H), 6.79 (d, *J* = 8.25 Hz, 1H), 5.82 - 5.96 (m, 1H), 5.27 (dd, *J* = 17.32, 1.44 Hz, 1H), 5.11 - 5.18 (m, 1H), 4.70 (td, *J* = 7.91, 5.82 Hz, 1H), 4.37 - 4.46 (m, 3H), 3.02 - 3.12 (m, 2H).

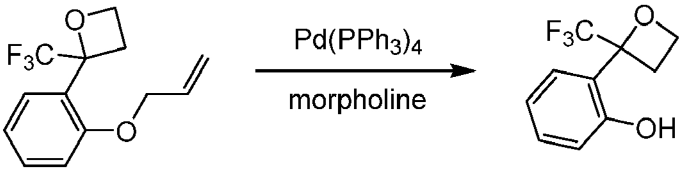


4 5

Two parallel reactions:

To a solution of Compound **4** (25 g, 96.81 mmol, 1.0 eq.) in DMF (200 mL) was added morpholine (9.28 g, 106.49 mmol, 9.37 mL, 1.1 eq.) and $Pd(PPh_3)_4$ (6.15 g, 5.32 mmol, 0.05 eq.). The mixture was stirred at 80 °C for 3hr. LC-MS showed compound **2** was fully consumed and the desired mass was detected. The reaction mixture was quenched with water (200 mL) diluted with ethyl acetate (200 mL) and the aqueous phase further extracted with ethyl acetate (200 mL x3). The combined organics were dried over $Na_2SO_4$, filtered and the filtrate was concentrated under reduced pressure to give a residue. The combined residues from the two reactions were purified by column chromatography ($SiO_2$, petroleum ether/ ethyl acetate = 20/1 to 3/1) to give compound 5 (40.0 g, 70 %) as a yellow oil. TLC: petroleum ether: ethyl acetate = 5:1. $R_f$ = 0.41; LC-MS: m/z 217.0 $[M-H]^-$; $^1H$ NMR (400MHz, $CDCl_3$) $\delta$ = 7.68 (br s, 1H), 7.14 - 7.26 (m, 1H), 6.78 - 6.93 (m, 3H), 4.73 - 4.91 (m, 2H), 3.25 (ddd, *J* = 11.83, 8.88, 6.53 Hz, 1H), 2.91 - 3.04 (m, 1H).

**General procedure A for the synthesis of 2-phenoxy-3-nitro(heteroarene) derivatives**

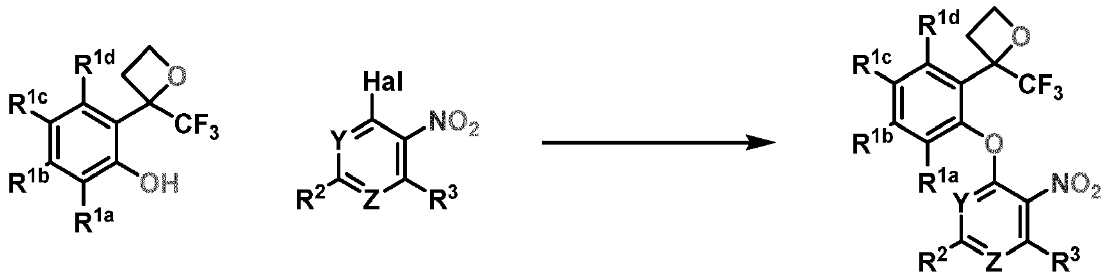

A solution containing an appropriate phenol (1.0 eq) and 2-halo-3-nitro(hetero)arene (1.1 eq.), base (1.1 eq.) in anhydrous solvent (0.1-0.5 M) was stirred at temperatures between 25-150 °C until LCMS analysis suggested the reaction had reached completion. The reaction was cooled, solvent removed *in vacuo* and the residue partitioned between ethyl acetate and water. The aqueous phase was further extracted with ethyl acetate (x3) and the organic phases then combined and washed with brine, dried over sodium sulfate, filtered and concentrated *in vacuo* to give a crude residue. The residue was either used without further purification, purified by flash-chromatography or recrystallized from an appropriate solvent.

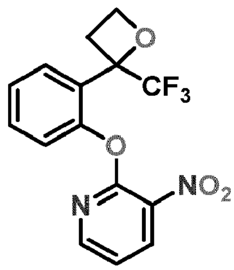

3-nitro-2-(2-(2-(trifluoromethyl)oxetan-2-yl)phenoxy)pyridine

A solution containing 2-[2-(trifluoromethyl)oxetan-2-yl]phenol (30.0 g, 137.51 mmol, 1.0 eq.), 2-fluoro-3-nitropyridine (39.08 g, 275.01 mmol, 2.0 eq.) and potassium carbonate (57.01 g, 412.52 mmol, 3.0 eq.) in N,N-dimethylformamide (300 mL), was stirred at 100 °C for 12 h. LC-MS showed the phenol was fully consumed and the desired mass was detected. The reaction mixture was quenched with water (500 mL), then diluted with ethyl acetate (300 mL). The aqueous layer was extracted with ethyl acetate (300 mL x3), the organic layers combined, dried over $Na_2SO_4$, filtered, and the filtrate concentrated under reduced pressure to give a residue. The residue was purified by column chromatography ($SiO_2$, petroleum ether/ethyl acetate = 20/1 to 1/1) to give the title compound as a yellow oil (57.0 g, 95%). TLC: petroleum ether: ethyl acetate = 2:1. $R_f$ = 0.66; LC-MS: m/z 341.0 $[M+H]^+$; $^1H$ NMR (400MHz, $CDCl_3$) $\delta$ = 8.35 - 8.44 (m, 2H), 7.68 (dd, *J* = 7.82, 1.22 Hz, 1H), 7.44 - 7.53 (m, 1H), 7.34 - 7.42 (m, 1H), 7.17 - 7.24 (m, 2H), 4.85 (td, *J* = 7.92, 5.68 Hz, 1H), 4.54 - 4.64 (m, 1H), 3.28 (t, *J* = 7.76 Hz, 2H).

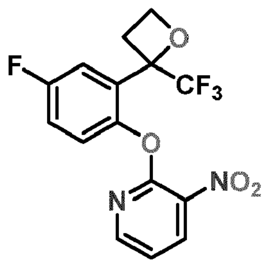

2-(4-fluoro-2-(2-(trifluoromethyl)oxetan-2-yl)phenoxy)-3-nitropyridine

Prepared according to general procedure **A.**

A solution containing 4-fluoro-2-[2-(trifluoromethyl)oxetan-2-yl]phenol (650 mg, 2.75 mmol), 2-chloro-3-nitropyridine (458 mg, 2.89 mmol) and caesium carbonate (897 mg, 2.75 mmol) in *anhydrous* N,N-dimethylformamide (15 mL), under argon, was stirred at 50 °C for 16 h. The reaction was cooled and the solvent removed *in vacuo.* The residue

was dissolved in the minimum volume of DMSO and purified by reverse-phase chromatography using a Biotage Isolera one 3.0 with 25 g C18 KP-SIL SNAP column linear gradient 30% - 95% acetonitrile in water (0.1% formic acid modifier) to give the title compound as a tan solid (777 mg, 89%). UPLC-MS: m/z 359.2 $[M+H]^+$; $^1H$ NMR (400 MHz, DMSO-$d_6$) $\delta$ = 8.59 (dd, *J* = 1.83, 8.07 Hz, 1H), 8.45 (dd, *J* = 1.83, 4.77 Hz, 1H), 7.36 - 7.48 (m, 3H), 7.28 (dd, *J* = 2.75, 8.99 Hz, 1H), 4.70 (dt, *J* = 5.87, 7.89 Hz, 1H), 4.52 (td, *J* = 5.87, 9.54 Hz, 1H), 3.10 - 3.19 (m, 1H), 2.89 - 3.09 (m, 1H).

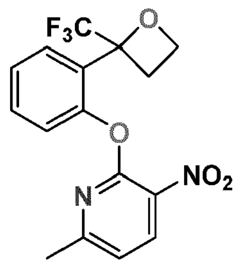

6-methyl-3-nitro-2-(2-(2-(trifluoromethyl)oxetan-2-yl)phenoxy)pyridine

To a solution of compound 2-[2-(trifluoromethyl)oxetan-2-yl]phenol (1.7 g, 7.79 mmol, 1.0 eq.) in DMF (20mL) was added $K_2CO_3$ (3.23 g, 23.37mmol, 3.0 eq.) and 2-fluoro-6-methyl-3-nitro-pyridine (2.43 g, 15.58 mmol, 2.0 eq.). The mixture was stirred at 100 °C for 12 hr LC-MS showed the phenol was fully consumed and the desired mass was detected. The reaction mixture wad diluted with $H_2O$ (30 mL) at 25°C, and then with EtOAc (30 mL). The aqueous phase was extracted with EtOAc (20 mL x3), dried over $Na_2SO_4$, filtered and the filtrate concentrated under reduced pressure to give a residue. The residue was purified by MPLC ($SiO_2$, petroleum ether: ethyl acetate= 15:1 to 5:1) to give the title material (1.8 g, 65%) as a yellow solid. LC-MS: m/z 355.1 $[M+H]^+$; $^1H$ NMR (400MHz, $CDCl_3$) $\delta$ = 8.28 (d, *J* = 8.2 Hz, 1H), 7.64 (d d, *J* = 1.2, 7.7 Hz, 1H), 7.45 - 7.38 (m, 1H), 7.35 - 7.29 (m, 1H), 7.16 (d d, *J* = 0.7, 8.2 Hz, 1H), 7.00 (d, *J* = 8.1 Hz, 1H), 4.83 (d t, *J* = 5.8, 7.9 Hz, 1H), 4.58 (td, *J* = 6.1, 8.7 Hz, 1H), 3.34 - 3.17 (m, 2H), 2.44 (s, 3H).

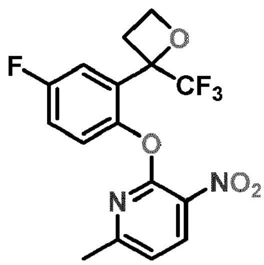

2-(4-fluoro-2-(2-(trifluoromethyl)oxetan-2-yl)phenoxy)-6-methyl-3-nitropyridine

Prepared according to general procedure **A.**

A solution containing 4-fluoro-2-[2-(trifluoromethyl)oxetan-2-yl]phenol (1000 mg, 1.0 eq.), 2-fluoro-6-methyl-3-nitropyridine (694 mg, 1.1 eq.) and caesium carbonate (1518 mg, 1.1 eq.) in *anhydrous* N,N-dimethylformamide (15 mL), under argon, was stirred at 50 °C for 16 h. The reaction was cooled and the solvent removed *in vacuo.* The residue was dissolved in the minimum volume of DMSO and purified by reverse-phase chromatography using a Biotage Isolera one 3.0 with 25 g C18 KP-SIL SNAP column linear gradient 30% - 95% acetonitrile in water (0.1% formic acid modifier) to give the title compound as a tan solid (1309 mg, 83%). UPLC-MS: m/z 373.3 $[M+H]^+$; $^1H$ NMR (400 MHz, $CDCl_3$) $\delta$ = 8.29 (d, J = 8.16 Hz, 1H), 7.37 (dd, J = 2.89, 9.03 Hz, 1H), 7.09 - 7.20 (m, 2H), 7.02 (d, J = 8.07 Hz, 1H), 4.83 (dt, J = 5.59, 7.98 Hz, 1H), 4.60 (dt, J = 5.69, 7.61 Hz, 1H), 3.26 (t, J = 7.79 Hz, 2H), 2.46 (s, 3H).

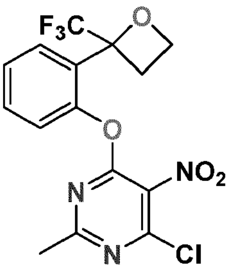

4-chloro-2-methyl-5-nitro-6-(2-(2-(trifluoromethyl)oxetan-2-yl)phenoxy)pyrimidine

Prepared according to general procedure **A.**

To a solution of 2-[2-(trifluoromethyl)oxetan-2-yl]phenol (1.80 g, 8.25 mmol, 1.0 eq.) in DMF (20 mL) was added $K_2CO_3$ (2.28 g, 16.50 mmol, 2 .0eq.) and 4,6-dichloro-2-methyl-5-nitro-pyrimidine (2.06 g, 9.90 mmol, 1.2 eq.). The mixture was stirred at 20 °C for 5 hr. LC-MS showed the phenol was fully consumed and the desired mass was detected. The reaction mixture was diluted with $H_2O$ (20 mL), and then extracted with DCM (20 mL). The combined organic layers were dried over $Na_2SO_4$, filtered, and the filtrate concentrated under reduced pressure to give the title compound as a yellow solid (crude 2.0 g) that was used directly in the next reaction step. LC-MS: m/z 390.1 $[M+H]^+$

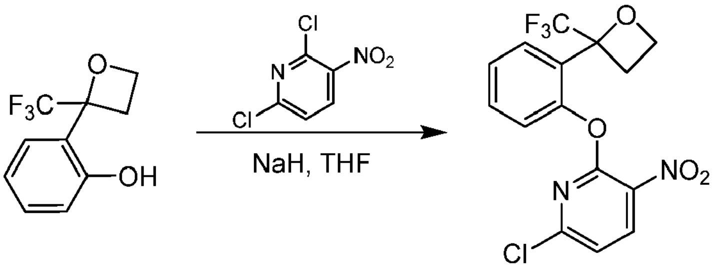


**3** **8**

To a solution of 2-[2-(trifluoromethyl)oxetan-2-yl]phenol (1000 mg, 4.58 mmol, 1.0 eq.) in THF (10 mL) was added sodium hydride (275 mg, 6.88 mmol, 60% wt., 1.5 eq.) at 0 °C and the reaction stirred for 5 min. 2,6-dichloro-3-nitro-pyridine (885 mg, 4.58 mmol, 1.0 eq.) was added and the mixture stirred at 20 °C for 2 hr. LC-MS showed the phenol was fully consumed and the desired mass was detected. The reaction mixture was quenched with $H_2O$ (20 mL) at 20°C, extracted with EtOAc (20 mL x3), dried over $Na_2SO_4$, filtered and the filtrate concentrated under reduced pressure to give a residue. The residue was purified by column chromatography ($SiO_2$, Petroleum ether/ Ethyl acetate = 1/ 0 to 3/ 1). To give compound **8** (1.28 g, 75%) as yellow oil. TLC (petroleum ether: ethyl acetate = 10:1, $R_f$ = 0.45); LC-MS: m/z 375.1 $[M+H]^+$.

**General Procedures for nitro reduction**

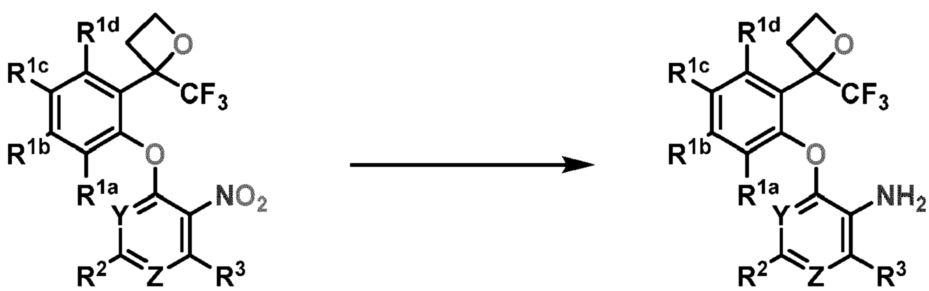

**General procedure 1 nitro reduction**

To a stirred solution of a representative nitro-(hetero)arene (1.0 eq) in methanol (0.27 M), under nitrogen, was added Pd/C (10% wt. 0.05-0.1 eq.). The mixture was degassed and purged with $H_2$ (15 psi, x3) and stirred at 25-40°C until such time that LC-MS showed complete conversion of the nito-(hetero)arene to the aniline. The reaction mixture was purged with nitrogen, filtered and the filtrate concentrated under reduced pressure. The target material was either used directly or purified by flash chromatography.

**General procedure 2 nitro reduction**

To a stirred solution of a representative nitro-(hetero)arene (1.0 eq) in tetrahydrofuran (0.27 M), under argon, in a flaks fitted with water-less condenser, was added zinc powder (5.0-10.0 eq.) followed by a solution of ammonium chloride in water (5.0-10.0 eq, 4.0 M). The reaction mixture was heated at 60 °C with rapid stirring until such time that UPLC-MS showed complete conversion of the nito-(hetero)arene to the aniline. If the reaction had not gone to completion within 8 h, then a second portion of zinc (5.0-10.0 eq.) and ammonium chloride (5.0-10.0 eq.) were added, and the reaction mixture stirred overnight. The reaction mixture was allowed to cool to room temperature, diluted with water and the biphasic mixture passed through a pad of Celite, eluting with ethyl acetate. The solution was concentrated *in vacuo,* the residue partitioned between water and dichloromethane, and the organic phase passed through a hydrophobic frit. The filtrate was concentrated *in vacuo* and the target material either used directly or purified by flash chromatography.

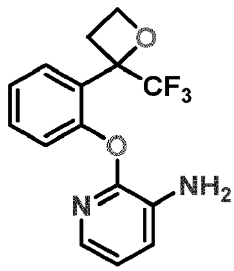

2-(2-(2-(trifluoromethyl)oxetan-2-yl)phenoxy)pyridin-3-amine

Prepared according to the general procedure **1** using 3-nitro-2-(2-(2-(trifluoromethyl)oxetan-2-yl)phenoxy)pyridine (28.0 g, 1.0 eq.) and Pd/C (10%wt., 10.0 g, 0.1 eq.) to give the title material as a white solid (25.0 g, 97%). LC-MS: m/z 311.2 $[M+H]^+$; $^1H$ NMR (400 MHz, $CDCl_3$) $\delta$ = 7.59 - 7.44 (m, 2H), 7.30 (ddd, *J* = 1.8, 7.4, 8.2 Hz, 1H), 7.22 - 7.08 (m, 1H), 6.99 (dd, *J* = 0.9, 8.3 Hz, 1H), 6.95 (dd, *J* = 1.6, 7.6 Hz, 1H), 6.79 (dd, *J* = 4.9, 7.6 Hz, 1H), 4.72 (dt, *J* = 5.6, 7.9 Hz, 1H), 4.45 (td, *J* = 5.9, 9.0 Hz, 1H), 3.83 (br. s., 2H), 3.18 - 2.90 (m, 2H).

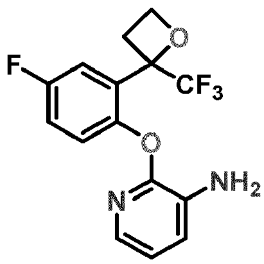

2-(4-fluoro-2-(2-(trifluoromethyl)oxetan-2-yl)phenoxy)pyridin-3-amine

Prepared according to the general procedure **2** using 2-(4-fluoro-2-(2-(trifluoromethyl)oxetan-2-yl)phenoxy)pyridin-3-amine (870 mg, 1.0 eq.) to give the title material as an off-white solid (650 mg, 82%). UPLC-MS: m/z 329.1 $[M+H]^+$; $^1H$ NMR (400 MHz, $CDCl_3$) $\delta$ = 7.59 (d, J = 4.40 Hz, 1H), 7.31 - 7.35 (m, 1H), 6.97 - 7.21 (m, 3H), 6.91 (dd, J = 4.77, 7.70 Hz, 1H), 4.74 - 4.90 (m, 1H), 4.56 (td, J = 5.87, 9.17 Hz, 1H), 3.93 (br. s., 2H), 2.98 - 3.24 (m, 2H).

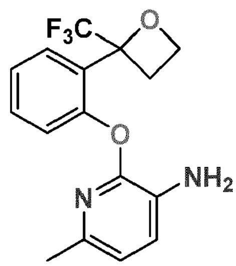

6-methyl-2-(2-(2-(trifluoromethyl)oxetan-2-yl)phenoxy)pyridin-3-amine

Prepared according to the general procedure **1** using 4 6-methyl-3-nitro-2-(2-(2-(trifluoromethyl)oxetan-2-yl)phenoxy)pyridine (1.8 g, 5.08 mmol, 1.0 eq.) and Pd/C (10% wt., 200 mg) to give the title material as a grey solid (1.6 g, 97%) LC-MS: m/z 325.2 $[M+H]^+$; $^1H$ NMR (400 MHz, $CDCl_3$) $\delta$ = 7.60 - 7.53 (m, 1H), 7.37 - 7.31 (m, 1H), 7.22 - 7.16 (m, 1H), 7.05 - 6.97 (m, 2H), 6.77 (d, *J* = 7.8 Hz, 1H), 4.83 (d t, *J* = 5.6, 7.9 Hz, 1H), 4.57 (td, *J* = 6.0, 8.7 Hz, 1H), 3.74 (b r s, 2H), 3.30 - 3.12 (m, 2H), 2.32 (s, 3H).

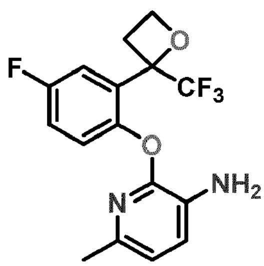

2-(4-fluoro-2-(2-(trifluoromethyl)oxetan-2-yl)phenoxy)-6-methylpyridin-3-amine

Prepared according to the general procedure **2** using 2-(4-fluoro-2-(2-(trifluoromethyl)oxetan-2-yl)phenoxy)-6-methyl-3-nitropyridine (575 mg) to give the title material as an off-white solid (529 mg, 100%). UPLC-MS: m/z 343.2 $[M+H]^+$; $^1H$ NMR (400 MHz, DMSO-$d_6$) $\delta$ 7.29 (ddd, *J* = 3.21, 8.00, 9.06 Hz, 1H), 7.20 (dd, *J* = 3.21, 9.17 Hz, 1H), 7.00 - 7.10 (m, 2H), 6.77 (d, *J* = 7.79 Hz, 1H), 4.81 (br s, 2H), 4.73 (dt, *J* = 5.78, 7.98 Hz, 1H), 4.55 (td, *J* = 5.82, 9.26 Hz, 1H), 3.19 - 3.28 (m, 1H), 3.10 (ddd, *J* = 6.05, 8.41, 12.59 Hz, 1H), 2.16 (s, 3H).

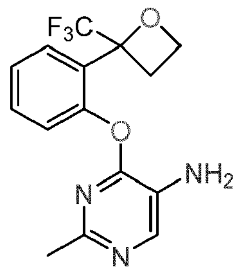

2-methyl-4-(2-(2-(trifluoromethyl)oxetan-2-yl)phenoxy)pyrimidin-5-amine

Prepared according to the general procedure **1** using 4-chloro-2-methyl-5-nitro-6-(2-(2-(trifluoromethyl)oxetan-2-yl)phenoxy)pyrimidine pyridine (1.9 g, 4.88 mmol, 1.0 eq.) and Pd/C (10% wt., 40 mg, 0.1 eq.) to give the title material as a yellow solid (1.4 g, crude) that was used directly without further purification. LC-MS: m/z 326.1 $[M+H]^+$.

**General Procedure B for isocyanate formation**

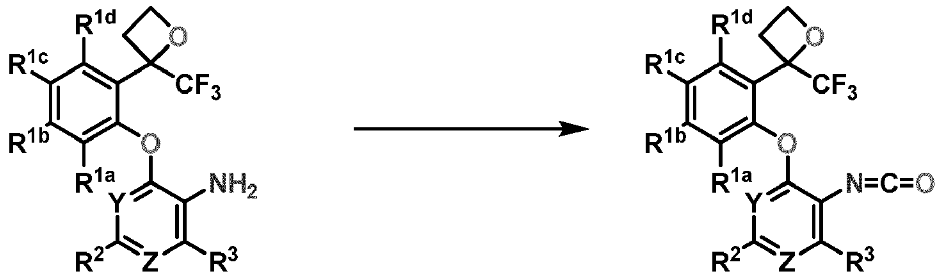

An oven-dried flask with stirrer was charged with a representative aniline (1.0 eq). The flask was fitted with a septum, evacuated and back-filled with argon (x3). *Anhydrous* dichloromethane (0.2M) was added and the reaction mixture cooled to 0 °C. Trichloromethyl chloroformate (0.85 eq) was added dropwise followed by 1,8-bis(dimethylamino)naphthalenediamine (2.1 eq) in a single portion. The reaction mixture was allowed to warm to room temperature and stirred until the reaction had gone to completion (2-24 h). The reaction mixture was washed with 0.5 N aq. HCl (x2), 1.0 N aq. NaOH (x1) and then brine. The organic phase was passed through a hydrophobic frit and concentrated *in vacuo* to give the target material, that was used directly without further purification.

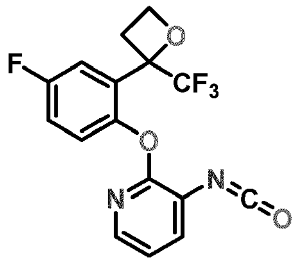

2-(4-fluoro-2-(2-(trifluoromethyl)oxetan-2-yl)phenoxy)-3-isocyanatopyridine

Prepared according to the general procedure **B** using 2-[4-fluoro-2-[2-(trifluoromethyl)oxetan-2-yl]phenoxy]pyridin-3-amine (645 mg) to give the title material as an orange oil that solidifies on standing to give a beige solid (659 mg, 95%). $^1$H NMR (400 MHz,$CDCl_3$) $\delta$ = 7.96 (dd, *J*=4.77, 1.47 Hz, 1H) 7.43 (dd, *J* = 7.70, 1.10 Hz, 1H) 7.37 (dd, *J* = 8.99, 3.12 Hz, 1H) 7.12 - 7.22 (m, 1H) 7.04 - 7.09 (m, 1H) 7.01 (dd, *J* = 7.70, 5.14 Hz, 1H) 4.77 - 4.87 (m, 1H) 4.52 - 4.63 (m, 1H) 3.16 (t, *J* = 7.89 Hz, 2H).

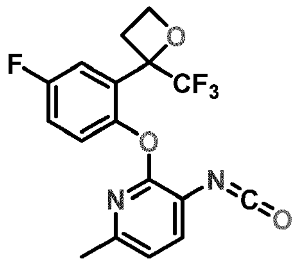

2-(4-fluoro-2-(2-(trifluoromethyl)oxetan-2-yl)phenoxy)-3-isocyanato-6-methylpyridine

Prepared according to the general procedure **B** using 2-(4-fluoro-2-(2-(trifluoromethyl)oxetan-2-yl)phenoxy)-6-methylpyridin-3-amine (1204 mg) to give the title material as an orange oil that solidifies on standing to give a beige solid (1241 mg, 96%). $^1$H NMR (400 MHz,$CDCl_3$) $\delta$ = 7.35 (dd, *J* = 3.12, 9.08 Hz, 1H), 7.23 - 7.32 (m, 1H), 7.12 (ddd, *J* = 3.12, 7.47, 9.03 Hz, 1H), 7.03 (dd, *J* = 4.68, 8.99 Hz, 1H), 6.83 (d, *J* = 7.79 Hz, 1H), 4.84 (dt, *J* = 5.64, 7.95 Hz, 1H), 4.59 (td, *J* = 6.17, 8.39 Hz, 1H), 3.43 (d, *J* = 2.57 Hz, 1H), 3.09 - 3.27 (m, 1H), 2.34 (s, 3H).

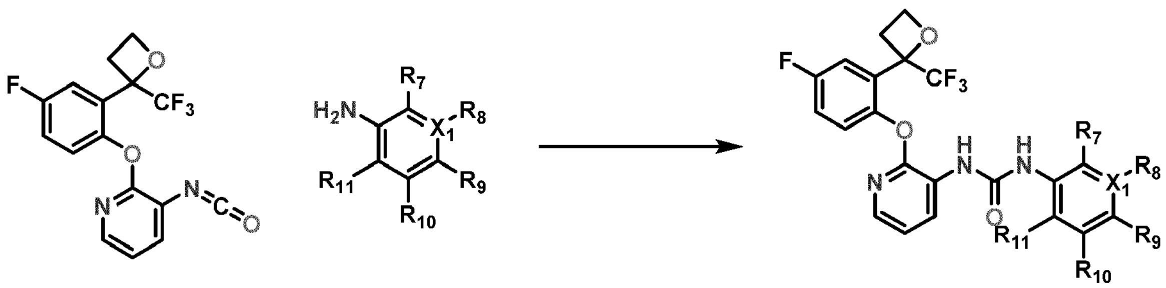

***General procedures for urea formations***

**General procedure 1 urea formation**

To a solution of representative aniline (1 eq.) and DIPEA (3.0 eq.) in dichloromethane (0.2M) was added a representative isocyanate (1-5 eq.). The mixture was stirred at 25 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 2 urea formation**

To a solution of representative aniline (1 eq.) and DIPEA (3.0 eq.) in dichloromethane (0.25 M) at 0 °C was added a solution of triphosgene (0.2 eq.) in dichloromethane 0.1 M). The mixture was stirred at 0 °C for 1 h. To the solution of *in-situ* formed isocyanate was added a representative aniline (1-2 eq.) and the reaction mixture stirred at 25 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 3 urea formation**

To a solution of representative aniline (1 eq.) and DIPEA (3.0 eq.) in dichloromethane (0.25 M) at 0 °C was added a solution of bis(trichloromethyl) carbonate (1.9 eq.) in dichloromethane 0.1 M). The mixture was stirred at 0 °C for 1 h. To the solution of *in-situ* formed isocyanate was added a representative aniline (1-2 eq.) and the reaction mixture stirred at 25 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 4 urea formation**

To a solution of a representative isocyanate (1 eq.) and DIPEA (2.0 eq.) in dichloromethane (0.25 M) was added a representative aniline (1-2 eq.) and the reaction mixture stirred at 25 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 5 urea formation**

To a solution of a representative isocyanate (1 eq.) and DIPEA (2.0 eq.) in toluene (0.25 M) was added a representative aniline (1-2 eq.) and the reaction mixture stirred at 60 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 6 urea formation**

To a 5 mL microwave vial was added a representative isocyanate (1.0 eq.) and representative aniline (1.0-1.2

eq.) The vial was sealed with a teflon-lined cap, evacuated and back-filled with argon (x3). Anhydrous toluene (0.2 M) was added and the reaction mixture was warmed to 60 °C and stirred until UPLC-MS showed complete conversion of the isocyanate starting material to the urea product. The solvent was removed *in vacuo* and the crude residue purified by flash chromatography using a Biotage Isolera one 3.0 using a 12 g C18 KP-SIL SNAP column linear gradient 15-40% - 95% acetonitrile in water (0.1% FA modifier) to give the title compounds.

**General procure 7 urea formation**

To a solution of representative aniline (1 eq.) and TEA (3.0 eq.) in toluene (0.2M) was added a representative isocyanate (1-5 eq.). The mixture was stirred at 50 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

**General procedure 8 urea formation**

To a solution of representative aniline (1 eq.) and TEA (2.0 eq.) in tetrahydrofuran (0.2M) was added a representative isocyanate (1-1.5 eq.). The mixture was stirred at 0-25 °C until the reaction was deemed complete. The reaction mixture was concentrated *in vacuo* to give a residue. The residue was purified by prep-HPLC (neutral condition) column: Waters Xbridge Prep OBD C18 150*40mm*10um;mobile phase: [water(10mM $NH_4HCO_3$)-ACN];B%: 45%-75%,8min.

*Example series* ***1***

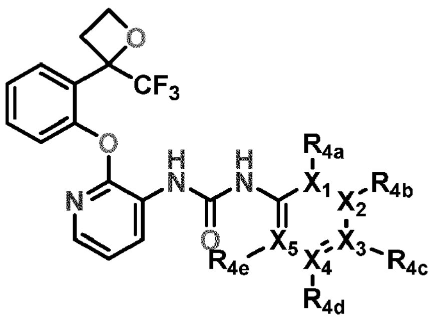

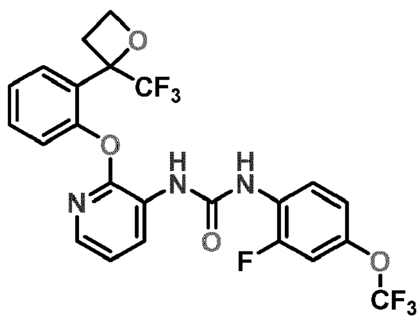

1-(2-fluoro-4-(trifluoromethoxy)phenyl)-3-(2-(2-(2-(trifluoromethyl)oxetan-2-**S9** yl)phenoxy)pyridin-3-yl)urea

Prepared according to **general procedure 4** using 3-isocyanato-2-(2-(2-(trifluoromethyl)oxetan-2-yl)phenoxy)pyridine (93 mg, 1.0 eq.) and 2-fluoro-4-(trifluoromethoxy)aniline (94 mg, 1.5 eq.) to give the title material as a white solid (42 mg, 25%). LC-MS: m/z 532.1 $[M+H]^+$; $^1H$ NMR (400MHz, $CDCl_3$) $\delta$ = 8.57 (dd, *J* = 1.7, 8.0 Hz, 1H), 8.32 - 8.11 (m, 1H), 7.89 - 7.76 (m, 2H), 7.68 (d, *J* = 7.9 Hz, 1H), 7.56 - 7.41 (m, 2H), 7.20 - 6.95 (m, 4H), 6.62 (br s, 1H), 5.05 - 4.88 (m, 1H), 4.68 (td, *J* = 5.7, 9.1 Hz, 1H), 3.53 - 2.96 (m, 2H).

**S13**

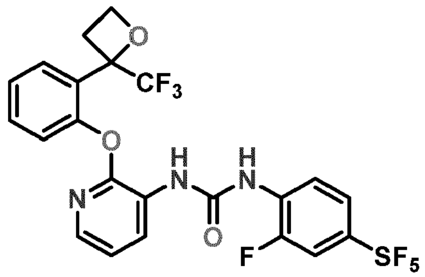

1-(2-fluoro-4-(pentafluoro-λ6-sulfaneyl)phenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (75 mg, 1.0 eq.) and 2-fluoro-4-(pentafluoro-$\lambda^6$-sulfanyl)aniline (64 mg, 1.2 eq.) to give the title material as a white solid (20 mg, 16%). LC-MS: m/z 574.0 $[M+H]^+$; $^1H$ NMR (400MHz, $CDCl_3$) $\delta$ = 8.56 (dd, *J* = 1.7, 7.9 Hz, 1H), 8.43 (t, J = 8.6 Hz, 1H), 8.06 (br s, 1H), 7.87 (dd, J = 1.6, 4.9 Hz, 1H), 7.80 (br d, *J* = 8.4 Hz, 1H), 7.61 - 7.52 (m, 2H), 7.48 (dt, *J* = 1.8, 7.9 Hz, 1H), 7.43 - 7.39 (m, 1H), 7.31 - 7.27 (m, 1H), 7.09 (dd, *J* = 4.9, 7.9 Hz, 1H), 6.88 (br d, *J* = 2.9 Hz, 1H), 4.97 (dt, *J* = 5.8, 7.9 Hz, 1H), 4.72 (td, *J* = 5.8, 9.1 Hz, 1H), 3.35 - 3.13 (m, 2H).

**S11**

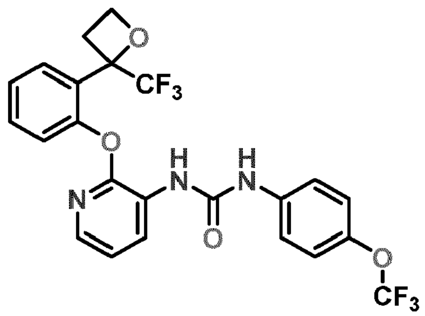

1-(4-(trifluoromethoxy)phenyl)-3-(2-(2-(2-(trifluoromethyl)oxetan-2-yl)phenoxy)pyridin-3-yl)urea

Prepared according to **general procedure 7** using 2-(2-(2-(trifluoromethyl)oxetan-2-yl)phenoxy)pyridin-3-amine (117 mg, 1 eq.) and 1-isocyanato-4-(trifluoromethoxy)benzene (157 mg, 1.5 eq.) to give the title material as a white solid (131 mg, 65%). LC-MS: m/z 514.0 $[M+H]^+$; $^1H$ NMR (400MHz, $CDCl_3$) 3.00 - 3.19 (m, 2 H) 4.58 (dt, *J* = 9.10, 5.83 Hz, 1 H) 4.83 (td, *J*=7.94, 5.63 Hz, 1 H) 6.48 (s, 1 H) 7.08 (dd, *J* = 8.00, 4.88 Hz, 1 H) 7.21 (d, *J* = 8.38 Hz, 2 H) 7.28 - 7.34 (m, 2 H) 7.35 - 7.48 (m, 4 H) 7.53 (dd, *J* = 7.75, 1.50 Hz, 1 H) 7.84 (dd, *J* = 4.88, 1.75 Hz, 1 H) 8.58 (dd, *J* = 8.00, 1.75 Hz, 1 H).

**S23**

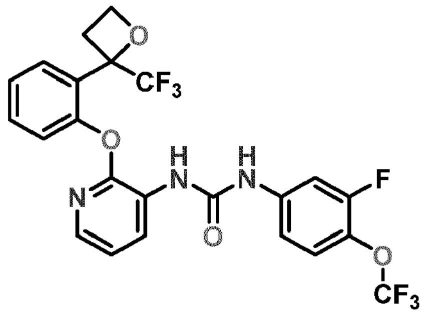

1-(3-fluoro-4-(trifluoromethoxy)phenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (108 mg, 1.0 eq.) and 3-fluoro-4-(trifluoromethoxy)aniline (94 mg, 1.5 eq.) to give the title material as a white solid (41 mg, 24%). LC-MS: m/z 532.0 $[M+H]^+$; $^1H$ NMR (400MHz, $CDCl_3$) $\delta$ = 8.57 (dd, *J*= 1.5, 7.9 Hz, 1H), 7.89 (dd, *J* = 1.6, 4.9 Hz, 1H), 7.61 - 7.44 (m, 4H), 7.43 - 7.31 (m, 2H), 7.29 - 7.22 (m, 1H), 7.17 - 7.01 (m, 2H), 6.62 (s, 1H), 4.99 - 4.50 (m, 2H), 3.30 - 3.00 (m, 2H).

**S25**

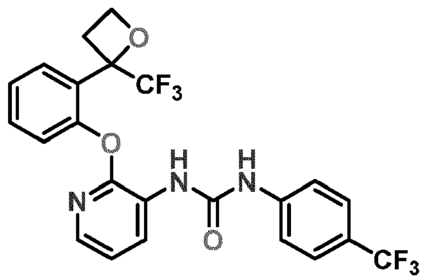

1-(2-(2-(2-(trifluoromethyl)oxetan-2-yl)phenoxy)pyridin-3-yl)-3-(4-(trifluoromethyl)phenyl)urea

Prepared according to **general procedure 5** using 3-isocyanato-2-(2-(2-(trifluoromethyl)oxetan-2-yl)phenoxy)pyridine (100 mg, 1.0 eq.) and 4-(trifluoromethyl) aniline (72 mg, 1.5 eq.) to give the title material as a white solid (18 mg, 12%). LC-MS: m/z 498.1 $[M+H]^+$; $^1H$ NMR (400MHz, $CDCl_3$) $\delta$ = 8.58 (dd, *J* = 7.95, 1.59 Hz, 1H) 7.86 (dd, *J* = 4.95, 1.65 Hz, 1H) 7.43 - 7.64 (m, 7H) 7.37 - 7.42 (m, 1H) 7.28 - 7.33 (m, 1H) 7.09 (dd, *J* = 7.89, 4.83 Hz, 1H) 6.57 (s, 1H) 4.81 - 4.93 (m, 1H) 4.62 (dt, *J* = 9.05, 5.75 Hz, 1H) 3.03 - 3.24 (m, 2H).

**S27**

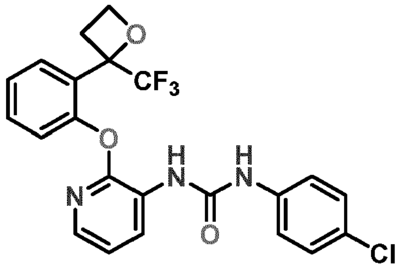

1-(4-chlorophenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (70 mg, 1.0 eq.) and 4-chloroaniline (40 mg, 1.5 eq.) to give the title material as a pale yellow solid (40 mg, 41%). LC-MS: m/z 462.1 [M-H]$^-$; $^1$H NMR (400MHz, $CDCl_3$) $\delta$ = 8.58 (dd, *J* = 8.00, 1.63 Hz, 1H) 7.85 (dd, *J* = 4.88, 1.75 Hz, 1H) 7.54 (dd, *J* = 7.75, 1.38 Hz, 1H) 7.44 (td, *J* = 7.82, 1.75 Hz, 1H) 7.23 - 7.41 (m, 7H) 7.07 (dd, *J* = 7.88, 4.88 Hz, 1H) 6.59 (s, 1H) 4.83 (td, *J* = 7.91, 5.69 Hz, 1H) 4.58 (dt, *J* = 9.04, 5.86 Hz, 1H) 2.96 - 3.15 (m, 2H).

**S28**

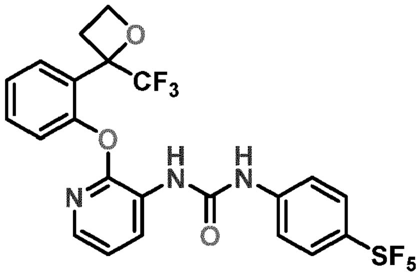

1-(4-(pentafluoro-$\lambda^6$-sulfaneyl)phenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1 eq.) and 4-(pentafluoro-λ6-sulfanyl)aniline (98 mg, 1.5 eq.) to give the title material as a white solid (23 mg, 14%). LC-MS: m/z 556.0 [M+H]$^+$; $^1$H NMR (400MHz, $CDCl_3$) $\delta$ = 8.47 (dd, *J* = 7.94, 1.69 Hz, 1H) 7.78 (dd, *J* = 4.88, 1.63 Hz, 1H) 7.58 - 7.71 (m, 2H) 7.33 - 7.54 (m, 5H) 7.26 - 7.33 (m, 1H) 7.13 - 7.25 (m, 1H) 6.99 (dd, *J* = 8.00, 4.88 Hz, 1H) 6.68 (s, 1H) 4.76 (td, *J* = 7.94, 5.63 Hz, 1H) 4.52 (dt, *J* = 9.01, 5.88 Hz, 1H) 2.97 - 3.18 (m, 2H).

**S29**

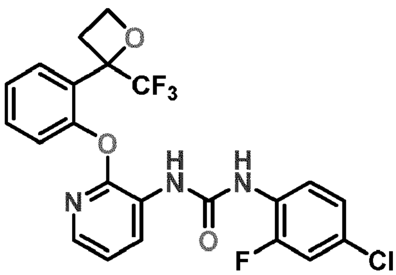

1-(4-chloro-2-fluorophenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (70 mg, 1.0 eq.) and 4-chloro-2-fluoroaniline (45 mg, 1.5 eq.) to give the title material as a pale yellow solid (31 mg, 31%). LC-MS: m/z 480.1 [M-H]$^-$; $^1$H NMR (400MHz, $CDCl_3$) $\delta$ = 8.58 (dd, *J* = 7.94, 1.69 Hz, 1H) 8.10 - 8.23 (m, 1H) 7.86 (dd, *J* = 4.88, 1.75 Hz, 1H) 7.81 (br. s., 1H) 7.66 (d, *J* = 8.25 Hz, 1H) 7.39 - 7.53 (m, 2H) 7.28 - 7.34 (m, 1H) 7.13 - 7.21 (m, 2H) 7.09 (dd, *J* = 8.00, 4.88 Hz, 1H) 6.64 (br. s., 1H) 4.95 (td, *J* = 7.94, 5.75 Hz, 1H) 4.69 (dt, *J* = 9.13, 5.82 Hz, 1H) 3.08 - 3.31 (m, 2H).

**S39**

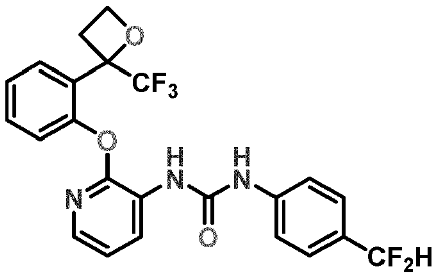

1-(4-(difluoromethyl)phenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (55 mg, 1.0 eq.) and 4-(difluoromethyl)aniline (35 mg, 1.5 eq.) to give the title material as a brown solid (8 mg, 10%). LC-MS: m/z 480.1 $[M+H]^+$; $^1H$ NMR (400MHz, $CDCl_3$) δ = 8.74 - 8.52 (m, 1H), 7.84 (dd, *J* = 1.3, 4.8 Hz, 1H), 7.56 - 7.40 (m, 7H), 7.34 - 7.28 (m, 2H), 7.07 (dd, *J* = 4.9, 7.9 Hz, 1H), 6.79 - 6.45 (m, 2H), 4.83 (br d, *J* = 5.7 Hz, 1H), 4.64 - 4.45 (m, 1H), 3.27 - 3.03 (m, 2H).

**S43**

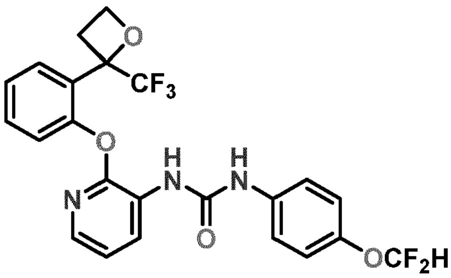

1-(4-(difluoromethoxy)phenyl)-3-(2-(2-(2-(trifluoromethyl)oxetan-2-yl)phenoxy)pyridin-3-yl)urea

Prepared according to **general procedure 3** using 3-isocyanato-2-(2-(2-(trifluoromethyl)oxetan-2-yl)phenoxy)pyridine was prepared *in situ* from 2-(2-(2-(trifluoromethyl)oxetan-2-yl)phenoxy)pyridin-3-amine (300 mg, 1.0 eq.) and reacted with 4-(difluoromethoxy)aniline (185 mg, 1.2 eq.) to give the title material as a pale yellow solid (171 mg, 36%). LC-MS: m/z 496.0 $[M+H]^+$; $^1H$ NMR (400MHz, $CD_3OD$) δ = 8.50 (dd, *J* = 1.6, 7.9 Hz, 1H), 7.73 (dd, *J* = 1.6, 4.9 Hz, 1H), 7.58 (dd, *J* = 1.3, 7.8 Hz, 1H), 7.51 - 7.41 (m, 3H), 7.37 - 7.26 (m, 1H), 7.13 - 7.07 (m, 3H), 7.04 (d, *J*= 8.1 Hz, 1H), 6.96 - 6.51 (m, 1H), 4.77 (dt, *J* = 5.8, 7.9 Hz, 1H), 4.53 (td, *J* = 5.7, 9.3 Hz, 1H), 3.29 - 3.19 (m, 1H), 3.16 - 3.05 (m, 1H).

**S47**

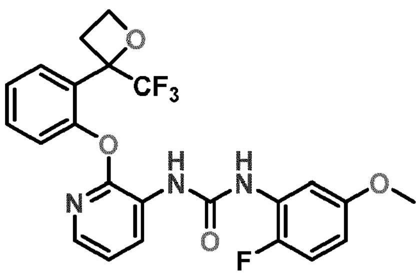

1-(2-fluoro-5-methoxyphenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (150 mg, 1.0 eq.) and 2-fluoro-4-methoxyaniline (63 mg, 1.0 eq.) to give the title material as a white solid (30 mg, 14%). LC-MS: m/z 478.0 $[M+H]^+$; $^1H$ NMR (400MHz, $CDCl_3$) δ = 8.57 - 8.73 (m, 1H) 7.73 - 8.00 (m, 3H) 7.65

(d, *J* = 8.13 Hz, 1H) 7.45 - 7.56 (m, 2H) 7.27 - 7.39 (m, 1H) 7.11 (dd, J= 7.88, 4.88 Hz, 1H) 6.97 - 7.08 (m, 1H) 6.69 (br. s., 1H) 6.58 (dt, *J* = 8.91, 3.49 Hz, 1H) 4.89 - 5.08 (m, 1H) 4.70 (dt, *J* = 9.13, 5.75 Hz, 1H) 3.84 (s, 3H) 3.12 - 3.39 (m, 2H).

S51

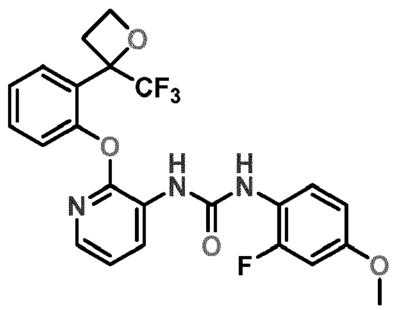

1-(2-fluoro-4-methoxyphenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 2-fluoro-4-methoxyaniline (63 mg, 1.5 eq.) to give the title material as a white solid (52 mg, 36%). LC-MS: m/z 476.1 $[M-H]^-$; $^1H$ NMR (400MHz, $CDCl_3$) $\delta$ = 8.61 (dd, *J* = 8.00, 1.63 Hz, 1H) 7.83 (dd, *J* = 4.88, 1.63 Hz, 1H) 7.71 (t, *J* = 8.82 Hz, 1H) 7.50 - 7.57 (m, 1H) 7.41 - 7.50 (m, 2H) 7.23 - 7.41 (m, 2H) 7.06 (dd, *J* = 8.00, 4.88 Hz, 1H) 6.65 - 6.78 (m, 2H) 6.39 (s, 1H) 4.86 (td, *J* = 7.88, 5.75 Hz, 1H) 4.59 (dt, *J* = 9.13, 5.82 Hz, 1H) 3.80 (s, 3H) 2.97 - 3.20 (m, 2H).

**S52**

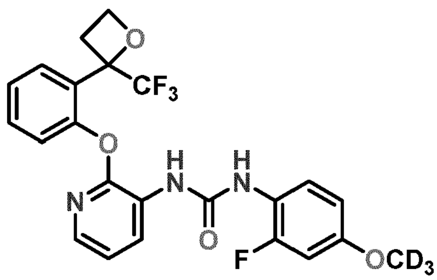

1-(2-fluoro-4-(methoxy-$d_3$)phenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (93 mg, 1.0 eq.) and 2-fluoro-4-methoxyaniline (63 mg, 1.5 eq.) to give the title material as a white solid (43 mg, 32%). LC-MS: m/z 481.2 $[M+H]^+$; $^1H$ NMR (400MHz, $CDCl_3$) $\delta$ = 9.02 (s, 1H), 8.72 (s, 1H), 8.51 (dd, *J* = 1.7, 7.9 Hz, 1H), 7.91 (t, *J*=9.3 Hz, 1H), 7.74 (dd, *J* = 17, 4.8 Hz, 1H), 7.55 - 7.44 (m, 1H), 7.37 - 7.28 (m, 1H), 7.17 - 7.05 (m, 2H), 6.91 (dd, *J* = 2.8, 13.0 Hz, 1H), 6.77 (td, *J* = 1.4, 9.0 Hz, 1H), 4.78 - 4.68 (m, 1H), 4.55 (td, *J* = 5.8, 9.4 Hz, 1H), 3.45 - 3.37 (m, 1H), 3.13 - 3.01 (m, 1H), 2.68 (s, 1H).

**S53**

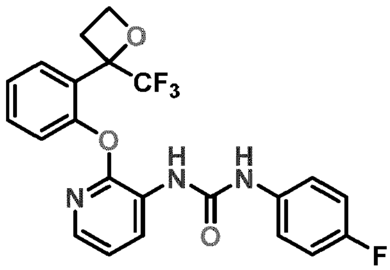

1-(4-fluorophenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 4-fluoroaniline (25 mg, 1.0 eq.) to give the title material as a white solid (50 mg, 38%). LC-MS: m/z 448.1 $[M+H]^+$; $^1H$ NMR (400MHz, $CDCl_3$) $\delta$ = 8.59 (dd, *J* = 1.5, 8.0 Hz, 1H), 7.83 (dd, *J* = 1.6, 4.9 Hz, 1H), 7.54 (d, *J* = 7.7 Hz, 1H), 7.47 - 7.39 (m, 1H), 7.34 (dd, *J* = 4.6, 8.9 Hz, 2H), 7.31 - 7.28 (m, 1H), 7.26 - 7.20 (m, 2H), 7.10 - 6.99 (m, 3H), 6.35 (s, 1H), 4.87 - 4.75 (m, 1H), 4.56 (td, *J* = 5.8,9.1 Hz, 1H), 3.12 - 2.94 (m, 2H).

**S55**

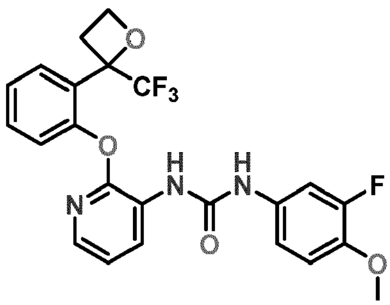

1-(3-fluoro-4-methoxyphenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 3-fluoro-4-methoxyaniline (63 mg, 1.5 eq.) to give the title material as a white solid (29 mg, 14%). LC-MS: m/z 478.1 $[M+H]^+$; $^1H$ NMR (400MHz, $CDCl_3$) $\delta$ = 8.52 (d, *J* = 8.00 Hz, 1H) 7.71 - 7.81 (m, 1H) 7.46 (d, *J* = 7.50 Hz, 1H) 7.31 - 7.40 (m, 1H) 7.22 (s, 1H) 7.07 - 7.17 (m, 3H) 6.90 - 7.04 (m, 2H) 6.76 - 6.88 (m, 1H) 6.24 (s, 1H) 4.68 - 4.79 (m, 1H) 4.47 (dt, *J* = 9.04, 5.74 Hz, 1H) 3.80 (s, 3H) 2.83 - 3.07 (m, 2H).

**S60**

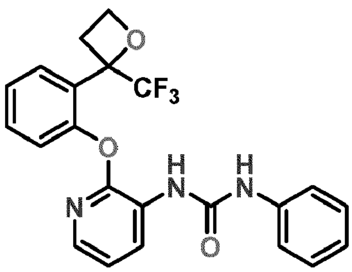

1-phenyl-3-(2-(2-(2-(trifluoromethyl)oxetan-2-yl)phenoxy)pyridin-3-yl)urea

Prepared according to **general procedure 1** using 2-(2-(2-(trifluoromethyl)oxetan-2-yl)phenoxy)pyridin-3-amine (300 mg) and phenylisocyanate (550 mg, 4.8 eq.) in dichloromethane (5 mL) at room temperature to give the title material as a white solid (134 mg, 32%). LC-MS: m/z 430.0 $[M+H]^+$; $^1H$ NMR (400 MHz, DMSO-$d_6$) $\delta$ = 9.33 (s, 1H), 8.51 (dd, *J* = 1.7, 7.9 Hz, 1H), 8.38 (s, 1H), 7.74 (dd, *J* = 1.8, 4.8 Hz, 1H), 7.53 - 7.43 (m, 4H), 7.33 - 7.26 (m, 3H), 7.15 - 7.07 (m, 2H), 6.99 (t, *J* = 7.3 Hz, 1H), 4.79 - 4.64 (m, 1H), 4.53 (td, *J* = 5.8, 9.3 Hz, 1H), 3.40 - 3.35 (m, 1H), 3.05 (ddd, *J* = 6.0, 8.3, 12.5 Hz, 1H).

**S61**

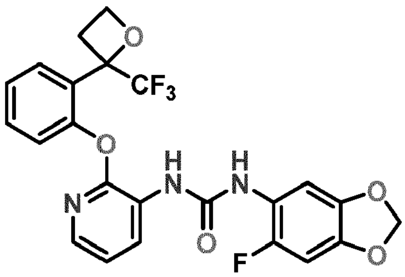

1-(6-fluorobenzo[d][1,3]dioxol-5-yl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 6-fluoro-1,3-benzodioxol-5-amine (69 mg, 1.5 eq.) to give the title material as a white solid (49 mg, 80%). LC-MS: m/z 490.1 $[M-H]^-$; $^1H$ NMR (400MHz, $CDCl_3$) $\delta$ = 8.60 (dd, *J* = 7.94, 1.56 Hz, 1H) 7.84 (dd, *J* = 4.88, 1.63 Hz, 1H) 7.36 - 7.66 (m, 5H) 7.26 - 7.34 (m, 1H) 7.07 (dd, *J* = 7.88, 4.88 Hz, 1H) 6.67 (d, *J* = 9.76 Hz, 1H) 6.43 (br. s., 1H) 5.98 (d, *J* = 1.63 Hz, 2H) 4.76 - 5.01 (m, 1H) 4.62 (dt, *J* = 9.04, 5.86 Hz, 1H) 3.04 - 3.33 (m, 2H).

**S62**

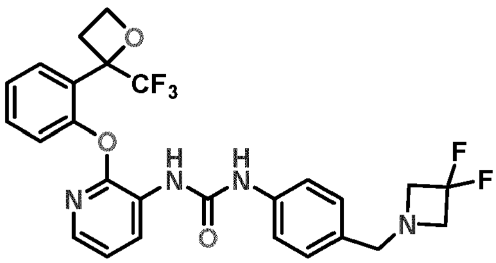

1-(4-((3,3-difluoroazetidin-1-yl)methyl)phenyl)-3-(2-(2-(2-(trifluoromethyl)oxetan-2-yl)phenoxy)pyridin-3-yl)urea

Prepared according to **general procedure 2.** 3-isocyanato-2-(2-(2-(trifluoromethyl)oxetan-2-yl)phenoxy)pyridine was prepared *in situ* from 2-(2-(2-(trifluoromethyl)oxetan-2-yl)phenoxy)pyridin-3-amine (90 mg, 1.0 eq.) and reacted 4-((3,3-difluoroazetidin-1-yl)methyl)aniline (69 mg, 1.2 eq.) to give the title material as an off-white solid (4 mg, 2%). LC-MS: m/z 535.1 $[M+H]^+$; $^1H$ NMR (400 MHz, DMSO-$d_6$) $\delta$ = 9.37 (s, 1H), 8.51 (dd, *J* = 1.6, 8.0 Hz, 1H), 8.40 (s, 1H), 7.73 (dd, *J* = 1.8, 4.8 Hz, 1H), 7.54 - 7.44 (m, 2H), 7.41 (d, *J* = 8.6 Hz, 2H), 7.32 (dt, *J* = 1.1, 7.6 Hz, 1H), 7.22 (d, *J* = 8.3 Hz, 2H), 7.12 (dd, *J* = 4.9, 8.0 Hz, 1H), 7.09 (dd, *J* = 0.8, 8.2 Hz, 1H), 4.75 - 4.67 (m, 1H), 4.53 (td, *J* = 5.8, 9.3 Hz, 1H), 3.64 (s, 2H), 3.55 (t, *J* = 12.5 Hz, 4H), 3.41 - 3.34 (m, 1H), 3.05 (ddd, *J* = 6.1, 8.2, 12.6 Hz, 1H).

**S65**

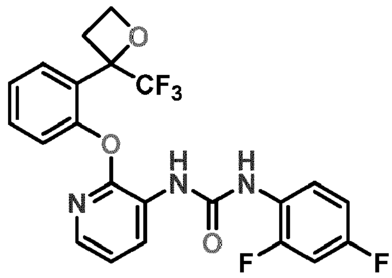

1-(2,4-difluorophenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 2,4-difluoroaniline (29 mg, 1.5 eq.) to give the title material as a white solid (48 mg, 34%). LC-MS: m/z 466.1 $[M+H]^+$; $^1H$ NMR (400MHz, $CDCl_3$) $\delta$ = 8.58 (dd, *J* = 1.5, 8.0 Hz, 1H), 8.10 - 7.99 (m, 1H), 7.84 (dd, *J* = 1.7, 4.8 Hz, 1H), 7.66 (br s, 1H), 7.55 (br d, *J* = 8.8 Hz, 1H), 7.50 - 7.42 (m, 2H), 7.31 - 7.28 (m, 1H), 7.08 (dd, *J* = 5.0, 8.0 Hz, 1H), 6.95 - 6.86 (m, 2H), 6.48 (br s, 1H), 4.97 - 4.86 (m, 1H), 4.65 (td, *J* = 5.8, 9.1 Hz, 1H), 3.25 - 3.08 (m, 2H).

**S70**

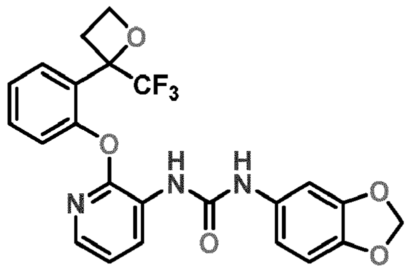

1-(benzo[*d*][1,3]dioxol-5-yl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 1,3-benzodioxol-5-amine (62 mg, 1.5 eq.) to give the title material as a white solid (52 mg, 37%). LC-MS: m/z 474.0 $[M+H]^+$; $^1H$ NMR (400MHz, $CDCl_3$) $\delta$ = 8.61 (dd, *J* = 7.94, 1.56 Hz, 1H) 7.81 (dd, *J* = 4.88, 1.63 Hz, 1H) 7.52 - 7.64 (m, 1H) 7.36 - 7.47 (m, 1H) 7.19 - 7.33 (m, 2H) 7.00 - 7.17 (m, 2H) 6.90 (d, *J* = 1.63 Hz, 1H) 6.68 - 6.80 (m, 2H) 6.55 (s, 1H) 5.97 (d, *J* = 2.88 Hz, 2H) 4.72 - 4.92 (m, 1H) 4.53 (dt, *J* = 8.94, 5.85 Hz, 1H) 2.90 - 3.20 (m, 2H).

**S71**

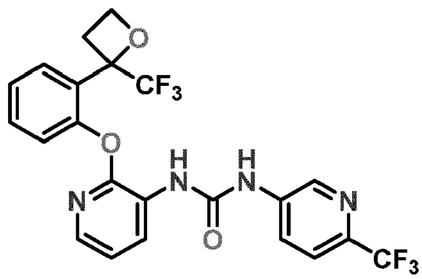

1-(2-(2-(2-(trifluoromethyl)oxetan-2-yl)phenoxy)pyridin-3-yl)-3-(6-(trifluoromethyl)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 6-(trifluoromethyl)pyridin-3-amine (72 mg, 1.5 eq.) to give the title material as a white solid (42 mg, 28%). LC-MS: m/z 499.0 $[M+H]^+$; $^1H$ NMR (400MHz, $CDCl_3$) δ = 8.49 - 8.67 (m, 2H) 8.28 (dd, *J* = 8.68, 1.96 Hz, 1H) 7.90 (dd, *J* = 4.89, 1.47 Hz, 1H) 7.69 (d, *J* = 8.44 Hz, 2H) 7.38 - 7.61 (m, 3H) 7.24 - 7.36 (m, 1H) 7.03 - 7.17 (m, 2H) 4.78 - 4.95 (m, 1H) 4.53 - 4.71 (m, 1H) 3.03 - 3.28 (m, 2H).

**S72**

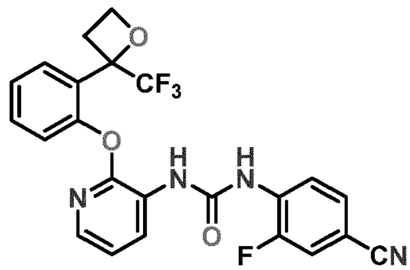

1-(4-cyano-2-fluorophenyl)-3-(2-(2-(2-(trifluoromethyl)oxetan-2-yl)phenoxy)pyridin-3-yl)urea

Prepared according to **general procedure 5** using 3-isocyanato-2-(2-(2-(trifluoromethyl)oxetan-2-yl)phenoxy)pyridine (100 mg, 1.0 eq.) and 4-amino-3-fluoro-benzonitrile (61 mg, 1.5 eq.) to give the title material as a white solid (16 mg, 11%). LC-MS: m/z 473.1 $[M+H]^+$; $^1H$ NMR (400MHz, $CDCl_3$) δ = 8.56 (dd, *J* = 1.5, 7.9 Hz, 1H), 8.50 (t, *J* = 8.3 Hz, 1H), 8.14 (br s, 1H), 7.91 - 7.80 (m, 2H), 7.55 - 7.46 (m, 2H), 7.43 - 7.37 (m, 2H), 7.31 - 7.28 (m, 1H), 7.09 (dd, *J*= 4.9, 7.9 Hz, 1H), 6.95 (br d, *J*= 3.7 Hz, 1H), 5.04 - 4.92 (m, 1H), 4.79 - 4.69 (m, 1H), 3.33 - 3.18 (m, 2H).

**S78**

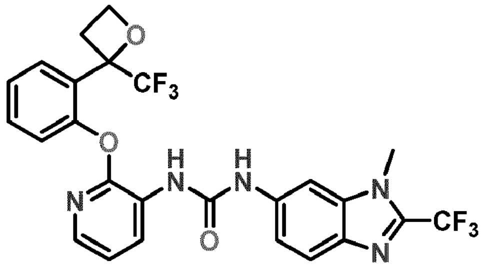

1-(1-methyl-2-(trifluoromethyl)-1H-benzo[*d*]imidazol-6-yl)-3-(2-(2-(2-(trifluoromethyl)oxetan-2-yl)phenoxy)pyridin-3-yl)urea

Prepared according to **general procedure 2.** 3-isocyanato-2-(2-(2-(trifluoromethyl)oxetan-2-yl)phenoxy)pyridine was prepared *in situ* from 2-(2-(2-(trifluoromethyl)oxetan-2-yl)phenoxy)pyridin-3-amine (300 mg, 1 eq.) and reacted with 2-(trifluoromethyl)-1-methyl-1H-benzo[d]imidazol-6-amine (250 mg, 1.2 eq.) to give the title material as a pale yellow solid (250 mg, 47%). LC-MS: m/z 552.0 $[M+H]^+$; $^1H$ NMR (400MHz, $CD_3OD$) δ = 8.54 (dd, *J* = 1.6, 8.0 Hz, 1H), 8.01 (d, *J* = 1.8 Hz, 1H), 7.74 (dd, *J* = 1.6, 4.9 Hz, 1H), 7.62 (d, *J* = 8.9 Hz, 1H), 7.58 (dd, *J* = 1.3, 7.8 Hz, 1H), 7.52 (dd, *J* = 1.9, 8.9 Hz, 1H), 7.48 - 7.41 (m, 1H), 7.34 - 7.28 (m, 1H), 7.12 (dd, *J* = 4.9, 8.0 Hz, 1H), 7.05 (d, *J* = 8.1 Hz, 1H), 4.77 (dt, *J* = 5.7, 7.9 Hz, 1H), 4.53 (td, *J* = 5.8, 9.2 Hz, 1H), 4.00 (s, 3H), 3.29 - 3.20 (m, 1H), 3.16 - 3.05 (m, 1H).

**S79**

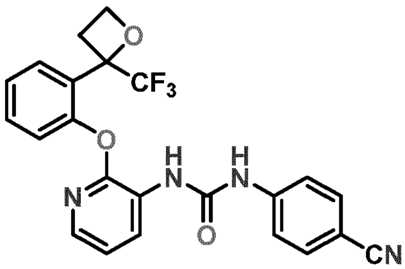

1-(4-cyanophenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (70 mg, 1.0 eq.) and 4-aminobenzonitrile (37 mg, 1.5 eq.) to give the title material as a pale yellow solid (18 mg, 19%). LC-MS: m/z 453.1 $[M+H]^+$; $^1H$ NMR (400MHz, $CDCl_3$) $\delta$ = 8.58 (dd, *J* = 7.88, 1.63 Hz, 1H) 7.88 (dd, *J* = 4.88, 1.63 Hz, 1H) 7.54 - 7.67 (m, 5H) 7.49 - 7.54 (m, 1H) 7.39 - 7.49 (m, 2H) 7.30 - 7.35 (m, 1H) 7.11 (dd, *J* = 7.94, 4.94 Hz, 1H) 6.77 (s, 1H) 4.88 (td, *J* = 7.91, 5.69 Hz, 1H) 4.64 (dt, *J* = 8.91, 5.93 Hz, 1H) 3.11 - 3.26 (m, 2H).

**S85**

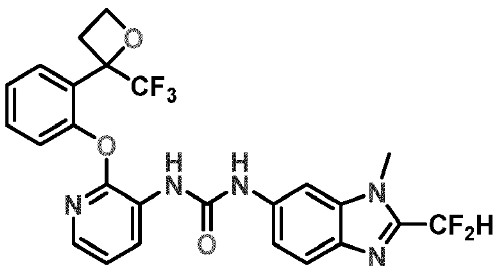

1-(2-(difluoromethyl)-1-methyl-1*H*-benzo[*d*]imidazol-6-yl)-3-(2-(2-(2-(trifluoromethyl)oxetan-2-yl)phenoxy)pyridin-3-yl)urea

Prepared according to **general procedure 2.** 3-isocyanato-2-(2-(2-(trifluoromethyl)oxetan-2-yl)phenoxy)pyridine was prepared *in situ* from 2-(2-(2-(trifluoromethyl)oxetan-2-yl)phenoxy)pyridin-3-amine (300 mg, 1.0 eq.) and reacted with 2-(difluoromethyl)-1-methyl-1H-benzo[d]imidazol-6-amine (229 mg, 1.2 eq.) to give the title material as a white solid (262 mg, 50%). LC-MS: m/z 534.0 $[M+H]^+$; $^1H$ NMR (400MHz, $CD_3OD$) $\delta$ = 8.53 (dd, *J* = 1.5, 8.0 Hz, 1H), 7.95 (d, *J* = 1.8 Hz, 1H), 7.73 (dd, *J* = 1.5, 4.9 Hz, 1H), 7.57 (d, *J* = 8.8 Hz, 2H), 7.50 - 7.41 (m, 2H), 7.34 - 7.27 (m, 1H), 7.24 - 6.95 (m, 3H), 4.82 - 4.68 (m, 1H), 4.53 (td, J=5.7, 9.2 Hz, 1H), 3.98 (s, 3H), 3.29 - 3.19 (m, 1H), 3.17 - 2.99 (m, 1H).

**S36**

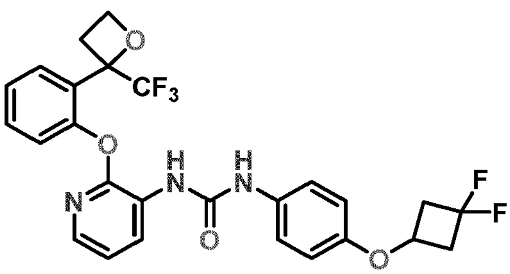

1-(4-(3,3-difluorocyclobutoxy)phenyl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (50 mg, 1.0 eq.) and 4-(3,3-difluorocyclobutoxy)aniline (20 mg, 0.7 eq.) to give the title material as a white solid (29 mg, 53%). LC-MS: m/z 536.1 $[M+H]^+$; $^1H$ NMR (400MHz, $CDCl_3$) $\delta$ = 8.62 (d, *J* = 8.0 Hz, 1H), 7.81 (d, *J* = 4.6 Hz, 1H), 7.54 (d, *J* = 7.8 Hz, 1H), 7.42 (t, *J* = 7.8 Hz, 1H), 7.32 - 7.23 (m, 3H), 7.18 (s, 1H), 7.13 (d, *J* = 8.3 Hz, 1H), 7.06 (dd, *J* = 4.7, 7.8 Hz, 1H), 6.78 (d, *J* = 8.6 Hz, 2H), 6.27 (s, 1H), 4.87 - 4.73 (m, 1H), 4.66 - 4.46 (m, 2H), 3.15 - 2.90 (m, 4H), 2.83 - 2.64 (m, 2H).

**S46**

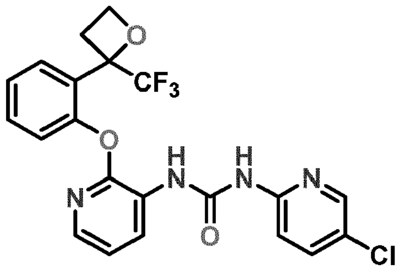

1-(5-chloropyridin-2-yl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (70 mg, 1.0 eq.) and 5-chloropyridin-2-amine (32 mg, 1.2 eq.) to give the title material as a pale yellow solid (43 mg, 45%). LC-MS: m/z 465.0 $[M+H]^+$; $^1H$ NMR (400MHz, $CDCl_3$) $\delta$ = 11.89 (br s, 1H), 8.86 - 8.56 (m, 2H), 8.27 (s, 1H), 7.98 - 7.78 (m, 2H), 7.66 (d, *J* = 7.8 Hz, 1H), 7.51 - 7.40 (m, 1H), 7.40 - 7.31 (m, 1H), 7.09 (dd, *J* = 4.8, 7.9 Hz, 1H), 7.05 - 6.94 (m, 2H), 4.91 - 4.70 (m, 1H), 4.54 (td, *J* = 5.7, 9.3 Hz, 1H), 3.34 - 3.14 (m, 1H), 3.12 - 2.97 (m, 1H).

**S38**

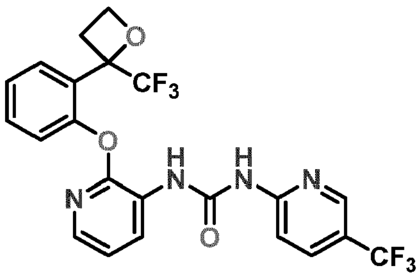

1-(2-(2-(2-(trifluoromethyl)oxetan-2-yl)phenoxy)pyridin-3-yl)-3-(5-(trifluoromethyl)pyridin-2-yl)urea

Prepared according to **general procedure 4** using 3-isocyanato-2-(2-(2-(trifluoromethyl)oxetan-2-yl)phenoxy)pyridine (70 mg, 1.0 eq.) and 5-(trifluoromethyl)pyridin-2-amine (41 mg, 1.2 eq.) to give the title material as a white solid (38 mg, 37%). LC-MS: m/z 499.0 $[M+H]^+$; $^1H$ NMR (400MHz, $CDCl_3$) $\delta$ = 11.69 (br s, 1H), 8.73 (dd, *J* = 1.4, 7.9 Hz, 1H), 8.16 (br s, 1H), 7.96 (d, *J* = 2.4 Hz, 1H), 7.87 (dd, *J* = 1.5, 4.9 Hz, 1H), 7.72 - 7.55 (m, 2H), 7.49 - 7.39 (m, 1H), 7.38 - 7.29 (m, 1H), 7.15 - 6.94 (m, 2H), 6.84 (br d, *J* = 8.9 Hz, 1H), 4.87 - 4.74 (m, 1H), 4.55 (td, *J* = 5.6, 9.2 Hz, 1H), 3.31 - 3.15 (m, 1H), 3.13 - 2.99 (m, 1H).

**S50**

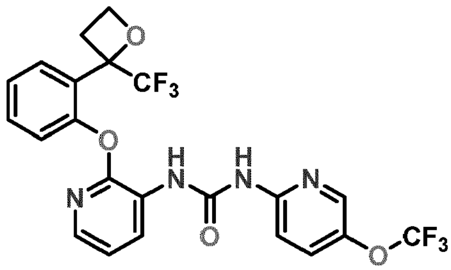

1-(5-(trifluoromethoxy)pyridin-2-yl)-3-(2-(2-(2-(trifluoromethyl)oxetan-2-yl)phenoxy)pyridin-3-yl)urea

Prepared according to **general procedure 4** using 3-isocyanato-2-(2-(2-(trifluoromethyl)oxetan-2-yl)phenoxy)pyridine (70 mg, 1.0 eq.) and 5-(trifluoromethoxy)pyridin-2-amine (45 mg, 1.2 eq.) to give the title material as a white solid (49 mg, 46%). LC-MS: m/z 515.0.0 $[M+H]^+$; $^1H$ NMR (400MHz, $CDCl_3$) $\delta$ = 11.63 (br s, 1H), 8.85 - 8.62 (m, 1H), 7.95 (d, *J* = 2.4 Hz, 1H), 7.91 - 7.84 (m, 1H), 7.71 (br s, 1H), 7.65 (d, *J* = 7.5 Hz, 1H), 7.56 (br d, *J* = 8.8 Hz, 1H), 7.47 - 7.38 (m, 1H), 7.32 (t, *J* = 7.5 Hz, 1H), 7.08 (dd, *J* = 4.9, 7.9 Hz, 1H), 7.01 (d, *J* = 8.1 Hz, 1H), 6.88 (br d, *J* = 9.0 Hz, 1H), 4.88 - 4.73 (m, 1H), 4.61 - 4.48 (m, 1H), 3.29 - 3.15 (m, 1H), 3.12 - 2.99 (m, 1H).

**Example Series 2**

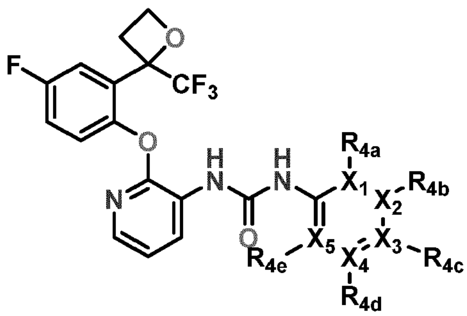

**S26**

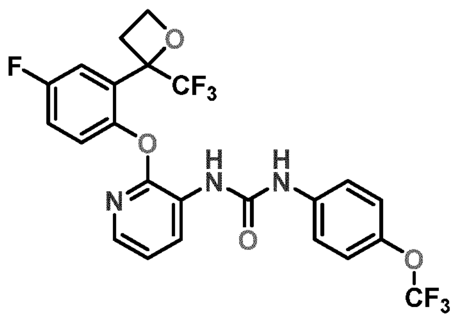

1-(2-(4-fluoro-2-(2-(trifluoromethyl)oxetan-2-yl)phenoxy)pyridin-3-yl)-3-(4-(trifluoromethoxy)phenyl)urea

Prepared according to the general procedure **6** using 2-(4-fluoro-2-(2-(trifluoromethyl)oxetan-2-yl)phenoxy)-3-isocyanatopyridine (40 mg, 1.0 eq.) and 4-trifluormethoxyaniline (20 mg, 1.0 eq.) to give the title material as a white solid (60 mg, 100%). UPLC-MS: m/z 532.1 $[M+H]^+$; : (400 MHz, DMSO-$d_6$) $\delta$ = 9.54 (s, 1H), 8.49 (dd, *J* = 1.65, 7.89 Hz, 1H), 8.42 (s, 1H), 7.76 (dd, *J* = 1.65, 4.95 Hz, 1H), 7.52 - 7.62 (m, 2H), 7.20 - 7.41 (m, 5H), 7.14 (dd, *J* = 4.77, 8.07 Hz, 1H), 4.65 - 4.78 (m, 1H), 4.56 (td, *J* = 5.87, 9.17 Hz, 1H), 3.23 - 3.36 (m, 1H), 3.06 (ddd, *J* = 6.24, 8.25, 12.65 Hz, 1H).

S30

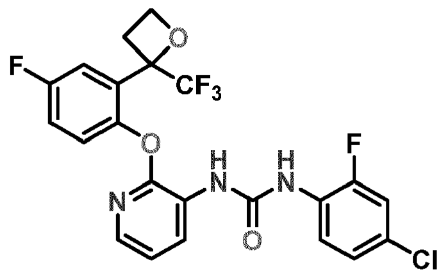

1-(4-chloro-2-fluorophenyl)-3-(2-(4-fluoro-2-(2-(trifluoromethyl)oxetan-2-yl)phenoxy)pyridin-3-yl)urea

Prepared according to the general procedure **6** using 2-(4-fluoro-2-(2-(trifluoromethyl)oxetan-2-yl)phenoxy)-3-isocyanatopyridine (40 mg, 1.0 eq.) and 4-chloro-2-fluoroaniline (16 mg, 1.0 eq.) to give the title material as a white solid (30 mg, 56%). UPLC-MS: m/z 500.1/502.0 $[M+H]^+$; $^1H$ NMR (400 MHz, DMSO-$d_6$) $\delta$ = 9.36 (d, *J* = 2.20 Hz, 1H), 8.94 (s, 1H), 8.50 (dd, *J* = 1.65, 7.89 Hz, 1H), 8.21 (t, *J* = 8.99 Hz, 1H), 7.76 (dd, *J* = 1.65, 4.95 Hz, 1H), 7.48 (dd, *J* = 2.38, 11.19 Hz, 1H), 7.37 (dt, *J* = 3.30, 8.44 Hz, 1H), 7.17 - 7.31 (m, 3H), 7.14 (dd, *J* = 4.77, 8.07 Hz, 1H), 4.71 (d, *J* = 5.87 Hz, 1H), 4.50 - 4.63 (m, 1H), 3.29 - 3.39 (m, 1H), 3.00-3.12 (m, 1H).

**S68**

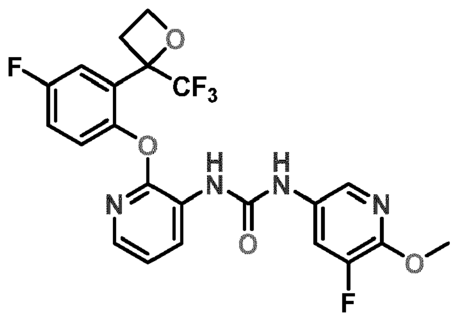

1-(2-(4-fluoro-2-(2-(trifluoromethyl)oxetan-2-yl)phenoxy)pyridin-3-yl)-3-(5-fluoro-6-methoxypyridin-3-yl)urea

Prepared according to the general procedure 6 using 2-(4-fluoro-2-(2-(trifluoromethyl)oxetan-2-yl)phenoxy)-3-isocyanatopyridine (40 mg, 1.0 eq.) and 5-fluoro-6-methoxy-pyridin-3-amine (19 mg, 1.2 eq.) to give the title material as a white solid (50.0 mg, 89%). UPLC-MS: m/z 497.1 $[M+H]^+$; $^1H$ NMR (400 MHz, DMSO-$d_6$) $\delta$ = 9.51 (s, 1H), 8.40 - 8.54 (m, 2H), 7.90 - 8.01 (m, 2H), 7.76 (dd, *J* = 1.65, 4.95 Hz, 1H), 7.36 (ddd, *J* = 3.30, 7.89, 8.99 Hz, 1H), 7.18 - 7.29 (m, 2H), 7.14 (dd, *J* = 4.77, 7.70 Hz, 1H), 4.72 (dt, *J* = 5.87, 7.89 Hz, 1H), 4.56 (td, *J*= 6.01, 9.26 Hz, 1H), 3.92 (s, 3H), 3.23 - 3.32 (m, 1H), 3.07 (ddd, *J* = 5.87, 8.44, 12.47 Hz, 1H).

**S57**

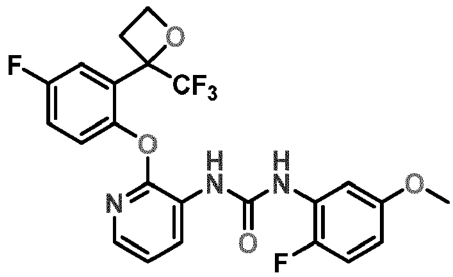

1-(2-(4-fluoro-2-(2-(trifluoromethyl)oxetan-2-yl)phenoxy)pyridin-3-yl)-3-(2-fluoro-5-methoxyphenyl)urea

Prepared according to the general procedure **6** 2-(4-fluoro-2-(2-(trifluoromethyl)oxetan-2-yl)phenoxy)-3-isocyanatopyridine (40 mg, 1.0 eq.) and 2-fluoro-5-methoxyaniline (19 mg, 1.2 eq.) to give the title material as a white solid (56 mg, 100%). UPLC-MS: m/z 496.1 $[M+H]^+$; $^1H$ NMR (400 MHz, DMSO-$d_6$) $\delta$ = 9.27 (d, *J* = 2.20 Hz, 1H), 8.96 (s, 1H), 8.52 (dd, *J* = 1.65, 7.89 Hz, 1H), 7.84 (dd, *J* = 3.12, 6.79 Hz, 1H), 7.76 (dd, *J* = 1.83, 4.77 Hz, 1H), 7.37 (ddd, *J* = 3.30, 7.89, 8.99 Hz, 1H), 7.21 - 7.29 (m, 2H), 7.10 - 7.21 (m, 2H), 6.57 (td, *J* = 3.58, 8.99 Hz, 1H), 4.66 - 4.80 (m, 1H), 4.57 (td, *J* = 6.01, 9.26 Hz, 1H), 3.73 (s, 3H), 3.32 - 3.41 (m, 1H), 3.05 (ddd, *J*= 6.05, 8.44, 12.65 Hz, 1H).

**S49**

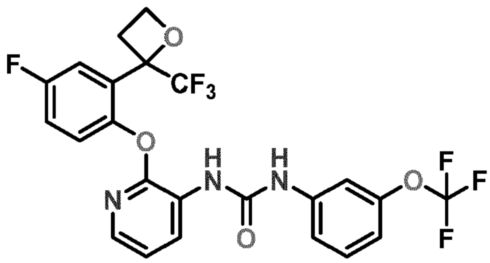

1-(2-(4-fluoro-2-(2-(trifluoromethyl)oxetan-2-yl)phenoxy)pyridin-3-yl)-3-(3-(trifluoromethoxy)phenyl)urea

Prepared according to the general procedure **6** using 2-(4-fluoro-2-(2-(trifluoromethyl)oxetan-2-yl)phenoxy)-3-isocyanatopyridine (40 mg, 1.0 eq.) and 3-trifluoromethoxyaniline (24 mg, 1.2 eq.) to give the title material as a white solid (51 mg, 85%). UPLC-MS: m/z 532.2 $[M+H]^+$; $^1H$ NMR (400 MHz, DMSO-$d_6$) $\delta$ = 9.64 (s, 1H), 8.48 (dd, *J* = 1.65, 7.89 Hz, 1H), 8.44 (s, 1H), 7.77 (dd, *J* = 1.83, 4.77 Hz, 1H), 7.73 (s, 1H), 7.32 - 7.46 (m, 2H), 7.20 - 7.32 (m, 3H), 7.14 (dd, *J* = 4.77, 8.07 Hz, 1H), 6.98 (d, *J* = 8.07 Hz, 1H), 4.64 - 4.77 (m, 1H), 4.56 (td, *J* = 5.87, 9.17 Hz, 1H), 3.22 - 3.31 (m, 1H), 3.07 (ddd, *J* = 5.87, 8.44, 12.47 Hz, 1H).

**S40**

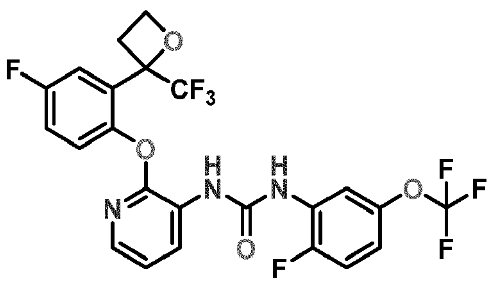

1-(2-(4-fluoro-2-(2-(trifluoromethyl)oxetan-2-yl)phenoxy)pyridin-3-yl)-3-(2-fluoro-5-(trifluoromethoxy)phenyl)urea

Prepared according to the general procedure 6 using 2-(4-fluoro-2-(2-(trifluoromethyl)oxetan-2-yl)phenoxy)-3-isocyanatopyridine (40 mg, 1.0 eq.) and 2-fluoro-5-trifluoromethoxyaniline (26 mg, 1.2 eq.) to give the title material as a white solid (31 mg, 50%). UPLC-MS: m/z 550.2 $[M+H]^+$; $^1H$ NMR (400 MHz, DMSO-$d_6$) $\delta$ = 9.40 (d, *J* = 1.83 Hz, 1H), 8.95 (s, 1H), 8.50 (dd, *J* = 1.83, 8.07 Hz, 1H), 8.28 (t, *J* = 9.17 Hz, 1H), 7.77 (dd, *J* = 1.83, 4.77 Hz, 1H), 7.48 (dd, *J* = 2.57, 11.37 Hz, 1H), 7.33 -7.41 (m, 1H), 7.20 - 7.29 (m, 3H), 7.15 (dd, *J* = 4.95, 7.89 Hz, 1H), 4.66 - 4.77 (m, 1H), 4.56 (td, *J* = 6.01, 9.26 Hz, 1H), 3.33 - 3.41 (m, 1H), 3.05 (ddd, *J* = 6.24, 8.34, 12.56 Hz, 1H).

**S21**

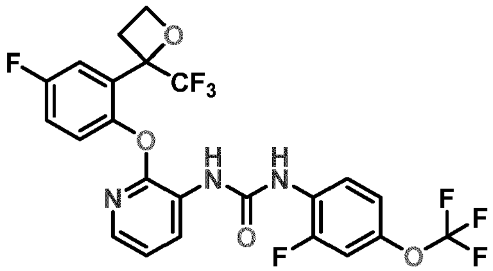

1-(2-(4-fluoro-2-(2-(trifluoromethyl)oxetan-2-yl)phenoxy)pyridin-3-yl)-3-(2-fluoro-4-(trifluoromethoxy)phenyl)urea

Prepared according to the general procedure **6** using 2-(4-fluoro-2-(2-(trifluoromethyl)oxetan-2-yl)phenoxy)-3-isocyanatopyridine (40 mg, 1.0 eq.) and 2-fluoro-4-trifluoromethoxyaniline (26 mg, 1.2 eq.) to give the title material as a white solid (43 mg, 69%). UPLC-MS: m/z 550.1 $[M+H]^+$; $^1H$ NMR (400 MHz, DMSO-$d_6$) $\delta$ = 9.55 (d, *J* = 2.20 Hz, 1H), 9.04 (s, 1H), 8.50 (dd, *J* = 1.65, 7.89 Hz, 1H), 8.29 (dd, *J* = 2.57, 6.60 Hz, 1H), 7.78 (dd, *J* = 1.83, 4.77 Hz, 1H), 7.31 - 7.46 (m, 2H), 7.22 - 7.31 (m, 2H), 7.15 (dd, *J* = 4.77, 8.07 Hz, 1H), 6.95 - 7.10 (m, 1H), 4.67 - 4.76 (m, 1H), 4.56 (td, *J* = 5.87, 9.17 Hz, 1H), 3.33 - 3.40 (m, 1H), 3.33 (s, 1H), 3.06 (ddd, *J* = 6.05, 8.44, 12.65 Hz, 1H).

**Example Series 3**

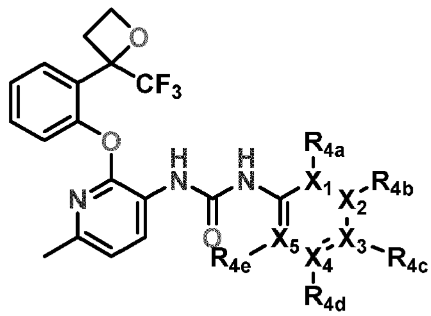

**S2**

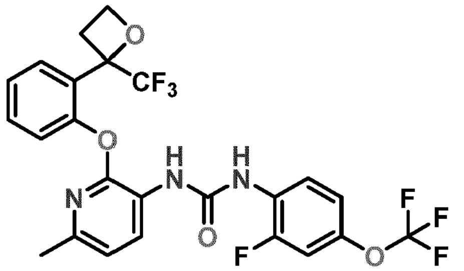

1-(2-fluoro-4-(trifluoromethoxy)phenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (70 mg, 1.0 eq.) and 2-fluoro-4-(trifluoromethoxy)aniline (47 mg, 1.2 eq.) to give the title material as a white solid (16 mg, 15%). LC-MS: m/z 546.0 $[M+H]^+$; $^1H$ NMR (400MHz, $CDCl_3$) $\delta$ = 8.43 (d, *J* = 8.1 Hz, 1H), 8.24 (t, *J* = 9.1 Hz, 1H), 7.82 - 7.70 (m, 2H), 7.47 - 7.37 (m, 2H), 7.07 - 6.92 (m, 4H), 6.59 (br s, 1H), 5.00 - 4.93 (m, 1H), 4.75 - 4.68 (m, 1H), 3.24 (t, *J* = 7.6 Hz, 2H), 2.43 (s, 3H).

**S4**

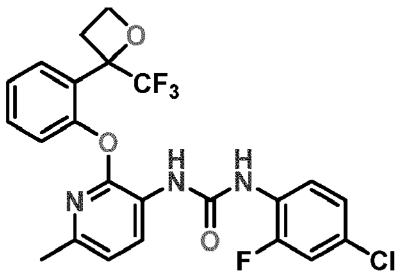

1-(4-chloro-2-fluorophenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (70 mg, 1.0 eq.) and 2-fluoro-4-chloroaniline (35 mg, 1.2 eq.) to give the title material as a white solid (24 mg, 24%). LC-MS: m/z 496.2 $[M+H]^+$; $^1H$ NMR (400MHz, $CDCl_3$) $\delta$ = 8.43 (d, *J* = 8.1 Hz, 1H), 8.15 (t, *J* = 8.6 Hz, 1H), 7.78 (br. s., 1H), 7.70 (d, *J* = 7.9 Hz, 1H), 7.48 - 7.40 (m, 2H), 7.26 - 7.20 (m, 1H), 7.17 - 7.09 (m, 2H), 6.94 (d, *J* = 8.0 Hz, 1H), 6.55 (br. s., 1H), 4.96 (d, *J* = 5.8 Hz, 1H), 4.71 (d, *J* = 5.5 Hz, 1H), 3.24 (t, *J* = 7.8 Hz, 2H), 2.42 (s, 3H).

**S7**

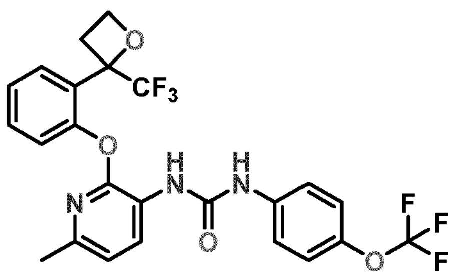

1-(6-methyl-2-(2-(2-(trifluoromethyl)oxetan-2-yl)phenoxy)pyridin-3-yl)-3-(4-(trifluoromethoxy)phenyl)urea

Prepared according to **general procedure 8** using 6-methyl-2-(2-(2-(trifluoromethyl)oxetan-2-yl)phenoxy)pyridin-3-amine (100 mg, 1.0 eq.) and 1-isocyanato-4-(trifluoromethoxy)benzene (63 mg, 1.0 eq.) to give the title material

as a white solid (58 mg, 35%). LC-MS: m/z 528.0 $[M+H]^+$; $^1H$ NMR (400MHz, $CDCl_3$) $\delta$ = 8.43 (d, *J* = 8.1 Hz, 1H), 7.50 (d, *J* = 7.6 Hz, 1H), 7.44 -7.37 (m, 3H), 7.33 (br d, *J* = 7.1 Hz, 2H), 7.27 -7.24 (m, 1H), 7.18 (br d, *J* = 8.6 Hz, 2H), 6.94 (d, *J* = 8.1 Hz, 1H), 6.40 (s, 1H), 4.91 - 4.82 (m, 1H), 4.67 - 4.59 (m, 1H), 3.21 - 3.12 (m, 2H), 2.40 (s, 3H).

**S18**

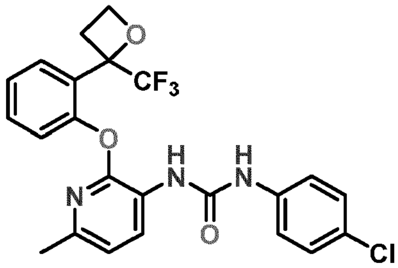

1-(4-chlorophenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (70 mg, 1.0 eq.) and 4-chloroaniline (31 mg, 1.2 eq.) to give the title material as a white solid (58 mg, 61%). LC-MS: m/z 478.3 $[M+H]^+$; $^1H$ NMR (400MHz, $CDCl_3$) $\delta$ = 8.44 (d, *J* = 7.9 Hz, 1H), 7.51 (d, *J* = 7.9 Hz, 1H), 7.46 - 7.36 (m, 1H), 7.36 - 7.20 (m, 7H), 6.94 (d, *J* = 8.3 Hz, 1H), 6.31 (br. s., 1H), 4.96 - 4.78 (m, 1H), 4.73 - 4.56 (m, 1H), 3.15 (t, *J* = 7.6 Hz, 2H), 2.39 (s, 3H).

**S44**

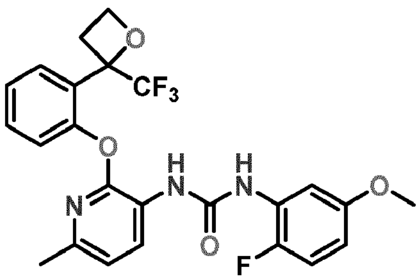

1-(2-fluoro-5-methoxyphenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (100 mg, 1.0 eq.) and 2-fluoro-5-methoxyaniline (60 mg, 1.5 eq.) to give the title material as a white solid (18 mg, 19%). LC-MS: m/z 492.0 $[M+H]^+$; $^1H$ NMR (400MHz, $CDCl_3$) $\delta$ = 8.46 (d, *J* = 8.1 Hz, 1H), 7.54 (d, *J* =7.7 Hz, 1H), 7.47 -7.37 (m, 1H), 7.29 -7.24 (m, 4H), 7.09 - 6.93 (m, 2H), 6.72 (td, *J* = 3.1, 8.7 Hz, 1H), 6.36 (s, 1H), 4.92 - 4.59 (m, 2H), 3.90 (s, 3H), 3.15 (t, *J* = 7.5 Hz, 2H), 2.41 (s, 3H).

**S67**

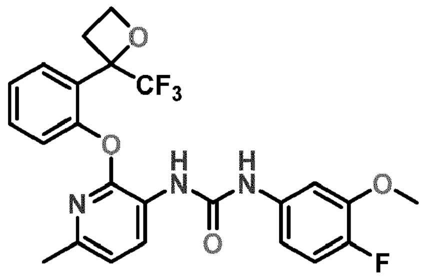

1-(4-fluoro-3-methoxyphenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (60 mg, 1.0 eq.) and 4-fluoro-3-methoxyaniline (60 mg, 1.5 eq.) to give the title material as a white solid (33 mg, 24%). LC-MS: m/z 492.1 $[M+H]^+$; $^1H$ NMR (400MHz, $CDCl_3$) $\delta$ = 8.47 (d, *J* = 8.1 Hz, 1H), 7.89 - 7.66 (m, 3H), 7.51 - 7.40 (m, 2H), 7.29 - 7.22 (m, 1H), 7.08 - 6.93 (m, 2H), 6.70 - 6.52 (m, 2H), 5.08 - 4.62 (m, 2H), 4.04 - 3.64 (m, 3H), 3.25 (t, *J*= 7.8 Hz, 2H), 2.44 (s, 3H).

**S15**

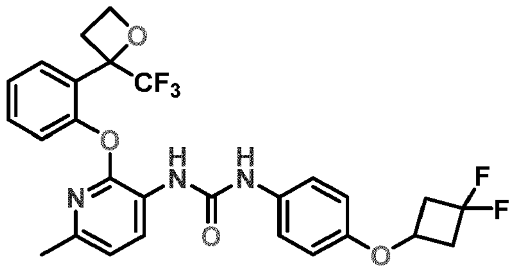

1-(4-(3,3-difluorocyclobutoxy)phenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (50 mg, 1.0 eq.) and 4-(3,3-difluorocyclobutoxy)aniline (34 mg, 1.2 eq.) to give the title material as a white solid (18 mg, 23%). LC-MS: m/z 492.1 $[M+H]^+$; $^1H$ NMR (400MHz, $CDCl_3$) $\delta$ = 8.45 (d, *J* = 8.1 Hz, 1H), 7.51 (d, *J* = 8.1 Hz, 1H), 7.40 - 7.33 (m, 1H), 7.25 - 7.24 (m, 1H), 7.23 - 7.20 (m, 2H), 7.13 (d, *J* = 7.5 Hz, 1H), 7.08 (s, 1H), 6.91 (d, *J* = 8.0 Hz, 1H), 6.78 - 6.71 (m, 2H), 6.17 (s, 1H), 4.88 - 4.76 (m, 1H), 4.63 - 4.51 (m, 2H), 3.15 - 2.97 (m, 4H), 2.72 (ddt, *J* = 5.5, 13.0, 15.4 Hz, 2H), 2.35 (s, 3H).

**S17**

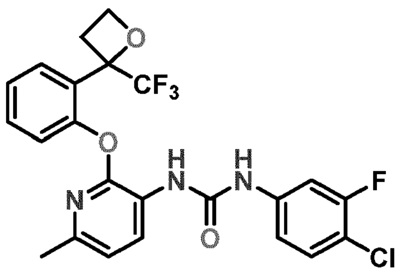

1-(4-chloro-3-fluorophenyl)-3-(6-methyl-2-(2-(2-(trifluoromethyl)oxetan-2-yl)phenoxy)pyridin-3-yl)urea

Prepared according to **general procedure 4** using 3-isocyanato-6-methyl-2-(2-(2-(trifluoromethyl)oxetan-2-yl)phenoxy)pyridine (75 mg, 1.0 eq.) and 4-chloro-3-fluoroaniline (62 mg, 2.0 eq.) to give the title material as a white solid (48 mg, 45%). LC-MS: m/z 496.1 $[M+H]^+$; $^1H$ NMR (400MHz, $CDCl_3$) δ = 8.41 (d, *J* = 8.0 Hz, 1H), 7.54 -7.35 (m, 5H), 7.33 - 7.23 (m, 2H), 7.05 - 6.97 (m, 1H), 6.94 (d, *J* = 8.1 Hz, 1H), 6.56 (s, 1H), 4.94 - 4.84 (m, 1H), 4.72 - 4.60 (m, 1H), 3.30 - 3.07 (m, 2H), 2.41 (s, 3H).

**Example Series 4**

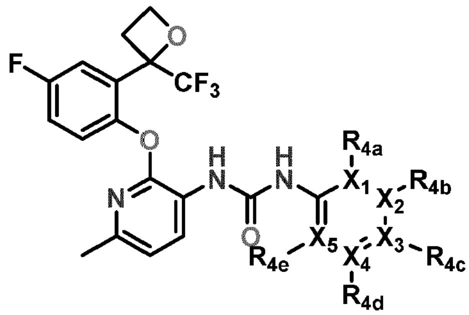

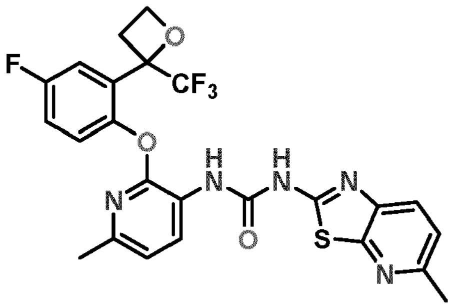

1-(2-(4-fluoro-2-(2-(trifluoromethyl)oxetan-2-yl)phenoxy)-6-methylpyridin-3-yl)-3-(5-methylthiazolo[5,4-*b*]pyridin-2-yl)urea

**S34**

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 5-chlorothiazolo[5,4-b]pyridin-2-amine (21 mg, 1.2 eq.) to give the title material as an off-white solid (43 mg, 53%). UPLC-MS: m/z 554.1/556.0 $[M+H]^+$; $^1H$ NMR (400 MHz, DMSO-$d_6$) δ = 11.76 (br. s., 1H) 8.78 (s, 1H) 8.33 (d, *J* = 7.70 Hz, 1H) 8.05 (d, *J* = 8.44 Hz, 1H) 7.53 (d, *J* = 8.44 Hz, 1H) 7.31 - 7.42 (m, 1H) 7.18 - 7.28 (m, 2H) 7.05 (d, *J* = 8.07 Hz, 1H) 4.66 - 4.82 (m, 1H) 4.56 (dt, *J* = 9.26, 5.82 Hz, 1H) 3.20 - 3.28 (m, 1H) 3.10 (ddd, *J* = 12.75, 8.34, 6.05Hz, 1H) 2.27(s, 3H).

**S56**

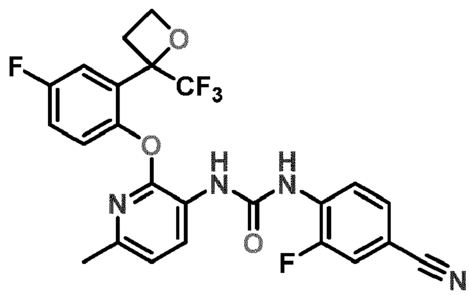

1-(4-cyano-2-fluorophenyl)-3-(2-(4-fluoro-2-(2-(trifluoromethyl)oxetan-2-yl)phenoxy)-6-methylpyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 4-amino-3-fluoro-benzonitrile (16 mg, 1.2 eq.) to give the title material as a white solid (14 mg, 29%). UPLC-MS: 505.2 $[M+H]^+$; $^1H$ NMR (400 MHz, DMSO-$d_6$) $\delta$ = 9.61 (br. s., 1H), 8.98 (s, 1H), 8.43 (t, *J* = 8.25 Hz, 1H), 8.34 (d, *J* = 7.70 Hz, 1H), 7.88 (dd, *J* = 1.83, 11.37 Hz, 1H), 7.66 (dd, *J* = 1.28, 8.62 Hz, 1H), 7.36 (ddd, *J* = 3.30, 7.98, 8.89 Hz, 1H), 7.25 (dd, *J* = 3.30, 9.17 Hz, 1H), 7.19 (dd, *J* = 4.77, 8.80 Hz, 1H), 7.02 (d, *J* = 8.07 Hz, 1H), 4.66 - 4.78 (m, 1H), 4.57 (td, *J* = 5.73, 9.45 Hz, 1H), 3.29 - 3.37 (m, 1H), 3.05 (ddd, *J* = 6.24, 8.25, 12.65 Hz, 1H), 2.25 (s, 3H).

**S66**

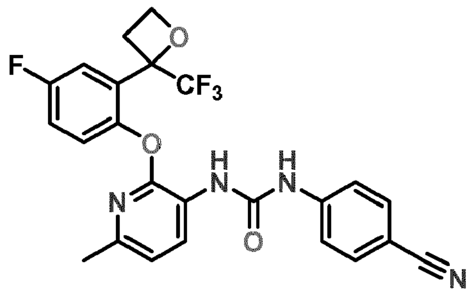

1-(4-cyanophenyl)-3-(2-(4-fluoro-2-(2-(trifluoromethyl)oxetan-2-yl)phenoxy)-6-methylpyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 4-aminobenzonitrile (14 mg, 1.2 eq) to give the title material as a white solid (37 mg, 80%). UPLC-MS: 487.2 $[M+H]^+$; $^1H$ NMR (400 MHz, DMSO-$d_6$) $\delta$ = 9.75 (s, 1H), 8.37 - 8.50 (m, 1H), 8.26 - 8.33 (m, 1H), 7.69 - 7.80 (m, 2H), 7.59 - 7.68 (m, 2H), 7.35 (ddd, *J* = 3.30, 7.98, 8.89 Hz, 1H), 7.25 (dd, *J* = 2.93, 9.17 Hz, 1H), 7.19 (dd, *J* = 4.77, 8.80 Hz, 1H), 7.01 (d, *J* = 8.07 Hz, 1H), 4.66 - 4.78 (m, 1H), 4.56 (td, *J* = 5.73, 9.45 Hz, 1H), 3.23 - 3.31 (m, 1H), 3.06 (ddd, *J* = 5.87, 8.44, 12.47 Hz, 1H), 2.25 (s, 3H).

**S54**

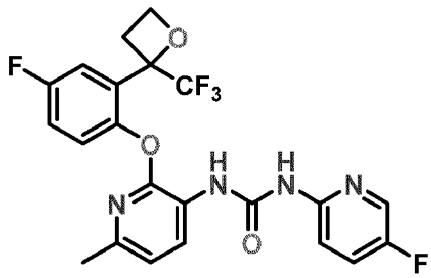

1-(2-(4-fluoro-2-(2-(trifluoromethyl)oxetan-2-yl)phenoxy)-6-methylpyridin-3-yl)-3-(5-fluoropyridin-2-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) 5-fluoropyridin-2-amine (18 mg, 1.2 eq.) to give the title material as a white solid (34 mg, 65%). UPLC-MS: 481.1 $[M+H]^+$; $^1H$ NMR (400 MHz, DMSO-$d_6$) $\delta$ = 10.12 (br. s., 1H), 9.98 (s, 1H), 8.43 (d, *J* = 8.07 Hz, 1H), 8.03 (d, *J* = 2.93 Hz, 1H), 7.74 (ddd, *J* = 3.12, 8.25, 9.17 Hz, 1H), 7.53 (d, *J* = 5.50 Hz, 1H), 7.34 (ddd, *J* = 3.30, 7.89, 8.99 Hz, 1H), 7.26 (dd, *J* = 3.30, 9.17 Hz, 1H), 7.15 (dd, *J* = 4.77, 9.17 Hz, 1H), 7.02 (d, *J* = 8.07 Hz, 1H), 4.71 (dt, *J* = 5.87, 7.89 Hz, 1H), 4.56 (td, *J* = 5.87, 9.17 Hz, 1H), 3.21 -3.31 (m, 1H), 3.05 (ddd, *J* = 6.24, 8.34, 12.56 Hz, 1H), 2.25 (s, 3H).

**S48**

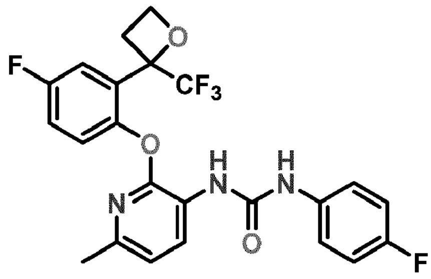

1-(2-(4-fluoro-2-(2-(trifluoromethyl)oxetan-2-yl)phenoxy)-6-methylpyridin-3-yl)-3-(4-fluorophenyl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 4-fluoroaniline (18 mg, 1.2 eq.) to give the title material as a white solid (40 mg, 62%). UPLC-MS: 480.2 $[M+H]^+$; $^1H$ NMR (400 MHz, DMSO-$d_6$) $\delta$ = 9.28 (s, 1H), 8.33 (d, *J* = 8.07 Hz, 1H), 8.20 (s, 1H), 7.40 - 7.50 (m, 2H), 7.35 (ddd, *J* = 3.12, 7.98, 9.08 Hz, 1H), 7.25 (dd, *J* = 3.12, 8.99 Hz, 1H), 7.09 - 7.20 (m, 3H), 6.99 (d, *J* = 8.44 Hz, 1H), 4.66 - 4.78 (m, 1H), 4.57 (td, *J* = 5.87, 9.17 Hz, 1H), 3.26 - 3.31 (m, 1H), 3.06 (ddd, *J* = 5.87, 8.44, 12.47 Hz, 1H), 2.24 (s, 3H).

**S16**

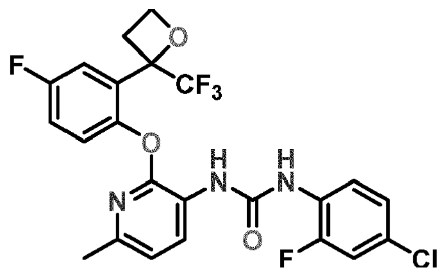

1-(4-chloro-2-fluorophenyl)-3-(2-(4-fluoro-2-(2-(trifluoromethyl)oxetan-2-yl)phenoxy)-6-methylpyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) 4-chloro2-fluoroaniline (24 mg, 1.2 eq.) to give the title material as a white solid (40 mg, 57%). UPLC-MS: 514.1/516.1 $[M+H]^+$; $^1H$ NMR (400 MHz, DMSO-$d_6$) $\delta$ = 9.25 (d, *J* = 2.20 Hz, 1H), 8.79 (s, 1H), 8.34 (d, *J* = 8.07 Hz, 1H), 8.19 (t, *J* = 8.99 Hz, 1H), 7.47 (dd, *J* = 2.38, 11.19 Hz, 1H), 7.35 (ddd, *J* = 3.30, 7.89, 8.99 Hz, 1H), 7.23 - 7.29 (m, 2H), 7.18 (dd, *J* = 4.77, 9.17 Hz, 1H), 7.00 (d, *J* = 8.07 Hz, 1H), 4.66-4.78 (m, 1H), 4.57 (td, *J* = 5.87, 9.17 Hz, 1H), 3.36 (d, *J* = 3.67 Hz, 1H), 3.05 (ddd, *J* = 6.05, 8.44, 12.65 Hz, 1H), 2.24 (s, 3H).

**S32**

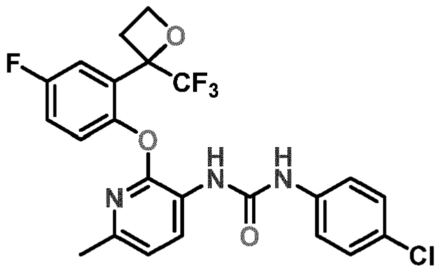

1-(4-chlorophenyl)-3-(2-(4-fluoro-2-(2-(trifluoromethyl)oxetan-2-yl)phenoxy)-6-methylpyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 4-chloroaniline (23 mg, 1.2 eq.) to give the title material as a white solid (50 mg, 68%). UPLC-MS: 496.1/498.1 $[M+H]^+$; $^1H$ NMR (400 MHz, DMSO-$d_6$) $\delta$ = 9.37 (s, 1H), 8.33 (d, *J* = 8.07 Hz, 1H), 8.25 (s, 1H), 7.43 - 7.53 (m, 2H), 7.31 - 7.39 (m, 3H), 7.25 (dd, *J* = 3.30, 9.17 Hz, 1H), 7.18 (dd, *J* = 4.77, 9.17 Hz, 1H), 7.00 (d, *J* = 8.07 Hz, 1H), 4.72 (dt, *J* = 5.87, 8.07 Hz, 1H), 4.56 (td, *J* = 5.78, 9.35 Hz, 1H), 3.24 - 3.32 (m, 1H), 3.06 (ddd, *J* = 6.24, 8.34, 12.56 Hz, 1H), 2.24 (s, 3H).

**S77**

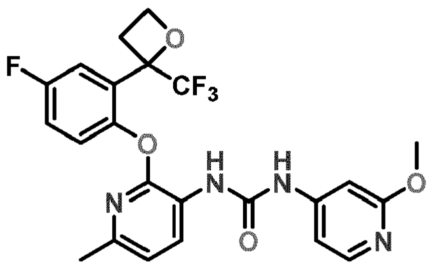

1-(2-(4-fluoro-2-(2-(trifluoromethyl)oxetan-2-yl)phenoxy)-6-methylpyridin-3-yl)-3-(2-methoxypyridin-4-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 2-methoxypyridin-4-amine (22 mg, 1.2 eq) to give the title material as a white solid (47 mg, 64%). UPLC-MS: 493.2 $[M+H]^+$; $^1H$ NMR (400 MHz, $CDCl_3$) $\delta$ = 8.39 (d, J = 7.98 Hz, 1H), 8.02 (d, J = 5.69 Hz, 1H), 7.45 (s, 1H), 7.19 - 7.29 (m, 2H), 7.05 - 7.17 (m, 2H), 6.84 - 6.96 (m, 3H), 4.84 (dt, J = 5.73, 7.86 Hz, 1H), 4.51 - 4.69 (m, 1H), 3.92 (s, 3H), 3.02 - 3.18 (m, 2H), 2.37 (s, 3H).

**S41**

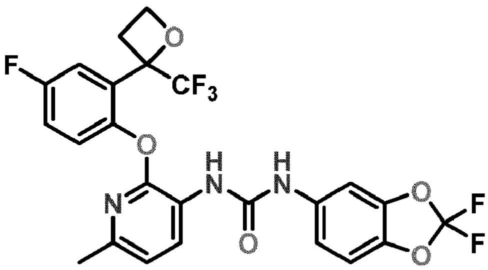

1-(2,2-difluorobenzo[*d*][1,3]dioxol-5-yl)-3-(2-(4-fluoro-2-(2-(trifluoromethyl)oxetan-2-yl)phenoxy)-6-methylpyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 2,2-Difluoro-5-amino benzodioxole (31 mg. 1.2 eq.) to give the title material as a white solid (54 mg, 81%). UPLC-MS: m/z 542.1 $[M+H]^+$; $^1H$ NMR (400 MHz, DMSO-$d_6$) $\delta$ = 9.45 (s, 1H), 8.32 (d, *J* = 8.07 Hz, 1H), 8.25 (s, 1H), 7.68 (d, *J* = 2.20 Hz, 1H), 7.29 - 7.39 (m, 2H), 7.25 (dd, *J* = 3.30, 9.17 Hz, 1H), 7.18 (dd, *J* = 4.77, 8.80 Hz, 1H), 7.05 (dd, *J* = 2.20, 8.80 Hz, 1H), 7.00 (d, *J* = 8.44 Hz, 1H), 4.72 (dt, *J* = 5.87, 7.89 Hz, 1H), 4.56 (td, *J* = 5.87, 9.17 Hz, 1H), 3.24-3.32 (m, 1H), 3.06 (ddd, *J* = 6.05, 8.34, 12.75 Hz, 1H), 2.24 (s, 3H).

**S42**

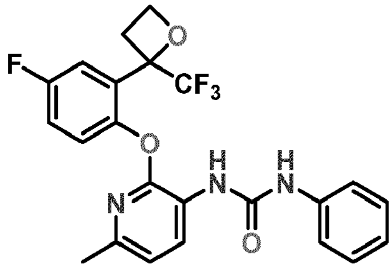

1-(2-(4-fluoro-2-(2-(trifluoromethyl)oxetan-2-yl)phenoxy)-6-methylpyridin-3-yl)-3-phenylurea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and aniline (17 mg, 1.2 eq.) to give the title material as a white solid (51 mg, 69%). UPLC-MS: m/z 462.2 $[M+H]^+$; $^1H$ NMR (400 MHz, DMSO-$d_6$) $\delta$ = 9.24 (s, 1H), 8.36 (d, *J* = 7.98 Hz, 1H), 8.23 (s, 1H), 7.39 - 7.49 (m, 2H), 7.22 - 7.39 (m, 4H), 7.18 (dd, *J* = 4.72, 9.03 Hz, 1H), 6.92 - 7.04 (m, 2H), 4.66 - 4.79 (m, 1H), 4.57 (td, *J* = 5.86, 9.28 Hz, 1H), 3.25 - 3.40 (m, 1H), 3.06 (ddd, *J*= 6.01, 8.32, 12.63 Hz, 1H), 2.20 - 2.30 (m, 3H).

**S19**

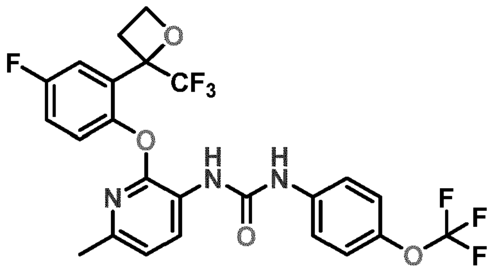

1-(2-(4-fluoro-2-(2-(trifluoromethyl)oxetan-2-yl)phenoxy)-6-methylpyridin-3-yl)-3-(4-(trifluoromethoxy)phenyl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 4-(trifluoromethoxy)aniline (32 mg, 1.2 eq.) to give the title material as a white solid (79 mg, 82%). UPLC-MS: m/z 546.2 $[M+H]^+$; $^1H$ NMR (400 MHz, DMSO-$d_6$) $\delta$ = 9.44 (s, 1H), 8.33 (d, *J* = 8.07 Hz, 1H), 8.26 (s, 1H), 7.50 - 7.64 (m, 2H), 7.22 - 7.40 (m, 4H), 7.19 (dd, J = 4.77, 9.08 Hz, 1H), 7.00 (d, *J* = 8.16 Hz, 1H), 4.72 (dt, *J* = 5.78, 7.93 Hz, 1H), 4.56 (td, *J* = 5.88, 9.33 Hz, 1H), 3.23 - 3.38 (m, 1H), 3.06 (ddd, J = 6.14, 8.46, 12.63 Hz, 1H), 2.24 (s, 3H).

**S75**

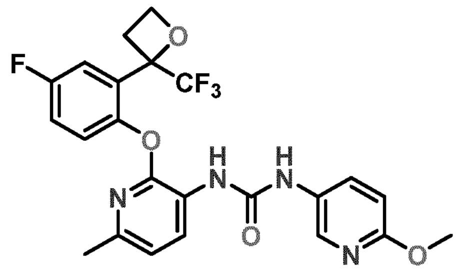

1-(2-(4-fluoro-2-(2-(trifluoromethyl)oxetan-2-yl)phenoxy)-6-methylpyridin-3-yl)-3-(6-methoxypyridin-3-yl)urea

Prepared according to the general procedure **6** using 2-(4-fluoro-2-(2-(trifluoromethyl)oxetan-2-yl)phenoxy)-3-isocyanato-6-methylpyridine (35 mg, 1.0 eq.) and 6-methoxypyridin-3-amine (22 mg, 1.2 eq.) to give the title material as a white solid (66 mg, 74%). UPLC-MS: m/z 493.2 $[M+H]^+$; $^1H$ NMR (400 MHz, DMSO-$d_6$) δ = 9.17 (s, 1H), 8.33 (d, *J* = 8.07 Hz, 1H), 8.22 (s, 1H), 8.19 (d, *J* = 2.38 Hz, 1H), 7.82 (dd, *J* = 2.84, 8.89 Hz, 1H), 7.35 (ddd, *J* = 3.26, 7.93, 8.99 Hz, 1H), 7.25 (dd, *J* = 3.12, 9.17 Hz, 1H), 7.17 (dd, *J* = 4.72, 9.03 Hz, 1H), 6.99 (d, *J* = 8.16 Hz, 1H), 6.80 (d, *J* = 8.80 Hz, 1H), 4.65 - 4.79 (m, 1H), 4.57 (td, *J* = 5.89, 9.31 Hz, 1H), 3.25 - 3.32 (m, 1H), 3.07 (ddd, *J* = 6.05, 8.39, 12.61 Hz, 1H), 2.24 (s, 3H).

**Example Series 5**

**S45**

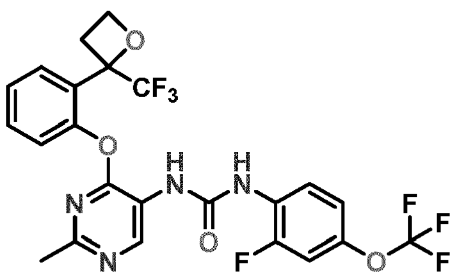

1-(2-fluoro-4-(trifluoromethoxy)phenyl)-3-(2-methyl-4-(2-(2-(trifluoromethyl)oxetan-2-yl)phenoxy)pyrimidin-5-yl)urea

Prepared according to **general procedure 4** using 5-isocyanato-2-methyl-4-(2-(2-(trifluoromethyl)oxetan-2-yl)phenoxy)pyrimidine (70 mg, 1.0 eq.) and 2-fluoro-4-(trifluoromethoxy)aniline (47 mg, 1.2 eq.) to give the title material as a white solid (23 mg, 11%). LC-MS: m/z 547.3 $[M+H]^+$; $^1H$ NMR (400MHz, $CDCl_3$) δ = 8.25 (t, *J* = 9.1 Hz, 1H), 7.89 (br d, *J* = 8.5 Hz, 1H), 7.74 (br s, 1H), 7.59 - 7.47 (m, 1H), 7.44 - 7.31 (m, 3H), 7.14 - 6.96 (m, 2H), 6.69 (br s, 1H), 5.04 - 4.91 (m, 1H), 4.77 - 4.63 (m, 1H), 3.35 - 3.10 (m, 2H), 2.61 (s, 3H).

**S58**

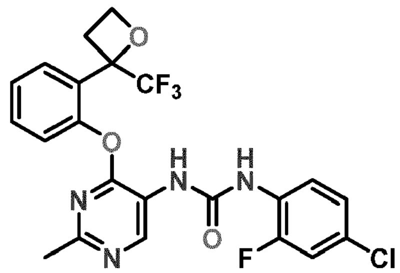

1-(4-chloro-2-fluorophenyl)-3-(2-methyl-4-(2-(2-(trifluoromethyl)oxetan-2-yl)phenoxy)pyrimidin-5-yl)urea

Prepared according to **general procedure 4** using 5-isocyanato-2-methyl-4-(2-(2-(trifluoromethyl)oxetan-2-yl)phenoxy)pyrimidine (70 mg, 1.0 eq.) and 4-chloro-2-fluoroaniline (35 mg, 1.2 eq.) to give the title material as a white solid (34 mg, 32%). LC-MS: m/z 497.0 $[M+H]^+$; $^1H$ NMR (400MHz, $CDCl_3$) $\delta$ = 9.32 (s, 1H), 8.17 (t, *J* = 8.9 Hz, 1H), 7.85 (d, *J* = 8.3 Hz, 1H), 7.69 (br s, 1H), 7.52 (dt, *J* = 2.0, 7.8 Hz, 1H), 7.41 - 7.30 (m, 2H), 7.20 - 7.07 (m, 2H), 6.69 (br s, 1H), 4.95 (dt, *J* = 5.7, 7.9 Hz, 1H), 4.69 (td, *J* = 5.7, 9.4 Hz, 1H), 3.32 - 3.09 (m, 2H), 2.59 (s, 3H).

**S81**

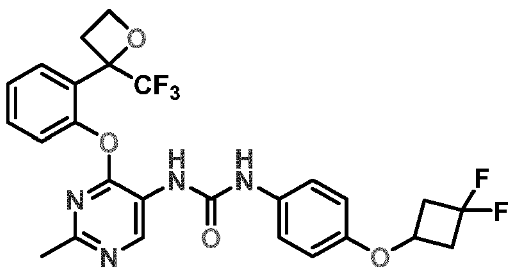

1-(4-(3,3-difluorocyclobutoxy)phenyl)-3-(2-methyl-4-(2-(2-(trifluoromethyl)oxetan-2-yl)phenoxy)pyrimidin-5-yl)urea

Prepared according to **general procedure 4** 5-isocyanato-2-methyl-4-(2-(2-(trifluoromethyl)oxetan-2-yl)phenoxy)pyrimidine (50 mg, 1.4 eq.) and 4-(3,3-difluorocyclobutoxy)aniline (20 mg, 1.2 eq.) to give the title material as a white solid (24 mg, 24%). LC-MS: m/z 551.0 $[M+H]^+$; $^1H$ NMR (400MHz, $CDCl_3$) $\delta$ = 9.35 (s, 1H), 7.54 - 7.43 (m, 2H), 7.38 - 7.28 (m, 4H), 7.14 - 6.96 (m, 1H), 6.81 (d, *J* = 8.8 Hz, 2H), 6.64 - 6.33 (m, 1H), 4.84 - 4.71 (m, 1H), 4.61 (br s, 1H), 4.54 - 4.45 (m, 1H), 3.15 - 2.99 (m, 3H), 2.96 (br s, 1H), 2.83 - 2.64 (m, 2H), 2.53 (s, 3H).

**S59**

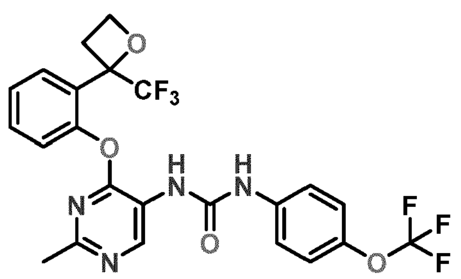

1-(2-methyl-4-(2-(2-(trifluoromethyl)oxetan-2-yl)phenoxy)pyrimidin-5-yl)-3-(4-(trifluoromethoxy)phenyl)urea

Prepared according to **general procedure 8** using 2-methyl-4-(2-(2-(trifluoromethyl)oxetan-2-yl)phenoxy)pyrimidin-5-amine (100 mg, 1.0 eq.) and 1-isocyanato-4-(trifluoromethoxy)benzene (47 mg, 1.2 eq.) to give the title material as a white solid (36 mg, 22%). LC-MS: m/z 529.0 $[M+H]^+$; $^1H$ NMR (400MHz, $CDCl_3$) $\delta$ = 9.31 (s, 1H), 7.54 - 7.39 (m, 5H), 7.38 - 7.31 (m, 1H), 7.30 (br s, 1H), 7.21 (br d, *J* = 8.5 Hz, 2H), 6.84 (br s, 1H), 4.87 - 4.75 (m, 1H), 4.56 (td, J=5.8, 9.2 Hz, 1H), 3.21 - 2.99 (m, 2H), 2.55 (s, 3H).

**S69**

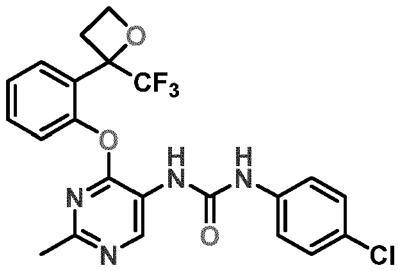

1-(4-chlorophenyl)-3-(2-methyl-4-(2-(2-(trifluoromethyl)oxetan-2-yl)phenoxy)pyrimidin-5-yl)urea

Prepared according to **general procedure 4** using 5-isocyanato-2-methyl-4-(2-(2-(trifluoromethyl)oxetan-2-yl)phenoxy)pyrimidine (70 mg, 1.0 eq.) and 4-chloroaniline (31 mg, 1.2 eq.) to give the title material as a white solid (19 mg, 20%). LC-MS: m/z 479.0 $[M+H]^+$; $^1$H NMR (400MHz, $CDCl_3$) $\delta$ = 9.31 (s, 1H), 7.55 - 7.42 (m, 3H), 7.40 - 7.29 (m, 5H), 7.20 (br s, 1H), 6.65 (br s, 1H), 4.91 - 4.73 (m, 1H), 4.64 - 4.47 (m, 1H), 3.21 - 2.95 (m, 2H), 2.54 (s, 3H).

**S64**

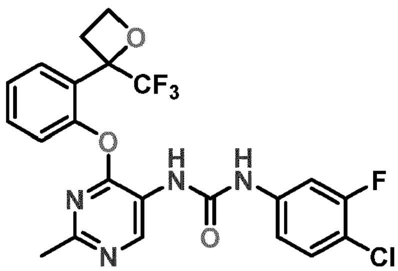

1-(4-chloro-3-fluorophenyl)-3-(2-methyl-4-(2-(2-(trifluoromethyl)oxetan-2-yl)phenoxy)pyrimidin-5-yl)urea

Prepared according to **general procedure 4** using 5-isocyanato-2-methyl-4-(2-(2-(trifluoromethyl)oxetan-2-yl)phenoxy)pyrimidine (75 mg, 1 eq.) and 4-chloro-3-fluoroaniline (31 mg, 1.0 eq.) to give the title material as an off-white solid (38 mg, 36%). LC-MS: m/z 497.1 $[M+H]^+$; $^1$H NMR (400MHz, $CDCl_3$) $\delta$ = 9.28 (s, 1H), 7.55 -7.40 (m, 4H), 7.40 - 7.30 (m, 2H), 7.24 (s, 1H), 7.05 (d, *J* = 7.4 Hz, 1H), 6.74 (s, 1H), 4.97 - 4.75 (m, 1H), 4.58 (td, *J* = 5.8, 9.3 Hz, 1H), 3.26 - 3.12 (m, 1H), 3.06 (t, *J* = 8.6 Hz, 1H), 2.67 - 2.52 (m, 3H.

**Example Series 6**

**S20**

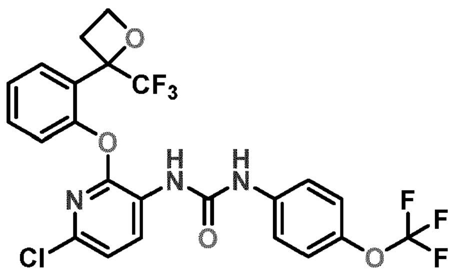

1-(6-chloro-2-(2-(2-(trifluoromethyl)oxetan-2-yl)phenoxy)pyridin-3-yl)-3-(4-(trifluoromethoxy)phenyl)urea

Prepared according to **general procedure 1** using 6-chloro-2-(2-(2-(trifluoromethyl)oxetan-2-yl)phenoxy)pyridin-3-amine (230 mg, 1.0 eq.) and 1-isocyanato-4-(trifluoromethoxy)benzene (407 mg, 3.0 eq.) to give the title material as a yellow oil (49 mg, 13%). LC-MS: m/z 548.1 $[M+H]^+$; $^1H$ NMR (400MHz, $CDCl_3$) $\delta$ = 8.56 (d, *J* = 8.4 Hz, 1H), 7.52 - 7.38 (m, 6H), 7.33 - 7.27 (m, 1H), 7.20 (d, *J* = 8.6 Hz, 2H), 7.09 (d, *J* = 8.4 Hz, 1H), 6.51 (s, 1H), 4.93 - 4.57 (m, 2H), 3.22 - 3.07 (m, 2H).

**S14**

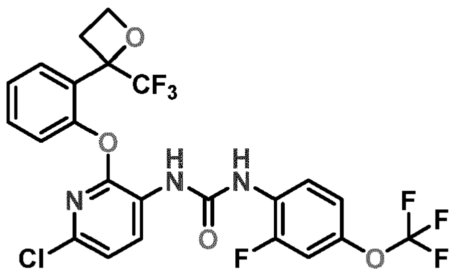

1-(6-chloro-2-(2-(2-(trifluoromethyl)oxetan-2-yl)phenoxy)pyridin-3-yl)-3-(2-fluoro-4-(trifluoromethoxy)phenyl)urea

Prepared according to **general procedure 4** using 6-chloro-2-(2-(2-(trifluoromethyl)oxetan-2-yl)phenoxy)pyridin-3-amine (344 mg, 1.0 eq.) and 2-fluoro-4-(trifluoromethoxy)aniline (272 mg, 1.5 eq.) to give the title material as a white solid. (161 mg, 31%). LC-MS: m/z 566.1 $[M+H]^+$; $^1H$ NMR (400MHz, $CDCl_3$) $\delta$ = 8.48 (d, *J* = 8.4 Hz, 1H), 8.22 - 8.13 (m, 1H), 7.98 (br s, 1H), 7.80 (br d, *J* = 8.3 Hz, 1H), 7.46 - 7.39 (m, 1H), 7.31 - 7.20 (m, 2H), 7.05 - 6.91 (m, 3H), 6.59 (br d, *J* = 3.1 Hz, 1H), 4.99 - 4.86 (m, 1H), 4.67 (td, *J* = 5.8, 9.2 Hz, 1H), 3.26 -3.08 (m, 2H).

**S22**

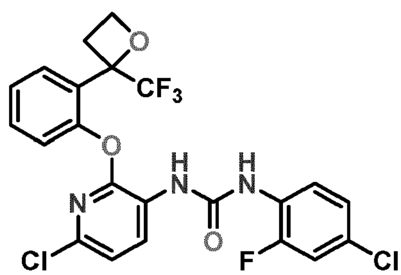

1-(6-chloro-2-(2-(2-(trifluoromethyl)oxetan-2-yl)phenoxy)pyridin-3-yl)-3-(4-chloro-2-fluorophenyl)urea

Prepared according to **general procedure 4** using 6-chloro-2-(2-(2-(trifluoromethyl)oxetan-2-yl)phenoxy)pyridin-3-amine (322 mg, 1.0 eq.) and 4-chloro-2-fluoro-aniline (190 mg, 1.5 eq.) to give the title material as a white solid. (39 mg, 9%). LC-MS: m/z 516.1 $[M+H]^+$; $^1H$ NMR (400MHz, $CDCl_3$) $\delta$ = 8.58 (d, *J* = 8.4 Hz, 1H), 8.22 - 8.12 (m, 1H), 8.04 (br. s., 1H), 7.89 (d, *J* = 8.4 Hz, 1H), 7.52 (ddd, *J* = 1.8, 7.2, 8.6 Hz, 1H), 7.41 - 7.35 (m, 1H), 7.34 - 7.29 (m, 1H), 7.22 - 7.14 (m, 2H), 7.11 (d, *J* = 8.4 Hz, 1H), 6.65 (d, *J* = 3.0 Hz, 1H), 5.00 (dt, *J* = 5.7, 7.9 Hz, 1H), 4.76 (td, *J* = 5.8, 9.2 Hz, 1H), 3.40 - 3.14 (m, 2H).

**S24**

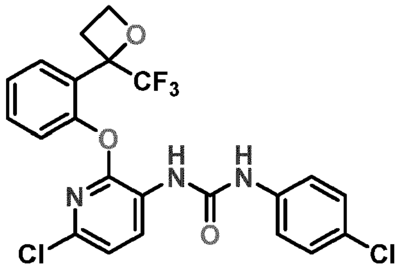

1-(6-chloro-2-(2-(2-(trifluoromethyl)oxetan-2-yl)phenoxy)pyridin-3-yl)-3-(4-chlorophenyl)urea

Prepared according to **general procedure 4** using 6-chloro-2-(2-(2-(trifluoromethyl)oxetan-2-yl)phenoxy)pyridin-3-amine (300 mg, 1.0 eq.) and 4-chloroaniline (167 mg, 1.5 eq.) to give the title material as a white solid. (67 mg, 15%). LC-MS: m/z 498.1 $[M+H]^+$; $^1H$ NMR (400MHz, $CDCl_3$) $\delta$ = 8.47 (d, *J* = 8.3 Hz, 1H), 7.48 - 7.30 (m, 4H), 7.29 - 7.15 (m, 5H), 7.00 (d, *J* = 8.4 Hz, 1H), 6.41 (s, 1H), 4.77 (dt, *J* = 5.7, 7.9 Hz, 1H), 4.54 (td, *J* = 5.8, 9.2 Hz, 1H), 3.15 - 2.93 (m, 2H).

**Example Series 7: aryl, alkyl ureas**

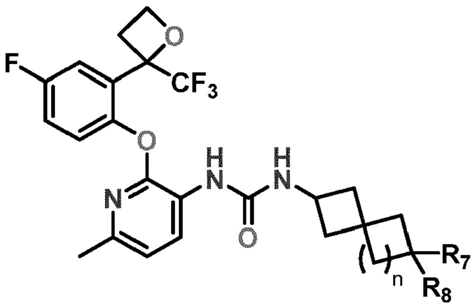

**General procedure for aryl,alkyl urea formation**

To a 5 mL microwave vial was added 2-(4-fluoro-2-(2-(trifluoromethyl)oxetan-2-yl)phenoxy)-3-isocyanato-6-methylpyridine (1.0 eq) and representative amine (1.2 eq) The vial was sealed with a teflon-lined cap, evacuated and back-filled with argon (x3). Anhydrous tetrahydrofuran (0.1M) and N,N-diisopropylethylamine (1.2 eq) was added and the reaction mixture was warmed to 60 °C and stirred until UPLC-MS showed complete conversion of the isocyanate to urea product. The solvent was removed *in vacuo* and the crude residue purified by flash chromatography using a Biotage Isolera one 3.0 using a 12 g C18KP-SIL SNAP column linear gradient 5% - 95% acetonitrile in water (0.1% FA modifier) to give the title compound.

**S63**

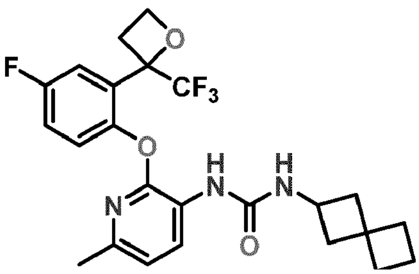

1-(2-(4-fluoro-2-(2-(trifluoromethyl)oxetan-2-yl)phenoxy)-6-methylpyridin-3-yl)-3-(spiro[3.3]heptan-2-yl)urea

Prepared according to the general procedure **4** using spiro[3.3]heptan-2-amine, hydrochloride (23.2 mg) to give the title material as a white solid (55 mg, 77%). UPLC-MS: m/z 480.2 $[M+H]^+$; $^1H$ NMR (400 MHz, DMSO-$d_6$) $\delta$ = 8.30 (d, *J* = 7.98 Hz, 1H), 7.76 (s, 1H), 7.32 (ddd, *J* = 3.30, 7.93, 9.03 Hz, 1H), 7.23 (dd, *J* = 3.16, 9.22 Hz, 1H), 7.10 (dd, *J* = 4.72, 9.03 Hz, 1H), 7.03 (d, *J* = 7.61 Hz, 1H), 6.92 (d, *J* = 8.07 Hz, 1H), 4.73 (dt, *J* = 5.87, 7.93 Hz, 1H), 4.56 (td, *J* = 5.87, 9.26 Hz, 1H), 3.96 (q, *J* = 8.31 Hz, 1H), 3.21 - 3.38 (m, 1H), 3.02 (ddd, *J* = 6.05, 8.41, 12.59 Hz, 1H), 2.32 (dt, 3.90, 7.40 Hz, 2H), 2.20 (s, 3H), 2.01 (t, *J* = 7.11 Hz, 2H), 1.86 - 1.94 (m, 2H), 1.69 - 1.84 (m, 4H).

**S86**

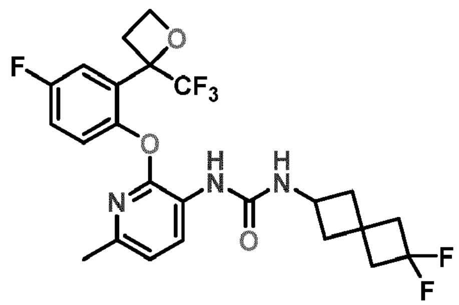

1-(6,6-difluorospiro[3.3]heptan-2-yl)-3-(2-(4-fluoro-2-(2-(trifluoromethyl)oxetan-2-yl)phenoxy)-6-methylpyridin-3-yl)urea

Prepared according to the general procedure **4** using 2,2-difluorospiro[3.3]heptan-6-amine;hydrochloride (11.4 mg) to give the title material as a white solid (36 mg, 95%). %). UPLC-MS: m/z 516.2 $[M+H]^+$; $^1H$ NMR (400 MHz, DMSO-$d_6$) $\delta$ 8.29 (d, *J* = 7.98 Hz, 1H), 7.79 (s, 1H), 7.32 (ddd, *J* = 3.26, 7.91, 9.01 Hz, 1H), 7.23 (dd, *J* = 3.21, 9.17 Hz, 1H), 7.06 - 7.12 (m, 2H), 6.93 (d, *J* = 8.07 Hz, 1H), 4.67 - 4.82 (m, 1H), 4.56 (td, *J* = 5.87, 9.35 Hz, 1H), 3.94-4.15 (m, 1H), 3.25 - 3.31 (m, 1H), 3.02 (ddd, *J* = 6.10, 8.41, 12.63 Hz, 1H), 2.66 (t, *J* = 12.56 Hz, 2H), 2.53 - 2.61 (m, 2H), 2.35 - 2.45 (m, 2H), 1.99 (ddd, *J* = 3.90, 8.53, 12.24 Hz, 2H).

**S90**

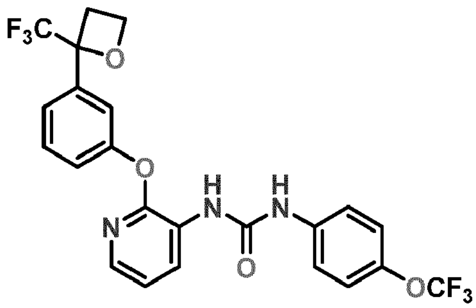

1-(4-(trifluoromethoxy)phenyl)-3-(2-(3-(2-(trifluoromethyl)oxetan-2-yl)phenoxy)pyridin-3-yl)urea

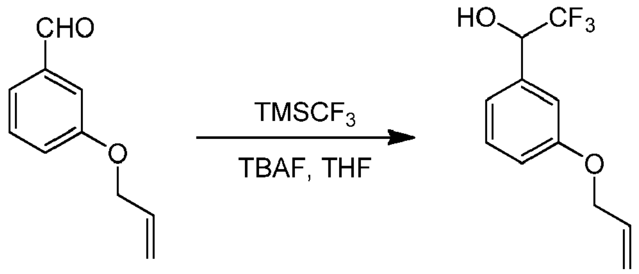


2 3

To a solution of **compound 2** (20 g, 123.3 mmol, 1 eq.) in THF (600 mL) was added TBAF (1 M, 308 mL, 2.5 eq.) at 20 °C. Then, trimethyl(trifluoromethyl)silane (43.84 g, 308.3 mmol, 2.5 eq.) was added dropwise at 20 °C and the mixture stirred at 20 °C for 12 hr. The residue was then diluted with ethyl acetate 500 mL, and the combined organic layers were washed with $H_2O$ (500 mL x3), dried over $Na_2SO_4$, filtered and the filtrate concentrated under reduced pressure to give a residue. The residue was purified by column chromatography (SiO2, petroleum ether/ ethyl acetate = 1/0 to 1/1) to give **Compound 3** (34.5 g, 148.6 mmol, 60% yield) as a yellow oil. TLC: (petroleum ether: ethyl acetate = 3:1, $R_f$= 0.48); $^1$H NMR (400MHz, $CDCl_3$) $\delta$ = 7.20 (t, *J* = 7.9 Hz, 1H), 7.01 - 6.90 (m, 2H), 6.89 - 6.77 (m, 1H), 5.94 (tdd, *J* = 5.3, 10.6, 17.2 Hz, 1H), 5.32 (dd, *J* = 1.5, 17.3 Hz, 1H), 5.19 (dd, *J*= 1.4, 10.5 Hz, 1H), 4.84 (q, *J*= 6.8 Hz, 1H), 4.43 (d, *J*= 5.3 Hz, 2H), 4.00 (q, *J* = 7.2 Hz, 1H).

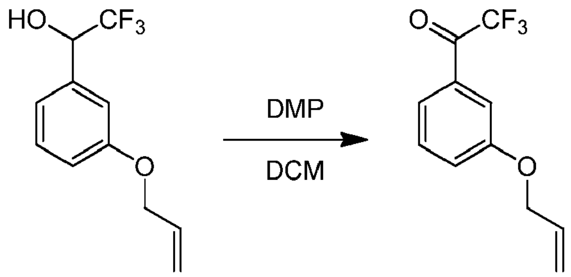


3 4

To a solution of **compound 3** (28.18 g, 121.4 mmol, 1.0 eq.) in DCM (845 mL) was added DMP (77.21 g, 182 mmol, 1.5 eq.). The mixture was stirred at 25 °C for 12 hr. TLC indicated **compound 3** was consumed completely and one new spot formed. The reaction mixture was quenched by the addition of $H_2O$ (500 mL) at 25°C, and the biphasic mixture further extracted with ethyl acetate (400 mL x3). The combined organic layers were dried over $Na_2SO_4$, filtered and the filtrate concentrated under reduced pressure to give a residue. The residue was purified by column chromatography (SiO2, petroleum ether/ ethyl acetate = 1/0 to 1/ 1) to give **compound 4** (23.11 g, 100.4 mmol, 83% yield) as a yellow oil.). TLC: petroleum ether: ethyl acetate = 3:1. $R_f$ = 0.34; $^1$H NMR (400MHz, $CDCl_3$) $\delta$ = 7.58 - 7.73 (m, 2H), 7.44 - 7.54 (m, 1H), 7.29 (br d, J=7.34 Hz, 1H), 6.01 - 6.17 (m, 1H), 5.27 - 5.52 (m, 2H), 4.63 (br d, J=5.13 Hz, 2 H).

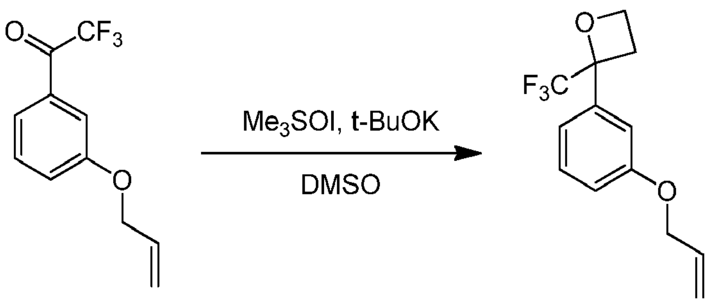


4 5

To a solution of trimethylsulfoxonium iodide (84.67 g, 384.7 mmol, 4.0 eq.) in DMSO (300 mL) was added t-BuOK (43.17 g, 384.7 mmol, 4.0 eq.) at 25 °C. The mixture was stirred at 25°C for 10 min, and then a solution of **compound 4** (22.14 g, 96.2 mmol, 1.0 eq.) in DMSO (150 mL) was added. The resulting mixture was stirred at 25 °C

for 12 hr. TLC indicated **compound 4** was consumed completely and one new spot had formed. The reaction mixture was quenched with $H_2O$ (600 mL) at 0 °C, and then extracted with ethyl acetate (500 mL x4). The combined organic layers were dried over $Na_2SO_4$, filtered and the filtrate concentrated under reduced pressure to give a residue. The residue was purified by column chromatography (SiO2, petroleum ether/ ethyl acetate = 1/0 to 1/1) to give **compound 5** (15.60 g, 55.8 mmol, 58%) as a yellow oil. TLC: petroleum ether: ethyl acetate = 10:1. $R_f$ = 0.52; $^1H$ NMR (400MHz, $CDCl_3$) $\delta$ = 7.24 (t, *J* = 7.9 Hz, 1H), 6.97 - 6.88 (m, 2H), 6.85 (ddd, *J* = 0.8, 2.5, 8.3 Hz, 1H), 5.98 (tdd, *J* = 5.3, 10.6, 17.2 Hz, 1H), 5.35 (qd, 1.5, 17.3 Hz, 1H), 5.26 - 5.12 (m, 1H), 4.80 - 4.62 (m, 1H), 4.59 - 4.41 (m, 3H), 3.16 (ddd, *J* = 6.3, 8.8, 11.6 Hz, 1H), 2.83 (dddd, *J* = 0.8, 8.1, 9.3, 10.5 Hz, 1H).

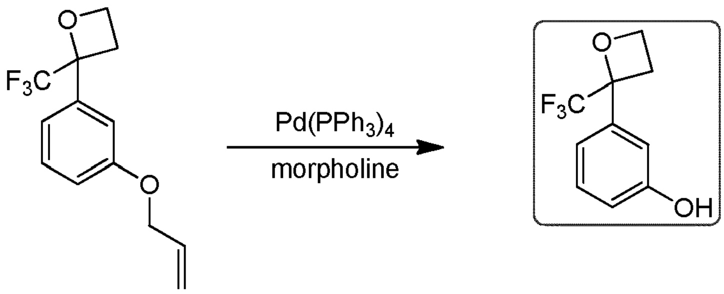


**5** **6**

To a solution of **compound 5** (15.6 g, 60.41 mmol, 1.0 eq.) and morpholine (5.79 g, 66.45 mmol, 5.85 mL, 1.1 eq.) in DMF (160 mL) was added $Pd(PPh_3)_4$ (6.98 g, 6.04 mmol, 0.1 eq.) under an $N_2$ atmosphere. The mixture was stirred under $N_2$ at 80 °C for 3 hr. LC-MS showed **compound 5** was completely consumed and one main peak with the desired mass was detected. The reaction mixture was cooled to room temperature, diluted with $H_2O$ (500 mL) and extracted with ethyl acetate (500 mL x3). The combined organic layers were dried over $Na_2SO_4$, filtered and the filtrate concentrated under reduced pressure to give a residue. The residue was purified by column chromatography (SiO2, petroleum ether/ ethyl acetate = 1/0 to 1/1) to give **compound 6** (12.94 g, 59.31 mmol, 98% yield) as a yellow oil). TLC: petroleum ether: ethyl acetate = 3:1, $R_f$ = 0.65; LC-MS: m/z 217.0 $[M-H]^-$; $^1H$ NMR (400MHz, $CDCl_3$) $\delta$ = 7.40 - 7.25 (m, 1H), 7.04 - 6.93 (m, 2H), 6.88 (ddd, 1.1, 2.4, 8.1 Hz, 1H), 6.01 (br. s., 1H), 4.94 - 4.77 (m, 1H), 4.62 (td, *J* = 6.0, 9.1 Hz, 1H), 3.25 (ddd, *J* = 6.3, 8.8, 11.7 Hz, 1H), 3.00 - 2.76 (m, 1H).

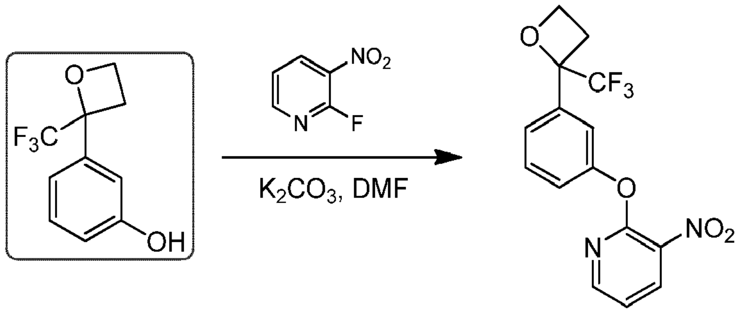


**6** **7**

To a solution of 2-fluoro-3-nitro-pyridine (651 mg, 4.58 mmol, 1.0 eq.) in DMF (10 mL) was added $K_2CO_3$ (1.90 g, 13.75 mmol, 3.0 eq.) and **compound 6** (1.00 g, 4.58 mmol, 1.0 eq.) at 25°C. The mixture was stirred at 100 °C for 12 hr. LC-MS showed **compound 6** was consumed completely and one main peak with the desired mass was detected. The residue was purified by column chromatography ($SiO_2$, petroleum ether/ ethyl acetate=1/0 to 1/ 1) to give **compound 7** (1.08 g, 69%) as a yellow oil. TLC: petroleum ether: ethyl acetate = 3:1, $R_f$ = 0.15; LC-MS: m/z 341.0 $[M+H]^+$.

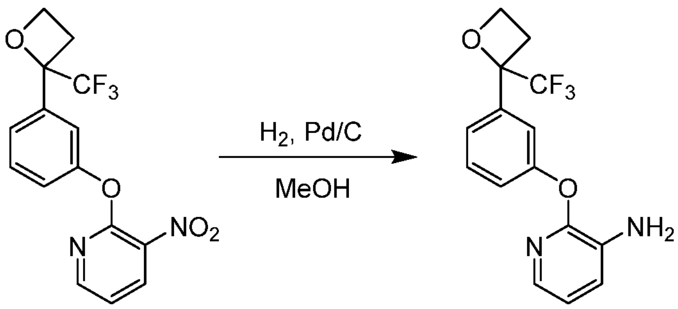


7 8

To a solution of **compound 7** (500 mg, 1.47 mmol, 1.0 eq.) in MeOH (5 mL) was added Pd/C (10% wt., 0.20 g) under $N_2$ atmosphere. The suspension was degassed and purged with $H_2$ (x3). The mixture was stirred under $H_2$ (15 Psi.) at 25 °C for 3 hr. LC-MS showed **compound 7** was consumed completely and one main peak with desired mass was detected. The reaction mixture was concentrated under reduced pressure to give a residue of **compound 8** (300 mg, crude) as a brown oil. No further purification. LC-MS: m/z 311.0 $[M+H]^+$.

**S90**

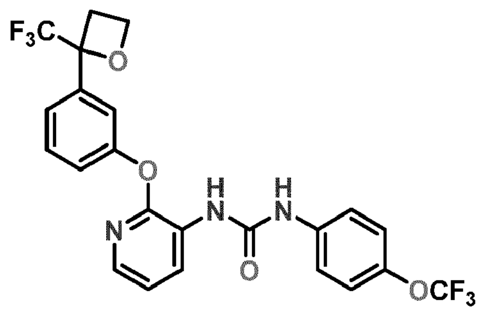

1-(4-(trifluoromethoxy)phenyl)-3-(2-(3-(2-(trifluoromethyl)oxetan-2-yl)phenoxy)pyridin-3-yl)urea

Prepared according to **general procedure 8** using 2-(3-(2-(trifluoromethyl)oxetan-2-yl)phenoxy)pyridin-3-amine (300 mg, 1.0 eq.) and 1-isocyanato-4-(trifluoromethoxy)benzene (196 mg, 1.0 eq.) to give the title material as a white solid (18 mg, 4%). LC-MS: m/z 514.1 $[M+H]^+$; $^1H$ NMR (400MHz, $CDCl_3$) $\delta$ = 8.49 (dd, $J$ = 1.6, 7.9 Hz, 1H), 7.71 (dd, $J$ = 1.6, 4.9 Hz, 1H), 7.37 - 7.26 (m, 4H), 7.18 - 7.04 (m, 5H), 7.04 - 6.98 (m, 1H), 6.95 (dd, $J$ = 4.9, 7.9 Hz, 1H), 4.81 - 4.67 (m, 1H), 4.48 (td, $J$ = 5.9, 9.1 Hz, 1H), 3.14 (ddd, $J$ = 6.4, 8.7, 11.7 Hz, 1H), 2.90 - 2.68 (m, 1H).

**S84**

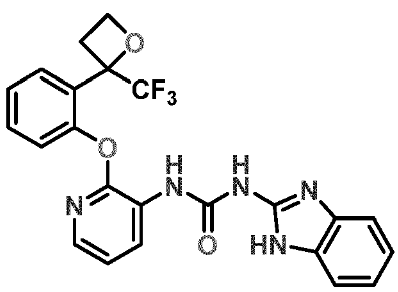

1-(1*H*-benzo[*d*]imidazol-2-yl)-3-(2-(2-(2-(trifluoromethyl)oxetan-2-yl)phenoxy)pyridin-3-yl)urea

Prepared according to **general procedure 2** using using 2-(2-(2-(trifluoromethyl)oxetan-2-yl)phenoxy)pyridin-3-amine (300 mg, 1.0 eq.) and 1H-benzimidazol-2-amine (154 mg, 1.2 eq.) to give the title material as a white solid (75 mg, 16%). LC-MS: m/z 470.0 $[M+H]^+$; $^1H$ NMR (400MHz, $CD_3OD$) $\delta$ = 8.69 (dd, *J* = 1.4, 7.9 Hz, 1H), 7.77 (dd, *J* = 1.4, 4.8 Hz, 1H), 7.60 (d, *J* = 7.9 Hz, 1H), 7.50 - 7.42 (m, 1H), 7.37 - 7.29 (m, 3H), 7.17 - 7.10 (m, 3H), 7.06 (d, *J* = 8.1 Hz, 1H), 4.79 - 4.71 (m, 1H), 4.51 (td, *J* = 5.7, 9.3 Hz, 1H), 3.42 - 3.35 (m, 1H), 3.12 (ddd, *J* = 6.0, 8.4, 12.4 Hz, 1H).

**Synthetic route to S73**

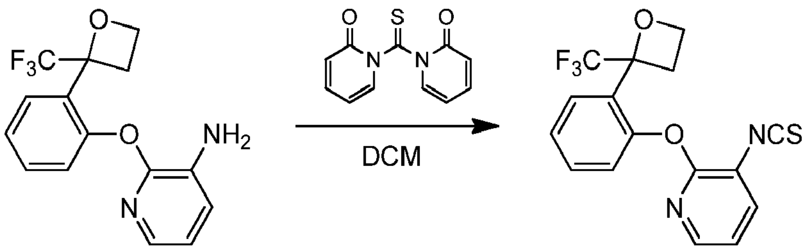


1 2

To a solution of **compound 1** (1.00 g, 3.23 mmol, 1.0 eq.) in DCM (20 mL) was added 1-(2-oxopyridine-1-carbothioyl)pyridin-2-one (826 mg, 3.56 mmol, 1.1 eq.). The mixture was stirred at 25°C for 2 hr. TLC showed compound **1** was completely consumed, and one major new spot was detected. The reaction mixture was concentrated under reduced pressure to give a residue. The residue was purified by column chromatography ($SiO_2$, petroleum ether/ethyl acetate=1/0 to 5/1) to give **compound 2** (520 mg, 46%) as a white solid. TLC: petroleum ether: ethyl acetate = 4:1, $R_f$ = 0.54; LC-MS: m/z 353.0. $[M+H]^+$.

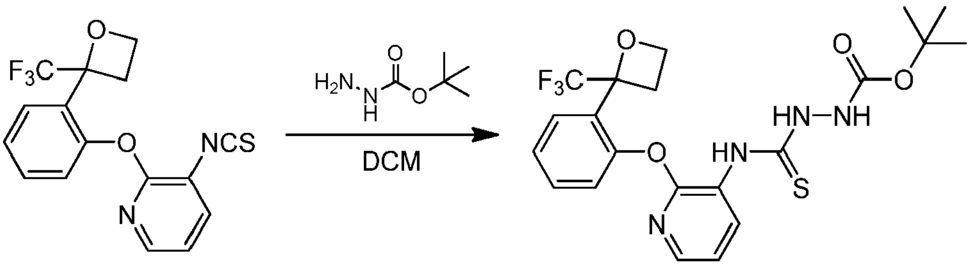


2 3

To a solution of **compound 2** (520 mg, 1.48 mmol, 1.0 eq.) in DCM (10 mL) was added tert-butyl N-aminocarbamate (234 mg, 1.77 mmol, 1.2 eq.). The mixture was stirred at 25°C for 12 hr. The reaction mixture was concentrated under reduced pressure to give a residue. **Compound 3** (1.0 g) was obtained as a white solid and used directly in the next step without further purification. LC-MS: m/z 485.1 $[M+H]^+$

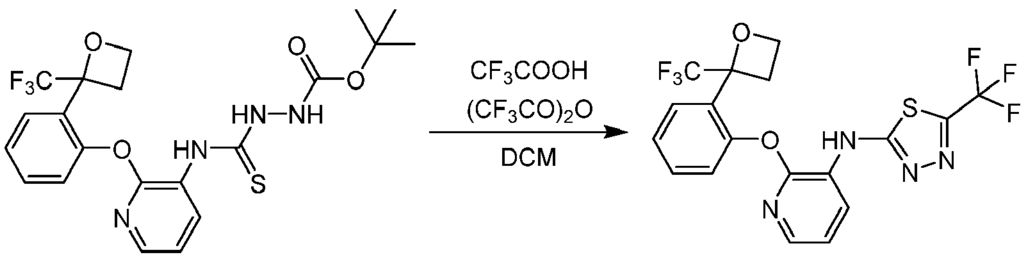


3

To a solution of **compound 3** (1.0 g, 2.06 mmol, 1.0 eq.) in DCM (10 mL) was added TFA (5.65 g, 49.54 mmol,

3.67 mL, 24 eq.). The mixture was stirred at 25°C for 4 hr. (2,2,2-trifluoroacetyl) 2,2,2-trifluoroacetate (2.17 g, 10.32 mmol, 1.44 mL, 5.0 eq.) was then added and the mixture was stirred at 25°C for 12 hr. The reaction mixture was concentrated under reduced pressure to give a residue. The residue was purified by prep-HPLC (neutral condition) column: Phenomenex Gemini NX-C18(75*30mm*3um); mobile phase: [water(10mM $NH_4HCO_3$)-ACN];B%: 35%-60%,10min to give the title material (261 mg, 27%) as a white solid. LC-MS: m/z 463.0 $[M+H]^+$; $^1H$ NMR (400 MHz, DMSO-$d_6$) δ = 10.16 (br. s, 1H), 8.65 (dd, *J* = 1.5, 7.9 Hz, 1H), 7.87 (dd, *J* = 1.6, 4.9 Hz, 1H), 7.55 - 7.42 (m, 2H), 7.32 (dt, *J* = 0.9, 7.5 Hz, 1H), 7.24 (dd, *J* = 4.9, 7.9 Hz, 1H), 7.12 (d, *J* = 8.1 Hz, 1H), 4.74 - 4.64 (m, 1H), 4.51 (td, *J* = 5.9, 9.3 Hz, 1H), 3.42 - 3.34 (m, 1H), 3.00 (ddd, *J* = 6.0, 8.3, 12.6 Hz, 1H).

**S82**

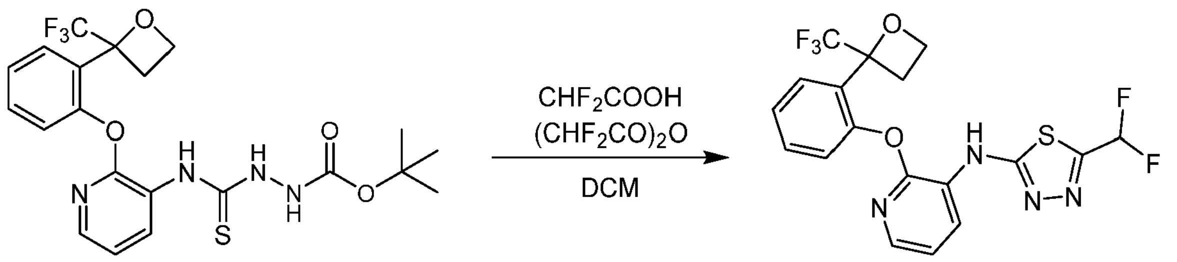


**3**

5-(difluoromethyl)-*N*-(2-(2-(2-(trifluoromethyl)oxetan-2-yl)phenoxy)pyridin-3-yl)-1,3,4-thiadiazol-2-amine

To a solution of **compound 3** (412 mg, 0.85 mmol, 1.0 eq.) in DCM (10 mL) was added 2,2-difluoroacetic acid (1.96 g, 20.44 mmol, 1.28 mL, 24 eq.) and and (2,2-difluoroacetyl) 2,2-difluoroacetate (741 mg, 4.26 mmol, 5 eq.). The mixture was stirred at 25°C for 12 hr. The reaction mixture was concentrated under reduced pressure to give a residue. The residue was purified by prep-HPLC (column: Phenomenex Luna C18 75*30mm*3um; phase: [water(0.2% FA)-MeOH];B% : 35%-65% ,8min) to give the title material (30 mg, 8%) as a white solid. LC-MS: m/z 445.0 $[M+H]^+$; $^1H$ NMR (400 MHz, DMSO-$d_6$) δ = 10.48 (br. s., 1H), 8.70 (d, *J* = 7.6 Hz, 1H), 7.85 (d, *J* = 3.5 Hz, 1H), 7.61 - 7.43 (m, 3H), 7.41 (s, 1H), 7.38 - 7.29 (m, 1H), 7.29 - 7.19 (m, 1H), 7.13 (d, *J* = 8.1 Hz, 1H), 4.83 - 4.62 (m, 1H), 4.53 (td, *J* = 5.7, 9.3 Hz, 1H), 3.09 - 2.90 (m, 1H).

**S76**

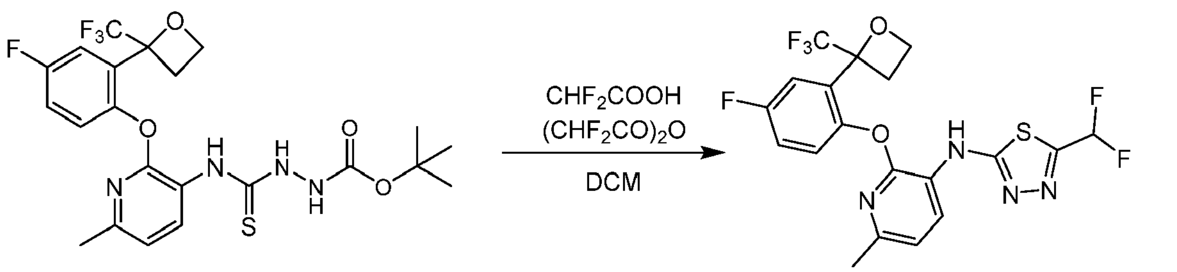


**3**

5-(difluoromethyl)-*N*-(2-(4-fluoro-2-(2-(trifluoromethyl)oxetan-2-yl)phenoxy)-6-methylpyridin-3-yl)-1,3,4-thiadiazol-2-amine

Preparation analogous to **S82**

To a solution of **compound 3** (58 mg, 0.14 mmol, 1.0 eq.) in DCM (1.0 mL) was added 2,2-difluoroacetic acid (500 mg, 5.21 mmol, 36 eq.) and (2,2-difluoroacetyl) 2,2-difluoroacetate (29 mg, 0.17 mmol, 1.2 eq.). The mixture was

stirred at 25°C for 24 hr. The reaction mixture was concentrated under reduced pressure to give a residue. The residue was purified by reverse-phase flash chromatography using a Biotage Isolera one 3.0 with a 12 g C18 SNAP column linear gradient (30% - 95%) acetonitrile in water (0.1% formic acid modifier) to give the title material (14 mg, 20%) as a white solid. UPLC-MS: m/z 475.1 $[M-H]^-$; $^1H$ NMR (400 MHz, DMSO-$d_6$) $\delta$ = 10.33 (br. s., 1H), 8.48 (d, *J* = 7.98 Hz, 1H), 7.29 - 7.45 (m, 2H), 7.16 - 7.29 (m, 2H), 7.09 (dd, *J* = 0.55, 7.98 Hz, 1H), 4.70 (dt, *J* = 5.78, 7.93 Hz, 1H), 4.55 (td, *J* = 5.87, 9.26 Hz, 1H), 3.26 - 3.40 (m, 1H), 3.00 (ddd, *J* = 6.14, 8.48, 12.70 Hz, 1H), 2.28 (2, 3H).

**Synthetic route to S80**

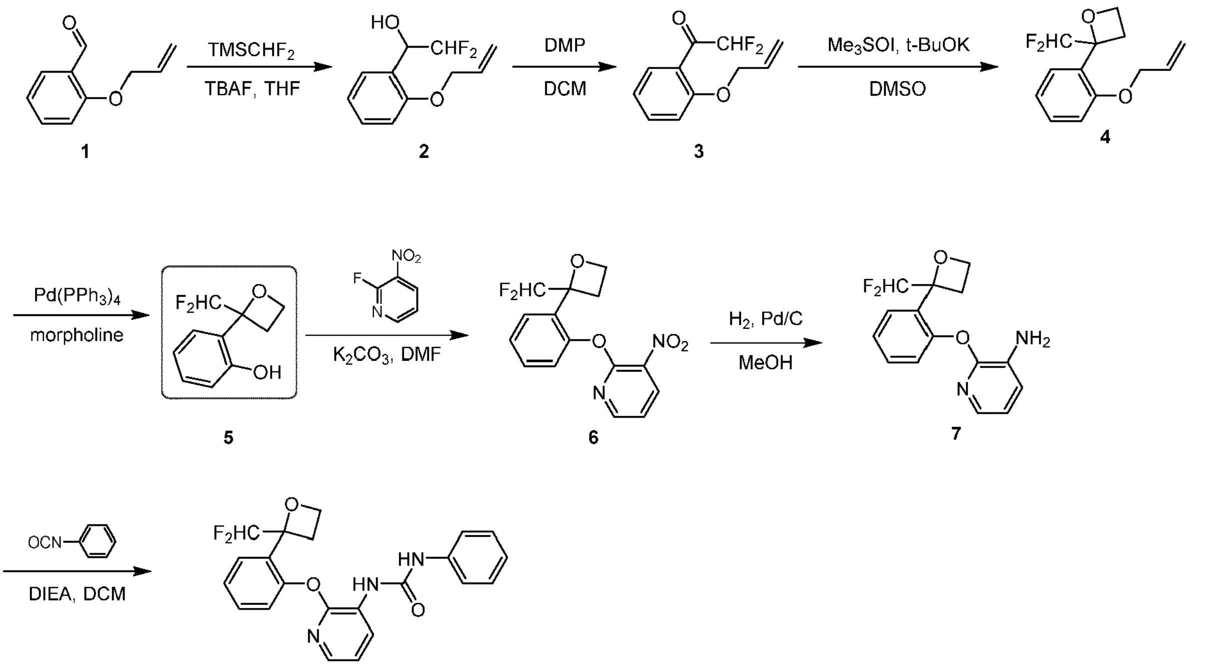


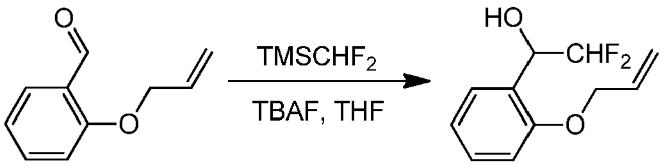


**1** **2**

To a solution of compound **1** (5.0 g, 30.83 mmol, 1.0 eq.) in THF (300 mL) was added TBAF (1.0 M, 30.83 mL, 1.0 eq.) and difluoromethyl(trimethyl)silane (9.57 g, 77.07 mmol, 2.5 eq.). The mixture was stirred at 20 °C for 12 hr. TLC indicated compound **1** was completely consumed and 4 new spots had formed. The reaction mixture was concentrated under reduced pressure to remove solvent. The residue was purified by column chromatography ($SiO_2$, petroleum ether/ ethyl acetate=1/0 to 1/ 1) to give compound **2** (1.98 g, 30%) as a light yellow solid. TLC: petroleum ether: ethyl acetate = 1:1, $R_f$ = 0.63.

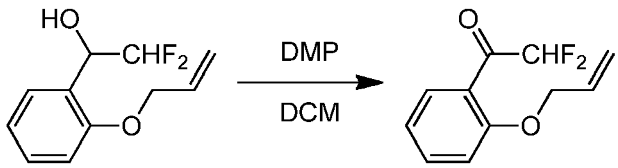


**2** **3**

To a solution of compound **2** (3.23 g, 15.08 mmol, 1.0 eq.) in DCM (30 mL) was added DMP (8.31 g, 19.60 mmol, 1.3 eq.). The mixture was stirred at 20 °C for 12 hr. TLC indicated compound **2** was consumed completely and

one new spot formed. The reaction mixture was concentrated under reduced pressure to remove solvent and then the residue was purified by column chromatography ($SiO_2$, petroleum ether/ ethyl acetate=1/ 0 to 1/ 1) to give compound 3 (1.21 g, 38%) as a light yellow solid. TLC: petroleum ether: ethyl acetate = 1:1, $R_f$ = 0.60.

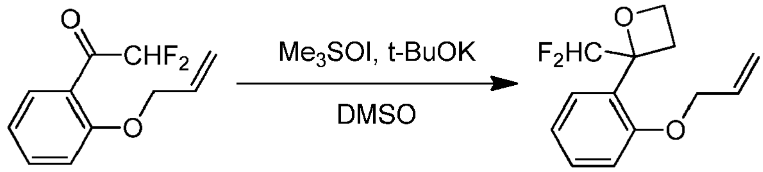


3 4

To a solution of trimethylsulfoxonium iodide (3.73 g, 16.97 mmol, 4.0 eq.) in DMSO (9 mL) was added t-BuOK (1.90 g, 16.97 mmol, 4.0 eq.) at 20 °C. The mixture was stirred at 20 °C for 10 min, then compound **3** (0.90 g, 4.24 mmol, 1.0 eq.) in DMSO (6 mL) was added to the mixture at 20 °C and stirred at 20 °C for 12 hr. TLC indicated compound 3 was completely consumed and one new spot had formed. The reaction mixture was concentrated under reduced pressure to remove solvent and then the residue was purified by prep-TLC ($SiO_2$, petroleum ether: ethyl acetate = 5:1) to give compound **4** (0.70 g, 69%) as a light yellow solid. TLC: petroleum ether: ethyl acetate = 5:1, $R_f$ = 0.39.

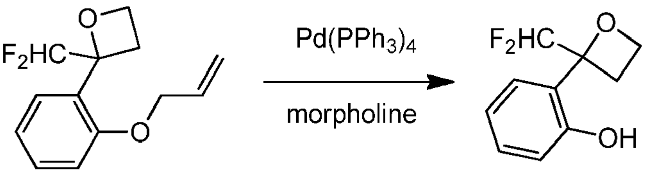


4 5

To a solution of compound **4** (704 mg, 2.93 mmol, 1.0 eq.) in DMF (10 mL) was added $Pd(PPh_3)_4$ (339 mg, 0.29 mmol, 0.1 eq.) and morpholine (281 mg, 3.22 mmol, 283 μL, 1.1 eq.). The mixture was stirred at 80 °C for 3 hr. LC-MS showed compound 4 was completely consumed and one main peak with the desired m/z was detected. The reaction mixture was diluted with water (30 mL) and extracted with ethyl acetate (10 mL x4). The organic phases were combined, filtered and the filtrate concentrated under reduced pressure to give a residue. The residue was purified by prep-TLC ($SiO_2$, petroleum ether: ethyl acetate = 3:1) to give compound **5** (500 mg, 85%) as a yellow solid. TLC: petroleum ether: ethyl acetate = 3:1, $R_f$ = 0.31; LC-MS: m/z 199.0. $[M-H]^-$.

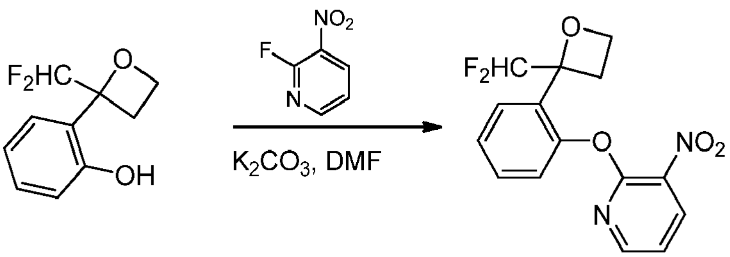


5 6

To a solution of compound **5** (300 mg, 1.50 mmol, 1.0 eq.) in DMF (6 mL) was added $K_2CO_3$ (621 mg, 4.50 mmol, 3.0 eq.) and 2-fluoro-3-nitro-pyridine (213 mg, 1.50 mmol, 1.0 eq.) at 20 °C. The mixture was stirred at 100 °C for 12 hr. LC-MS showed compound 5 was completely consumed and that one main peak with the desired m/z was detected. The reaction mixture was concentrated under reduced pressure to remove solvent and the residue diluted with water (20 mL) and extracted with ethyl acetate (10 mL x4). The organic fractions were combined, dried over $Na_2SO_4$, filtered and the filtrate concentrated under reduced pressure to give a residue. The residue was purified by column chromatography ($SiO_2$, petroleum ether/ethyl acetate=1/ 0 to 1/ 1) to give compound 6 (200 mg, 41%) as a yellow solid. TLC: petroleum ether: ethyl acetate = 1:1, $R_f$ = 0.31; LC-MS: m/z 323.1 $[M+H]^+$.

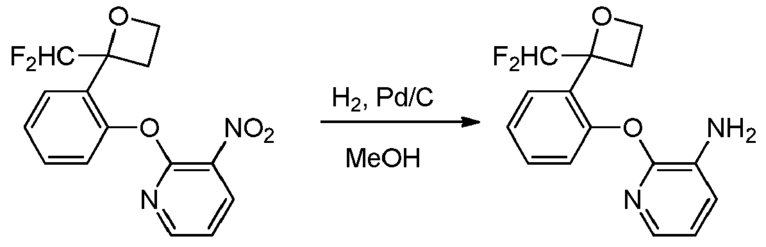


6 7

A mixture of compound 6 (180 mg, 0.57 mmol, 1.0 eq.), Pd/C (60 mg, 0.056 mmol, 10% wt.) in MeOH (2 mL) was degassed and purged with $H_2$ (15 PSI, x3), and then the mixture was stirred at 20 °C for 1 hr under a $H_2$ atmosphere. TLC indicated compound 6 was completely consumed and one new spot had formed. The reaction mixture was concentrated under reduced pressure to remove solvent and the residue was purified by prep-TLC ($SiO_2$, petroleum ether: ethyl acetate 3:1) to give compound **7** (160 mg, 0.55 mmol, 98%) as a yellow solid. TLC: petroleum ether: ethyl acetate = 3:1, $R_f$ = 0.35.

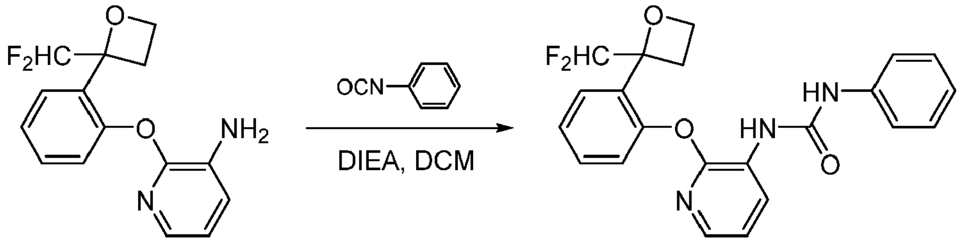


7

Prepared according to **general procedure 1** using compound **7** (140 mg, 1.0 eq.) and isocyanatobenzene (700 mg, 12.3 eq.) to give the title material as a white solid (30 mg, 15%). LC-MS: m/z 412.2 $[M+H]^+$; $^1H$ NMR (400MHz, $CDCl_3$) $\delta$ = 8.56 (dd, *J* = 1.8, 8.0 Hz, 1H), 7.90 - 7.70 (m, 2H), 7.40 (dd, *J* = 1.4, 7.7 Hz, 1H), 7.34 - 7.22 (m, 5H), 7.17 (br d, *J* = 7.9 Hz, 1H), 7.09 - 6.97 (m, 2H), 6.62 (br s, 1H), 6.17 - 5.65 (m, 1H), 5.01 - 4.14 (m, 2H), 3.30 - 2.47 (m, 2H).

**Synthetic route to S74**

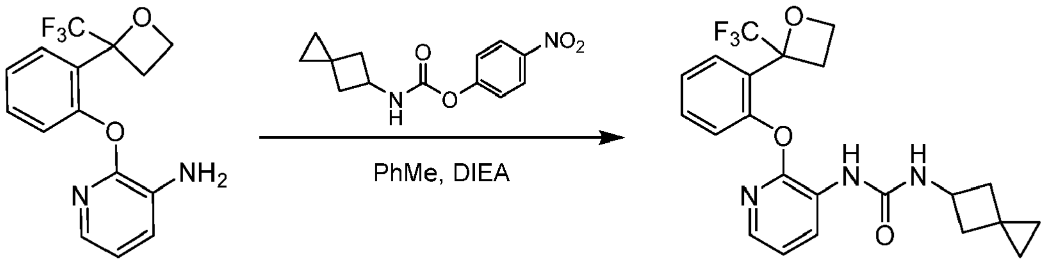


3

1-(spiro[2.3]hexan-5-yl)-3-(2-(2-(2-(trifluoromethyl)oxetan-2-yl)phenoxy)pyridin-3-yl)urea

To a solution of compound **3** (120 mg, 0.387 mmol, 1.0 eq.) in toluene (3 mL) was added DIPEA (299 mg, 2.32 mmol, 0.40 mL, 6.0 eq.) and (4-nitrophenyl) N-spiro[2.3]hexan-5-ylcarbamate (203 mg, 0.77 mmol, 2.0 eq.). The mixture was stirred at 100 °C for 12 hr. LC-MS showed consumption of compound 3. The reaction mixture was concentrated under reduced pressure to remove solvent. The residue was purified by prep-HPLC (column: Waters Xbridge Prep OBD C18 150*40mm*10um; mobile phase: [water (10mM NH4HCO3)-ACN]; B%: 45%-75%,8min) to give the title material (68 mg, 40%) as a white solid. LC-MS: m/z 434.0 $[M+H]^+$; $^1H$ NMR (400MHz, $CDCl_3$) $\delta$ = 8.58 (dd, *J* = 1.7, 7.9 Hz, 1H), 7.78 (dd, *J* = 1.8, 4.9 Hz, 1H), 7.58 (dd, *J* = 1.6, 7.8 Hz, 1H), 7.43 (ddd, *J* = 1.8, 7.4, 8.2 Hz, 1H), 7.33 - 7.28 (m, 1H), 7.17 (d, *J* = 8.0 Hz, 1H), 7.04 (dd, *J* = 4.9, 80 Hz, 1H), 6.93 (s, 1H), 4.88 - 4.80 (m, 1H), 4.80 - 4.71 (m, 1H), 4.57 (td, *J* = 5.8, 9.2 Hz, 1H), 4.50 - 4.34 (m, 1H), 3.17 - 2.99 (m, 2H), 2.46 - 2.32 (m, 2H), 2.19 (dd, *J* = 7.3, 12.3 Hz, 2H), 0.56 -

0.47 (m, 2H), 0.47 - 0.39 (m, 2H).

3-oxetanes

**Representative procedure for 3-oxetane containing compounds S87**

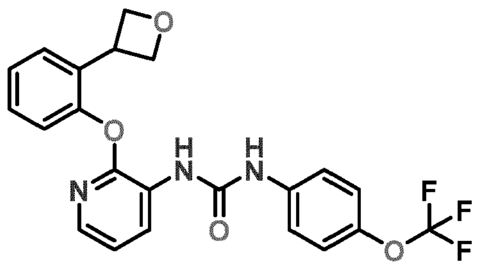

An oven-dried 5 mL vial with stirrer and Teflon-lined cap was charged with 1-[2-(2-bromophenoxy)-3-pyridyl]-3-[4-(trifluoromethoxy)phenyl]urea (47 mg, 0.10 mmol, 1.0 eq.), 3-bromooxetane (28.5 uL, 0.30 mmol, 3.0 eq.), 2,6-lutidine (47 uL, 0.40 mmol, 4.0 eq.), tris(trimethylsilyl)silane (46 uL, 0.15 mmol), and [4,4-bis(1,1-dimethylethyl)-2,2-bipyridine-N1,N1']bis[3,5-difluoro-2-[5-(trifluoromethyl)-2-pyridinyl-N]phenyl-C]Iridium(III) hexafluorophosphate (1.1 mg, 0.99 umol, 0.01 eq.). A stock solution (100 uL in anhydrous DME) containing nickel(II) chloride ethylene glycol dimethyl ether complex (0.11 mg, 0.05 umol, 0.005 eq.) and 4,4'-di-tert-butyl-2,2'-dipyridyl (0.13 mg, 0.05 umol, 0.005 eq.) was added and the vial was capped, sealed with parafilm, evacuated and backfilled with argon (x3). Anhydrous DME (pre-sparged for 10 min with argon) was added. The reaction mixture was sparged with argon for 5 min, pre-stirred for 5 min and then irradiated with blue LED light (40 W, kessil lamp, 456 nM) for 6 h. The solvent was removed in vacuo and the residue purified by flash chromatography using a Biotage Isolera one 3.0 using an 10 g KP-SIL SNAP column linear gradient (5% - 30%) ethyl acetate in cyclohexane to give the title material (8 mg, 18%) as a white solid. UPLC-MS: m/z 446.1 $[M+H]^+$; $^1H$ NMR (400 MHz, DMSO-$d_6$) $\delta$ = 9.45 (s, 1H), 8.63 (s, 1H), 8.46 (dd, *J* = 1.74, 7.89 Hz, 1H), 7.44 - 7.58 (m, 4H), 7.19 - 7.31 (m, 4H), 7.02 - 7.07 (m, 1H), 6.99 (dd, *J* = 4.86, 7.89 Hz, 1H), 4.55 - 4.63 (m, 4H), 4.19 (quin, *J* = 7.89 Hz, 1H).

**S88**

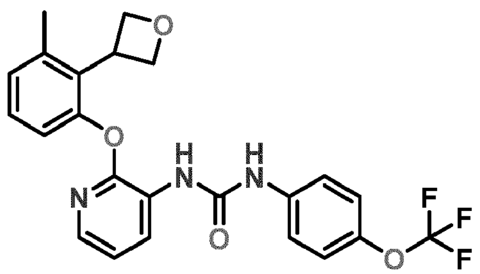

Prepared according to the representative procedure using 1-[2-(2-bromo-3-methyl-phenoxy)-3-pyridyl]-3-[4-(trifluoromethoxy)phenyl]urea (50 mg, 0.10 mmol), to give the title material (8 mg, 18%) as a white solid. UPLC-MS: m/z 460.1 $[M+H]^+$; $^1H$ NMR (400 MHz, DMSO-$d_6$) $\delta$ = 9.51 (s, 1H), 8.62 (s, 1H), 8.51 (dd, *J* = 1.74, 7.89 Hz, 1H), 7.64 (dd, *J* = 1.74, 4.86 Hz, 1H), 7.50 - 7.59 (m, 2H), 7.28 (dd, *J* = 0.83, 9.08 Hz, 2H), 7.10 - 7.21 (m, 1H), 7.00 - 7.09 (m, 2H), 6.84 (d, *J* = 7.52 Hz, 1H), 4.85 (dd, *J* = 5.73, 8.94 Hz, 2H), 4.61 (dd, *J* = 5.64, 8.67 Hz, 2H), 4.45 (quin, *J* = 8.78 Hz, 1H), 2.14 (s, 3H).

**S92**

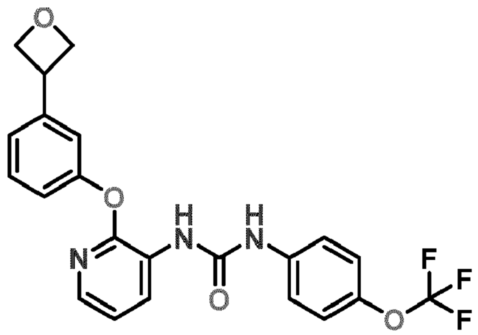

Prepared according to the representative procedure using 1-[2-(3-bromophenoxy)-3-pyridyl]-3-[4-(trifluoromethoxy)phenyl]urea (47 mg, 0.10 mmol), to give the title material (34 mg, 76%) as a white solid. UPLC-MS: m/z 446.1 $[M+H]^+$; $^1H$ NMR (400 MHz, DMSO-$d_6$) $\delta$ = 9.46 (s, 1H), 8.63 (s, 1H), 8.47 (dd, *J* = 1.74, 7.98 Hz, 1H), 7.58 - 7.66 (m, 1H), 7.44 - 7.51 (m, 2H), 7.32 - 7.39 (m, 1H), 7.17 - 7.26 (m, 3H), 7.13 (t, *J* = 2.02 Hz, 1H), 6.97 - 7.06 (m, 2H), 4.86 (dd, *J* = 5.91, 8.39 Hz, 2H), 4.53 (dd, *J* = 6.01, 6.74 Hz, 2H), 4.07 - 4.31 (m, 1H).

**S91**

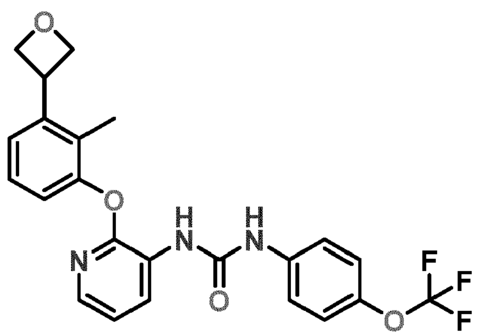

Prepared according to the representative procedure using 1-[2-(3-bromo-2-methyl-phenoxy)-3-pyridyl]-3-[4-(trifluoromethoxy)phenyl]urea (96 mg, 0.20mmol, 1.0 eq.), to give the title material (79 mg, 92%) as a white solid. UPLC-MS: m/z 460.1 $[M+H]^+$; $^1H$ NMR (400 MHz, DMSO-$d_6$) $\delta$ = 9.47 (s, 1H), 8.70 (s, 1H), 8.47 (dd, *J* = 1.70, 7.93 Hz, 1H), 7.56 (dd, *J* = 1.70, 4.91 Hz, 1H), 7.46 - 7.53 (m, 2H), 7.18 - 7.28 (m, 4H), 6.91 - 7.03 (m, 2H), 4.88 (dd, *J* = 5.78, 8.44 Hz, 2H), 4.66 (dd, *J* = 5.73, 7.38 Hz, 2H), 4.42 - 4.55 (m, 1H), 1.82 (s, 3H).

**Representative procedure for the synthesis of N-aryl azetidine containing compounds S94**

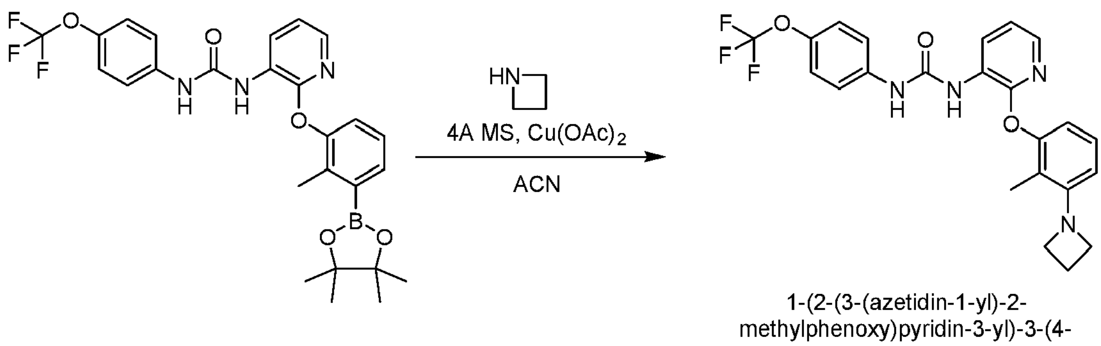


1

1-(2-(3-(azetidin-1-yl)-2-methylphenoxy)pyridin-3-yl)-3-(4-(trifluoromethoxy)phenyl)urea

To a solution of compound **1** (0.1 g, 189 umol, 1.0 eq.) in acetonitrile (5 mL) was added azetidine (22 mg, 378

umol, 25.508 uL, 2.0 eq.), 4A MS (0.1 g) and $Cu(OAc)_2$ (38 mg, 208 umol, 1.1 eq.). The mixture was stirred at 80 °C for 12hr. LCMS showed compound **1** was consumed and the desired mass was detected. To the reaction mixture was added $Na_2CO_3$ (1.0 g) and it was then stirred for 30 min, filtered, and the filtrate concentrated under reduced pressure. The residue was purified by MPLC (column: Phenomenex Gemini-NX 150*30mm*5um;mobile phase: [water(10mM $NH_4HCO_3$)-ACN];B%: 42%-62%,8min) to give the title material (8 mg, 9%) as a yellow solid. LC-MS: m/z 459.2 $[M+H]^+$; $^1H$ NMR (400MHz, methanol-$d_4$) δ = 8.59 (d, *J* = 7.89 Hz, 1H), 7.62 (d, *J* = 5.04 Hz, 1H), 7.62 (d, *J* = 5.04 Hz, 1H), 7.55 (d, *J* = 8.99 Hz, 2H), 7.22 (d, *J* = 8.77 Hz, 2H), 7.09 - 7.16 (m, 1H), 7.04 (dd, *J* = 7.78, 4.93 Hz, 1H), 6.56 (d, *J* = 7.89 Hz, 1H), 6.50 (d, *J*=8.11 Hz, 1 H), 3.94 (t, *J* = 7.13 Hz, 4H), 2.29 (quin, *J* = 7.13 Hz, 2H), 2.01 (s, 3H).

**S93**

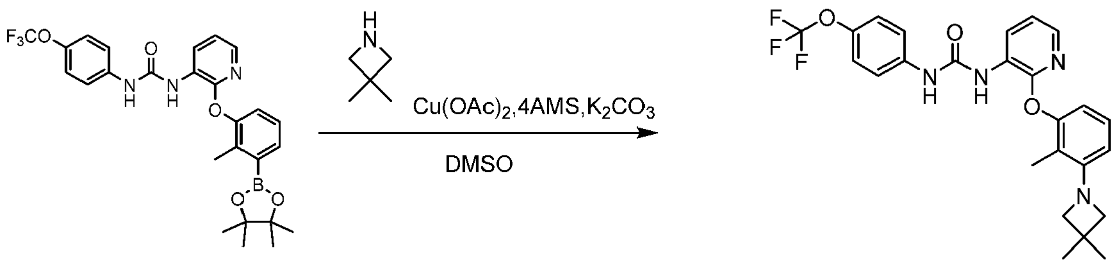


**1**

1-(2-(3-(3,3-dimethylazetidin-1-yl)-2-methylphenoxy)pyridin-3-yl)-3-(4-(trifluoromethoxy)phenyl)urea

To a solution of compound **1** (0.1 g, 189 umol, 1.0 eq.) in DMSO (2 mL) was added 4A MS (5 mg), 3,3-dimethylazetidine hydrochloride (46 mg, 378 umol, 2 .0 eq.), $K_2CO_3$ (52 mg, 378 umol, 2.0 eq.) and $Cu(OAc)_2$ (38 mg, 208 umol, 1.1 eq.). The mixture was stirred at 60 °C for 12 hr. LCMS showed compound 1 was consumed and the desired mass was detected. The reaction mixture was concentrated under reduced pressure to give a residue. The residue was purified by prep-HPLC (column: Waters Xbridge Prep OBD C18 150*40mm*10um; mobile phase: [water (10mM $NH_4HCO_3$)-ACN]; B%: 60%-85%,8min) to give the title material (9 mg, 10%) as a white solid. LC-MS: m/z 487.1 $[M+H]^+$; $^1H$ NMR (400MHz, $CDCl_3$) δ = 8.54 - 8.36 (m, 1H), 7.64 (dd, *J* = 0.9, 4.8 Hz, 1H), 7.35 - 7.22 (m, 4H), 7.06 (br d, *J* = 8.6 Hz, 2H), 7.01 - 6.91 (m, 1H), 6.86 (dd, *J* = 4.9, 7.9 Hz, 1H), 6.34 (br d, *J* = 7.9 Hz, 1H), 6.22 (br d, *J* = 8.0 Hz, 1H), 3.49 (s, 4H), 1.80 (s, 3H), 1.20 (s, 6H).

**Representative procedure for the synthesis of 2-azetidine containing compounds**

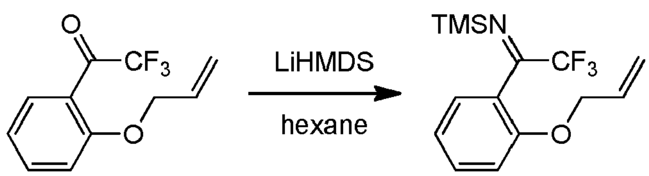


**1** **2**

To a solution of compound **1** (10 g, 43.44 mmol, 1.0 eq.) in hexane (200 mL) was added LiHMDS (1.0 M, 52 mL, 1.2 eq.) at 0°C. The mixture was stirred at 0°C for 0.5 hr and then 15°C for another 2 hr. TLC (petroleum ether: ethyl acetate = 5:1) showed compound 1 was consumed completely, and one new spot ($R_f$ = 0.58) was observed. The reaction mixture was quenched by the addition of ice-cold $H_2O$ (100 mL) at 0°C, and then washed with brine (200 mL x2). The combined organic layers were dried over $Na_2SO_4$, filtered and concentrated under reduced pressure to give a residue. Compound **2** (13 g, crude) was obtained as yellow oil.

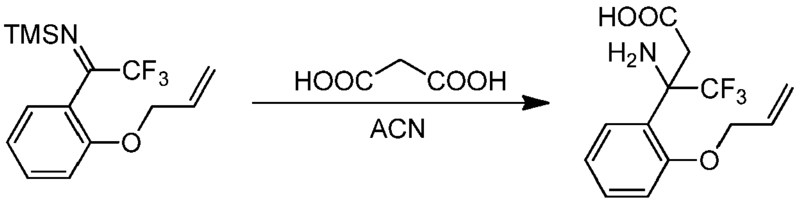


2 3

To a solution of compound **2** (12 g, 39.82 mmol, 1.0 eq.) in acetonitrile (200 mL) was added malonic acid (12.43 g, 119.45 mmol, 12.43 mL, 3.0 eq.). The mixture was stirred at 15°C for 12 hr. The reaction mixture was concentrated under reduced pressure to give a residue. The crude product was purified by reversed-phase HPLC(TFA condition) (column: C18 20-35um 100A 3000g; mobile phase: [water-ACN]; B%:20-40% 30min;40-40% 20min@250mL/min). Compound **3** (11 g, 38.03 mmol, 95.51% yield) was obtained as yellow gum. LC-MS: m/z 288.0 $[M+H]^+$; (400 MHz, DMSO-$d_6$) $\delta$ = 7.56 (d, *J* = 7.8 Hz, 1H), 7.23 (t, *J* = 7.6 Hz, 1H), 7.01 (d, *J* = 8.3 Hz, 1H), 6.88 (t, *J* = 7.5 Hz, 1H), 6.13 - 6.01 (m, 1H), 5.47 - 5.37 (m, 1H), 5.27 (br d, *J*= 10.5 Hz, 1H), 4.59 (br d, *J*= 4.1 Hz, 2H), 3.01 (d, *J*= 15.0 Hz, 1H), 2.43 (d, *J*= 15.0 Hz, 1H).

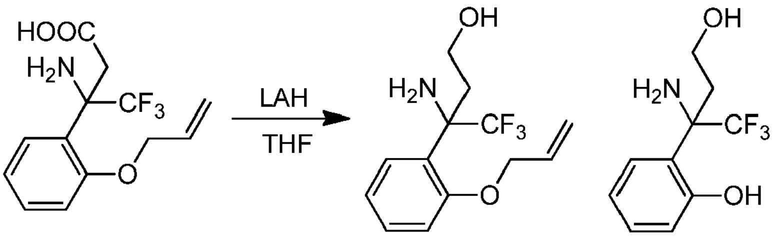


3 4 4A

To a solution of compound **3** (2.0 g, 6.91 mmol, 1.0 eq.) in THF (30 mL) was added $LiAlH_4$ (1.05 g, 27.66 mmol, 4.0 eq.) at 0 °C. The mixture was stirred at 25°C for 12hr. The reaction mixture was quenched by addition of sat. aqueous $MgSO_4$ solution (3 mL) at 0°C, and then diluted with EtOAc (50 mL) and filtered. The filter cake was suspended in EtOAc (50 mL), agitated and the slurry filtered (x2). The combined filtrates were dried over $Na_2SO_4$, filtered and concentrated under reduced pressure to give a residue. A mixture of compound **4** and compound **4A** (900 mg, crude) was obtained as yellow oil.

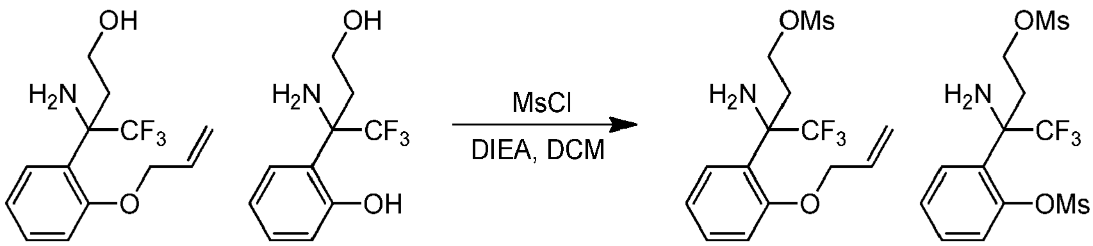


4 4A 5 5A

2 parallel reactions were set. To a mixture of compound **4** (3.0 g, 12.75 mmol, 1.0 eq.), compound **4A** (12.75 mmol, 1.0 eq.) and DIPEA (4.95 g, 38.25 mmol, 6.66 mL, 3.0 eq.) in DCM (30 mL) was added methanesulfonyl chloride(3.67 g, 32.00 mmol, 2.48 mL, 2.5 eq.) at 0 °C. The mixture was stirred at 0 °C for 1 hr. The two reactions were combined for work-up. The reaction mixture was quenched by addition of $H_2O$ (40 mL) and then the combined organic layers were dried over $Na_2SO_4$, filtered and concentrated under reduced pressure to give a residue. A mixture of compound **5** and compound **5A** (9.6 g, crude) was obtained as a red oil.

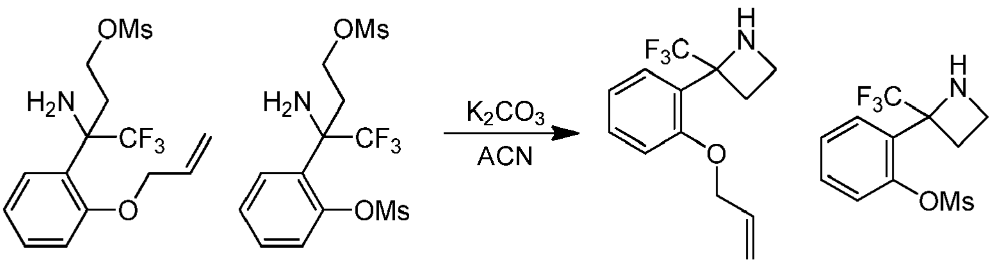


5 5A 6 6A

A mixture of compound **5** (4.8 g, 12.26 mmol, 1.0 eq.), compound **5A** (1.0 eq.) in acetonitrile (40 mL) was added $K_2CO_3$ (6.78 g, 49.04 mmol, 4.0 eq.). The mixture was stirred at 80°C for 12 hr. The reaction mixture was filtered and concentrated under reduced pressure to give a residue. The residue was dissolved in EtOAc (100 mL), washed with brine (100 mL x2). The combined organic layers were dried over $Na_2SO_4$, filtered and then concentrated under reduced pressure to give a residue. The residue was purified by column chromatography ($SiO_2$, petroleum ether/ ethyl acetate = 1/ 0 to 1/ 1) to give compound **6** (1.7 g, 28%) as a red oil. TLC: petroleum ether: ethyl acetate = 3:1, $R_f$ = 0.53; LC-MS: m/z 258.4 $[M+H]^+$; $^1H$ NMR (400MHz, $CDCl_3$) $\delta$ = 7.33 - 7.24 (m, 3H), 7.03 - 6.98 (m, 1H), 6.91 (d, *J* = 8.3 Hz, 1H), 6.09 - 5.97 (m, 1H), 5.41 (dd, *J* = 1.5, 17.2 Hz, 1H), 5.27 (dd, *J* = 1.4, 10.7 Hz, 1H), 4.63 - 4.51 (m, 2H), 3.79 - 3.69 (m, 1H), 3.54 - 3.42 (m, 1H), 3.02 - 2.95 (m, 1H), 2.94 - 2.84 (m, 1H). and compound **6A** (2.6 g, 37%) as a red oil. TLC: petroleum ether: ethyl acetate = 2:1, $R_f$ = 0.13; LC-MS: m/z 296.1 $[M+H]^+$; $^1H$ NMR (400MHz, $CDCl_3$) $\delta$ = 7.60 (d, *J* = 8.4 Hz, 1H), 7.42 - 7.28 (m, 3H), 3.89 (q, *J* = 7.9 Hz, 1H), 3.49 - 3.39 (m, 1H), 3.24 (s, 3H), 3.01 - 2.94 (m, 2H).

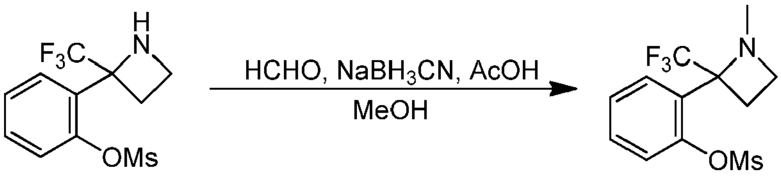


6A 7

To a solution of compound **6A** (200 mg, 0.68 mmol, 1.0 eq.) and formaldehyde (274 mg, 3.39 mmol, 252 uL, 37% wt., 5.0 eq.) in MeOH (10 mL) was added $NaBH_3CN$ (170 mg, 2.71 mmol, 4.0 eq.) and AcOH (41 mg, 0.68 mmol, 39 uL, 1.0 eq.) at 0 °C. The mixture was stirred at 15°C for 12 hr. The reaction mixture was concentrated under reduced pressure and the residue dissolved in $H_2O$ (5 mL), then extracted with EtOAc (10 mL x3). The combined organic layers were dried over $Na_2SO_4$, filtered and the filtrate concentrated under reduced pressure to give a residue. Compound 7 (280 mg, crude) was obtained as a yellow oil.

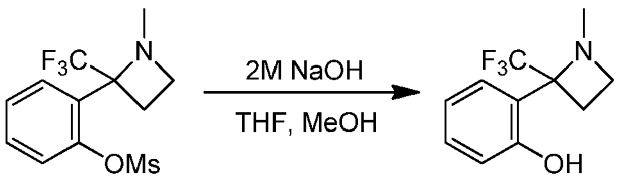


7 8

To a solution of compound **7** (140 mg, 453 umol, 1.0 eq.) in THF (2 mL) and MeOH (2 mL) was added NaOH (2.0 M, 566 uL, 2.5 eq.). The mixture was stirred at 30 °C for 3 hr. The reaction mixture was concentrated under reduced pressure to give a residue. Compound **8** (100 mg, crude) was obtained as a yellow oil and used directly in the next reaction.

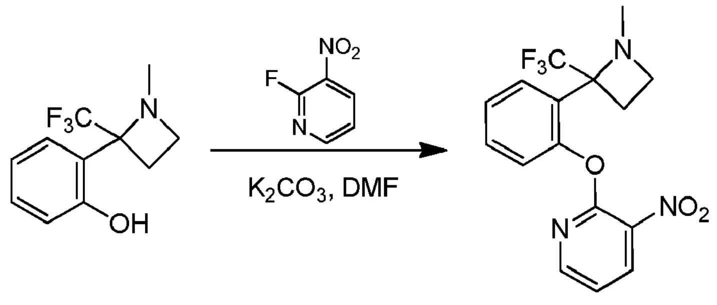


**8** **9**

To a solution of compound **8** (100 mg, 433 umol, 1.0 eq.) in DMF (2 mL) was added $K_2CO_3$ (179 mg, 1.30 mmol, 3.0 eq.) and 2-fluoro-3-nitro-pyridine (74 mg, 519 umol, 1.2 eq.). The mixture was stirred at 15 °C for 12 hr. The reaction mixture was quenched with $H_2O$ (5 mL), and then extracted with EtOAc (10 mL x3). The combined organic layers were dried over $Na_2SO_4$, filtered and the filtrate concentrated under reduced pressure to give a residue. The residue was purified by prep-TLC ($SiO_2$, petroleum ether: ethyl acetate = 2:1) to give compound **9** (70 mg, 28.0%) as a yellow oil. TLC: petroleum ether: ethyl acetate = 2:1, $R_f$ = 0.42; LC-MS: m/z 354.2 $[M+H]^+$; $^1H$ NMR (400MHz, $CDCl_3$) $\delta$ = 8.37 - 8.33 (m, 2H), 7.54 (dd, $J$ = 1.5, 7.8 Hz, 1H), 7.41 - 7.35 (m, 1H), 7.33 - 7.28 (m, 1H), 7.19 - 7.14 (m, 1H), 7.09 (d, $J$ = 8.1 Hz, 1H), 3.51 (br s, 1H), 3.41 - 3.28 (m, 1H), 2.84 - 2.67 (m, 5H).

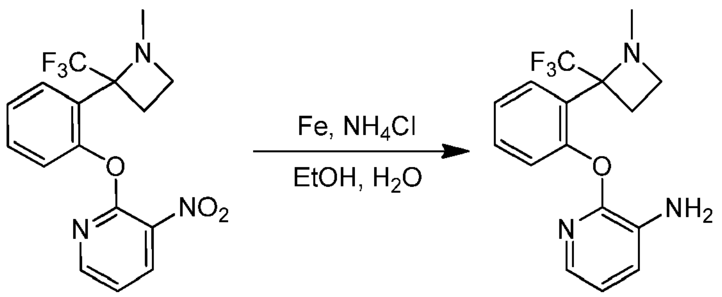


**9** **10**

To a solution of compound **9** (55 mg, 156 umol, 1.0 eq.) in EtOH (1 mL) and $H_2O$ (1 mL) was added Fe (87 mg, 1.56 mmol, 10.0 eq.) and $NH_4Cl$ (83 mg, 1.56 mmol, 10.0 eq.). The mixture was stirred at 50°C for 2 hr. The reaction mixture was filtered and concentrated under reduced pressure to give a residue. The residue was purified by prep-HPLC (TFA condition) column: Phenomenex Luna 80*30mm*3um;mobile phase: [water(0.1%TFA)-ACN];B%: 15%-45%,8min to give compound **10** (20 mg, 40%) as a brown solid. LC-MS: m/z 324.2 $[M+H]^+$; $^1H$ NMR (400MHz, $CDCl_3$) $\delta$ = 7.44 (dd, $J$ = 1.3, 7.9 Hz, 1H), 7.39 - 7.33 (m, 2H), 7.23 - 7.18 (m, 1H), 7.13 (dd, $J$ = 1.5, 7.7 Hz, 1H), 6.94 - 6.88 (m, 2H), 3.56 (br s, 1H), 3.47 (br d, $J$ = 7.5 Hz, 1H), 2.94 - 2.63 (m, 5H).

**S89**

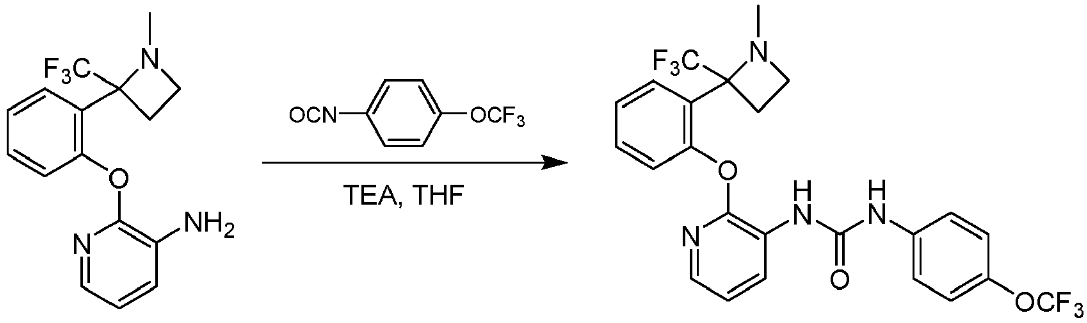


10

To a solution of compound **10** (15 mg, 46 umol, 1.0 eq.) and TEA (9.4 mg, 93 umol, 12.9 uL, 2.0 eq.) in THF (3 mL) was added 1-isocyanato-4-(trifluoromethoxy)benzene (11.3 mg, 56 umol, 8.4 uL, 1.2 eq.) at 0 °C. The mixture was stirred at 15°C for 2 hr. The reaction mixture was concentrated under reduced pressure to give a residue. The residue was purified by prep-HPLC (neutral condition) column: Waters Xbridge BEH C18 100*30mm*10um; mobile phase: [water(10mM $NH_4HCO_3$)-ACN];B%: 50%-80%,8min to give the title compound (20 mg, 80%) as a white solid. ; LC-MS: m/z 527.0 $[M+H]^+$; $^1H$ NMR (400MHz, methanol-$d_4$) δ = 8.53 (dd, *J* = 1.6, 8.0 Hz, 1H), 7.74 (dd, *J* = 1.6, 4.9 Hz, 1H), 7.57 - 7.54 (m, 1H), 7.54 - 7.52 (m, 1H), 7.49 (dd, *J* = 1.3, 7.9 Hz, 1H), 7.40 - 7.34 (m, 1H), 7.29 - 7.24 (m, 1H), 7.21 (d, *J* = 8.8 Hz, 2H), 7.11 (dd, *J* = 4.9, 8.0 Hz, 1H), 6.95 (dd, *J* = 1.1, 8.3 Hz, 1H), 3.51 - 3.44 (m, 1H), 3.40 - 3.32 (m, 1H), 2.90 - 2.79 (m, 1H), 2.74 - 2.64 (m, 4H).

**Representative procedures for chiral separation of racemic compounds**

Chiral separation of **S11**

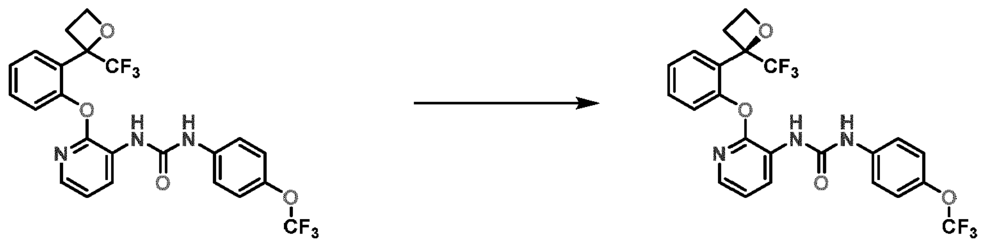

1-(4-(trifluoromethoxy)phenyl)-3-(2-(2-(2-(trifluoromethyl)oxetan-2-yl)phenoxy)pyridin-3-yl)urea

(*R*)-1-(4-(trifluoromethoxy)phenyl)-3-(2-(2-(2-(trifluoromethyl)oxetan-2-yl)phenoxy)pyridin-3-yl)urea

+

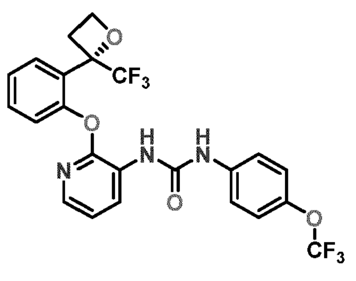

(*S*)-1-(4-(trifluoromethoxy)phenyl)-3-(2-(2-(2-(trifluoromethyl)oxetan-2-yl)phenoxy)pyridin-3-yl)urea

The racemic compound S11 (1954 mg) was separated by SFC (column: DAICEL CHIRALPAK AD(250mm*30mm,10um);mobile phase: $CO_2$/[0.1%$NH_3H_2O$ MEOH];B%: 17% - 17%,4 min) to give compound S110 (610 mg, 31%) and S111 (543 mg, 28%) as white solids.

**S110** LC-MS: m/z 514.2 $[M+H]^+$; **Analytical SFC Analysis:** Chiralpak AD-3 ($CO_2$/MeOH(0.1%IPA)) = 95/5 - 50/50, 2.5 mL $min^{-1}$, 35 °C) $tR_{major}$ = 0.87 min (area = 99.7%); $tR_{minor}$ = 0.81 min (area = 0.3%); *ee* = 99.4%;

$[\alpha]_D^{20}$ = -8.83° (c = 0.79, MeOH).

**S111** LC-MS: m/z 514.2 [M+H]$^+$; **Analytical SFC Analysis:** Chiralpak AD-3 ($CO_2$/MeOH(0.1%IPA)) = 95/5 - 50/50, 2.5 mL min$^{-1}$, 35 °C) $tR_{major}$ = 0.81 min (area = 100.0%); *ee* = 100%; $[\alpha]_D^{20}$ = +10.06° (c = 0.63, MeOH).

Chiral separation of **S9**

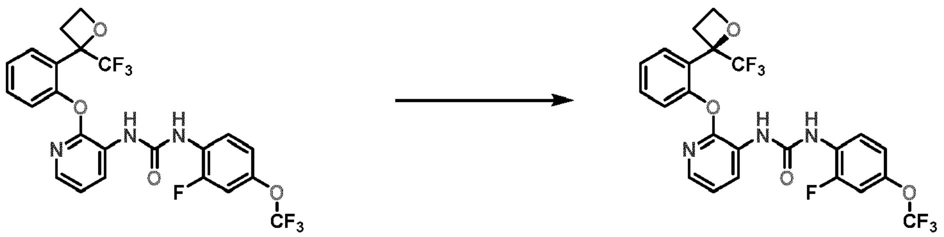

1-(2-fluoro-4-(trifluoromethoxy)phenyl)-3-(2-(2-(2-(trifluoromethyl)oxetan-2-yl)phenoxy)pyridin-3-yl)urea

(*R*)-1-(2-fluoro-4-(trifluoromethoxy)phenyl)-3-(2-(2-(2-(trifluoromethyl)oxetan-2-yl)phenoxy)pyridin-3-yl)urea

\+

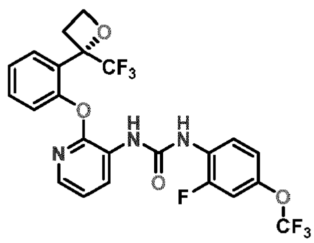

(*S*)-1-(2-fluoro-4-(trifluoromethoxy)phenyl)-3-(2-(2-(2-(trifluoromethyl)oxetan-2-yl)phenoxy)pyridin-3-yl)urea

The racemic compound S9 (250 mg) was separated by SFC (column: DAICEL CHIRALPAK AD(250mm*30mm, 10um); mobile phase: $CO_2$/[0.1%$NH_3H_2O$ MEOH];B%: 10% - 10%,10 min) to give compound S112 (85 mg, 34%) and S107 (68 mg, 27%) as white solids.

**S107** LC-MS: m/z 532.0 [M+H]$^+$; **Analytical SFC Analysis:** Chiralpak AD-3 ($CO_2$/MeOH(0.1%IPA)) = 90/10 - 50/50, 2.5 mL min$^{-1}$, 35 °C) $tR_{major}$ = 1.49 min (area = 100.0%); *ee* = 100.0%.

**S112** LC-MS: m/z 532.0 [M+H]$^+$; **Analytical SFC Analysis:** Chiralpak AD-3 ($CO_2$/MeOH(0.1%IPA)) = 90/10 - 50/50, 2.5 mL min$^{-1}$, 35 °C) $tR_{minor}$ = 1.50 min (area = 1.6%); $tR_{major}$ = 1.60 min (area = 98.4%); *ee* = 96.8%.

Chiral separation of **S2**

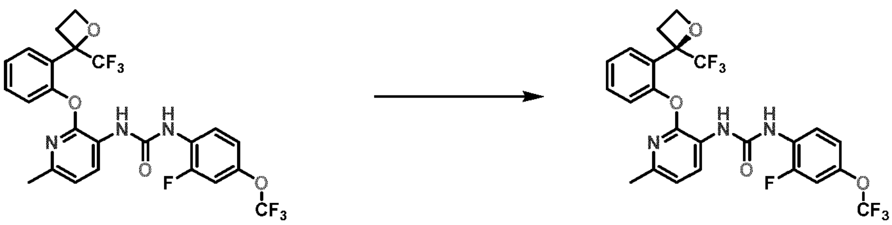

1-(2-fluoro-4-(trifluoromethoxy)phenyl)-3-(6-methyl-2-(2-(2-(trifluoromethyl)oxetan-2-yl)phenoxy)pyridin-3-yl)urea

(*R*)-1-(2-fluoro-4-(trifluoromethoxy)phenyl)-3-(6-methyl-2-(2-(2-(trifluoromethyl)oxetan-2-yl)phenoxy)pyridin-3-yl)urea

+

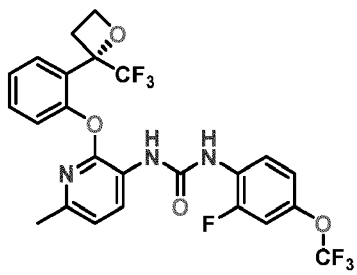

(*S*)-1-(2-fluoro-4-(trifluoromethoxy)phenyl)-3-(6-methyl-2-(2-(2-(trifluoromethyl)oxetan-2-yl)phenoxy)pyridin-3-yl)urea

The racemic compound S2 (164 mg) was separated by SFC (column: DAICEL CHIRALPAK AD(250mm*30mm, 10um);mobile phase: $CO_2$/[0.1%$NH_3H_2O$ MEOH];B%: 12.5% - 12.5%,10 min) to give compound S113 (43 mg, 26%) and S104 (41 mg, 25%) as white solids.

**S104** LC-MS: m/z 546.0 $[M+H]^+$; **Analytical SFC Analysis:** Chiralpak AD-3 ($CO_2$/MeOH(0.1%IPA)) = 90/10 - 50/50, 2.5 mL $min^{-1}$, 35 °C) $tR_{major}$ = 1.50 min (area = 99.9%); $tR_{minor}$ = 1.84 min (area = 0.14%); *ee* = 99.7%.

S113 LC-MS: m/z 546.0 $[M+H]^+$; **Analytical SFC Analysis:** Chiralpak AD-3 ($CO_2$/MeOH(0.1%IPA)) = 90/10 - 50/50, 2.5 mL $min^{-1}$, 35 °C) $tR_{minor}$ = 1.45 min (area = 0.51%); $tR_{major}$ = 1.79 min (area = 98.4%); *ee* = 99.0%.

Chiral separation of **S4**

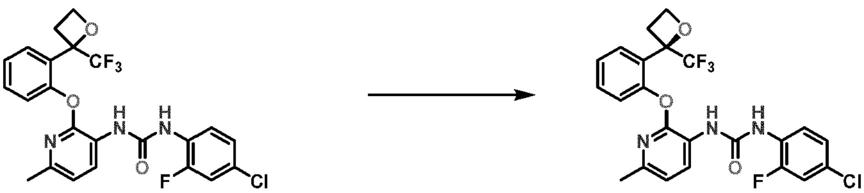

1-(4-chloro-2-fluorophenyl)-3-(6-methyl-2-(2-(2-(trifluoromethyl)oxetan-2-yl)phenoxy)pyridin-3-yl)urea

(*R*)-1-(4-chloro-2-fluorophenyl)-3-(6-methyl-2-(2-(2-(trifluoromethyl)oxetan-2-yl)phenoxy)pyridin-3-yl)urea

+

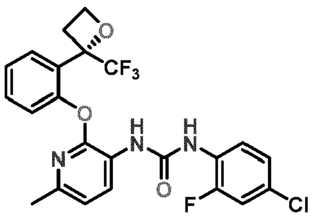

(*S*)-1-(4-chloro-2-fluorophenyl)-3-(6-methyl-2-(2-(2-(trifluoromethyl)oxetan-2-yl)phenoxy)pyridin-3-yl)urea

The racemic compound S4 (1673mg) was separated by SFC (column: DAICEL CHIRALPAK AD(250mm*30mm, 10um);mobile phase: $CO_2$/[0.1%$NH_3H_2O$ MEOH];B%: 27% - 27%, 10 min) to give compound S109 (47 mg, 27%) and S114 (48 mg, 28%) as yellow solids.

**S109** LC-MS: m/z 496.0 $[M+H]^+$; **Analytical SFC Analysis:** Chiralpak AD-3 ($CO_2$/MeOH(0.1%IPA)) = 90/10 - 50/50, 2.5 mL $min^1$, 35 °C) $tR_{major}$ = 2.35 min (area = 99.9%); $tR_{minor}$ = 2.74 min (area = 0.10%); *ee* = 99.8%.

**S114** LC-MS: m/z 496.0 $[M+H]^+$; **Analytical SFC Analysis:** Chiralpak AD-3 ($CO_2$/MeOH(0.1%IPA)) = 90/10 - 50/50, 2.5 mL $min^{-1}$, 35 °C) $tR_{minor}$ = 2.31 min (area = 0.30%); $tR_{major}$ = 2.70 min (area = 99.7%); *ee* = 99.4%.

Chiral separation of **S89**

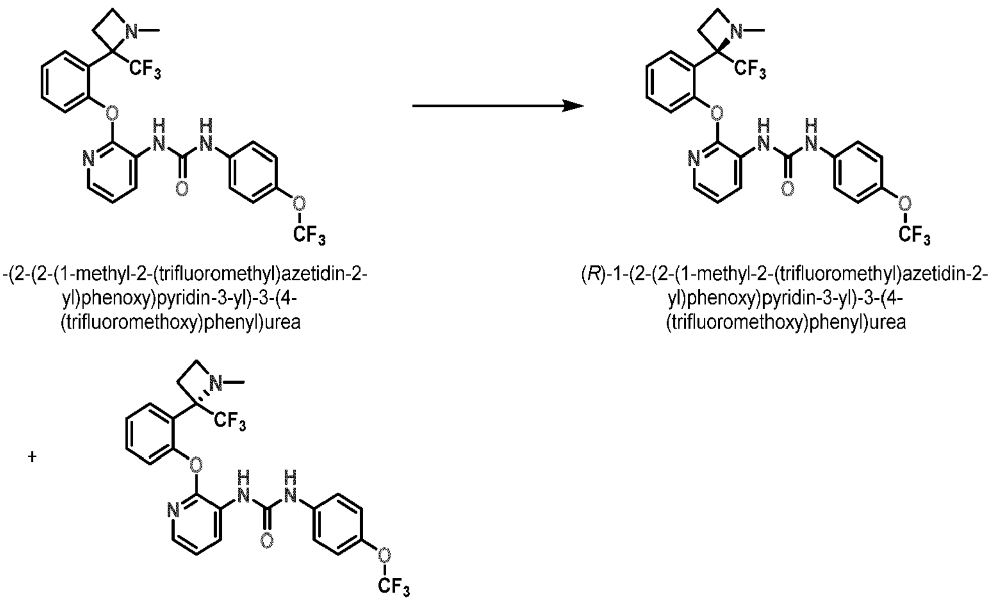

1-(2-(2-(1-methyl-2-(trifluoromethyl)azetidin-2-yl)phenoxy)pyridin-3-yl)-3-(4-(trifluoromethoxy)phenyl)urea

(*R*)-1-(2-(2-(1-methyl-2-(trifluoromethyl)azetidin-2-yl)phenoxy)pyridin-3-yl)-3-(4-(trifluoromethoxy)phenyl)urea

+

(*S*)-1-(2-(2-(1-methyl-2-(trifluoromethyl)azetidin-2-yl)phenoxy)pyridin-3-yl)-3-(4-(trifluoromethoxy)phenyl)urea

The racemic compound S89 (100 mg) was separated by SFC (column: DAICEL CHIRALPAK AD(250mm*30mm,10um);mobile phase: $CO_2$/[0.1%$NH_3H_2O$ EtOH];B%: 14% - 14%,6 min) to give compound S105 (40 mg, 40%) and S115 (38 mg, 38%) as white solids respectively.

**S105** LC-MS: m/z 527.1 $[M+H]^+$; **Analytical SFC Analysis:** Chiralpak AD-3 ($CO_2$/EtOH (0.1%IPA)) = 95/5 - 50/50, 4.0 mL $min^{-1}$, 35 °C) $tR_{major}$ = 1.04 min (area = 99.8%); $tR_{minor}$ = 1.50 min (area = 0.16%); *ee* = 99.7%.

**S115** LC-MS: m/z 527.1 $[M+H]^+$; **Analytical SFC Analysis:** Chiralpak AD-3 ($CO_2$/EtOH(0.1%IPA)) = 95/5 - 50/50, 4.0 mL $min^{-1}$, 35 °C) $tR_{minor}$ = 1.03 min (area = 2.27%); $tR_{major}$ = 1.44 min (area = 97.7%); *ee* = 95.5%.

***Representative procedure for preparation of compounds via chiral separation of intermediates***

Chiral separation of racemic 2-(2-(2-(trifluoromethyl)oxetan-2-yl)phenoxy)pyridin-3-amine **7**

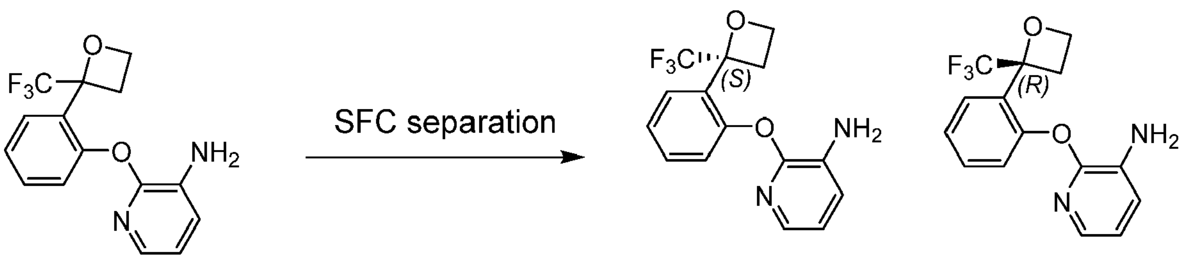


**7** **7A** **7B**

The racemic compound 7 (1000 mg) was separated by SFC (column: DAICEL CHIRALCEL OJ(250mm*30mm,10um);mobile phase: $CO_2$[0.1%$NH_3H_2O$ IPA];B%: 18%-18%,5min) to give compounds **7A** (414 mg, 41%) and **7B** (408 mg, 41%) as white solids. **7A** LC-MS: m/z 311.1 $[M+H]^+$; Analytical SFC Analysis: Chiralpak AD-3 (CO2/IPA(0.1%IPA)) = 90/10 - 50/50, 2.5 mL $min^{-1}$, 35 °C) $tR_{major}$ = 2.06 min (area = 100.0%); *ee* = 100.0%.

**7B** LC-MS: m/z 311.1 $[M+H]^+$; Analytical SFC Analysis: Chiralpak AD-3 ($CO_2$/IPA(0.1%IPA)) = 90/10 - 50/50, 2.5 mL $min^{-1}$, 35 °C) $tR_{major}$ = 2.33 min (area = 100.0%); *ee* = 100.0%.

**S101**

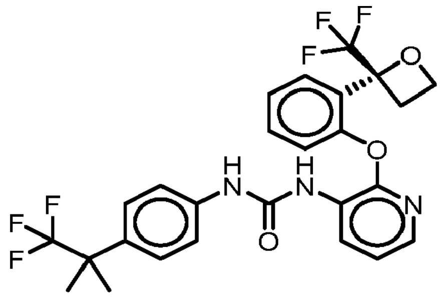

Prepared according to **general procedure 2** using (*R*)-2-(2-(2-(trifluoromethyl)oxetan-2-yl)phenoxy)pyridin-3-amine (60 mg, 1.0 eq.) and 4-(2,2,2-trifluoro-1,1-dimethylethyl)aniline (43.21 mg, 1.1 eq.) to give the title material as a white solid (61 mg, 59%). LC-MS: m/z 540.1 $[M+H]^+$; $^1H$ NMR (400MHz, $CDCl_3$) $\delta$ = 8.61 (dd, *J* = 8.00, 1.63 Hz, 1H), 7.83 (dd, *J* = 4.88, 1.63 Hz, 1H), 7.53 (d, *J* = 7.75 Hz, 1H), 7.41 - 7.50 (m, 3H), 7.38 (br d, *J* = 8.63 Hz, 3H), 7.28 - 7.32 (m, 1H), 7.26 (br s, 1H), 7.08 (dd, *J* = 8.00, 4.88 Hz, 1H), 6.46 (s, 1H), 4.76 - 4.88 (m, 1H), 4.57 (dt, *J* = 8.85, 5.96 Hz, 1H), 2.99 - 3.18 (m, 2H), 1.56 (d, *J* = 4.00 Hz, 6H).

**Synthesis of reference compound RS1**

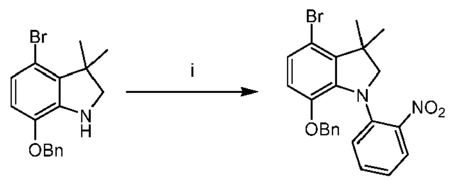


**1A** **2A**

STEP 1: To a solution of 7-(benzyloxy)-4-bromo-3,3-dimethylindoline **1A** (3.0 g, 9.03 mmol, 1.0 eq.) in toluene (30 mL) was added $Cs_2CO_3$ (8.83 g, 27.09 mmol, 3.0 eq.), 1-bromo-2-nitro-benzene (2.19 g, 10.84 mmol, 1.2 eq.), *rac*-BINAP (562 mg, 0.90 mmol, 0.1 eq.) and $Pd_2(dba)_3$ (413 mg, 0.52 mmol, 0.05 eq.). The mixture was degassed and purged with $N_2$ (x3), and then the mixture was stirred at 110 °C for 12 hr under $N_2$ atmosphere. The reaction mixture was diluted with $H_2O$ (30 mL), and then extracted with EtOAc (30mL x3). The combined organic layers were washed with brine (50 mL), dried over $Na_2SO_4$, filtered and the filtrate concentrated under reduced pressure to give a residue. The residue was purified by column chromatography ($SiO_2$, petroleum ether/ethyl acetate=0/1 to 9/1). 7-(benzyloxy)-4-bromo-3,3-dimethyl-1-(2-nitrophenyl)indoline **2A** (3.26 g, 80%) was obtained as red solid. LC-MS: m/z 453.2/455.2$[M+H]^+$.

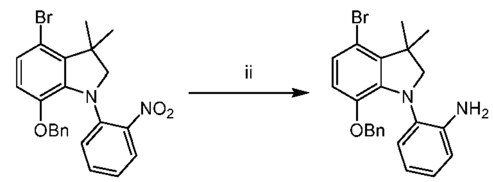


**2A** **1**

STEP 2: A mixture of 7-(benzyloxy)-4-bromo-3,3-dimethyl-1-(2-nitrophenyl)indoline **2A** (1.30 g, 2.87 mmol, 1.0 eq.) and Fe (801 mg, 14.34 mmol, 5.0 eq.) in AcOH (30 mL) was stirred at 80 °C for 3 hr. LC-MS showed that compound **2A** was completely consumed and the desired product mass was detected. The reaction mixture was concentrated under reduced pressure to remove AcOH. Then the residue was taken-up in $H_2O$ (50 mL) and extracted with EtOAc (50 mL x3). The combined organic layers were washed with sat. $NaHCO_3$ solution (50 mL x3), dried over $Na_2SO_4$, filtered and the filtrate concentrated under reduced pressure to give a residue. 2-(7-(benzyloxy)-4-bromo-3,3-dimethylindolin-1-yl)aniline **1** (1.36 g, crude) was obtained as black oil and used directly in the next reaction without further purification. LC-MS: m/z 423.0/425.0$[M+H]^+$.

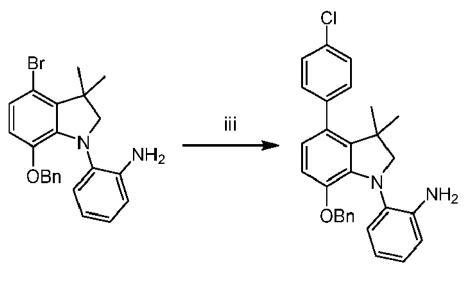


1 2

STEP 3: To a mixture of 2-(7-(benzyloxy)-4-bromo-3,3-dimethylindolin-1-yl)aniline **1** (300 mg, 0.71 mmol, 1.0 eq.), (4-chlorophenyl)boronic acid (133 mg, 0.85 mmol, 1.2 eq.), $Cs_2CO_3$ (577 mg, 1.77 mmol, 2.5 eq.) in dioxane (5 mL) and $H_2O$ (0.5 mL) was added Pd(dppf)$Cl_2$ (52 mg, 71 umol, 0.1 eq.). The mixture was degassed and purged with $N_2$ (x3) and then the mixture was stirred at 80 °C for 12 hr under a $N_2$ atmosphere. LC-MS showed that compound **1** was completely consumed and the desired product mass was detected. The reaction mixture was diluted with $H_2O$ (10 mL) and extracted with EtOAc (20 mL x3). The combined organic layers were washed with brine (50 mL), dried over $Na_2SO_4$, filtered and the filtrate concentrated under reduced pressure to give a residue. The residue was purified by prep-TLC ($SiO_2$, petroleum ether: ethyl acetate = 4:1) to give 2-(7-(benzyloxy)-4-(4-chlorophenyl)-3,3-dimethylindolin-1-yl)aniline **2** (200 mg, 62%) as a brown solid. LC-MS: m/z 455.2 $[M+H]^+$.

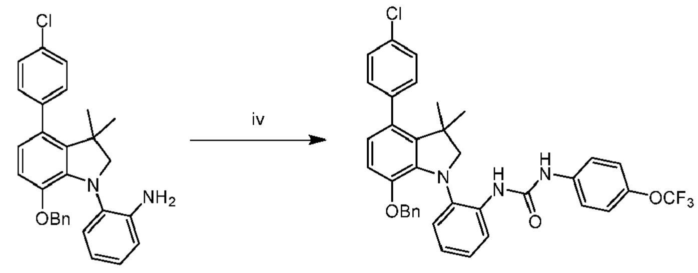


2 3

STEP 4: To a solution of 2-(7-(benzyloxy)-4-(4-chlorophenyl)-3,3-dimethylindolin-1-yl)aniline **2** (160 mg, 0.35 mmol, 1.0 eq.) and TEA (36 mg, 0.35 mmol, 49 uL, 1.0 eq.) in THF (2 mL) was added 1-isocyanato-4-(trifluoromethoxy)benzene (86 mg, 0.42 mmol, 64 uL, 1.2 eq.) at 0°C. The mixture was stirred at 15°C for 2 hr. LC-MS showed that compound **2** was completely consumed and the desired product mass was detected. The reaction mixture was concentrated under reduced pressure to give a residue. 1-(2-(7-(Benzyloxy)-4-(4-chlorophenyl)-3,3-dimethylindolin-1-yl)phenyl)-3-(4-(trifluoromethoxy)phenyl)urea (200 mg, crude) was obtained as a dark-brown oil.

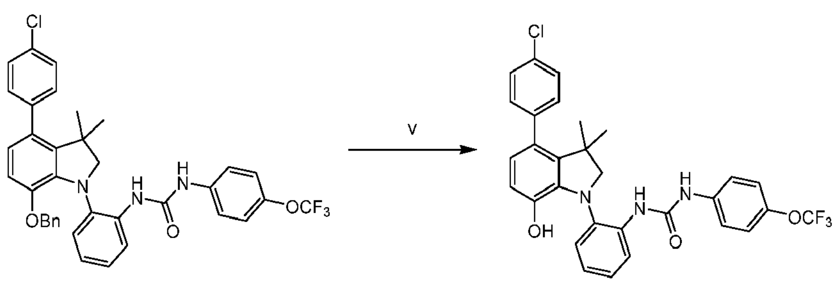


**3** **RS1**

**1-(2-(4-(4-chlorophenyl)-7-hydroxy-3,3-dimethylindolin-1-yl)phenyl)-3-(4-(trifluoromethoxy)phenyl)urea (RS1)**

STEP 5: A mixture of 1-(2-(7-(benzyloxy)-4-(4-chlorophenyl)-3,3-dimethylindolin-1-yl)phenyl)-3-(4-(trifluoromethoxy)phenyl)urea **3** (190 mg, 0.29 mmol, 1.0 eq.) and Raney Ni (95 mg, 1.62 mmol, 5.6 eq.) in MeOH (2 mL) was degassed and purged with $H_2$ (x3), and then the mixture was stirred at 50°C for 6 hr under $H_2$ (15 psi) atmosphere. LC-MS showed that compound **3** was completely consumed and the desired product mass was detected. The reaction mixture was filtered and concentrated under reduced pressure to give a residue. The residue was purified by prep-HPLC (basic condition LC) column: Phenomenex Gemini-NX C18 75*30mm*3um;mobile phase: [water(10mM $NH_4HCO_3$)-ACN];B%: 75%-95%,8min to give the title material (115 mg, 70%) as a white solid. LC-MS: m/z 568.0 $[M+H]^+$; $^1H$ NMR (400MHz, Methanol-$d_4$) $\delta$ = 7.92 - 7.83 (m, 1H), 7.56 - 7.47 (m, 2H), 7.42 - 7.34 (m, 2H), 7.31 - 7.24 (m, 2H), 7.20 - 7.14 (m, 2H), 7.12 - 7.06 (m, 1H), 7.03 - 6.94 (m, 2H), 6.66 - 6.47 (m, 2H), 3.74 (d, *J*= 9.9 Hz, 1H), 3.12 (d, *J*= 9.9 Hz, 1H), 1.08 (s, 6H).

**Synthesis of reference compound RS2**

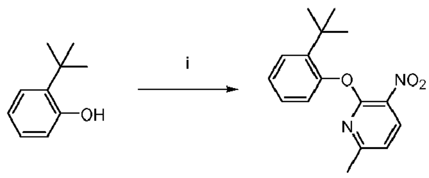


1 2

STEP 1: To a solution of 2-tert-butylphenol **1** (1.01 g, 6.66 mmol, 1.02 mL, 1.0 eq.) in DMF (10 mL) was added $K_2CO_3$ (2.76 g, 19.97 mmol, 3.0 eq.) and 2-fluoro-6-methyl-3-nitropyridine (1.04 g, 6.66 mmol, 1.0 eq.). The mixture was stirred at 80 °C for 12 hr. LC-MS showed the main peak was the desired mass. The reaction mixture was concentrated under reduced pressure to remove solvent. The residue was purified by column chromatography ($SiO_2$, petroleum ether/ethyl acetate = 1/0 to 1/1) to give 2-(2-(tert-butyl)phenoxy)-6-methyl-3-nitropyridine **3** (1.5 g, 5.24 mmol, 79% yield) as a light yellow solid. LC-MS: m/z 287.1 $[M+H]^+$.

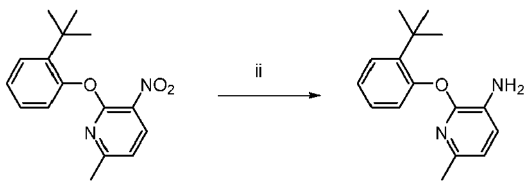


2 3

STEP 2: To a solution of 2-(2-(tert-butyl)phenoxy)-6-methyl-3-nitropyridine **3** (500 mg, 1.75 mmol, 1.0 eq.) in MeOH (10 mL) was added Pd/C (10% wt., 200 mg, 0.19 mmol, .1 eq.) under $N_2$. The suspension was degassed under vacuum and purged with $H_2$ (15 psi) several times. The mixture was stirred under $H_2$ (15 psi) at 20 °C for 2 hr. LC-MS showed that compound **3** was completely consumed and the desired product mass was detected. The reaction mixture was purged with $N_2$, filtered and the filtrate concentrated under reduced pressure. 2-(2-(tert-butyl)phenoxy)-6-methylpyridin-3-amine **3** (447 mg, crude) was obtained as a white solid and used without further purification. LC-MS: m/z 257.1 $[M+H]^+$.

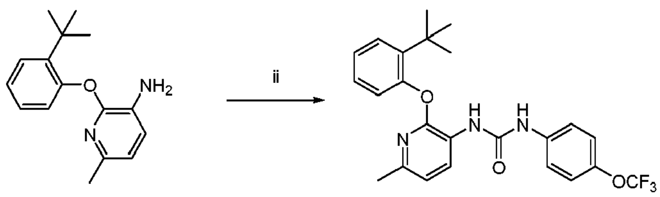


3 RS2

**1-(2-(2-(tert-butyl)phenoxy)-6-methylpyridin-3-yl)-3-(4-(trifluoromethoxy)phenyl)urea (RS2)**

STEP 3: To a solution of 2-(2-(tert-butyl)phenoxy)-6-methylpyridin-3-amine **3** (447 mg, 1.74 mmol, 1.0 eq.) in THF (5 mL) was added TEA (529 mg, 5.23 mmol, 728 uL, 3.0 eq.) and 1-isocyanato-4-(trifluoromethoxy) benzene (354 mg, 1.74 mmol, 262 uL, 1.0 eq.). The mixture was stirred at 0 °C for 1 hr. LC-MS showed that compound **3** was completely consumed and the desired product mass was detected. The reaction mixture was concentrated under reduced pressure

to remove solvent. The residue was purified by prep-HPLC (column: Waters Xbridge Prep OBD C18 150*40mm*10um; mobile phase: [water(0.05% $NH_3H_2O$+10mM $NH_4HCO_3$)-ACN];B%: 55%-85%,8min) to give the title compound (371 mg, 46%) as a light yellow solid. LC-MS: m/z 460.2 $[M+H]^+$; $^1H$ NMR (400MHz, $CDCl_3$) $\delta$ = 8.41 - 8.38 (m, 1H), 8.39 (d, *J* = 8.0 Hz, 1H), 8.75 - 8.26 (m, 1H), 7.44 - 7.41 (m, 1H), 7.45 - 7.36 (m, 1H), 7.37 (d, *J* = 9.0 Hz, 1H), 7.21 - 7.11 (m, 4H), 7.02 (s, 1H), 6.90 - 6.81 (m, 2H), 6.52 (s, 1H), 2.31 (s, 3H), 1.34 (s, 9H).

## Claims

1. A compound that (a) is of formula (I) or formula (II):

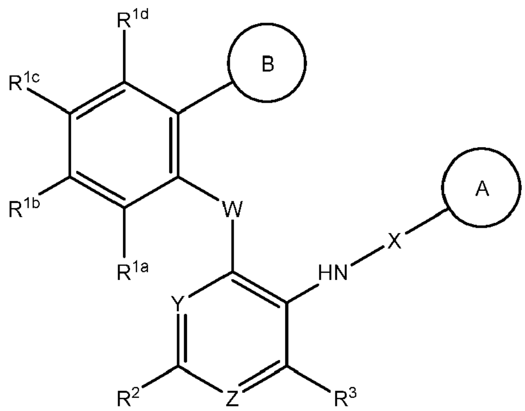

(I)

5

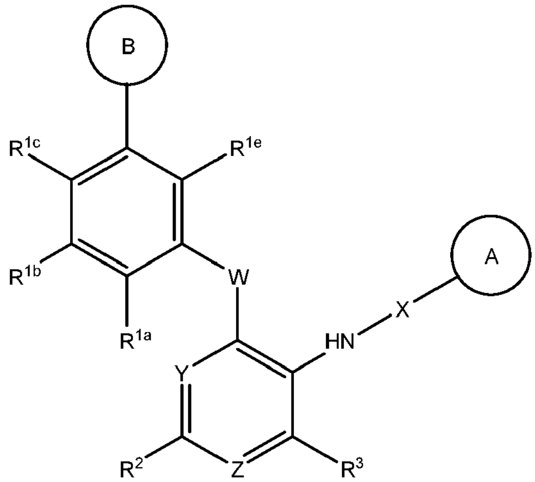

(II)

;

or (b) is a tautomer, N-oxide, pharmaceutically acceptable salt, or solvate thereof; wherein:
ring A is $C_{3-10}$ carbocycle substituted with 0-4 $R^4$, or is 5- to 10-membered heterocycle comprising carbon ring atoms and 1-4 heteroatom ring atoms selected from N, $NR^5$, O and $S(O)_p$, wherein said heterocycle is substituted with 0-4 $R^4$, with the proviso that ring A is not:

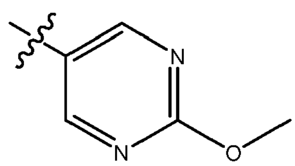

or

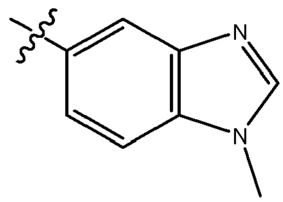

,

and with the proviso that when ring A is a 2-pyridinyl ring of formula (III), there is no $R^4$ substituent at the pyridinyl 3-position marked by * in formula (III)

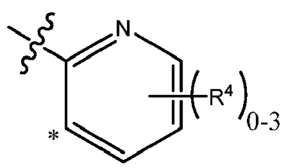

(III)

;

ring B is selected from:

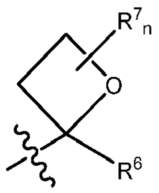 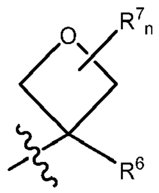 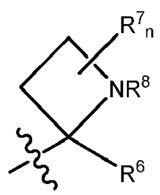 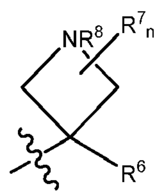

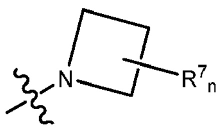 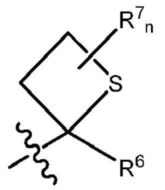 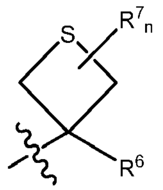 ;

W is O, NH, or S;

X is -CO-NH- or bond;

Y is N or CH;

Z is N or CH;

$R^{1a}$ and $R^{1b}$ are, independently at each occurrence, H, F, Cl, Br, -OH, or $-C_{1-6}$ alkyl substituted with 0-3 substituents selected from F or Cl;

$R^{1c}$, $R^{1d}$ and $R^{1e}$ are, independently at each occurrence, H, F, Cl, Br, or $-C_{1-6}$ alkyl substituted with 0-3 substituents selected from F or Cl;

$R^2$ is H, F, Cl, Br, or $-C_{1-6}$ alkyl substituted with 0-3 substituents selected from F or Cl;

$R^3$ is H or OH;

$R^4$ is, independently at each occurrence, F, Cl, Br, $-C_{rA}F_{(2rA+1)}$, $-C_{rA}F_{2rA}H$, $-(CR^{9A}{}_2)_{tA}-NR^{10}R^{11}$, -CN, $-OC_{rA}F_{(2rA+1)}$, $-OC_{rA}F_{2rA}H$, $-SF_5$, $-C_{1-6}$ alkyl substituted with 0-3 substituents selected from $R^{12}$, $-C_{3-6}$ cycloalkyl substituted with 0-3 substituents selected from $R^{12}$, $-O-C_{1-6}$ alkyl substituted with 0-3 substituents selected from $R^{12}$, $-O-C_{3-6}$ cycloalkyl substituted with 0-3 substituents selected from $R^{12}$, or 3- to 6- membered heterocyclic ring comprising: carbon ring atoms and 1-3 heteroatom ring atoms selected from N, $NR^5$, O and $S(O)_p$, wherein said heterocyclic ring is substituted with 0-3 substituents selected from F, Cl, $-C_{rAA}F_{(2rAA+1)}$, $-C_{rAA}F_{2rAA}H$, or $C_{1-6}$ alkyl substituted with 0-3 substituents selected from $R^{12}$;

alternatively, two $R^4$ groups on the same carbon ring atom combine to form a $C_{3-6}$ carbocycle substituted with 0-2 substituents selected from $R^{13}$;

$R^5$ is, independently at each occurrence, H, $-C_{rB}F_{(2rB+1)}$, $-C_{rB}F_{2rB}H$ or $-C_{1-6}$ alkyl substituted with 0-3 substituents selected from F or Cl;

$R^6$ is H, $-C_{rC}F_{(2rC+1)}$, $-C_{rC}F_{2rC}H$, or $C_{1-6}$ alkyl;

$R^7$ is $-C_{rD}F_{(2rD+1)}$, $-C_{rD}F_{2rD}H$, or $C_{1-6}$ alkyl;

Alternatively, two $R^7$ groups on the same carbon ring atom form =O;

$R^8$ is H, $-C_{rE}F_{(2rE+1)}$, $-C_{rE}F_{2rE}H$, $C_{1-6}$ alkyl substituted with 0-3 substituents selected from F and Cl, or $-(CR^{9B}{}_2)_{tB}-C_{3-10}$ carbocycle substituted with 0-3 substituents selected from F, Cl or $C_{1-6}$ alkyl;

$R^{9A}$ is, independently at each occurrence, H, $-C_{rF}F_{(2rF+1)}$, $-C_{rF}F_{2rF}H$, or $C_{1-6}$ alkyl;

alternatively, two $R^{9A}$ groups on the same carbon atom combine to form a 3- to 7-membered carbocyclic ring;

$R^{9B}$ is, independently at each occurrence, H, $-C_{rG}F_{(2rG+1)}$, $-C_{rG}F_{2rG}H$, or $C_{1-6}$ alkyl;

alternatively, two $R^{9B}$ groups on the same carbon atom combine to form a 3- to 7-membered carbocyclic ring;

$R^{10}$ is $-C_{1-6}$ alkyl substituted with 0-3 substituents selected from F or Cl;

$R^{11}$ is H or $-C_{1-6}$ alkyl substituted with 0-3 substituents selected from F or Cl;

alternatively, $R^{10}$ and $R^{11}$ on the same nitrogen atom combine to form a 3- to 6-membered heterocyclic ring comprising: the said nitrogen atom as a heteroatom ring atom, one or more carbon ring atoms and 0, 1 or 2 further heteroatom ring atoms selected from N, $NR^{14}$, O and $S(O)_p$, wherein said heterocyclic ring is substituted with 0-3 substituents selected from F or Cl;

$R^{12}$ is, independently at each occurrence, F, Cl, $-CF_3$, $-CF_2H$, $-C_2F_5$, $-C_2F_4H$, $-OCH_3$,$-OCH_2CH_3$, $-N(CH_3)_2$, $-N(CH_3)(CH_2CH_3)$, $-N(CH_2CH_3)_2$;

$R^{13}$ is, independently at each occurrence, F or Cl;

$R^{14}$ is H, $-C_{rH}F_{(2rH+1)}$, $-C_{rH}F_{2rH}H$ or $-C_{1-6}$ alkyl substituted with 0-3 substituents selected from F or Cl;

n is 0, 1 or 2;

p is, independently at each occurrence, 0, 1 or 2;

rA is, independently on each substituent in which it occurs, 1-6;
rAA is, independently on each substituent in which it occurs, 1-6;
rB is, independently on each substituent in which it occurs, 1-6;
rC is, independently on each substituent in which it occurs, 1-6;
rD is, independently on each substituent in which it occurs, 1-6;
rE is, independently on each substituent in which it occurs, 1-6;
rF is, independently on each substituent in which it occurs, 1-6;
rG is, independently on each substituent in which it occurs, 1-6;
rH is, independently on each substituent in which it occurs, 1-6;
tA is, independently at each occurrence, 1, 2 or 3; and
tB is, independently at each occurrence, 1, 2 or 3.

**2.** The compound according to claim 1, wherein ring A is a phenyl ring substituted with 0-4 $R^4$.

**3.** The compound according to claim 1 or 2, wherein:

$R^4$ is, independently at each occurrence, F, Cl, $-C_{rA}F_{(2rA+1)}$, $-C_{rA}F_{2rA}H$, $-CH_2-NR^{10}R^{11}$, -CN, $-OC_{rA}F_{(2rA+1)}$, $-OC_{rA}F_{2rA}H$, $-SF_5$, $-C_{1-3}$ alkyl substituted with 0-2 substituents selected from $R^{12}$, $-O-C_{1-3}$ alkyl substituted with 0-2 substituents selected from $R^{12}$, $O-C_{3-5}$ cycloalkyl substituted with 0-2 substituents selected from $R^{12}$, or 3- to 5- membered heterocyclic ring comprising: carbon atoms and 1 heteroatom selected from O, wherein said heterocyclic ring is substituted with 0-2 substituents selected from F, Cl, $-C_{rAA}F_{(2rAA+1)}$, $-C_{rAA}F_{2rAA}H$, or $C_{1-3}$ alkyl substituted with 0-2 substituents selected from $R^{12}$;
$R^{10}$ and $R^{11}$ on the same nitrogen atom combine to form a 3- to 5- membered heterocyclic ring comprising: carbon ring atoms and 1-2 heteroatom ring atoms selected from N, $NR^{14}$ and O and $S(O)_p$, wherein said heterocyclic ring is substituted with 0-3 substituents selected from F or Cl;$R^{12}$ is F, Cl, $-CF_3$, $-CF_2H$, $-C_2F_5$, or $-C_2F_4H$,;
$R^{14}$ is H, $-CH_3$ or $-CH_2CF_3$;
rA is, independently on each substituent in which it occurs, 1-3; and
rAA is, independently on each substituent in which it occurs, 1-3.

**4.** The compound according to any one of claims 1 to 3, wherein ring A is

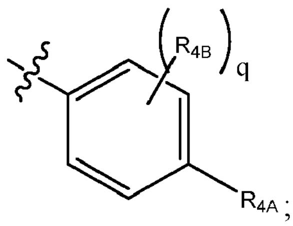

wherein:

$R_{4A}$ is $R_4$;
$R_{4B}$ is independently at each occurrence F or Cl and
q is 0-2.

**5.** The compound according to any one of claims 1 to 4, wherein ring B is

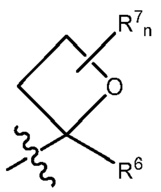 or 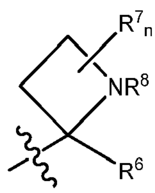 .

**6.** The compound according to any one of claims 1 to 5, wherein $R^6$ is $-CF_3$ and n is 0.

**7.** The compound according to any one of claims 1 to 6, wherein W is O, X is -CO-NH-, Y is N, and Z is CH.

**8.** The compound according to any one of claims 1 to 7, wherein:

the formula (I) or formula (II) is formula (I);
$R^{1a}$ and $R^{1d}$ are H;
$R^{1b}$ and $R^{1c}$ are independently selected from H or F;
$R^2$ is H, Cl, or $-CH_3$; and
$R^3$ is H.

**9.** The compound according to any one of claims 1 to 8, wherein the formula (I) or formula (II) is formula (I):

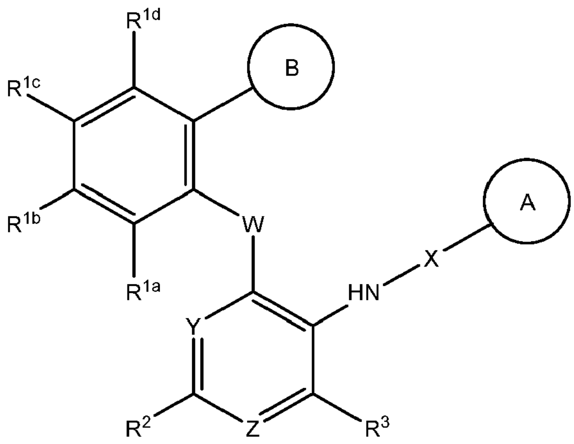

(I)

wherein:

ring A is

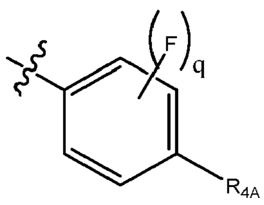

;

ring B is selected from:

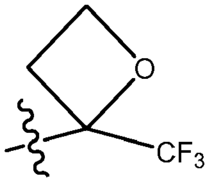

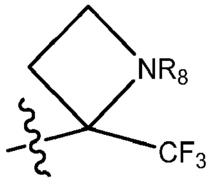

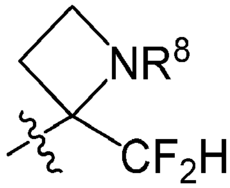

;

W is O;
X is -CO-NH-;
Y is N;
Z is CH;
$R^{1a}$ and $R^{1d}$ are H;

$R^{1b}$ and $R^{1c}$ are independently selected from H or F;
$R^2$ is H, Cl or $-CH_3$;
$R^3$ is H;
$R^{4A}$ is F, Cl, $-CF_3$, $-OCF_3$, $-SF_5$, $-C_{1-3}$ alkyl substituted with 0-2 $CF_3$ substituents,

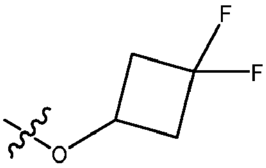 , or 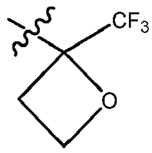 ;

q is 0, 1 or 2; and
$R^8$ is $-CH_3$, $-C_2H_5$, $-CH_2CH_2F$, $-CH_2C(CH_3)_3$, benzyl or -methylcyclopropyl.

**10.** The compound according to claim 1, wherein the formula (I) or formula (II) is selected from:

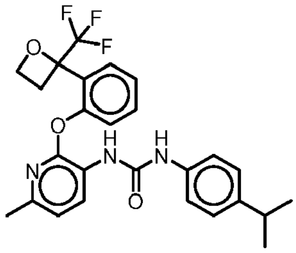 , 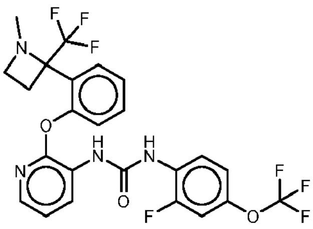 , 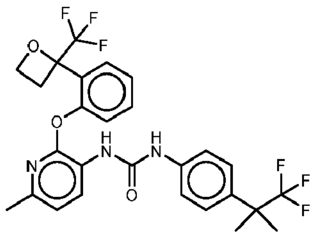

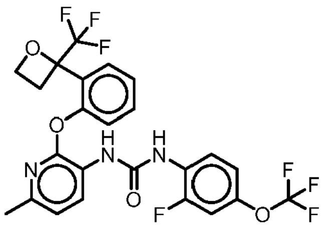 , 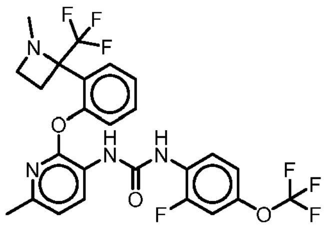 , 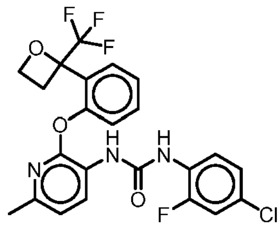 ,

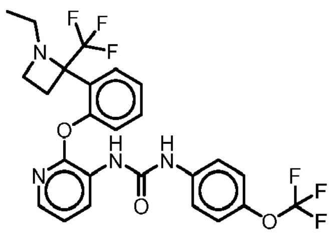 , 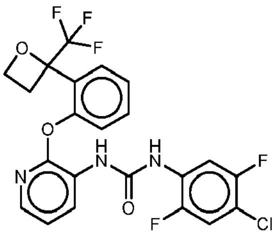 , 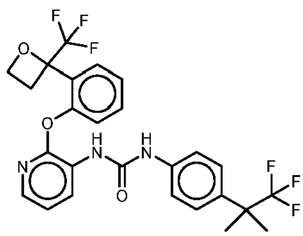 ,

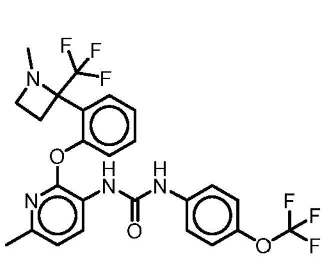 , 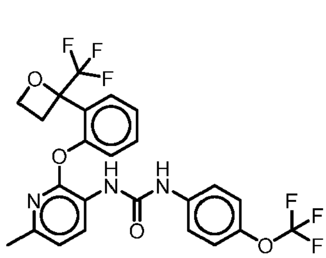 , 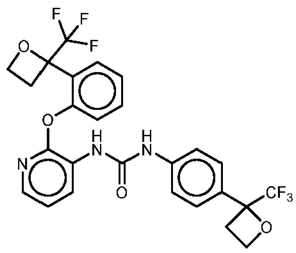 ,

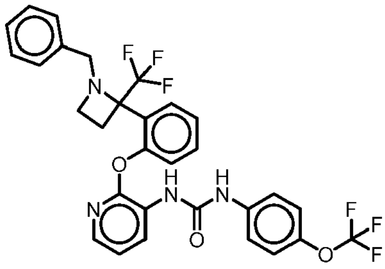, 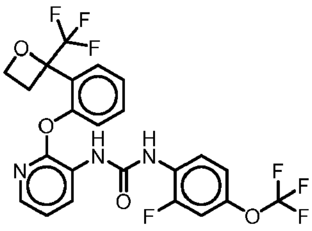, 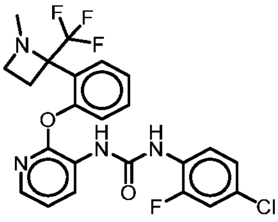,

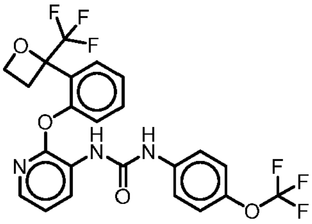, 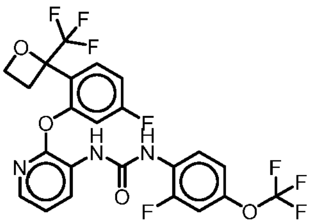, 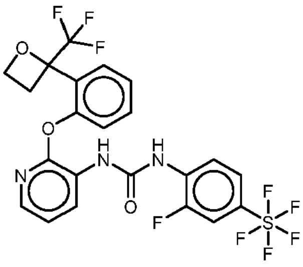

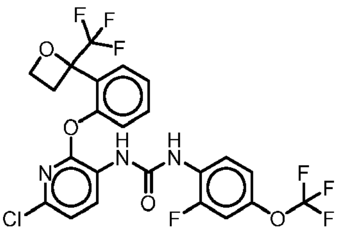, 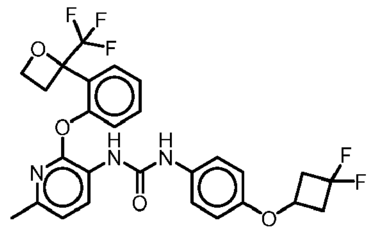, 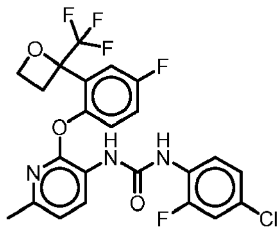,

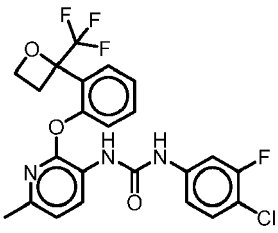, 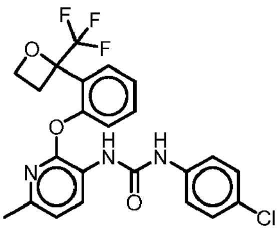, 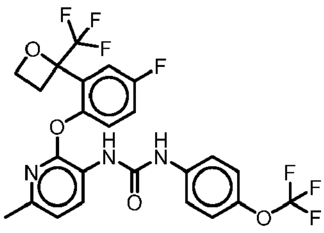,

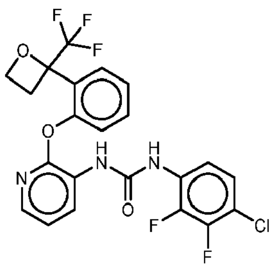, 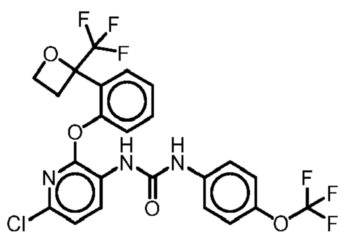, 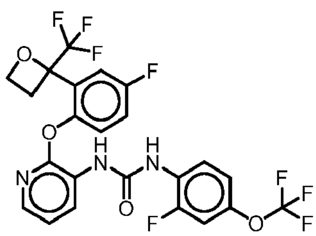,

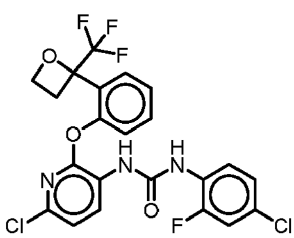, 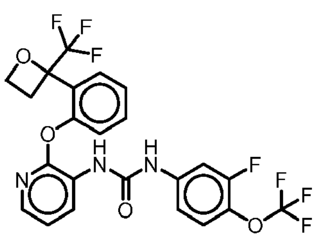, 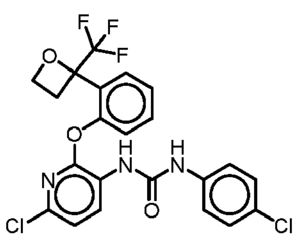,

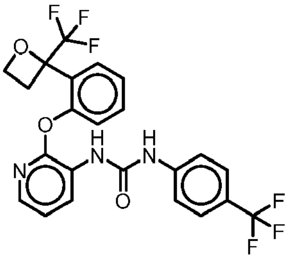, 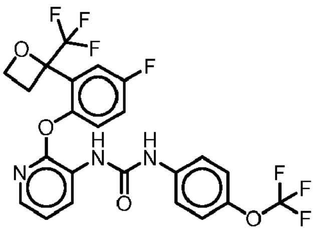, 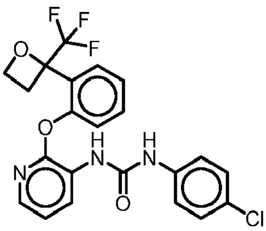,

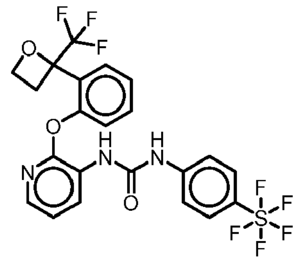, 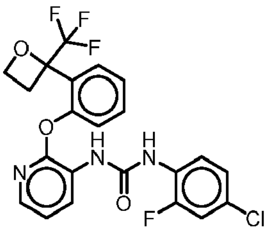, 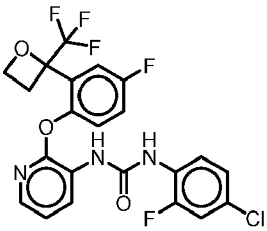,

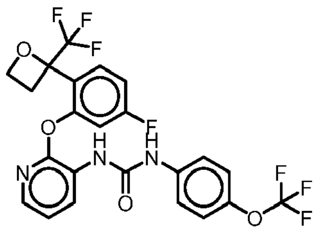, 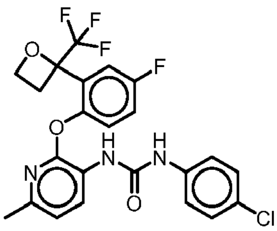, 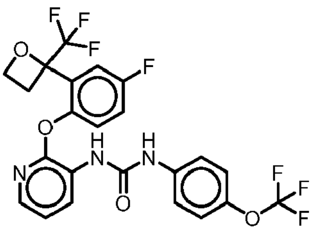,

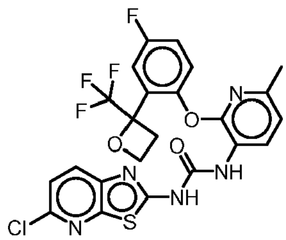, 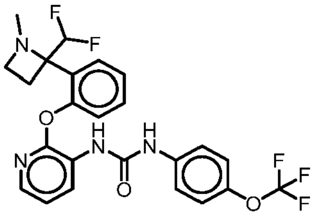, 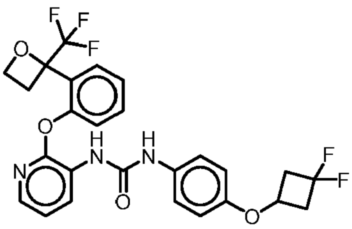,

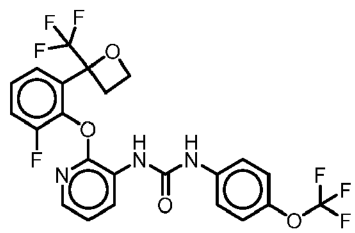, 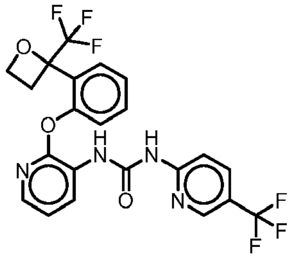, 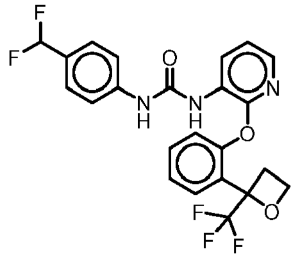,

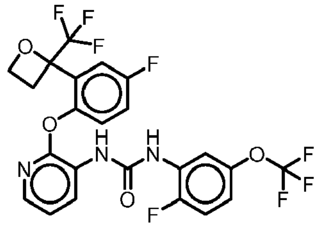, 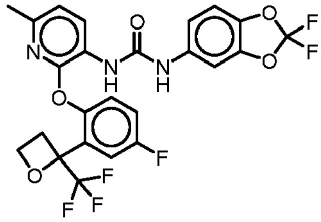, 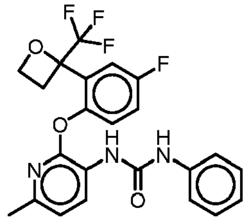,

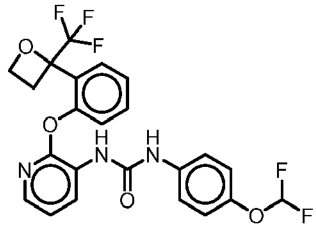, 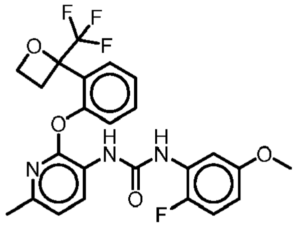, 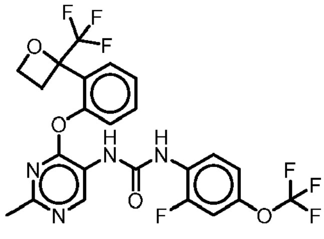,

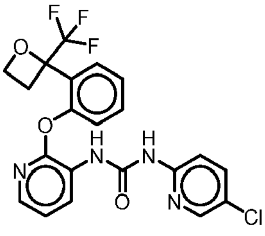, 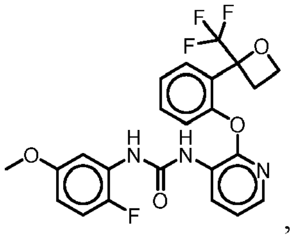, 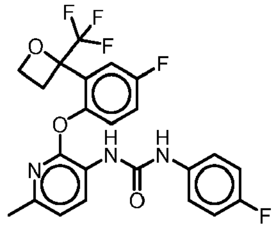,

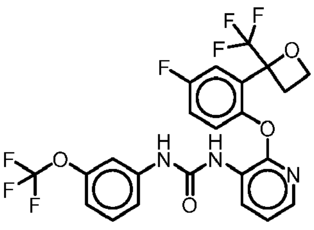, 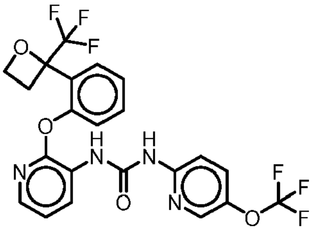, 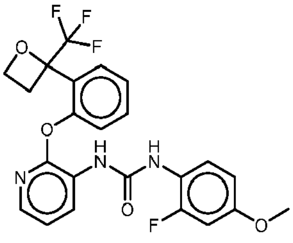,

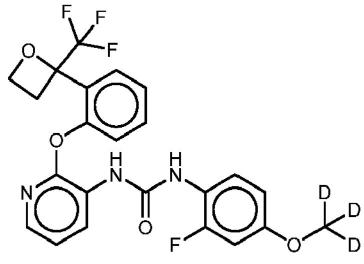, 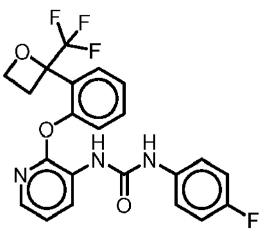, 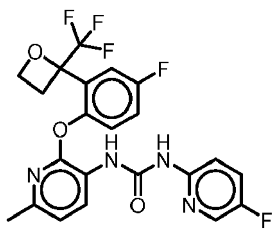,

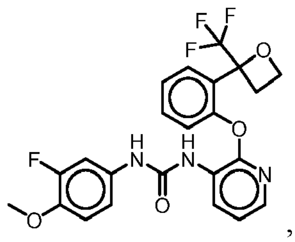, 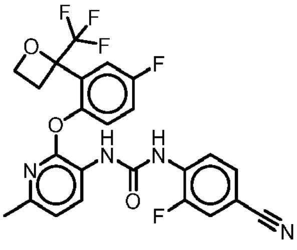, 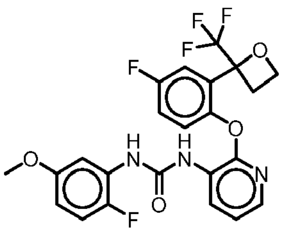,

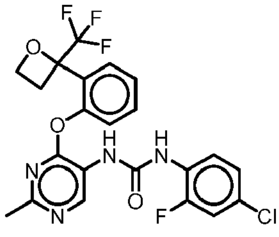, 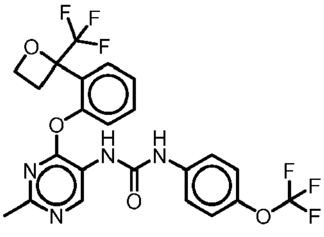, 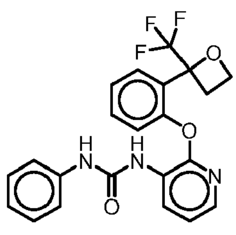,

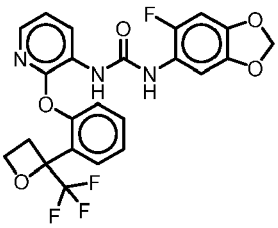, 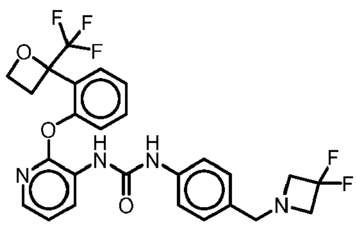, 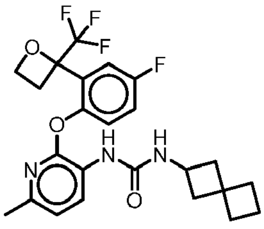,

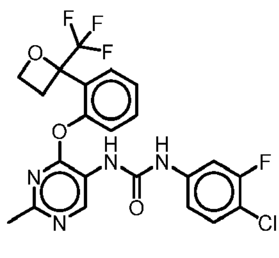, 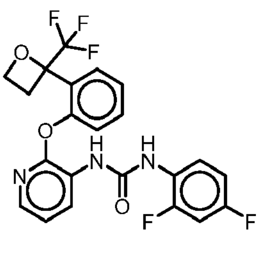, 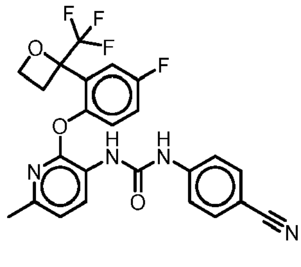,

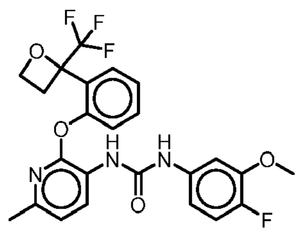, 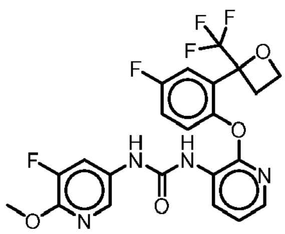, 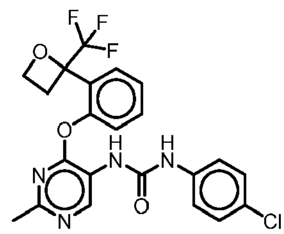,

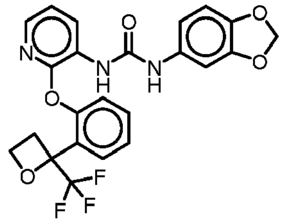, 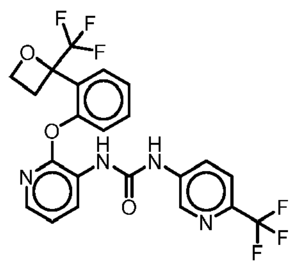, 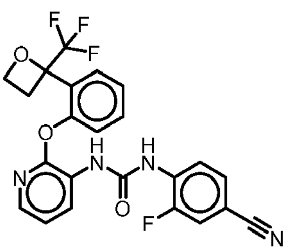,

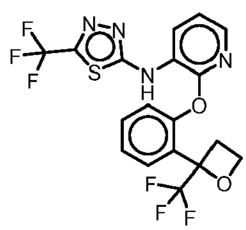, 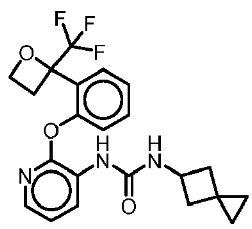, 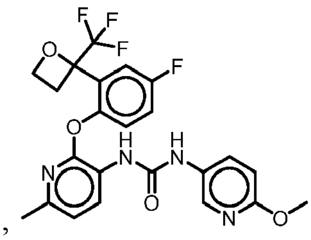, 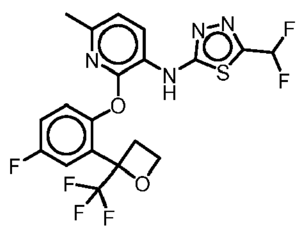,

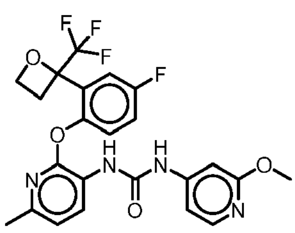, 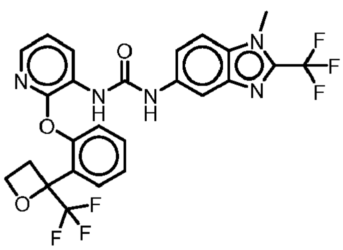, 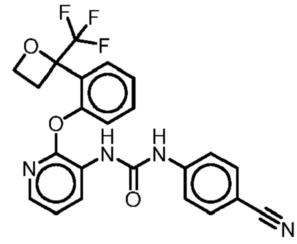,

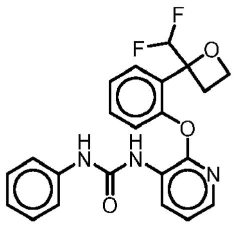,
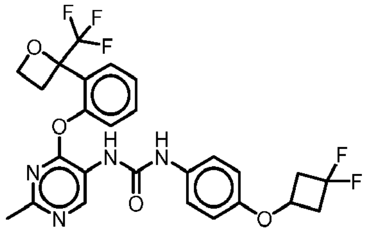,
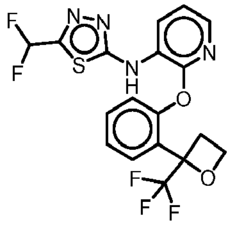,

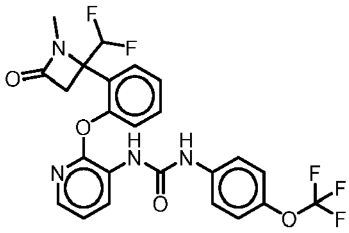,
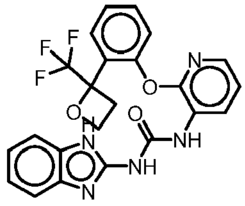,
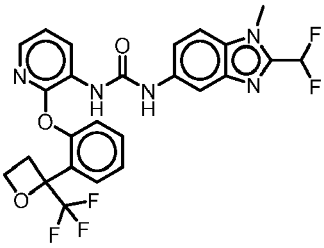,

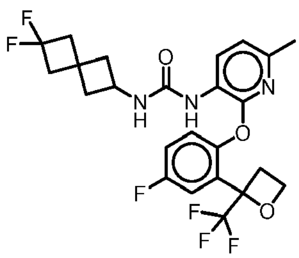,
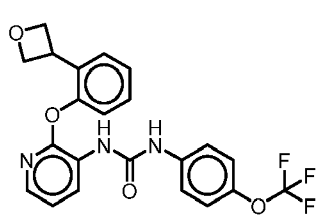,
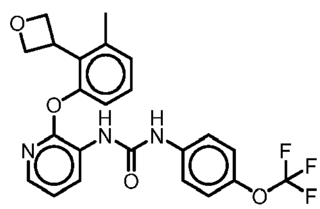,

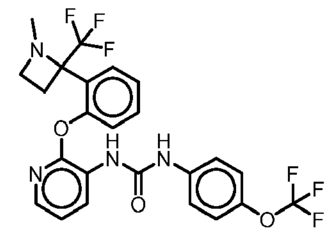,
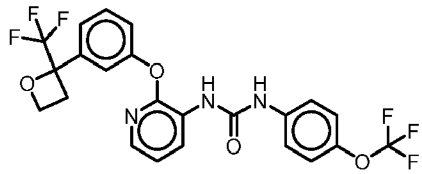,

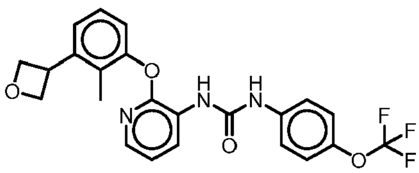,
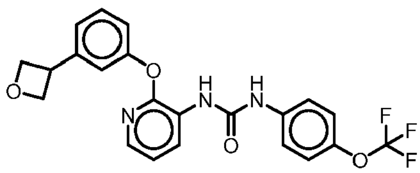,

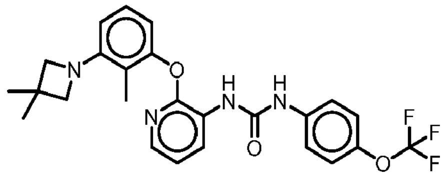,
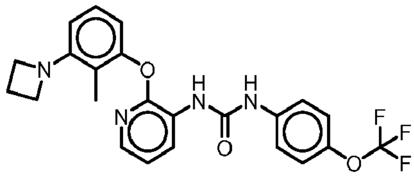,

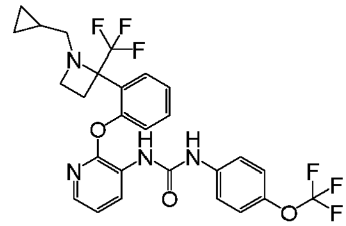, 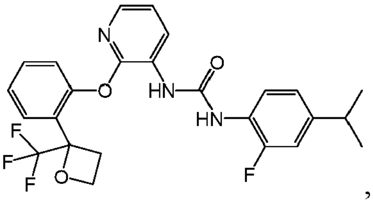, 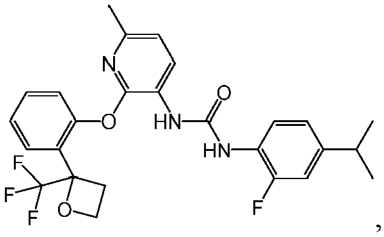,

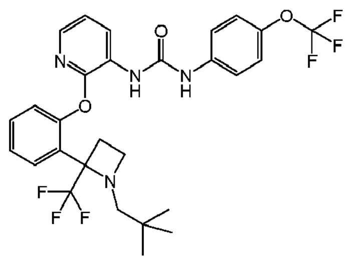, 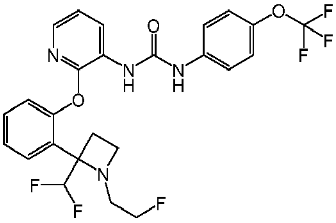, 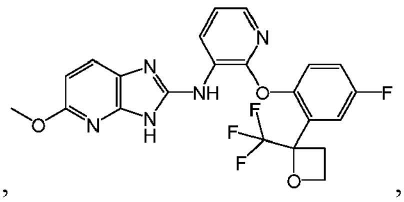,

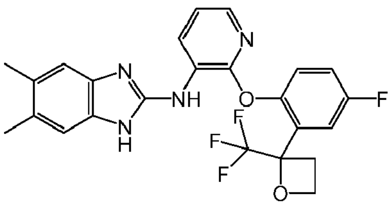, 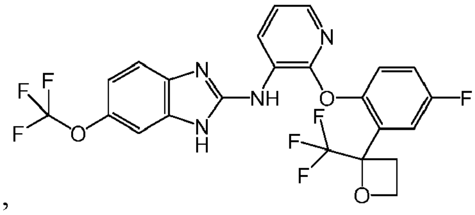.

**11.** The compound according to claim 1, wherein the formula (I) or formula (II) is selected from:

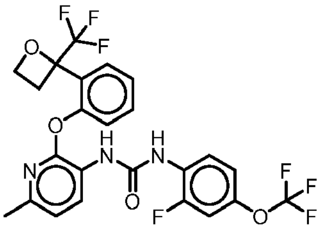, 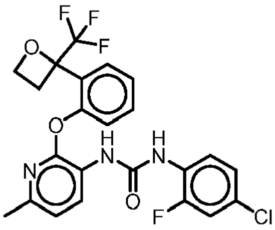, 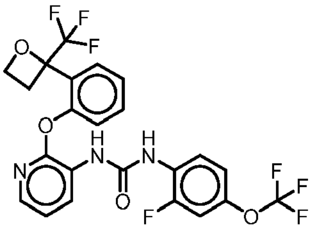,

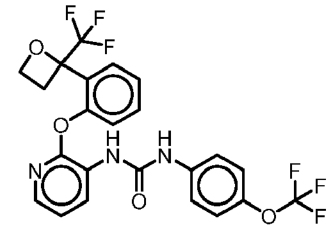, and 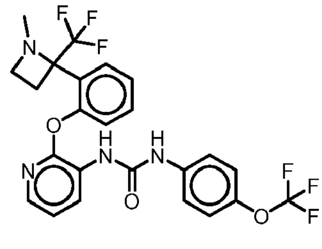;

optionally wherein the formula (I) or formula (II) is

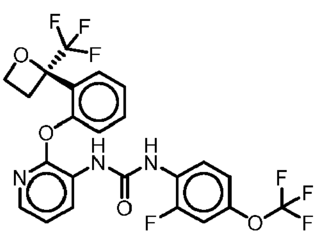.

**12.** A compound that: (a) is of formula (I) or formula (II):

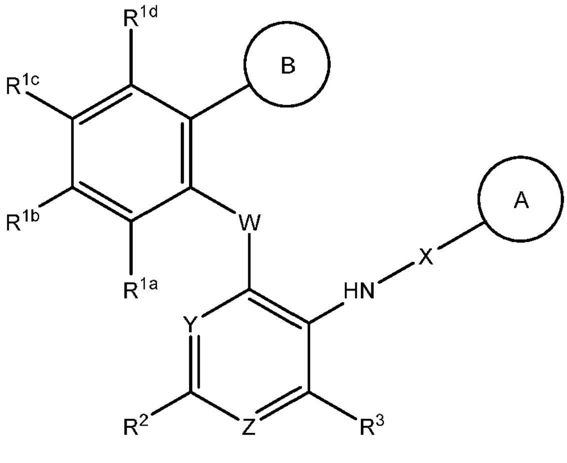

(I)

5

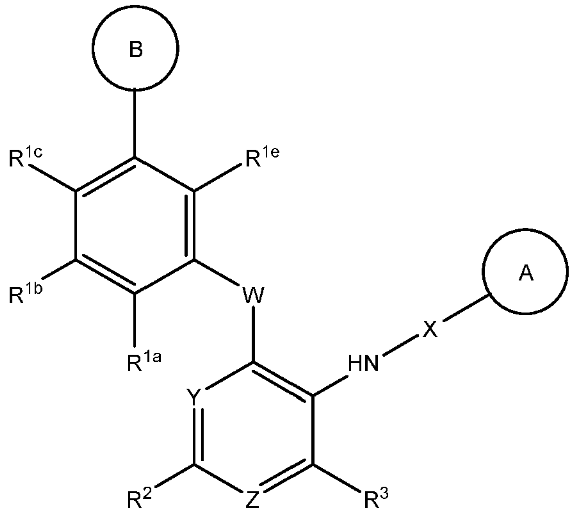

(II)

;

or (b) is a tautomer, N-oxide, pharmaceutically acceptable salt, or solvate thereof, wherein:

ring A is $C_{3-10}$ carbocycle substituted with 0-4 $R^4$, or is 5- to 10-membered heterocycle comprising carbon ring atoms and 1-4 heteroatom ring atoms selected from N, $NR^5$, O and $S(O)_p$, wherein said heterocycle is substituted with 0-4 $R^4$;

ring B is:

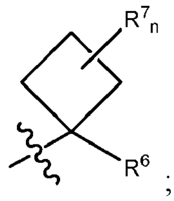

;

W is O, NH, or S;

X is -CO-NH- or bond;

Y is N or CH;

Z is N or CH;

$R^{1a}$ and $R^{1b}$ are, independently at each occurrence, H, F, Cl, Br, -OH, or $-C_{1-6}$ alkyl substituted with 0-3 substituents selected from F or Cl;

$R^{1c}$, $R^{1d}$ and $R^{1e}$ are, independently at each occurrence, H, F, Cl, Br, or $-C_{1-6}$ alkyl substituted with 0-3 substituents selected from F or Cl;

$R^2$ is H, F, Cl, Br, or $-C_{1-6}$ alkyl substituted with 0-3 substituents selected from F or Cl;

$R^3$ is H or OH;

$R^4$ is, independently at each occurrence, F, Cl, Br, $-C_{rA}F_{(2rA+1)}$, $-C_{rA}F_{2rA}H$,$-(CR^{9A}2)_{tA}-NR^{10}R^{11}$, -CN, $-OC_{rA}F_{(2rA+1)}$, $-OC_{rA}F_{2rA}H$, $-SF_5$, $-C_{1-6}$ alkyl substituted with 0-3 substituents selected from $R^{12}$, $-C_{3-6}$ cycloalkyl substituted with 0-3 substituents selected from $R^{12}$, $-O-C_{1-6}$ alkyl substituted with 0-3 substituents selected from $R^{12}$, $O-C_{3-6}$ cycloalkyl substituted with 0-3 substituents selected from $R^{12}$, or 3- to 6- membered heterocyclic ring comprising: carbon ring atoms and 1-3 heteroatom ring atoms selected from N, $NR^5$, O and $S(O)_p$, wherein said heterocyclic ring is substituted with 0-3 substituents selected from F, Cl, $-C_{rAA}F_{(2rAA+1)}$, $-C_{rAA}F_{2rAA}H$, or $C_{1-6}$ alkyl substituted with 0-3 substituents selected from $R^{12}$;

alternatively, two $R^4$ groups on the same carbon ring atom combine to form a $C_{3-6}$ carbocycle substituted with 0-2 substituents selected from $R^{13}$;

$R^5$ is, independently at each occurrence, H, $-C_{rB}F_{(2rB+1)}$, $-C_{rB}F_{2rB}H$ or $-C_{1-6}$ alkyl substituted with 0-3 substituents selected from F or Cl;

$R^6$ is H, $-C_{rC}F_{(2rC+1)}$, $-C_{rC}F_{2rC}H$, or $C_{1-6}$ alkyl;
$R^7$ is $-C_{rD}F_{(2rD+1)}$, $-C_{rD}F_{2rD}H$, or $C_{1-6}$ alkyl;
alternatively, two $R^7$ groups on the same carbon ring atom form =O;
$R^{9A}$ is, independently at each occurrence, H, $-C_{rF}F_{(2r+1)}$, $-C_{rF}F_{2rF}H$, or $C_{1-6}$ alkyl;
alternatively, two $R^9$ groups on the same carbon atom combine to form a 3- to 7-membered carbocyclic ring;
$R^{10}$ is $-C_{1-6}$ alkyl substituted with 0-3 substituents selected from F or Cl;
$R^{11}$ is H or $-C_{1-6}$ alkyl substituted with 0-3 substituents selected from F or Cl;
alternatively, $R^{10}$ and $R^{11}$ on the same nitrogen atom combine to form a 3- to 6-membered heterocyclic ring comprising: the said nitrogen atom as a heteroatom ring atom, one or more carbon ring atoms and 0, 1 or 2 further heteroatom ring atoms selected from N, $NR^{14}$, O and $S(O)_p$, wherein said heterocyclic ring is substituted with 0-3 substituents selected from F or Cl;
$R^{12}$ is, independently at each occurrence, F, Cl, $-CF_3$, $-CF_2H$, $-C_2F_5$, $-C_2F_4H$, $-OCH_3$, $-OCH_2CH_3$, $-N(CH_3)_2$, $-N(CH_3)(CH_2CH_3)$, $-N(CH_2CH_3)_2$;
$R^{13}$ is, independently at each occurrence, F or Cl;
$R^{14}$ is H, $-C_{rH}F_{(2rH+1)}$, $-C_{rH}F_{2rH}H$ or $-C_{1-6}$ alkyl substituted with 0-3 substituents selected from F or Cl;
n is 0, 1 or 2;
p is, independently at each occurrence, 0, 1 or 2;
rA is, independently on each substituent in which it occurs, 1-6;
rAA is, independently on each substituent in which it occurs, 1-6;
rB is, independently on each substituent in which it occurs, 1-6;
rC is, independently on each substituent in which it occurs, 1-6;
rD is, independently on each substituent in which it occurs, 1-6;
rF is, independently on each substituent in which it occurs, 1-6;
rH is, independently on each substituent in which it occurs, 1-6; and
tA is, independently at each occurrence, 1, 2 or 3.

**13.** A pharmaceutical composition comprising a compound according to any one of claims 1 to 12 in association with one or more pharmaceutically acceptable carriers.

**14.** A compound according to any of claims 1 to 12, or a pharmaceutical composition according to claim 13, for use as a medicament.

**15.** A compound according to any of claims 1 to 12, or a pharmaceutical composition according to claim 13, for use in a method for treating and/or preventing a CNS disorder, wherein the CNS disorder is selected from Alzheimer's disease, pain, epilepsy, stroke, traumatic brain injury, amyotrophic lateral sclerosis, Huntington's disease, Parkinson's disease and frontotemporal dementia.

Europäisches Patentamt
European Patent Office
Office européen des brevets

# EUROPEAN SEARCH REPORT

Application Number
EP 21 17 5671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2005/113511 A1 (SQUIBB BRISTOL MYERS CO [US]) 1 December 2005 (2005-12-01)<br>* paragraphs [0001] - [0011], [0802] - [0804]; claims 1, 4, 12, 16, 20-25; examples 106, 124, 164, 414, 643 *<br>----- | 1-15 | INV.<br>C07D405/12<br>C07D405/14<br>C07D417/14<br>C07D401/12<br>C07D513/04<br>A61K31/4427<br>A61K31/506<br>A61P25/00 |
| A,D | WO 2005/113537 A2 (SQUIBB BRISTOL MYERS CO [US]) 1 December 2005 (2005-12-01)<br>* paragraphs [0001] - [0011], [0380] - [0382]; claims 1, 10, 11, 13, 17-22; example 39 *<br>----- | 1-15 | |
| A | CHAO HANNGUANG ET AL: "Discovery of 2-(Phenoxypyridine)-3-phenylureas as Small Molecule P2Y1 Antagonists",<br>JOURNAL OF MEDICINAL CHEMISTRY,<br>vol. 56, no. 4, 2013, pages 1704-1714, XP055850126,<br>ISSN: 0022-2623, DOI: 10.1021/jm301708u<br>* the whole document *<br>----- | 1-15 | |
| A | CONROY SEAN ET AL: "Drug-like Antagonists of P2Y Receptors - From Lead Identification to Drug Development",<br>JOURNAL OF MEDICINAL CHEMISTRY,<br>vol. 59, no. 22, 2016, pages 9981-10005, XP055850323,<br>ISSN: 0022-2623, DOI: 10.1021/acs.jmedchem.5b01972<br>Retrieved from the Internet:<br>URL:https://pubs.acs.org/doi/pdf/10.1021/acs.jmedchem.5b01972><br>* pages 9981-9986, introduction, drug-like antagonists of the P2Y1 receptor; page 9999, conclusion *<br>----- | 1-15 | TECHNICAL FIELDS SEARCHED (IPC)<br>C07D |

The present search report has been drawn up for all claims

1

| Place of search | Date of completion of the search | Examiner |
|---|---|---|
| Munich | 12 October 2021 | Ladenburger, Claude |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 17 5671

This annex lists the patent family members relating to the patent documents cited in the above-mentioned European search report. The members are as contained in the European Patent Office EDP file on
The European Patent Office is in no way liable for these particulars which are merely given for the purpose of information.

12-10-2021

| Patent document cited in search report | Publication date | Patent family member(s) | Publication date |
|---|---|---|---|
| WO 2005113511 A1 | 01-12-2005 | AT 460402 T | 15-03-2010 |
| | | AU 2005245389 A1 | 01-12-2005 |
| | | EP 1751113 A1 | 14-02-2007 |
| | | ES 2340179 T3 | 31-05-2010 |
| | | JP 2008500284 A | 10-01-2008 |
| | | KR 20070032648 A | 22-03-2007 |
| | | US 2005267119 A1 | 01-12-2005 |
| | | US 2008280905 A1 | 13-11-2008 |
| | | WO 2005113511 A1 | 01-12-2005 |
| WO 2005113537 A2 | 01-12-2005 | AU 2005245400 A1 | 01-12-2005 |
| | | CN 1984655 A | 20-06-2007 |
| | | CN 1984892 A | 20-06-2007 |
| | | DK 1750704 T3 | 02-11-2009 |
| | | EP 1750704 A2 | 14-02-2007 |
| | | ES 2328716 T3 | 17-11-2009 |
| | | HR P20090468 T1 | 30-09-2009 |
| | | JP 4795336 B2 | 19-10-2011 |
| | | JP 2007537273 A | 20-12-2007 |
| | | KR 20070011479 A | 24-01-2007 |
| | | PL 1750704 T3 | 29-01-2010 |
| | | PT 1750704 E | 06-10-2009 |
| | | SI 1750704 T1 | 31-12-2009 |
| | | US 2005261244 A1 | 24-11-2005 |
| | | WO 2005113537 A2 | 01-12-2005 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2005113511 A
- WO 2005113537 A
- WO 2006078621 A
- WO 2008023235 A
- WO 2014022253 A
- WO 2014022349 A
- WO 2014022343 A

### Non-patent literature cited in the description


- **DELAKATE A et al.** *Nat. Commun.,* 2014, vol. 5, 5422
- **REICHENBACH N et al.** *J. Exp. Med.,* 2018, vol. 215 (6), 1649-1663
- **REICHENBACH N et al.** *EMBO Mol Med.,* 2019, vol. 11, e9665
- **SHI A et al.** *Cereb Cortex.,* 2020, vol. 30 (3), 1272-1290
- **BARRAGÁN-IGLESIAS P et al.** *Brain Res.,* 2016, vol. 1636, 43-51
- **MITCHELL R et al.** *Mol Neurobiol.,* 2019, vol. 56 (8), 5917-5933
- **YANG CT et al.** *Sci Rep.,* 2019, vol. 9 (1), 16032
- **HOCKLEY JR et al.** *J Neurosci.,* 2016, vol. 36 (8), 2364-76
- **KWON SG et al.** *Brain Res Bull.,* 2017, vol. 130, 165-172
- **ALVAREZ-FERRADAS C et al.** *Glia.,* 2015, vol. 63 (9), 1507-21
- **ALVES M et al.** *J Neurosci.,* 2019, vol. 39 (27), 5377-5392
- **ALVES M et al.** *Front Pharmacol.,* 2020, vol. 10, 1558
- **ALVES M et al.** *Epilepsia,* 2017, vol. 58 (9), 1603-1614
- **NIKOLIC L et al.** *Glia,* 2018, vol. 66 (12), 2673-2683
- **WELLMANN M et al.** *Front Cell Neurosci.,* 2018, vol. 12, 446
- **CARMO MR et al.** *Eur J Neurosci.,* 2014, vol. 39, 614-622
- **CHIN Y et al.** *J Neuroinflammation,* 2013, vol. 10, 95
- **FORSTER D ; REISER G.** *Purinergic Signal.,* 2015, vol. 11, 441-454
- **FUKUMOTO Y et al.** *J Cereb Blood Flow Metab.,* 2019, vol. 39, 2144-2156
- **KUBOYAMA K et al.** *J Cereb Blood Flow Metab.,* 2011, vol. 31, 1930-1941
- **GIOVANNA, M. et al.** *PloS one,* 2014, vol. 9 (12), e115273
- **GUO, H. et al.** *Molecular Medicine Reports,* 2018, vol. 17, 1819-1824
- **CHOO AM et al.** *Brain,* 2013, vol. 136, 65-80
- **SHINOZAKI Y et al.** *Cell Rep.,* 2017, vol. 19, 1151-1164
- **CIEŚLAK M et al.** *Purinergic Signal.,* 2019, vol. 15 (1), 1-15
- **IZRAEL M et al.** *Front Neurosci.,* 2020, vol. 14, 824
- **JIANG MC et al.** *Neuroscience,* 2017, vol. 362, 33-46
- **PEHAR M et al.** *Curr Pharm Des.,* 2017, vol. 23, 5010-5021
- **WILTON DK ; STEVENS B.** *Neurobiol Dis.,* 2020, 143
- **MIYAZAKI I ; ASANUMA M.** *Cells,* 2020, vol. 9, 2623
- **RADFORD RA et al.** *Front Cell Neurosci.,* 2015, vol. 9, 414
- **YANG et al.** *J. Med. Chem.,* 2014, vol. 57 (14), 6150-6164
- **PENG et al.** *European Journal of Medicinal Chemistry,* 2018, vol. 158, 302-310
- **PARDRIDGE W M.** *Alzheimers Dement.,* 2009, vol. 5 (5), 427-43
- **WAGER, T.T et al.** *ACS Chem. Neurosci.,* 2010, vol. 1, 435-449
- Remington's Pharmaceutical Sciences. Mack Publishing Company, 1985, 1418
- **CHOU ; TALALAY.** *Adv. Enzyme Regul.,* 1984, vol. 22, 27-55
- Remington's Pharmaceutical Sciences. 1985
- Remington's Pharmaceutical Sciences. Mack Publishing Company
- **WAGER et al.** *ACS Chem. Neurosci.,* 2010, vol. 1, 435-449